(19) Europäisches Patentamt
European Patent Office
Office européen des brevets

(11) EP 3 313 983 B1

(12) **EUROPEAN PATENT SPECIFICATION**

(45) Date of publication and mention of the grant of the patent:
**08.04.2020 Bulletin 2020/15**

(21) Application number: **16735675.7**

(22) Date of filing: **23.06.2016**

(51) Int Cl.:
*C12N 5/00* (2006.01)
*A61K 41/00* (2020.01)
*C07K 17/14* (2006.01)
*G01N 33/543* (2006.01)
*C12N 15/62* (2006.01)

(86) International application number:
**PCT/GB2016/051882**

(87) International publication number:
**WO 2016/207638 (29.12.2016 Gazette 2016/52)**

# (54) CONTROLLED CELL DELIVERY VEHICLE AND TREATMENT OF TUMOURS

VEHIKEL FÜR GESTEUERTE ZELLFREISETZUNG UND BEHANDLUNG VON TUMOREN

VÉHICULE D'ADMINISTRATION CELLULAIRE CONTRÔLÉE ET TRAITEMENT DE TUMEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.06.2015 GB 201511159**

(43) Date of publication of application:
**02.05.2018 Bulletin 2018/18**

(73) Proprietor: **The University Of Nottingham**
**Nottingham, Nottinghamshire NG7 2TU (GB)**

(72) Inventor: **DIXON, James**
**of Nottingham**
**Nottingham**
**Nottinghamshire NG7 2RD (GB)**

(74) Representative: **Barker Brettell LLP**
**100 Hagley Road**
**Edgbaston**
**Birmingham B16 8QQ (GB)**

(56) References cited:
- **ZHANG QIANYU ET AL: "A pH-responsive [alpha]-helical cell penetrating peptide-mediated liposomal delivery system", BIOMATERIALS, vol. 34, no. 32, 24 July 2013 (2013-07-24) , pages 7980-7993, XP028686809, ISSN: 0142-9612, DOI: 10.1016/J.BIOMATERIALS.2013.07.014**
- **BRUNDEN K R ET AL: "PH-DEPENDENT BINDING OF SYNTHETIC BETA-AMYLOID PEPTIDES TO GLYCOSAMINOGLYCANS", JOURNAL OF NEUROCHEMISTRY, WILEY INTERSCIENCE, NEW YORK, NY, US, vol. 61, no. 6, 1 January 1993 (1993-01-01), pages 2147-2154, XP008039062, ISSN: 0022-3042, DOI: 10.1111/J.1471-4159.1993.TB07453.X**
- **JAMES E. DIXON ET AL: "Highly efficient delivery of functional cargoes by the synergistic effect of GAG binding motifs and cell-penetrating peptides", PROCEEDINGS OF THE NATIONAL ACADEMY OF SCIENCES, vol. 113, no. 3, 5 January 2016 (2016-01-05), pages E291-E299, XP055290762, US ISSN: 0027-8424, DOI: 10.1073/pnas.1518634113**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 313 983 B1

SEQUENCE LISTING

<110> The University of Nottingham

<120> CONTROLLED CELL DELIVERY VEHICLE AND TREATMENT OF TUMOURS

<130> JA78956P.WOP

<150> GB1511159.4
<151> 2015-06-24

<160> 52

<170> PatentIn version 3.5

<210> 1
<211> 21
<212> PRT
<213> Artificial Sequence

<220>
<223> Peptide

<400> 1

```
Lys Arg Lys Lys Lys Gly Lys Gly Leu Gly Lys Lys Arg Asp Pro Cys
1               5                   10                  15

Leu Arg Lys Tyr Lys
            20
```

<210> 2
<211> 21
<212> PRT
<213> Artificial Sequence

<220>
<223> Peptide

<400> 2

```
His Arg Lys Lys Lys Gly Lys Gly Leu Gly Lys Lys Arg Asp Pro Cys
1               5                   10                  15

Leu Arg Lys Tyr Lys
            20
```

<210> 3
<211> 21
<212> PRT
<213> Artificial Sequence

<220>
<223> Peptide

<400> 3

```
Lys Arg His Lys Lys Gly Lys Gly Leu Gly Lys Lys Arg Asp Pro Cys
1               5                   10                  15

Leu Arg Lys Tyr Lys
            20
```

<210> 4
<211> 21
<212> PRT
<213> Artificial Sequence

<220>
<223> Peptide

<400> 4

```
Lys Arg Lys His Lys Gly Lys Gly Leu Gly Lys Lys Arg Asp Pro Cys
1               5                   10                  15

Leu Arg Lys Tyr Lys
            20
```

<210> 5
<211> 21
<212> PRT
<213> Artificial Sequence

<220>
<223> Peptide

<400> 5

```
Lys Arg Lys Lys His Gly Lys Gly Leu Gly Lys Lys Arg Asp Pro Cys
1               5                   10                  15

Leu Arg Lys Tyr Lys
            20
```

<210> 6
<211> 21
<212> PRT
<213> Artificial Sequence

<220>
<223> Peptide

<400> 6

```
Lys Arg Lys Lys Lys Gly His Gly Leu Gly Lys Lys Arg Asp Pro Cys
1               5                   10                  15

Leu Arg Lys Tyr Lys
            20
```

<210> 7
<211> 21
<212> PRT
<213> Artificial Sequence

<220>
<223> Peptide

<400> 7

Lys Arg Lys Lys Lys Gly Lys Gly Leu Gly His Lys Arg Asp Pro Cys
1               5                   10                  15

Leu Arg Lys Tyr Lys
            20

<210> 8
<211> 21
<212> PRT
<213> Artificial Sequence

<220>
<223> Peptide

<400> 8

Lys Arg Lys Lys Lys Gly Lys Gly Leu Gly Lys His Arg Asp Pro Cys
1               5                   10                  15

Leu Arg Lys Tyr Lys
            20

<210> 9
<211> 21
<212> PRT
<213> Artificial Sequence

<220>
<223> Peptide

<400> 9

Lys Arg Lys Lys Lys Gly Lys Gly Leu Gly Lys Lys Arg Asp Pro Cys
1               5                   10                  15

Leu Arg His Tyr Lys
            20

<210> 10
<211> 21
<212> PRT
<213> Artificial Sequence

<220>
<223> Peptide

<400> 10

Lys Arg Lys Lys Lys Gly Lys Gly Leu Gly Lys Lys Arg Asp Pro Cys
1               5                   10                  15

Leu Arg Lys Tyr His
            20

<210> 11

<211> 21
<212> PRT
<213> Artificial Sequence

<220>
<223> Peptide

<400> 11

```
His Arg His His His Gly His Gly Leu Gly Lys Lys Arg Asp Pro Cys
1               5                   10                  15

Leu Arg Lys Tyr Lys
            20
```

<210> 12
<211> 21
<212> PRT
<213> Artificial Sequence

<220>
<223> Peptide

<400> 12

```
Lys Arg Lys Lys Lys Gly Lys Gly Leu Gly His His Arg Asp Pro Cys
1               5                   10                  15

Leu Arg His Tyr His
            20
```

<210> 13
<211> 21
<212> PRT
<213> Artificial Sequence

<220>
<223> Peptide

<400> 13

```
Lys Arg His Lys His Gly Lys Gly Leu Gly His Lys Arg Asp Pro Cys
1               5                   10                  15

Leu Arg His Tyr Lys
            20
```

<210> 14
<211> 21
<212> PRT
<213> Artificial Sequence

<220>
<223> Peptide

<400> 14

```
His Arg Lys His Lys Gly His Gly Leu Gly Lys His Arg Asp Pro Cys
1               5                   10                  15

Leu Arg Lys Tyr His
            20
```

<210> 15
<211> 21
<212> PRT
<213> Artificial Sequence

<220>
<223> Peptide

<400> 15

```
His Arg His His His Gly His Gly Leu Gly His His Arg Asp Pro Cys
1               5                   10                  15

Leu Arg His Tyr His
            20
```

<210> 16
<211> 18
<212> PRT
<213> Artificial Sequence

<220>
<223> Peptide

<400> 16

```
Gly Arg Pro Arg Glu Ser Gly Lys Lys Arg Lys Arg Lys Arg Leu Lys
1               5                   10                  15

Pro Thr
```

<210> 17
<211> 20
<212> PRT
<213> Artificial Sequence

<220>
<223> Peptide

<400> 17

```
Thr Tyr Ala Ser Ala Lys Trp Thr His Asn Gly Gly Glu Met Phe Val
1               5                   10                  15

                        Ala Leu Asn Gln
                                    20
```

<210> 18
<211> 20
<212> PRT
<213> Artificial Sequence

<220>
<223> Peptide

<400> 18

```
Thr Tyr Ala Ser Ala His Trp Thr His Asn Gly Gly Glu Met Phe Val
1               5                   10                  15

Ala Leu Asn Gln
            20
```

<210> 19
<211> 16
<212> PRT
<213> Artificial Sequence

<220>
<223> Peptide

<400> 19

```
Thr Tyr Arg Ser Arg Lys Tyr Thr Ser Trp Tyr Val Ala Leu Lys Arg
1               5                   10                  15
```

<210> 20
<211> 16
<212> PRT
<213> Artificial Sequence

<220>
<223> Peptide

<400> 20

```
Thr Tyr Arg Ser Arg His Tyr Thr Ser Trp Tyr Val Ala Leu Lys Arg
1               5                   10                  15
```

<210> 21
<211> 16
<212> PRT
<213> Artificial Sequence

<220>
<223> Peptide

<400> 21

```
Thr Tyr Arg Ser Arg Lys Tyr Thr Ser Trp Tyr Val Ala Leu His Arg

  1               5                   10                  15
```

<210> 22
<211> 16
<212> PRT
<213> Artificial Sequence

<220>
<223> Peptide

<400> 22

Thr Tyr Arg Ser Arg His Tyr Thr Ser Trp Tyr Val Ala Leu His Arg
1               5                   10                  15

<210> 23
<211> 15
<212> PRT
<213> Artificial Sequence

<220>
<223> Peptide

<400> 23

Arg Gln Ile Lys Trp Phe Gln Asn Arg Arg Met Lys Trp Lys Lys
1               5                   10                  15

<210> 24
<211> 11
<212> PRT
<213> Artificial Sequence

<220>
<223> Peptide

<400> 24

Tyr Gly Arg Lys Lys Arg Arg Gln Arg Arg Arg
1               5                   10

<210> 25
<211> 18
<212> PRT
<213> Artificial Sequence

<220>
<223> Peptide

<400> 25

Arg Gly Gly Arg Leu Ser Tyr Ser Arg Arg Arg Phe Ser Thr Ser Thr
1               5                   10                  15

Gly Arg

<210> 26
<211> 10
<212> PRT

<213> Artificial Sequence

<220>
<223> Peptide

<400> 26

```
Arg Arg Leu Ser Tyr Ser Arg Arg Arg Phe
1               5                   10
```

<210> 27
<211> 12
<212> PRT
<213> Artificial Sequence

<220>
<223> Peptide

<400> 27

```
Pro Ile Arg Arg Arg Lys Lys Leu Arg Arg Leu Lys
1               5                   10
```

<210> 28
<211> 12
<212> PRT
<213> Artificial Sequence

<220>
<223> Peptide

<400> 28

```
Arg Arg Gln Arg Arg Thr Ser Lys Leu Met Lys Arg
1               5                   10
```

<210> 29
<211> 15
<212> PRT
<213> Artificial Sequence

<220>
<223> Peptide

<400> 29

```
Arg Arg Arg Arg Asn Arg Thr Arg Arg Asn Arg Arg Arg Val Arg
1               5                   10                  15
```

<210> 30
<211> 19
<212> PRT
<213> Artificial Sequence

<220>
<223> Peptide

<400> 30

```
Lys Met Thr Arg Ala Gln Arg Arg Ala Ala Ala Arg Arg Asn Arg Trp
1               5                   10                  15

Thr Ala Arg
```

<210> 31
<211> 13
<212> PRT
<213> Artificial Sequence

<220>
<223> Peptide

<400> 31

```
Thr Arg Arg Gln Arg Thr Arg Arg Ala Arg Arg Asn Arg
1               5                   10
```

<210> 32
<211> 13
<212> PRT
<213> Artificial Sequence

<220>
<223> Peptide

<400> 32

```
Gly Arg Lys Lys Arg Arg Gln Arg Arg Arg Pro Pro Gln
1               5                   10
```

<210> 33
<211> 13
<212> PRT
<213> Artificial Sequence

<220>
<223> Peptide

<400> 33

```
Gly Arg Arg Arg Arg Arg Arg Arg Arg Arg Pro Pro Gln
1               5                   10
```

<210> 34
<211> 27
<212> PRT
<213> Artificial Sequence

<220>
<223> Peptide

<400> 34

Gly Trp Thr Leu Asn Ser Ala Gly Tyr Leu Leu Gly Lys Ile Asn Leu
1               5                   10                  15

Lys Ala Leu Ala Ala Leu Ala Lys Lys Ile Leu
            20                  25

<210> 35
<211> 17
<212> PRT
<213> Artificial Sequence

<220>
<223> Peptide

<400> 35

Lys Leu Ala Leu Lys Leu Ala Leu Lys Leu Ala Leu Ala Leu Lys Leu
1               5                   10                  15

Ala

<210> 36
<211> 27
<212> PRT
<213> Artificial Sequence

<220>
<223> Peptide

<400> 36

Met Gly Leu Gly Leu His Leu Leu Val Leu Ala Ala Ala Leu Gln Gly
1               5                   10                  15

Ala Trp Ser Gln Pro Lys Lys Lys Arg Lys Val
            20                  25

<210> 37
<211> 27
<212> PRT
<213> Artificial Sequence

<220>
<223> Peptide

<400> 37

Gly Ala Leu Phe Leu Gly Trp Leu Gly Ala Ala Gly Ser Thr Met Gly
1               5                   10                  15

Ala Trp Ser Gln Pro Lys Lys Lys Arg Lys Val
            20                  25

<210> 38

<211> 27
<212> PRT
<213> Artificial Sequence

<220>
<223> Peptide

<400> 38

```
Gly Ala Leu Phe Leu Gly Phe Leu Gly Ala Ala Gly Ser Thr Met Gly
1               5                   10                  15

Ala Trp Ser Gln Pro Lys Lys Lys Arg Lys Val
            20                  25
```

<210> 39
<211> 27
<212> PRT
<213> Artificial Sequence

<220>
<223> Peptide

<400> 39

```
Gly Ala Leu Phe Leu Gly Phe Leu Gly Ala Ala Gly Ser Thr Met Gly
1               5                   10                  15

Ala Trp Ser Gln Pro Lys Ser Lys Arg Lys Val
            20                  25
```

<210> 40
<211> 21
<212> PRT
<213> Artificial Sequence

<220>
<223> Peptide

<400> 40

```
Lys Glu Thr Trp Trp Glu Thr Trp Trp Thr Glu Trp Ser Gln Pro Lys
1               5                   10                  15

Lys Lys Arg Lys Val
            20
```

<210> 41
<211> 21
<212> PRT
<213> Artificial Sequence

<220>
<223> Peptide

<400> 41

Lys Glu Thr Trp Phe Glu Thr Trp Phe Thr Glu Trp Ser Gln Pro Lys
1               5                   10                  15

Lys Lys Arg Lys Val
            20

<210> 42
<211> 10
<212> PRT
<213> Artificial Sequence

<220>
<223> Peptide

<400> 42

Leu Lys Leu Lys Leu Lys Leu Lys Leu Lys
1               5                   10

<210> 43
<211> 10
<212> PRT
<213> Artificial Sequence

<220>
<223> Peptide

<400> 43

Lys Leu Lys Leu Lys Leu Lys Leu Lys Leu
1               5                   10

<210> 44
<211> 10
<212> PRT
<213> Artificial Sequence

<220>
<223> Peptide

<400> 44

Lys His His His His Lys His His His Lys
1               5                   10

<210> 45
<211> 10
<212> PRT
<213> Artificial Sequence

<220>
<223> Peptide

<400> 45

```
Lys Lys Lys His His His His His His His
1               5                   10
```

<210> 46
<211> 10
<212> PRT
<213> Artificial Sequence

<220>
<223> Peptide

<400> 46

```
His His His Lys Lys Lys His His His His
1               5                   10
```

<210> 47
<211> 10
<212> PRT
<213> Artificial Sequence

<220>
<223> Peptide

<400> 47

```
Lys Lys Lys Lys Lys His His His His His
1               5                   10
```

<210> 48
<211> 88
<212> DNA
<213> Artificial Sequence

<220>
<223> Synthetic delivery molecule

<220>
<221> misc_feature
<222> (64)..(64)
<223> n is a, c, g, or t

<220>
<221> n
<222> (74)..(74)
<223> n is any nucleotide or multiple nucleotide sequence

<400> 48

```
aagcgcaaga agaagggcaa aggcctgggc aagaagcgcg atccgtgcct gcgcaagtat      60

aagncgaaga cgcaggagac gtcgaagg                                          88
```

<210> 49
<211> 8
<212> PRT
<213> Artificial Sequence

<220>
<223> Peptide

<400> 49

```
Arg Arg Arg Arg Arg Arg Arg Arg
1               5
```

<210> 50
<211> 9
<212> PRT
<213> Artificial Sequence

<220>
<223> Peptide

<400> 50

```
Arg Lys Lys Arg Arg Gln Arg Arg Arg
1               5
```

<210> 51
<211> 8
<212> PRT
<213> Artificial Sequence

<220>
<223> Peptide

<400> 51

```
Lys Lys Lys Lys Lys Lys Lys Lys
1               5
```

<210> 52
<211> 8
<212> PRT
<213> Artificial Sequence

<220>
<223> Peptide

<400> 52

```
Arg Gln Arg Gln Arg Gln Arg Gln
1               5
```

## Description

The invention relates to a method of treatment for cancer and targeted intracellular delivery of cargo molecules into tumour cells. The invention further relates to controlled transduction of cargo molecules into living cells *in vitro.*

Chemotherapy cancer treatment typically comprises the use of non-targeted delivery of bioactive agents, which can kill or interfere with the function of tumour cells. However, many chemotherapy agents also affect cells of healthy non-cancerous tissue, which can cause significant side-effects and death for a patient. Therefore, there is an increasing prevalence of targeted therapies, which preferentially or specifically target cells of tumour tissue. For this, differences in tumour and healthy cells and tissue must be exploited by a therapeutic agent. A lack of oxygen commonly found in tumours (tumour hypoxia) can cause glycolytic behaviour in the cells of the tumour. Tumour cells have also been shown to undergo aerobic glycolysis, where they preferentially produce lactate from glucose even in an aerobic environment. This phenomenon is called the Warburg effect. These metabolic effects are implicated in causing the extracellular microenvironment of the tumour, particularly solid tumours, to be more acidic relative to the normal healthy tissue environment.

A number of anti-tumour therapies have been generated to exploit the acidic microenvironment effect, such as pH-sensitive liposomes, polymeric micelles and nanogels for pH-sensitive drug release. However, a need exists to improve on the delivery of anti-tumour therapy to tumours and particularly to intracellularly deliver anti-tumour agents directly into the tumour cells, whilst avoiding delivery into cells of healthy tissue.

The term hyperthermia when related to cancer therapy implies a type of treatment based on the generation of heat at the tumour site. Heating the local environment of the tumour aims at changing the physiology of the diseased cells, eventually leading to apoptosis. Normally hyperthermia is induced by external devices that transfer the energy to the tissues. So far there are different types of hyperthermia treatments available, but they all suffer from limitations, and are mainly used combined with conventional treatments such as chemotherapy or radiotherapy (Falk et al, 2001). Due to the ability to convert dissipated magnetic energy into thermal energy, magnetic materials have become a focus of interest for the use in this therapy. The recent introduction of magnetic nanoparticles (MNPs) has opened another field of promising research (Hergt, Dutz, Müller, & Zeisberger, 2006). The current aim of hyperthermia therapy research is to achieve the desired temperature enhancement for a particular application with the lowest concentration of MNPs possible. MNPs have been demonstrated to be superior to other materials used on hyperthermia due to their efficiency in transforming magnetic energy into heat even at low concentrations, allowing lower doses of particles (Kumar & Mohammad, 2011) (Byrne et al., 2014). Another advantage of MNPs is that they provide the opportunity of direct tumour targeting through blood circulation.

However, despite all these advantages of MNP there is still a lot of work to be done on optimizing their delivery and temperature control. For this purpose a specific delivery method of the nanoparticles to the tumour will enhance the specificity of the treatment and also avoid damaging of healthy tissues. It is known that particular cell types have an enhanced ability to internalize MNPs, however to optimize the therapeutic potential of hyperthermia the uptake of MNPs should be as close to complete as possible. There are several techniques that can provide enhanced delivery of MNPs to the cells. For delivery of MNP some examples are modification of the particles, or combination of them with transfection agents (Wang & Cuschieri, 2013), however these are often cytotoxic and non-specific. CPPs are small peptides that are able to ferry large molecules into cells independent of classical endocytosis (Beerens, Al Hadithy, Rots, & Haisma, 2003). The use of CPPs clinically has been inhibited due to incomplete delivery of a variety of cargoes both *in vivo* and *in vitro.*

In addition to tumour therapies, a need also exists to control intracellular delivery of cargo into cells *in vitro.*

Zhang et al, Biomaterials (2013) 34(32):7980-7993 describes a pH-responsive $\alpha$-helical cell penetrating peptide-mediated liposomal delivery system.

Brunden et al, J Neurochem (1993) 61(6):2147-2154 describes pH-dependent binding of synthetic beta-amyloid peptides to glycosaminoglycans.

Dixon et al, PNAS (2016) 113(5) E291-E299 describes highly efficient delivery of functional cargoes by the synergistic effect of GAG binding motifs and cell-penetrating peptides.

An aim of the present invention is to provide a method and associated delivery vehicle for controlled and targeted intracellular delivery of a cargo, such as MNPs, into a cell.

According to a first aspect of the invention, there is provided a pH mediated cell delivery vehicle comprising:

- a cargo or a cargo-binding molecule for binding to a cargo;
- a protein transduction domain; and
- a glycosaminoglycan (GAG) binding element, which is capable of binding to GAG on the surface of the cell, wherein the GAG binding element is a peptide which is modified to comprise one or more histidine residues which are capable of being protonated in an acidic environment.

In an embodiment comprising a cargo-binding molecule, the cargo may be bound to the cargo-binding molecule.

Advantageously, the provision of the pH mediated delivery vehicle of the present invention can increase the efficiency of transduction of a cargo into cells whilst also providing controlled and/or targeted delivery as it is dependent on (e.g. activated by) an acidic pH environment. The GAG binding element, such as HS-GAG binding element P21 (from a growth factor), in combination with a protein transduction domain, such as 8mer arginine peptide, and a cargo, greatly facilitates the uptake of very large quantities of the cargo into cells, such as mammalian cells. Not only do the cells take up the delivery molecule (by macro-pinocytosis) but the delivery molecules have been shown to traverse the cellular matrix and be delivered to the nucleus. This procedure is more effective than DNA-based delivery of proteins and the cargo is delivered to sites where activity is desired (including the nucleus). The pH mediation of such a molecule is provided by the one or more histidine residues provided in the GAG binding element which replace lysine residues. The histidine residues significantly hinder the function of the delivery vehicle in a neutral or alkaline pH environment as they do not provide the lysine residue function. However, histidine is protonated in an acidic pH environment which cause the histidine to behave like a lysine, thereby restoring function of the delivery vehicle which has been modified to comprise one or more histidine residues instead of lysine residues. Tumour cell specific delivery is achievable as the microenvironment of many tumours is acidic, which protonates the histidine residue and allows the delivery vehicle to function. *In vitro,* the activation of intracellular delivery can be controlled by pH adjustment of the extracellular environment.

Described herein is a method of treating acidic pH tumours, comprising administration of a pH sensitive delivery vehicle arranged to be activated by an acidic environment of the tumour, wherein the delivery vehicle comprises

- a cargo comprising an anti-tumour therapeutic agent;
- a protein transduction domain; and
- a glycosaminoglycan (GAG) binding element, which is capable of binding to GAG on the surface of the cell, wherein the GAG binding element is a peptide which is modified to comprise one or more histidine residues which are capable of being protonated in an acidic environment.

Described herein is a method of targeted delivery of a cargo into acidic pH tumours, comprising administration of a pH sensitive delivery vehicle arranged to be activated by an acidic environment of the tumour, wherein the delivery vehicle comprises

- a cargo;
- a protein transduction domain; and
- a glycosaminoglycan (GAG) binding element, which is capable of binding to GAG on the surface of the cell, wherein the GAG binding element is a peptide which is modified to comprise one or more histidine residues which are capable of being protonated in an acidic environment.

The term "activated" discussed herein in the context of the delivery vehicle is understood to mean that it switches from an inactive form that does not promote intracellular uptake into the cell to an active form that does promote intracellular uptake into the cell. Without being bound by theory, the activation is caused by protonation of the one or more histidine residues such that they have a function provided by lysine residues.

The inventive molecule delivery system herein may be referred to as pH mediated GET (GAG-binding enhanced transduction) or otherwise pH mediated Heparan-sulfate enhanced transduction domain (HETD)-mediated delivery. These terms may be used interchangeably. The delivery mechanism for this technology is termed Glycosaminoglycan-binding Enhanced Transduction (GET) and is described and demonstrated in international patent publication No. WO 2015/092417.

Reference to "histidine-modified" herein refers to the modified form of a GAG binding element where the wild-type sequence, which normally binds GAG is modified with one or more histidine residues in place of lysine residues of the wild-type. Reference to "wild-type" used herein in the context of the GAG binding element is understood to mean the GAG binding element without histidine residue modifications in place of lysine residues.

The acidic environment of a tumour comprises a lower pH relative to non-tumour tissue pH. In one embodiment, the acidic environment may comprise a pH of less than 7, for example a pH of 6.9 or less.

The GAG binding element may be a peptide which is modified to comprise two or more histidine residues which are capable of being protonated in an acidic environment. Alternatively, the GAG binding element may be a peptide which is modified to comprise three or more histidine residues which are capable of being protonated in an acidic environment. Alternatively, the GAG binding element may be a peptide which is modified to comprise four or more histidine residues which are capable of being protonated in an acidic environment. Alternatively, the GAG binding element may be a peptide which is modified to comprise 5, 6, 7, 8 or more histidine residues which are capable of being protonated in an acidic environment.

The GAG binding element may be a heparan sulphate glycosaminoglycan (HS-GAG) binding element, which

is capable of binding to HS-GAG on the surface of the cell, and which has been modified such that one or more lysine residues of the wild-type molecule have been substituted with histidine residues.

Heparan sulfate glycosaminoglycan (HS-GAG) is a proteoglycan in which two or three HS chains are attached in close proximity to cell surface or extracellular matrix proteins. It is in this form that HS binds to a variety of protein ligands and regulates a wide variety of biological activities, including developmental processes, angiogenesis, blood coagulation and tumour metastasis. Heparan sulfate is a member of the glycosaminoglycan family of carbohydrates and is very closely related in structure to heparin. Both consist of a variably sulfated repeating disaccharide unit. The most common disaccharide unit within heparan sulfate is composed of a glucuronic acid (GlcA) linked to N-acetylglucosamine (GlcNAc) typically making up around 50% of the total disaccharide units.

The wild-type GAG binding element may have specific affinity for GAG. The histidine-modified GAG binding element may have specific affinity for GAG in an acidic environment.

The HS-GAG binding element may have specific affinity for HS-GAG. The HS-GAG binding element may comprise a heparin binding domain (HBD), or a variant thereof. The HS-GAG binding element may comprise a heparin binding domain (HBD), or a variant thereof which has been modified such that one or more lysine residues of the wild-type molecule have been substituted with histidine residues. The heparin binding domain variant may comprise a truncated heparin binding domain, or an extended heparin binding domain. The GAG binding element may comprise any protein, peptide or molecule that specifically or preferentially binds to GAG, which has been modified such that one or more lysine residues of the wild-type molecule have been substituted with histidine residues. The HS-GAG binding element may comprise any protein, peptide or molecule that specifically or preferentially binds to HS-GAG, which has been modified such that one or more lysine residues of the wild-type molecule have been substituted with histidine residues.

The HS-GAG binding element may comprise at least part of the heparin binding domain of Heparin-Binding EGF-like Growth Factor (HB-EGF), which has been modified such that one or more lysine residues of the wild-type molecule have been substituted with histidine residues. The heparin binding domain may comprise P21 of HB-EGF, which has been modified such that one or more lysine residues of the wild-type molecule have been substituted with histidine residues. The heparin binding domain may comprise a truncated, extended, or functional variant of P21, which has been modified such that one or more lysine residues of the wild-type molecule have been substituted with histidine residues.

The HS-GAG binding element may comprise a heparin binding domain of a fibroblast growth factor, or a functional part or variant thereof, which has been modified such that one or more lysine residues of the wild-type molecule have been substituted with histidine residues.

The HS-GAG binding element may be selected from any of the group comprising FGF, antithrombin, such as ATIII, VEGF, BMPs, Wnts, Shh EGFs, and PDGF; or variants thereof. The HS-GAG binding element may comprise any of FGF2, FGF7, or PDGF. The HS-GAG binding element may comprise one or more of the heparan binding sulphate domains of any FGF protein (e.g. domains A, B or C). The HS-GAG binding element may comprise FGF4. The HS-GAG binding element may comprise FGF1 HBD A (heparan sulphate binding domain A (the first HBD domain of FGF1)), FGF2 HBD A (heparan sulphate binding domain A), FGF4 HBD A (heparan sulphate binding domain A), FGF1 HBD C (heparan sulphate binding domain C), FGF2 HBD B (heparan sulphate binding domain B), FGF2 HBD C (heparan sulphate binding domain C), FGF4 HBD C (heparan sulphate binding domain C), FGF7 HBD B (heparan sulphate binding domain B), FGF7 HBD C (heparan sulphate binding domain C), antithrombin, such as ATIII, VEGF, or PDGF, or variants thereof, and which has been modified such that one or more lysine residues of the wild-type molecule have been substituted with histidine residues.

The HS-GAG binding element may be selected from any of the group comprising Hepatocyte Growth Factor, Interleukin, morphogens, HS-GAG binding enzymes, Wnt/Wingless, Endostatin, viral protein, such as foot and mouth disease virus protein, annexin V, lipoprotein lipase; or HS-GAG binding fragments thereof. The HS-GAG binding element may comprise any protein, peptide or molecule capable of specifically binding HS-GAG, and which has been modified such that one or more lysine residues of the wild-type molecule have been substituted with histidine residues.

Reference to modification such that one or more lysine residues of the wild-type molecule have been substituted with histidine residues, may comprise a modification such that two or more lysine residues of the wild-type molecule have been substituted with histidine residues. In another embodiment, three or more lysine residues of the wild-type molecule have been substituted with histidine residues. In another embodiment, four or more lysine residues of the wild-type molecule have been substituted with histidine residues. In another embodiment, five or more lysine residues of the wild-type molecule have been substituted with histidine residues. In another embodiment, six or more lysine residues of the wild-type molecule have been substituted with histidine residues. In another embodiment, seven or more lysine residues of the wild-type molecule have been substituted with histidine residues. In another embodiment, eight or more lysine residues of the wild-type molecule have been substituted with histidine residues. In another embodiment, nine or more lysine residues of the wild-type molecule have been substituted with histidine residues.

Advantageously, the level of pH mediation can be tuned/controlled depending on the number of lysine to

histidine substitutions provided in the delivery vehicle. For, example, a greater number of histidine residues requires a more acidic environment to activate the delivery vehicle. Therefore, the delivery vehicle can be modified to deliver the cargo more or less as required depending on the pH of the cell or tissue environment.

A "variant" may be understood by the skilled person to include a functional variant, wherein there may be some sequence differences from the known, reported, disclosed or claimed sequence, but the variant may still bind to HS-GAG. Conservative amino acid substitutions are also envisaged within the meaning of "variant".

The HS-GAG binding element may comprise the amino acid sequence KRKKKGKGLGKKRDPCLRKYK (P21) (SEQ ID NO. 1), which has been modified such that one or more lysine residues have been substituted with histidine residues. For example, the HS-GAG binding element may comprise the amino acid sequence HRKKKGKGLGKKRDPCLRKYK (P21) (SEQ ID NO. 2). Alternatively, the HS-GAG binding element may comprise the amino acid sequence KRHKKGKGLGKKRDPCLRKYK (P21) (SEQ ID NO. 3). Alternatively, the HS-GAG binding element may comprise the amino acid sequence KRKHKGKGLGKKRDPCLRKYK (P21) (SEQ ID NO. 4). Alternatively, the HS-GAG binding element may comprise the amino acid sequence KRKKHGKGLGKKRDPCLRKYK (P21) (SEQ ID NO. 5). Alternatively, the HS-GAG binding element may comprise the amino acid sequence KRKKKGHGLGKKRDPCLRKYK (P21) (SEQ ID NO. 6). Alternatively, the HS-GAG binding element may comprise the amino acid sequence KRKKKGKGLGHKRDPCLRKYK (P21) (SEQ ID NO. 7). Alternatively, the HS-GAG binding element may comprise the amino acid sequence KRKKKGKGLGKHRDPCLRKYK (P21) (SEQ ID NO. 8). Alternatively, the HS-GAG binding element may comprise the amino acid sequence KRKKKGKGLGKKRDPCLRHYK (P21) (SEQ ID NO. 9). Alternatively, the HS-GAG binding element may comprise the amino acid sequence KRKKKGKGLGKKRDPCLRKYH (P21) (SEQ ID NO. 10). The sequences of SEQ ID NO: 1 to 10 may comprise a modification in which a lysine residue is substituted with a histidine residue. The sequences of SEQ ID NO: 1 to 10 may comprise two modifications in which lysine residues are substituted with histidine residues. The sequences of SEQ ID NO: 1 to 10 may comprise three modifications in which lysine residues are substituted with histidine residues. The sequences of SEQ ID NO: 1 to 10 may comprise four modifications in which lysine residues are substituted with histidine residues. The sequences of SEQ ID NO: 1 to 10 may comprise five modifications in which lysine residues are substituted with histidine residues. The sequences of SEQ ID NO: 1 to 10 may comprise six modifications in which lysine residues are substituted with histidine residues. The sequences of SEQ ID NO: 1 to 10 may comprise seven modifications in which lysine residues are substituted with histidine residues. The sequences of SEQ ID NO: 1 to 10 may comprise eight modifications in which lysine residues are substituted with histidine residues.

In one embodiment, the HS-GAG binding element may comprise the amino acid sequence HRHHHGHGLGKKRDPCLRKYK (P21Nacid) (SEQ ID NO: 11). In another embodiment, the HS-GAG binding element may comprise the amino acid sequence KRKKKGKGLGHHRDPCLRHYH (P21Cacid) (SEQ ID NO: 12). In another embodiment, the HS-GAG binding element may comprise the amino acid sequence KRHKHGKGLGHKRDPCLRHYK (P21Eacid) (SEQ ID NO: 13). In another embodiment, the HS-GAG binding element may comprise the amino acid sequence HRKHKGHGLGKHRDPCLRKYH (P21Oacid) (SEQ ID NO: 14). In another embodiment, the HS-GAG binding element may comprise the amino acid sequence HRHHHGHGLGHHRDPCLRHYH (P21acid / P21a) (SEQ ID NO: 15).

The HS-GAG binding element may comprise a sequence having at least 80% identity to SEQ ID NO. 1, which has been modified such that one or more lysine residues have been substituted with histidine residues. The HS-GAG binding element may comprise a sequence having at least 90% identity to SEQ ID NO. 1, which has been modified such that one or more lysine residues have been substituted with histidine residues. The HS-GAG binding element may comprise a sequence having at least 95% identity to SEQ ID NO. 1, which has been modified such that one or more lysine residues have been substituted with histidine residues. The HS-GAG binding element may comprise a sequence having at least 98% identity to SEQ ID NO. 1, which has been modified such that one or more lysine residues have been substituted with histidine residues. The HS-GAG binding element may comprise a sequence having at least 99% identity to SEQ ID NO. 1, which has been modified such that one or more lysine residues have been substituted with histidine residues.

The HS-GAG binding element may comprise the amino acid sequence G R P R E S G K K R K R K R L K P T (PDGF, SEQ ID NO. 16), which has been modified such that one lysine residue has been substituted with a histidine residue. The HS-GAG binding element may comprise the amino acid sequence G R P R E S G K K R K R K R L K P T (PDGF, SEQ ID NO. 16), which has been modified such that two lysine residues have been substituted with histidine residues. The HS-GAG binding element may comprise the amino acid sequence G R P R E S G K K R K R K R L K P T (PDGF, SEQ ID NO. 16), which has been modified such that three lysine residues have been substituted with histidine residues. The HS-GAG binding element may comprise the amino acid sequence G R P R E S G K K R K R K R L K P T (PDGF, SEQ ID NO. 16), which has been modified such that four lysine residues have been substituted with histidine residues. The HS-GAG binding element may comprise the amino acid sequence G R P R E S G K K R K R K R L K P T (PDGF, SEQ ID NO. 16), which has been modified such that five lysine residues have been substituted with histidine residues.

The HS-GAG binding element may comprise a sequence having at least 80% identity to SEQ ID NO. 16, which has been modified such that one or more lysine residues have been substituted with histidine residues. The HS-GAG

binding element may comprise a sequence having at least 90% identity to SEQ ID NO. 16, which has been modified such that one or more lysine residues have been substituted with histidine residues. The HS-GAG binding element may comprise a sequence having at least 95% identity to SEQ ID NO. 16, which has been modified such that one or more lysine residues have been substituted with histidine residues. The HS-GAG binding element may comprise a sequence having at least 98% identity to SEQ ID NO. 16, which has been modified such that one or more lysine residues have been substituted with histidine residues. The HS-GAG binding element may comprise a sequence having at least 99% identity to SEQ ID NO. 16, which has been modified such that one or more lysine residues have been substituted with histidine residues.

The HS-GAG binding element may comprise the amino acid sequence T Y A S A K W T H N G G E M F V A L N Q ((FGF7, HBD B) SEQ ID NO. 17). The HS-GAG binding element may comprise the amino acid sequence T Y A S A H W T H N G G E M F V A L N Q ((FGF7, HBD B) SEQ ID NO. 18).

The HS-GAG binding element may comprise a sequence having at least 80% identity to SEQ ID NO. 17, wherein the lysine residue is substituted with a histidine residue. The HS-GAG binding element may comprise a sequence having at least 90% identity to SEQ ID NO. 17, wherein the lysine residue is substituted with a histidine residue. The HS-GAG binding element may comprise a sequence having at least 95% identity to SEQ ID NO. 17, wherein the lysine residue is substituted with a histidine residue. The HS-GAG binding element may comprise a sequence having at least 98% identity to SEQ ID NO. 17, wherein the lysine residue is substituted with a histidine residue. The HS-GAG binding element may comprise a sequence having at least 99% identity to SEQ ID NO. 17, wherein the lysine residue is substituted with a histidine residue.

The HS-GAG binding element may comprise the amino acid sequence T Y R S R K Y T S W Y V A L K R (FGF2 HBD B SEQ ID NO. 19). The HS-GAG binding element may comprise the amino acid sequence T Y R S R H Y T S W Y V A L K R (FGF2 HBD B SEQ ID NO. 20). The HS-GAG binding element may comprise the amino acid sequence T Y R S R K Y T S W Y V AL H R (FGF2 HBD B SEQ ID NO. 21). The HS-GAG binding element may comprise the amino acid sequence T Y R S R H Y T S W Y V A L H R (FGF2 HBD B SEQ ID NO. 22).

The HS-GAG binding element may comprise a sequence having at least 80% identity to SEQ ID NO. 19, wherein one or both lysine residues are substituted with a histidine residue. The HS-GAG binding element may comprise a sequence having at least 90% identity to SEQ ID NO. 19, wherein one or both lysine residues are substituted with a histidine residue. The HS-GAG binding element may comprise a sequence having at least 95% identity to SEQ ID NO. 19, wherein one or both lysine residues are substituted with a histidine residue. The HS-GAG binding element may comprise a sequence having at least 98% identity to SEQ ID NO. 19, wherein one or both lysine residues are substituted with a histidine residue. The HS-GAG binding element may comprise a sequence having at least 99% identity to SEQ ID NO. 19, wherein one or both lysine residues are substituted with a histidine residue.

Sequence identity may be determined by standard BLASTP alignment parameters (provided by http://www.ncbi.nlm.nih.gov/).

In one embodiment, the GAG binding element may not comprise any of the protein transduction domains described herein. In one embodiment, the GAG binding element and the protein transduction domain are different. In one embodiment, the GAG binding element is not TAT.

The protein transduction domain may be hydrophilic or amphiphilic. The protein transduction domain may comprise a majority of hydrophilic amino acid residues. The protein transduction domain may comprise a majority of arginine and/or lysine amino acid residues. The protein transduction domain may comprise a periodic sequence, having a repeated amino acid sequence motif. The protein transduction domain may comprise penetratin, TAT such as HIV derived TAT, MAP, or transportan, pVec, or pep-1.

Where reference is made to a "majority" of residue, this may be understood by the skilled person to include greater than 50% of the residues. A majority may be 55%, 60%, 70%, 80%, 90% or 95% of the residues.

The protein transduction domain may be selected from any of the group comprising:

Penetratin or Antenapedia PTD RQIKWFQNRRMKWKK (SEQ ID NO: 23);
TAT YGRKKRRQRRR (SEQ ID NO: 24);
SynB1 RGGRLSYSRRRFSTSTGR (SEQ ID NO: 25);
SynB3 RRLSYSRRRF (SEQ ID NO: 26);
PTD-4 PIRRRKKLRRLK (SEQ ID NO: 27);
PTD-5 RRQRRTSKLMKR (SEQ ID NO: 28);
FHV Coat-(35-49) RRRRNRTRRNRRRVR (SEQ ID NO: 29);
BMV Gag-(7-25) KMTRAQRRAAARRNRWTAR (SEQ ID NO: 30);
HTLV-II Rex-(4-16) TRRQRTRRARRNR (SEQ ID NO: 31);
D-Tat GRKKRRQRRRPPQ (SEQ ID NO: 32);
R9-Tat GRRRRRRRRRPPQ (SEQ ID NO: 33);
Transportan GWTLNSAGYLLGKINLKALAALAKKIL (SEQ ID NO: 34) chimera;

MAP KLALKLALKLALALKLA (SEQ ID NO: 35);
SBP MGLGLHLLVLAAALQGAWSQPKKKRKV (SEQ ID NO: 36);
FBP GALFLGWLGAAGSTMGAWSQPKKKRKV (SEQ ID NO: 37);
MPG ac-GALFLGFLGAAGSTMGAWSQPKKKRKV-cya (SEQ ID NO: 38);
MPG(?NLS) ac- GALFLGFLGAAGSTMGAWSQPKSKRKV-cya (SEQ ID NO: 39);
Pep-1 ac-KETWWETWWTEWSQPKKKRKV-cya (SEQ ID NO: 40); and
Pep-2 ac-KETWFETWFTEWSQPKKKRKV-cya (SEQ ID NO: 41).

The protein transduction domain may comprise polyarginines, such as RxN (4<N<17) chimera, polylysines, such as KxN (4<N<17) chimera, (RAca)6R, (RAbu)6R, (RG)6R, (RM)6R, (RT)6R. (RS)6R, R10, (RA)6R, R7, or R8.

The protein transduction domain may comprise polyarginine or polylysine. The protein transduction domain may comprise an arginine and lysine repeat sequence. The protein transduction domain may comprise arginine residues, such as consecutive arginine residues. The protein transduction domain may consist essentially of arginine residues. The protein transduction domain may comprise arginine repeats, such as 4-20 arginine residues. The protein transduction domain may comprise 8 arginine residues. The protein transduction domain may comprise between about 6 and about 12 arginine residues. The protein transduction domain may comprise between about 7 and about 9 arginine residues.

The protein transduction domain may comprise between about 4 and about 12 amino acid residues. The protein transduction domain may comprise between about 6 and about 12 amino acid residues. The protein transduction domain may comprise between about 7 and about 9 amino acid residues. The protein transduction domain may comprise at least about 4 amino acid residues. The protein transduction domain may comprise at least about 6 amino acid residues.

The protein transduction domain may comprise lysine residues, such as consecutive lysine residues. The protein transduction domain may consist essentially of lysine residues. The protein transduction domain may comprise lysine repeats, such as 4-20 lysine residues. The protein transduction domain may comprise 8 lysine residues. The protein transduction domain may comprise between about 4 and about 12 lysine residues. The protein transduction domain may comprise between about 6 and about 12 lysine residues. The protein transduction domain may comprise between about 7 and about 9 lysine residues.

The protein transduction domain may comprise Q and R residues, such as consecutive QR repeat residues. The protein transduction domain may consist essentially of Q and R residues. The protein transduction domain may comprise QR repeats, such as 4-20 QR repeat residues. The protein transduction domain may comprise 8 QR repeat residues. The protein transduction domain may comprise between about 6 and about 12 QR repeat residues. The protein transduction domain may comprise between about 7 and about 9 QR repeat residues.

The protein transduction domain may comprise poly-histidine peptide. For example, the protein transduction domain may comprise between about 4 and 20 consecutive histidine residues. In one embodiment, the protein transduction domain comprises 10 consecutive histidine residues. The protein transduction domain may comprise between about 4 and 20 amino acids, wherein one residue is histidine. Alternatively, the protein transduction domain may comprise between about 4 and 20 amino acids, wherein two residues are histidine residues. Alternatively, the protein transduction domain may comprise between about 4 and 20 amino acids, wherein two residues are histidine residues. Alternatively, the protein transduction domain may comprise between about 4 and 20 amino acids, wherein three residues are histidine residues. Alternatively, the protein transduction domain may comprise between about 8 and 20 amino acids, wherein four residues are histidine residues. Alternatively, the protein transduction domain may comprise between about 4 and 20 amino acids, wherein five residues are histidine residues. Alternatively, the protein transduction domain may comprise between about 4 and 20 amino acids, wherein six residues are histidine residues. Alternatively, the protein transduction domain may comprise between about 4 and 20 amino acids, wherein at least seven residues are histidine residues. Alternatively, the protein transduction domain may comprise between about 4 and 20 amino acids, comprising arginine and histidine residues. The protein transduction may comprise any pH or light inducible cell penetrating peptide. The protein transduction domain may also bind GAG.

The protein transduction domains discussed herein may be further modified to substitute one or more lysine residues with histidine residues. The protein transduction domains discussed herein may be further modified to substitute two or more lysine residues with histidine residues. The protein transduction domains discussed herein may be further modified to substitute three or more lysine residues with histidine residues. The protein transduction domains discussed herein may be further modified to substitute 4, 5, 6, 7, 8 or more lysine residues with histidine residues. The histidine residues may be paired or grouped together.

The protein transduction domain may comprise a 50:50 mixture of lysine and histidine residues. In another embodiment, the protein transduction domain may comprise a 40:60 mixture of lysine and histidine residues. In another embodiment, the protein transduction domain may comprise a 30:70 mixture of lysine and histidine residues. The protein transduction domain may comprise the sequence LKLKLKLKLK (SEQ ID NO: 42) or KLKLKLKLKL(SEQ ID NO: 43). In another embodiment, the protein transduction domain may comprise the sequence KHHHHKHHHK(SEQ ID NO: 44). In another embodiment, the protein transduction domain may comprise the sequence KKKHHHHHHH (SEQ ID NO: 45).

In another embodiment, the protein transduction domain may comprise the sequence HHHKKKHHHH (SEQ ID NO: 46). In another embodiment, the protein transduction domain may comprise the sequence KKKKKHHHHH (SEQ ID NO: 47).

Providing histidine residues in the protein transduction domain advantageously provides additional pH control over the delivery molecule.

The cargo may be a molecular cargo. The cargo may comprise a protein. The cargo may comprise a peptide. The cargo may comprise a non-small molecule. The cargo may comprise a nanoparticle, such as a metal nanoparticle or polymer nanoparticle. The nanoparticle may be a rod, such as a metal rod. The nano-particle may be porous. The cargo may comprise a nano-structure. The cargo may comprise a superparamagnetic iron oxide nanoparticle (SPION). The cargo may comprise nucleic acid, such as a nucleic acid vector. The cargo may comprise oligonucleotide. The cargo may comprise any of siRNA, modified messenger RNAs (mRNAs), micro RNAs, DNA, PNA, LNA or constructs thereof.

In one embodiment, the cargo is an anti-tumour agent. The cargo may comprise any cytotoxic agent that is capable of cell killing once internalised. The skilled person will be familiar with a large library of anti-cancer drugs that may be delivered as cargo in accordance with the invention, which includes potential anti-cancer drugs that perhaps did not meet sufficient efficacy requirements, yet would be assisted in efficacy by the pH mediate delivery vehicle of the invention.

The cargo may comprise any of the group selected from alkylating agents, such as Bendamustine, Busulfan, Carmustine, Chlorambucil, Cyclophosphamide, Dacarbazine, Ifosfamide, Melphalan, Procarbazine, Streptozocin, or Temozolomide: antimetabolites, such as Asparaginase, Capecitabine, Cytarabine, 5- Fluoro Uracil, Fludarabine, Gemcitabine, Methotrexate, Pemetrexed, Raltitrexed; anti-tumour antibiotics such as Actinomycin D / Dactinomycin, Bleomycin, Daunorubicin, Doxorubicin, Doxorubicin (pegylated liposomal), Epirubicin, Idarubicin, Mitomycin, Mitoxantrone; Plant Alkaloids/ Microtubule Inhibitors such as Etoposide, Docetaxel, Irinotecan, Paclitaxel, Topotecan, Vinblastine, Vincristine, Vinorelbine; DNA linking agents such as Carboplatin, Cisplatin, Oxaliplatin; biological agents such as Alemtuzamab, BCG, Bevacizumab, Cetuximab, Denosumab, Erlotinib, Gefitinib, Imatinib, Interferon, Ipilimumab, Lapatinib, Panitumumab, Rituximab, Sunitinib, Sorafenib, Temsirolimus, Trastuzumab, bisphosphonates such as Clodronate, Ibandronic acid, Pamidronate, Zolendronic acid; and hormones or other agents such as Anastrozole, Abiraterone, Amifostine, Bexarotene, Bicalutamide, Buserelin, Cyproterone, Degarelix, Exemestane, Flutamide, Folinic acid, Fulvestrant, Goserelin, Lanreotide, Lenalidomide, Letrozole, Leuprorelin, Medroxyprogesterone, Megestrol, Mesna, Octreotide, Stilboestrol, Tamoxifen, Thalidomide, or Triptorelin; or combinations thereof.

The cargo may comprise a magnetic nanoparticle (MNP). The magnetic nanoparticle may be an anti-tumour agent. The MNP may comprise iron. The MNP may comprise Nanomag®-D MNPs ($Fe_3O_4$ core; Micromod). The MNP may be about 250nm or less in size. The MNP may be 100nm or less in size. The MNP may be 30nm or less in size.

In one embodiment, the cargo may comprise ricin toxin. In another embodiment, the cargo may comprise Sheperdin (SHEP). Sheperdin (SHEP) targets the interaction between Hsp90 and survivin resulting in the breakdown of the survivin. Survivin is upregulated in cancer cells and acts to prevent cell death. Therefore, through the delivery of SHEP as the cargo, any cells reliant on survivin can be selectively targeted, leading to cell death.

The cargo may comprise a physiologically or metabolically relevant protein. The cargo may comprise an intracellular protein. The cargo may comprise a signal protein, which is a protein involved in a signal pathway. The cargo may comprise a protein involved with regulation of expression or metabolism of a cell. The cargo may comprise a protein involved with cell division. The cargo may comprise a protein involved with cell differentiation, such as stem cell differentiation. The cargo may comprise a protein required for induction of pluripotent stem cells. The cargo may comprise a protein involved with cardiac cell differentiation. The cargo may comprise a marker, such as a protein marker. The cargo may comprise a bacterial, or bacterially derived protein. The cargo may comprise a mammalian, or mammalian derived protein. The cargo may be any peptide, polypeptide or protein. The cargo may comprise research, diagnostic or therapeutic molecules. The cargo may comprise a transcription modulator, a member of signal production. The cargo may comprise an enzyme or substrate thereof, a protease, an enzyme activity modulator, a perturbimer and peptide aptamer, an antibody, a modulator of protein-protein interaction, a growth factor, or a differentiation factor.

The cargo may be a pre-protein. For example, excision domains may be provided in the delivery molecule, which is arranged to be cleaved upon entry or after entry into the cell. The cargo may be a protein arranged to be post-translationally modified within the cell. The cargo may be arranged to be functional once inside the cell. For example, the cargo may not be functional until after transduction into the cell.

The cargo may comprise any intracellular molecule. The cargo may comprise any protein or molecule having an intracellular function (mode of action), intracellular receptor, intracellular ligand, or intracellular substrate. The cargo may comprise a protein or molecule that is naturally/normally internalised into a cell. The cargo may comprise a protein intended for delivery or display in the cell surface, such as a cell surface receptor. The cargo may be selected from any of the group comprising a therapeutic molecule; a drug; a pro-drug; a functional protein or peptide, such as an enzyme or a transcription factor; a microbial protein or peptide; and a toxin; or nucleic acid encoding thereof.

The cargo may be selected from any of the group comprising NANOG, NEO, MYOD, Cre, GATA4, TBX5, BAF60c and NKX2.5. The cargo may comprise RFP. The cargo may comprise Cre. The cargo may comprise a member

of the cardiac gene regulatory network. The cargo may comprise GATA4. The cargo may comprise TBX5. The cargo may comprise NKX2.5. The cargo may comprise BAF60c. The cargo may comprise Oct-3/4 (Pou5f1), Sox2, Lin28, Klf4, Nanog, Glis1 or c-Myc; or combinations thereof.

The cargo may be selected from any of the group comprising toxin, hormone transcription factors, such as jun, fos, max, mad, serum response factor (SRF), AP-1, AP2, myb, MyoD, myogenin, ETS-box containing proteins, TFE3, E2F, ATF1, ATF2, ATF3, ATF4, ZF5, NFAT, CREB, HNF4, C/EBP, SP1, CCAAT-box binding proteins, interferon regulation factor (IRF-1), Wilms tumor protein, ETS-binding protein, STAT, GATA-box binding proteins, e.g., GATA-3, transcription factor, such as HIF1a and RUNT, the forkhead family of winged helix proteins, carbamoyl synthetase I, ornithine transcarbamylase, arginosuccinate synthetase, arginosuccinate lyase, arginase, fumarylacetacetate hydrolase, phenylalanine hydroxylase, alpha-1 antitrypsin, glucose-6-phosphatase, porphobilinogen deaminase, factor VIII, factor IX, cystathione beta-synthase, branched chain ketoacid decarboxylase, isovaleryl-coA dehydrogenase, propionyl CoA carboxylase, methyl malonyl CoA mutase, glutaryl CoA dehydrogenase, beta-glucosidase, pyruvate carboxylate, hepatic phosphorylase, phosphorylase kinase, glycine decarboxylase, H-protein, T-protein, a cystic fibrosis transmembrane regulator (CFTR) sequence, a dystrophin cDNA sequence, Oct-3/4 (Pou5f1), Sox2, c-Myc, Klf4, RPE65 Nanog, and SoxB1; or fragments thereof, and/or combinations thereof.

The cargo may comprise non-covalently bound complexes such as protein-protein complexes, protein-mRNA, protein-non-coding RNA, protein-lipid and protein-small molecule complexes. Examples of such complexes are RISCs and spliceosomes.

The cargo may comprise a photo-reactive agent for photodynamic therapy in combination with the pH mediated delivery. The photo-reactive agent may comprise a compound capable of forming reactive oxygen species (ROS) upon excitation by light. The photo-reactive agent may comprise a porphyrin, chlorophyll or dye.

The method of treatment or targeted delivery described herein may further comprise photodynamic therapy in combination with the pH mediated delivery, wherein the pH mediated delivery vehicle is linked to a cargo comprising a photo-reactive agent as an anti-tumour therapeutic agent. The photodynamic therapy may comprise the step of exposing the tumour tissue to light which is capable of excitation of the photo-reactive agent thereby causing cell death within the tumour tissue.

The cargo may have a molecular weight of at least 1KDa. The cargo may have a molecular weight of at least 5KDa. The cargo may have a molecular weight of at least 10KDa. The cargo may have a molecular weight of at least 20KDa. The cargo may have a molecular weight of 400KDa or less. The cargo may have a molecular weight of 300KDa or less. The cargo may have a molecular weight of between about 0.5KDa and about 400kDa. The cargo may have a molecular weight of between about 1KDa and about 400kDa. The cargo may have a molecular weight of between about 0.5KDa and about 200kDa. The cargo may have a molecular weight of between about 1KDa and about 200kDa. The cargo may have a molecular weight of between about 2KDa and about 300kDa. The cargo may have a molecular weight of between about 20KDa and about 300kDa. The cargo may have a molecular weight of between about 20KDa and about 100kDa.

Where the cargo comprises amino acids, the cargo may be between about 20 and about 30,000 amino acids in length. The cargo may be between about 20 and about 10,000 amino acids in length. The cargo may be between about 20 and about 5,000 amino acids in length. The cargo may be between about 20 and about 1000 amino acids in length. The cargo may be at least about 20 amino acids in length. The cargo may be at least about 100 amino acids in length.

The cargo may be capable of binding, such as ionic or covalent binding, to the cargo-binding molecule. The cargo may be capable of binding, such as ionic or covalent binding, to the protein transduction domain and/or GAG binding element.

The cargo may comprise an element for binding to the cargo-binding molecule. The cargo may comprise biotin, or alternatively streptavidin. The cargo may be biotinylated. The cargo may comprise an affinity tag capable of binding to a complementary affinity tag on the cargo-binding molecule.

In one embodiment the cargo is bound to the cargo-binding molecule. The cargo may be bound to the cargo-binding molecule during manufacture of the delivery molecule, post-manufacture, prior to use, or during use.

The cargo-binding molecule may be a carrier for the cargo molecule. A single cargo-binding molecule may bind and carry multiple cargo molecules. The cargo-binding molecule may protect the cargo prior to internalisation into a cell. The cargo-binding molecule may be capable of binding to biotin on a biotinylated cargo. The cargo-binding molecule may be capable of binding to nucleic acid-based cargo. The cargo-binding molecule may be capable of binding to a peptide-based cargo. The cargo-binding molecule may be capable of binding to an antibody cargo, or fragment or mimetic thereof. The cargo-binding molecule may be capable of binding to a nanoparticle cargo, such as a metal or polymer nanoparticle. The cargo-binding molecule may be functionally inactive in a cell, but can carry or bind to an active cargo. The cargo-binding molecule may comprise a chemical linker molecule. The cargo-binding molecule may comprise an affinity tag. The cargo-binding molecule may comprise a peptide or protein. The cargo-binding molecule may comprise mSA2 (monomeric streptavidin 2). The cargo-binding molecule may comprise a nucleic acid interacting peptide, such

as LK15. The cargo-binding molecule may comprise an antibody binding molecule, such as an IgG binding protein. The IgG binding protein may comprise S. aureus IgG ninding protein SpAB. The skilled person will understand that any suitable pairs or groups of molecules may be used for the cargo and cargo-binding molecule provided that they have sufficient binding or affinity between them.

The bond or interaction between the cargo and the cargo-binding molecule may be reversible, or degradeable, for example in the intracellular environment.

The GAG binding element and protein transduction domain may be bound to the cargo and/or cargo-binding molecule by direct chemical conjugation or through a linker molecule. The GAG binding element and protein transduction domain may be bound to the cargo by direct chemical conjugation or through a linker molecule. The GAG binding element and protein transduction domain may be bound to the cargo-binding molecule by direct chemical conjugation or through a linker molecule. The GAG binding element, protein transduction domain and cargo may be a single fusion molecule (e.g. it may be encoded and transcribed as a single peptide molecule). The GAG binding element, protein transduction domain and cargo-binding molecule may be a single fusion molecule (e.g. it may be encoded and transcribed as a single peptide molecule). The protein transduction domain and GAG binding element may flank the cargo-binding molecule and/or cargo.

The delivery vehicle may comprise P21a (HRHHHGHGLGHHRDPCLRHYH (SEQ ID NO: 15)) and a protein transduction domain comprising or consisting of 10 histidine residues.

The combined GAG-binding element, the protein transduction domain and the cargo may be termed the "delivery molecule".

The delivery molecule may be between about 10 and about 30,000 amino acids in length. The delivery molecule may be between about 20 and about 30,000 amino acids in length. The delivery molecule may be between about 30 and about 30,000 amino acids in length. The delivery molecule may be between about 40 and about 30,000 amino acids in length. The delivery molecule may be between about 10 and about 10,000 amino acids in length. The delivery molecule may be between about 20 and about 10,000 amino acids in length. The delivery molecule may be between about 40 and about 10,000 amino acids in length. The delivery molecule may be between about 10 and about 3,000 amino acids in length. The delivery molecule may be between about 20 and about 3,000 amino acids in length. The delivery molecule may be between about 40 and about 3,000 amino acids in length. The delivery molecule may be between about 10 and about 1000 amino acids in length. The delivery molecule may be between about 20 and about 1000 amino acids in length. The delivery molecule may be between about 40 and about 1000 amino acids in length. The delivery molecule may be between about 40 and about 500 amino acids in length. The delivery molecule may be between about 10 and about 500 amino acids in length. The delivery molecule may be between about 20 and about 500 amino acids in length. The delivery molecule may be between about 100 and about 3,000 amino acids in length. The delivery molecule may be at least about 100 amino acids in length.

The delivery molecule may be a single fusion molecule. The cargo, HS-GAG binding element, and protein transduction domain may be fused together. The HS-GAG binding element and protein transduction domain may flank the cargo. The cargo, HS-GAG binding element, and protein transduction domain may be linked together by one or more linker molecules.

The delivery molecule may have a molecular weight of at least 1KDa. The delivery molecule may have a molecular weight of at least 5KDa. The delivery molecule may have a molecular weight of at least 10KDa. The delivery molecule may have a molecular weight of at least 20KDa. The delivery molecule may have a molecular weight of 400KDa or less. The delivery molecule may have a molecular weight of 300KDa or less. The delivery molecule may have a molecular weight of between about 0.5KDa and about 400kDa. The delivery molecule may have a molecular weight of between about 1KDa and about 400kDa. The delivery molecule may have a molecular weight of between about 0.5KDa and about 200kDa. The delivery molecule may have a molecular weight of between about 1KDa and about 200kDa. The delivery molecule may have a molecular weight of between about 2KDa and about 300kDa. The delivery molecule may have a molecular weight of between about 20KDa and about 300kDa. The delivery molecule may have a molecular weight of between about 20KDa and about 100kDa.

The delivery molecule may comprise a marker for identifying and/or tracking the location of the delivery molecule. The marker may comprise a fluorescence marker, or a radioisotope. The marker may comprise mRFP1 (monomeric red fluorescent protein). The marker may comprise mNectarine, such as pH-sensitive mNectarine. mNectarine, is appropriate to measure physiological pH changes in mammalian cells, because it has a pKa' of 6.9. The marker may comprise a red fluorescent protein (RFP) homologue of avGFP. The marker may comprise a fluorescent protein selected from the mFruit series RFPs, derived from tetrameric Discosoma RFP. The marker may comprise any of mTangerine, mOrange, mCherry, mStrawberry, yellow FP Citrine. mApple and TagRFP-T. The marker may be pH-sensitive. The marker may be used to confirm delivery of the delivery molecule into the cell or tissue. The marker may be cell-type specific, for example the marker may only be activated or fluoresce in specific cell types.

The delivery molecule may comprise a tag to aid in purification, isolation, detection and/or determination of location. The tag may be an affinity tag. The tag may be a peptide. The tag may be a FLAG-tag / FLAG octapeptide.

In one embodiment, the method of targeted delivery of a cargo into acidic pH tumours is *in vivo.*

According to another aspect of the invention, there is provided a method of pH mediated cell delivery *in vitro* comprising:

- providing cells *in vitro;*
- exposing the cells to the pH mediated delivery vehicle according to the invention;
- lowering the pH environment of the cells, such that the one or more lysine residues of the pH mediated delivery vehicle are protonated, thereby activating the pH mediated delivery vehicle for intracellular uptake/delivery.

Advantageously, the method of pH mediated cell delivery *in vitro* enables control of the intracellular delivery. The method may be for use in imaging.

The cells may be mammalian cells, such as human cells. The cells may be cancerous cells. The cells may be stem cells. The cells may be mutant cells. The cells may comprise a population of cells. The population of cells may be a mixed population of cell types. The cells may be mesenchymal stem cells. The cells may be embryonic stem cells. The cells may be pluripotent stem cells. The cells may be cells requiring functional restoration. The cells may be cardiac stem cells. The cells may be selected from any of the group comprising NIH3t3, CGR8, and HUES7.

The delivery molecule may be encoded by a nucleotide sequence comprising SEQ ID NO: 48, wherein one or more codons for lysine in SEQ ID NO: 48 may be substituted for codons encoding histidine. Two or more codons for lysine in SEQ ID NO: 48 may be substituted for codons encoding histidine. All codons for lysine in SEQ ID NO: 48 may be substituted for codons encoding histidine. Additionally or alternatively, one or more codons encoding the arginine residues of the 8R protein transduction domain may be substituted to codons encoding histidine. For example, all codons encoding the arginine residues of the 8R protein transduction domain may be substituted to codons encoding histidine. In another embodiment, the 8R encoded protein transduction domain may be substituted for codons encoding a 10H protein transduction domain, which is pH sensitive.

Example delivery molecule nucleotide sequence (P21-cargo-8R):

(SEQ ID NO: 48)

aagcgcaagaagaagggcaaaggcctgggcaagaagcgcgatccgtgcctgcgcaagtataagNcgaagacgcagga
gacgtcgaagg

N=cargo nucleic acid sequence of various length (i.e. the number of nucleotide residues may vary), or another molecular entity. Alternative codons for the same amino acid residue may also be provided throughout the sequence as appropriate.

According to another aspect of the present invention, there is provided a nucleic acid encoding the delivery molecule according to the invention.

The nucleic acid may be DNA. The nucleic acid may be a vector.

According to another aspect of the present invention, there is provided a pharmaceutical composition comprising a pH mediated delivery molecule according to the invention, and a pharmaceutically acceptable excipient.

Described herein, there is provided a pharmaceutical composition or delivery vehicle according to the invention herein for use in the treatment or prevention of a disease.

The delivery molecule may be transduced in the presence of, or co-administered with a vesicle/endosome release agent for promoting release of the delivery molecule from micropinocytic vesicles. The vesicle release agent may comprise chloroquine. The chloroquine concentration may be between about 1μM and about 100μM.

The delivery vehicle may further comprise an endosome release agent. The endosome release agent may comprise one or more trifluoromethylquinolines, for example four trifluoromethylquinolines as described in Lindberg et al. (International Journal of Pharmaceutics 441 (2013) 242- 247). For example, the endosome release agent may be linked to the delivery vehicle and may comprise the following structure:

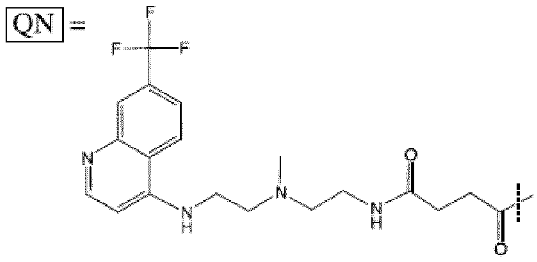

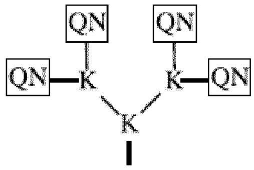

,

which is arranged to be linked to the delivery vehicle, optionally the link is to a lysine residue of the delivery vehicle.

The endosome release agent linked to a delivery vehicle described herein may comprise or consist of the following structure (P21-8R-QN1):
Stearyl-KRKKK1(QN-K3[QN]K2{QN-K3[QN]})GKGLGKKRDPCLRKYKRRRRRRRR, alternatively represented as:

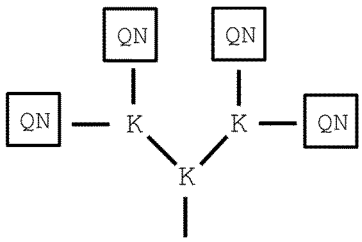

Stearyl-KRKKKGKGLGKKRDPCLRKYKRRRRRRRR-$NH_2$

,

wherein the peptide comprises one or more lysine to histidine substitutions as described herein for pH mediation.

The endosome release agent linked to a delivery vehicle described herein may comprise or consist of the following structure (P21-8R-QN2):
Stearyl-KRKKKGKGLGKKRDPCLRKYK1(QN-K3[QN]K2{QN-K3[QN]})RRRRRRRR, alternatively represented as:

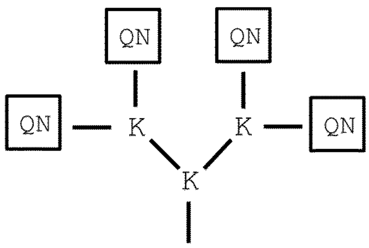

Stearyl-KRKKKGKGLGKKRDPCLRKYKRRRRRRRR-$NH_2$

,

wherein the peptide comprises one or more lysine to histidine substitutions as described herein for pH mediation.

The endosome release of the pH mediated delivery vehicle may use photochemical internalisation (PCI) technology that can enhance delivery of bioactive agents into the cytoplasm of a cell. In particular the pH mediated delivery vehicle may be further linked to a photosensitiser
Advantageously, the provision of the pH mediated delivery vehicle with a photosensitiser described herein can increase the efficiency and specific targeting of delivery and internalisation of the cargo cells by encouraging endosome release by excitation with light. This provides an additional layer of control of the delivery vehicle delivery by pH mediation and light-mediation via the action of the photosensitiser.

The term "Photosensitiser" used herein is understood to mean a photo-sensitive endo/lysosome release agent. Photosensitisers act by absorbing energy electromagnetic radiation in the form of ultraviolet or visible light and transferring it to adjacent molecules, potentiating a reaction. The photosensitiser may be photoxidative, causing release of reactive oxygen species (ROSs) upon excitation by light.

The photosensitiser may be a monofunctional photosensitiser. The photosensitising group may be selected from the group consisting of: lysomotropic weak bases, benzoporphyrins, haematoporphyrms, photofrin, naturally-occurring porphyrins, chlorins and bacteriochlorins, pheophorbides like pyropheophorbide a and its derivatives like Photochlor, chlorins, chlorin e6, mono-1-aspartyl derivative of chlorin e6, di-1-aspartyl derivative of chlorin e6, tin (IV) chlorin eβ, the palladium derivatives of naturally occurring bacteriochlorophylls like TOOKAD (Pd-bacteriopheophorbide), meta-tetrahydroxyphenyl chlorin (Foscan), benzoporphyrin derivatives, monobenzoporphyrin derivatives like verteporfm, phthalocyanines, sulphonated aluminium phthalocyanines (disulphonated and tetrasulphonated), naphthalocyanines, such as sulphonated aluminium naphthalocyanines and derivatives, purpurins like purpurin-18, tin and zinc derivatives of octaethylpurpurin, tin etiopurpurin, verdins, porphycenes, synthetic porphyrins, chlorins and bacteriochlorins, like the meso-triethynylporphyrins, metal free and metallated, core-modified porphyrins, expanded porphyrins (texaphyrins) like motexafin lutetium and motexafrn gadolinium, ketochlorins, hematoporphyrin derivatives, and cationic dyes and tetracyclines or derivatives thereof (Berg et al., (1997), J. Photochemistry and Photobiology, 65, 403-409).

Photosensitisers may comprise, but are not limited to, phenothiazinium derivatives like methylene blue, toluidine blue, cyanines such as merocyanine-540, acridine dyes, BODIPY dyes and aza- BODIPY derivatives, hypericin, halogenated squarine dyes and halogenated xanthene dyes like eosin and rose Bengal.

Porphyrin molecular structure includes four pyrrole rings linked together via methine bridges. They are natural compounds which are often capable of forming metal-complexes. For example in the case of the oxygen transport protein hemoglobin, an iron atom is introduced into the porphyrin core of heme B. Chlorins are large heterocyclic aromatic rings consisting, at the core, of three pyrroles and one pyrroline coupled through four methine linkages.

Other suitable photosensitisers for conjugation to the pH mediated delivery vehicle will readily occur to those skilled in the art.

In one embodiment, two or more photosensitisers may be linked to the pH mediated delivery vehicle. Alternatively, three or more photosensitisers may be linked to the pH mediated delivery vehicle In an embodiment wherein a plurality of photosensitisers are linked to a pH mediated delivery vehicle, the photosensitisers may be the same photosensitiser species, or different photosensitisers (e.g. combinations of the above photosensitisers may be provided).

In one embodiment, the photo-sensitive endosome release agent comprises formula (I):

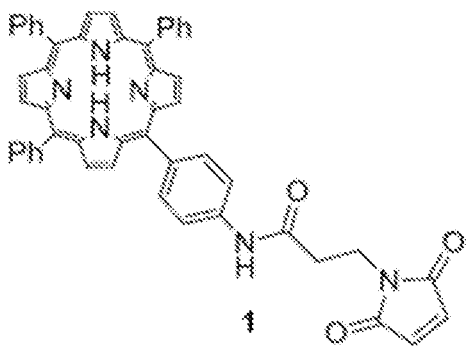

1

Formula (I).

In another embodiment, the photo-sensitive endosome release agent comprises formula (II):

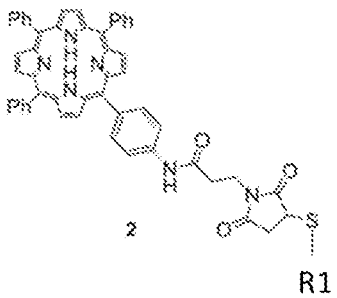

2

Formula (II)

wherein R1 is the pH mediated delivery vehicle or a linker to the pH mediated delivery vehicle.

The photosensitiser link to the pH mediated delivery vehicle may be a covalent bond, such as a disulphide bond. For example, sulphur of one of the amino acid residues, for example cysteine, of the pH mediated delivery vehicle may be involved in a disulphide bond with the photosensitiser in order to form the link. The skilled person will readily understand there are a number of methods, such as "click-chemistry", for linking chemical entities to peptides, such as the pH mediated delivery vehicle described herein. Publications WO2007042775, WO2013189663, and Wang et al. (2012. Journal of Controlled Release, 157 (2). pp. 305-313) describe suitable photosensitiser-to-peptide linking methods.

The pH mediated delivery vehicle may further comprise a linker molecule. The photosensitiser may be linked to the pH mediated delivery vehicle via the linker molecule. The linker molecule may comprise a polymer, a peptide or a small molecule (e.g. a molecule of less than 900Da). The linker may comprise an amino acid residue, such as lysine. The linker molecule may comprise one or more lysine residues linked to a lysine residue of the delivery vehicle.

The photosensitiser linked to a pH mediated delivery vehicle described herein may comprise or consist of the following structure (P21-8R-TPP1):

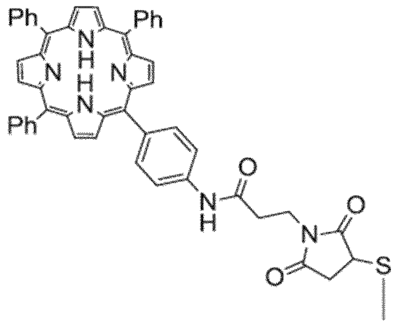

KRKKKGKGLGKKRDPCLRKYKRRRRRRRR-$NH_2$

,

wherein one or more of the lysine residues of the peptide are substituted with histidine in accordance with the description herein.

Alternative linking residues of the above pH mediated delivery vehicle may be considered by the skilled person.

Described herein is a method of treatment comprising administrating to a patient the photosensitiser linked to a pH mediated delivery vehicle described herein and a therapeutic cargo, and exposing the patient to light, thereby causing excitation of the photosensitiser and endosome/lysosome release of the therapeutic cargo into the cells of the patient which are exposed to the light.

The method of treatment may be suitable for any disease where specific tissue or regions of tissue need to be treated, such as a tumour. Cells or tissue may be selectively exposed to the light. Cells or tissue which are not to be treated for endosome/lysosome release may be masked/protected from the light source or kept dark.

Described herein is a method of treatment of cancer in a patient by pH mediated and light-assisted targeted cell killing, comprising:

- administrating a cytotoxic cargo and a photosensitiser linked to a pH mediated delivery vehicle described herein to the patient;
- exposing cells in the cancerous tissue with a light capable of exciting the photosensitiser, thereby releasing the cytotoxic cargo from the endosomes/lysosomes of the cell into the cell cytoplasm.

The light may be directed to specific tissue types, or specific regions of tissue. The light may be directed to cancerous tissue, such as a tumour. The light may be directed to the core of a tumour. Tissue which is not to be treated, for example healthy tissue, may be masked/protected from the light source or kept dark.

The light may be at a wavelength and intensity suitable to cause excitement of the photosensitiser, for example causing a photo-oxidative reaction of the photosensitiser. The light may be at least 420nm in wavelength. In one embodiment the light wavelength is in the red spectrum. In one embodiment the light wavelength is at least 590nm. The light exposure may vary between applications and patients.

The administration dose of the cargo and/or photosensitiser linked to a pH mediated delivery vehicle may be at a therapeutically effective amount.

Described herein is a method of imaging in vivo comprising administration of a pH mediated delivery molecule according to the invention to a subject; and
detecting and imaging the cargo location in the subject,
optionally wherein the cargo is a NMP.

The method of imaging may be for use in imaging tumour locations in a subject.

The skilled person will understand that optional features of one embodiment or aspect of the invention may be applicable, where appropriate, to other embodiments or aspects of the invention.

Embodiments of the invention will now be described in more detail, by way of example only, with reference to the accompanying drawings.

**Figure 1** pH stability of culture media. The three media studied in this experiment were: Buffered, Non-Buffered and CO2 independent. pH of the media after incubation overnight with cells with and without CO2 (A). pH variability calculated for each media after incubation overnight with cells in a non-CO2 (B) or 5% CO2 (C) atmosphere, plotted against initial pH.

**Figure 2** P218R and P21a10H mediated MNP delivery to cell monolayer in buffered media. Representative bright field images of Prussian blue stained MCF7 cells media at pH 7.6, 7.4, 7.2, 7.0, 6.8 and 6.6 treated with MNP; (A, D, G, J, M, P) with MNP+P218R peptide (B, E, H, K, N, Q) and with MNP+P21a10H peptide (C, F, I, L,O,R). Cells were treated overnight with 100μg/mL of MNP and 1 mM of peptide; scale bar 40 μm; n=1.

**Figure 3** P218R and P21a10H mediated MNP delivery to cell monolayer in non-buffered media. Representative bright field images of Prussian blue stained MCF7 cells media at pH 7.6, 7.4, 7.2, 7.0, 6.8 and 6.6 treated with MNP; (A, D, G, J, M, P) with MNP+P218R peptide (B, E, H, K, N, Q) and with MNP+P21a10H peptide (C, F, I, L,O,R). Cells were treated overnight with 100μg/mL of MNP and 1 mM of peptide; scale bar 40 μm; n=1.

**Figure 4** P218R and P21a10H mediated MNP delivery to cell monolayer in CO2 independent media. Representative bright field images of Prussian blue stained MCF7 cells at pH 7.6, 7.4, 7.2, 7.0, 6.8, 6.6, 6.4, 6.2 and 6.0 treated with MNP (A, D, G, J, M, P, S, V, Y) with MNP+P218R peptide (B, E, H, K, N, Q, T, W, Z) and with MNP+P21a10H peptide (C, F, I, L,O,R, U, X, 1). Cells were treated overnight with 100μg/mL of MNP and 1 mM of peptide; scale bar 40 μm; n=1.

**Figure 5** Protein binding assay. Percentage of peptide bound to the MNPs at each pH. Proteins used for this experiment were P21mR8R and P21amR10H, fluorescent versions of P218R and P21a10H. Samples were incubated for one hour at room temperature with 50μg of MNPs and with 20ug/ml of red fluorescent protein. Data reported as mean ± SEM; n=2.

**Figure 6** Slightly acidic conditions (pH6.6) promote P21 activity but inhibit 8R function. DMEM (no Sodium Biocarbonate or HEPES) pH adjusted with HCl.

**Figure 7** Mutations in some CPP to render activity acid inducible. Lysine (K) to Histidine (H). In acidic environments H is protonated and functions as a K mimic.

**Figure 8** 10H and CPP27 have acid inducible transduction activity. DMEM (no Sodium Biocarbonate or HEPES) pH adjusted with HCl.

**Figure 9** Mutations in P21 to render its activity acid inducible. Lysine (K) to Histidine (H). In acidic environments H is protonated and functions as a K mimic.

**Figure 10** K>H Mutations in P21 can render its activity acid inducible.

**Figure 11** P21 can Synergise with 10H and CPP27 acid inducible transduction activity.

**Figure 12** 8R can Synergise with K>H Mutations in P21 acid inducibly.

**Figure 13** Full protein for Acid inducibility.

**Figure 14** Treatment of Hela cells with 50 μM SHEP for 24h. Shows a decrease in viability as acidity increases; a significantly more viability loss when under the effect of P218RSHEP; and acid inducibility demonstrated by the inactivation of the GET system when not under the right conditions.

**Figure 15** Demonstration of acid inducibility. Hela cells + 10 μM SHEP treated for 24h in various pH conditions. P21a-10H-SHEP cytotoxic activity is increased with progressively more acidic microenvironment. This is demonstrated by trypan blue exclusion of live cells and blue staining of dead cells.

**Figure 16** P21 improves PTD-mediated transduction. (a) Schematic of the proteins created after screening domains which improve efficiency of protein delivery to cells. P21-mR is mRFP with an *N*-terminal fusion of the P21 domain of heparin-binding EGF (HB-EGF). P21-mR-8R is mRFP with N-terminal fusion of P21 and C-terminal fusion of 8R. (b) Fusion of P21 to mR-8R significantly improves transduction into NIH3t3 cells. Fluorescence microscopy images of NIH3t3 cells treated with proteins (20μg/ml) for twelve hours in standard media conditions. Scale bar, 100μm. (c) P21-mR-8R transduces efficiently into human and mouse embryonic stem cells (HUES7 and CGR-8, respectively) and human induced pluripotent stem cells (IPS2) and mouse cardiomyocyte cell line HL1. Flow cytometry analyses of the mR-8R-inefficiently transduced cell lines treated with proteins mR-8R (20μg/ml) for twelve hours. **(d)** P21-mR-8R initially strongly interacts with cell membranes and progressively transduces be localised peri-nuclearly. Fluorescence (top) and confocal laser scanning microscopy (bottom) images of NIH3t3 cells treated with P21-mR-8R (20μg/ml) for either 1 hour, 1 hour with washes and a further 5 hours incubation (in serum-free media) or 6 hours treatment. Cells were pre-incubated for 1 hour in serum-free media, transduced for the desired time in serum-free media. Scale bars, 50μm (top) and 10μm (bottom). **(e)** Enhancement of transduction mediated by P21 and 8R are affected by Trypsin proteolysis. Flow cytometry analyses NIH3t3 cells treated with proteins (20μg/ml) for 1 hour and a further 5 hour incubation (in serum-free media), with or without 10min pre-digestion with Trypsin or treatment with non-proteolytic cell dissociation solution (CDS). Cells were pre-incubated for 1 hour in serum-free media, treated with Trypsin and transduced for 1 hour in serum-free media. **(f)** Cell surface interaction of P21-containing proteins is disrupted by Tritonx100 treatment. Flow cytometry analyses NIH3t3 cells treated with proteins (20μg/ml) for 1 hour and a further 5 hour incubation (in serum-free media) with 10min pre-treatment of PBS or PBS containing 0.1% (v/v) Tritonx100 (Tx100). Cells were pre-incubated for 1 hour in serum-free media, treated with PBS or PBS with Tx100 and transduced for 1 hour in serum-free media. Error bars indicate s.d.

**Figure 17** P21 binds directly to Heparin and cell surface HS-GAG. **(a)** Soluble Heparin in media during transduction inhibits cell membrane interaction and transduction of P21-containing proteins. Fluorescence microscopy images of CGR-8 cells treated with P21-mR-8R (20μg/ml) for 6 hours in serum-free media containing 0 or 50μg/ml Heparin. Scale bar, 100μm. **(b)** Flow cytometry analyses NIH3t3 cells treated with P21-mR-8R (20μg/ml) for 6 hours with or without a variety of GAGs (50μg/ml) in serum-free medium. CS is Chondroitin sulphate. Cells were pre-incubated for 1 hour in serum-free media and transduced for 6 hours in serum-free media with or without GAGs. **(c-d)** Only high-doses of Heparin inhibit 8R activity whereas P21 activity is inhibited dose-dependently by Heparin in **(c)** NIH3t3 cells and **(d)** CGR-8 cells. **(e)** and **(f)** Cell surface Heparan sulphate is required for efficient P21-mediated protein delivery. Heparan sulphates/Heparin-containing FCS inhibits P21-mediated transduction but can also replace cell surface GAGs and mediate P21-transduction in cells deficient for Heparan sulphate. Fluorescence microscopy images of CGR-8 cells and *EXT1-/-* mESCs treated with P21-mR-8R (20μg/ml) for 6 hours in media containing 0 or 20% FCS. Scale bar, 100μm. Error bars indicate s.d.

**Figure 18** GET / HETD-mediated nuclear delivery of Cre Recombinase. **(a)** Schematic of the construct created to mark Cre activity in cells. Cre-mediated excision of a transcriptional STOP region flanked by *lox*P sites induces the constitutive expression of eGFP. Pr, promoter; βGal, β-galactosidase; Neo, Neomycin phosphotransferase. The NIH3t3 LSL-eGFP cell line was created by transfection and selection of NIH3t3 cells. **(b)** eGFP expression in untreated NIH3t3 LSP-eGFP cells or those transduced with SIN Cre lentivirus. Left shows fluorescence microscopy and right shows flow cytometry histogram of eGFP expression. Scale bar, 50μm **(c)** Scheme of testing transduction of Cre activity in NIH3t3 LSL-eGFP cells. Cells were transduced with Cre proteins for 1 hour, washed and cultured for 2 days before analyses. **(d-e)** P21-mR-Cre-8R is efficiently transduced and recombines DNA. **(d)** Fluorescence microscopy images Cre-transduced NIH3t3 LSL-eGFP with the variety of dosages. Scale bar, 50μm. **(e)** Flow

cytometry analyses of NIH3t3 LSL-eGFP cells transduced for 1 hour with mR-Cre, mR-Cre-8R and P21-mR-Cre-8R at a variety of dosages (0, 1, 10, 100 and 500μg/ml), washed and cultured for 2 days. Graph shows % recombination (i.e. % of eGFP+ve from total cell population). Error bars indicate s.d.

**Figure 19** GET / HETD-mediated delivery of domain position protein variants. **(a)** Schematic of the proteins created to test the effect of domain position on protein delivery to cells. **(b)** Fusion of P21 and 8R to mR in any orientation significantly improves transduction. Flow cytometry analyses of NIH3t3 and HUES7 cells incubated with the protein variants (20μg/ml) for twelve hours. Error bars indicate s.d.

**Figure 20** GET / HETD-mediated delivery of PTD protein variants. **(a)** Schematic of the proteins created to test the enhancing effect of P21 on other PTDs for protein delivery to cells. 8R is RRRRRRRR (SEQ ID NO: 49), TAT is HIV-1 TAT protein RKKRRQRRR (SEQ ID NO: 50), 8K is KKKKKKKK (SEQ ID NO: 51), 8RQ is RQRQRQRQ (SEQ ID NO: 52) **(b)** Fusion of P21 and any PTD to mR significantly improves transduction. Flow cytometry analyses of NIH3t3 and HUES7 cells incubated with the protein variants (20μg/ml) for twelve hours. Error bars indicate s.d.

**Figure 21** GET / HETDs can achieve higher intracellular levels of cargo delivery than transgenic systems. **(a)** Fluorometry of soluble extracts generated from NIH3t3 mR (transgenic NIH3t3 cells transduced with SIN mR) compared with those from NIH3t3 cells transduced for 6 hours with different doses of mR-8R or P21-mR-8R (0, 10, 20, 50, 100 or 200μg/ml in serum-free media) **(b)** Flow cytometry of NIH3t3 mR (transgenic NIH3t3 cells transduced with SIN mR) compared with those from NIH3t3 cells transduced for 6 hours with different doses of mR-8R or P21-mR-8R (0, 10, 20, 50, 100 or 200μg/ml in serum-free media). Fluorescence is normalised to untreated NIH3t3 cells. Error bars indicate s.d.

**Figure 22** HERD-mediated Cre Recombinase nuclear activity is promoted by vesicle escape but repressed by inhibitors of macropinocytosis or cholesterol depletion. NIH3t3: LSL-eGFP cells were pre-incubated in serum-free media (with or without drugs), transduced with Cre proteins (mR-Cre: 100μg/ml or P21-mR-8R: 10μg/ml) for 1 hour in serum-free media (with or without drugs), washed and cultured for 12 hours in full growth media (with or without drugs) and a further 36 hours in full growth media before analyses. **(a)** Methyl-β-cyclodextrin (used to deplete cholesterol) inhibits Cre transduction and recombination. (Methyl-β-cyclodextrin doses were 0, 1 2 and 5 mM). **(b)** Nystatin (a drug which sequesters cholesterol) inhibits Cre transduction and recombination. (Nystatin doses were 0, 10, 20 and 50μg/ml). **(c)** Amiloride (a specific inhibitor of $Na^+/H^+$ exchanged required for macropinocytosis) inhibits Cre transduction and recombination. (Amiloride doses were 0, 1, 5 or 10mM). **(d)** Cytochalasin D (an F-actin elongation inhibitor) inhibits Cre transduction and recombination. (Cytochalasin D doses were 0, 1, 5 or 10μM). **(e)** Chloroquine promotes the release of Cre from endosomal vesicles and increases recombination (Chloroquine doses were 0, 10 and 100μM). **(f)** Picogram per millilitre amounts is required to induce recombination with enhanced vesicle escape. The dose of transduced P21-mR-Cre-8R was varied in ten-fold dilutions (0-100μg/ml) with 1 hour incubation in the presence of Chloroquine. All data is presented as % of the maximal recombination. Error bars indicate s.d.

**Figure 23** GET / HETD-mediated transduction increases general cellular macropinocytosis. Flow cytometry of cells incubated in 70kDa FITC-Dextran and transduced with recombinant proteins. NIH3t3 cells were pre-incubated in serum-free media for 1 hour and transduced with mR, P21-mR, mR-8R or P21-mR-8R (20μg/ml in serum-free media) containing 70kDa FITC-Dextran (neutral) for 1 hour. Error bars indicate s.d.

**Figure 24** GET of non-protein Cargoes. **(a)** GET of biotinylated cargoes using monomeric streptavidin (mSA2). **(i)** Schematic of the mSA2 proteins engineered to bind to and transduce biotinylated cargoes. P21-8R was used as a non-interacting control, mSA2 as a non-transducing control, and P21-mSA2-8R as the test protein. **(ii)** Schematic of the antibody (Ab) complexes of a biotinylated primary (1°) antibody (Goat anti-rabbit; GtαRb) bound to an FITC-conjugated secondary (2°) antibody (Rabbit anti-mouse; Rb αMu) used to test activity. **(iii)** GET-delivery of Ab complexes were visible by fluorescence microscopy (scale bar, 50μm). With co-incubation of P21-mSA2-8R (10μg/ml, bottom image), Ab complexes were efficiency delivered to cells **(iv)** Flow cytometry demonstrating that 1°/2° Ab complexes (1μg/ml) are taken into NIH3t3 cells poorly by direct incubation or when co-incubated with mSA2 only. **(b)** GET of nucleic acids by employing LK15 peptide. **(i)** Schematic of the LK15 proteins engineered to bind to and transduce nucleic acids. **(ii)** Transfection of human mesenchymal stem cells (iHMSCs) using GET-LK15. Initially we assessed binding capacity of LK15 peptides for plasmid (p)DNA (SIN GFP, to express GFP on transfection), modified synthetic messenger RNA (modRNA) (Miltenyi Biotech; to express GFP on transfection) and small-inhibitory (si)RNAs (labelled with FAM fluorophore to detect delivery). After optimising ratios we transfected iHMSCs with P21-LK15-8R and pDNA (10μg), modRNA (10μg) or siRNA (1μg) and visualised transfection by fluorescence microscopy (scale bar, 100μm). **(iii)** Quantification of GET-LK15 transfection of iHMSCs by flow cytometry (%

transfection efficiency or relative fluorescence for siRNA) compared to lipofectamine (LIPO)2000 as a commercial standard. Error bars indicate s.d. **(c)** GET of Magnetic Nanoparticles. **(i)** Schematic of the P21-8R peptide synthesised and test magnetic nanoparticles (MNPs). We tested 250nm Nanomag-D dextran shell/iron oxide core MNPs and conjugated P21-8R peptide to surface COOH groups. (ii) MNPs are taken into NIH3t3 cells most efficiently in serum-free media (SFM; left panel). Light microscopy images of Prussian blue iron stained NIH3t3 cells treated with MNPs (50μg/ml) for twelve hours in standard media conditions (10% FCS) or SFM. Conjugation of P21-8R to MNPs significantly increases cellular uptake in both 10% FCS and SFM conditions (circular image is of entire well, scale bar, 100μm).

**Figure 25** Ligand auto-labelling of intracellular and extracellular membrane-anchored HALO proteins. **(a)** Schematic of the HALO (intracellular) and LAMP2b-HALO (extracellular membrane-anchored) transgenic SIN lentivirus constructs. In LAMP2b-HALO the expressed protein is localised to the cell membrane by the signal peptide (SIG) which is cleaved and presented on the extracellular side of the cell membrane **(b)** NIH3t3 cells transgenic for intracellular HALO protein (NIH3t3-HALO) are only efficiently labelled by cell permeant ligands ($HALO^{TAG}$ Oregon Green). NIH3t3 cells transgenic for membrane-anchored extracellular HALO protein (NIH3t3-LAMP2b) is efficiently labelled by both cell permeant and cell impermeant ligands ($HALO^{TAG}$ $Alexafluor^{488}$). Data shows flow cytometry of the NIH3t3 cell-lines incubated in ligand (1μM) for 15mins, followed by 3 media washes and a 15mins incubation to remove unbound ligand. Error bars indicate s.d. This provides an assay to assess intra- verses extracellular localisation of HALO proteins.

**Figure 26** Ligand labelling of GET-HALO proteins demonstrates rapid cell binding and transduction. **(a)** Schematic of HALO proteins created (as described for mRFP in fig 1). **(b)** P21-HALO-8R and P21-HALO efficiently bind NIH3t3 cells but do not significantly internalise with a 1 hour incubation. (c) Bound P21-HALO-8R efficiently transduces into NIH3t3 cells with further incubation (1h-5h). Bound P21-HALO does not as efficiently enter cells and remains bound to cell membrane with further incubation. Data shows flow cytometry analyses of NIH3t3 cells treated with proteins (20μg/ml) for 1 hour followed direct ligand labelling (1h) or further incubation of 5 hours (1h-5h). Error bars indicate s.d.

**Figure 27** pH-sensitivity of GET-mNectarine (mNect) proteins demonstrates rapid cell binding and transduction. **(a)** Schematic of HALO proteins created (as described for mRFP in fig 1). **(b-c)** GET-mNect or GET-mR proteins (20μg/ml) were transduced into NIH3t3 cells for 1h (to demonstrate membrane binding activity), 1h followed by a further 5h incubation without protein (1h-5h) (to demonstrate transduction activity) or 6h (to demonstrate sustained delivery). Flow cytometry was used to compare intensities of mNect and mR GET-proteins. Fluorescence signal from transducing mNect proteins (unlike mR versions) is rapidly lost after internalisation new to endosomal acidification and protein unfolding. Error bars indicate s.d. **(d-f)** GET-mNect proteins (20μg/ml) were transduced into NIH3t3 cells for the same regimes but washed in DMEM at pH7.5 or pH5.5 before cytometry. Membrane localised mNect protein fluorescence is extinguished by pH5.5 but is retained at pH7.5 indicating at 1h incubations leave P21-mNect-8R external to cells, bound to membranes and not protected from pH-mediated unfolding. 1h-5h incubations demonstrate that P21-mNect-8R localisation is shifted and protected from pH-mediated unfolding demonstrating internalisation of the protein and protection by the cell membrane. Error bars indicate s.d.

**Figure 28** GET protein must be delivered intracellularly to allow successful re-transduction **(a)** Scheme of testing the effect of re-transduction of GET proteins in NIH3t3 cells. Cells were pre-incubated in fresh media for 1 hour and transduced with P21-mR-8R (20μg/ml) for 1 hour. Cells were then either analysed for fluorescence or re-transduced with P21-mR-8R (20μg/ml) for a further 1 hour. This re-transduction was either immediate (0h) or with a 1-6 hour incubation between re-transduction before fluorescence analyses by flow cytometry. Immediate re-transduction is inhibited whereas >1 hour incubation between transductions allows the most efficient re-transduction of GET-protein. Error bars indicate s.d. * $p<0.05$.

**Figure 29** GET is biocompatible in multiple clinically relevant cell types. Cell lines were transduced with P21-mR-8R at 20 or 200μg/ml over 24 hours and assessed by trypan blue for cell viability (cell lines were those described in Fig. 1 including rat aortic smooth muscle cells (rSMC) and neonatal cardiomyocytes (rCMs)). Viability remained high in all cell types for both concentrations tested. Error bars indicate s.d.

**Figure 30** A: Prussian blue staining of Nanomag particles incubated with 3t3 cells for 24 hours, B: Iron assay results for the amount of iron per cell after the 24 hour incubation.

**Figure 31** Graph showing NIH3T3, CGR-8 and HUES7 cells treated with mRFP conjugated peptides (20ug/ml) for 12h. Flow cytometry analysis was used to quantify fluorescence (relative fluorescence units (RFU)) of cells. Error

bars indicate s.d, n=3.

**Figure 32** Graph showing the increase in transduction of mRFP into cells by modified CPPs (HS-GAG binding domain mRFP 8R) over an unmodified CPP (mRFP 8R). NIH3T3, CGR-8 and HUES7 cells were treated with mRFP conjugated peptides (20ug/ml) for 12h. Error bars indicate s.d, n=3.

**Figure 33** Efficient delivery of mRFP to cells via modified peptides shown to promote GET. Fluorescence microscopy images of NIH3T3, CGR8 and HUES-7 cells treated with P21 mRFP 8R, FGF2B mRFP 8R, FGF7B mRFP 8R and PDGF mRFP 8R peptides (20μg/ml) for twelve hours. Scale bar, 100μm.

**Figure 34** Optimization of (+/-) charge ratio of P21 LK15 8R to pSIN GFP using YO-PRO-1 assay. Graph shows a decrease in % of fluorescence as P21 LK15 8R binds pSIN GFP. Error bars indicate s.d., n=3.

**Figure 35** Examples of flow cytometry dot plots showing GFP expression of NIH3T3 cells following transfection with pSIN GFP for 6h. Following transfection, cells were fixed at a 48h time-point. Flow cytometry analysis was used to quantify % of GFP positive cells.

**Figure 36** Transfection optimization of pSIN GFP into NIH3T3 cells by P21 LK15 8R peptide. Cells were treated with the optimum (+/-) charge ratio of P21 LK15 8R to pSIN GFP of 2:1, respectively. Optimization was carried out at varying transfection times (3, 6 and 24h) in (A) 10% serum transfection media (B) serum free transfection media. Following transfection, cells were fixed at a 48h time-point. Flow cytometry analysis was used to quantify % of GFP positive cells. Error bars indicate s.d., n=3.

**Figure 37** Graph showing the optimization of the transfection of pSIN GFP into NIH3T3 cells by Lipofectamine2000, in serum free conditions. Flow cytometry analysis was used to quantify % of GFP positive cells. Error bars indicate s.d., n=3.

**Figure 38** MNP delivery to cell monolayer in FCS. Representative bright field images of Prussian blue stained NIH 3t3 cells. A, cells without MNPs, B, cells treated with MNP; cells treated with MNP and P218R. Cells were treated overnight with 50 μg/mL of MNP and 0.2 mM of peptide; scale bar 40 μm; n=1.

**Figure 39** MNP delivery to cell monolayer in FCS. Representative bright field images of Prussian blue stained NIH 3t3 cells. Cells treated with NH2 functionalized MNPs with (D) and without (A) P218R B, cells treated with MNP; Cells treated with plain MNPs with (E) and without (B) P218R: Cells treated with COOH-PEG functionalized MNPs with (F) and without (C) P218R B, scale bar 40 μm; n=1.

**Figure 40** MNP delivery to cell monolayer in FCS. Representative bright field images of Prussian blue staining. GIN 8 treated with MNP with (D) and without (A) P218R B, KNS-42 treated with MNP with (E) and without (B) U87 treated with MNP with (F) and without (C) P218R B, scale bar 50 μm; n=1.

**Figure 41** TEM imaging of MNPs on NIH 3t3s, A and C, delivery of MNP only (50 and 500 ug/ml), showing small internalization of particles within vesicles (arrowhead); scale scale bar = 0.5 and 5 μm respectively. B and D, delivery of MNP (50 and 500 ug/ml) with P218R, showing increased MNP uptake and concentration of the particles within vesicles (arrowhead); scale bar 2 and 5 μm respectively.

**Figure 42** TEM imaging of 20 nm MNPs on NIH 3t3s, A, delivery of MNP with P218R (50 ug/ml), showing internalization of particles within vesicles (arrowhead); scale scale bar = 2 μm. B, delivery of MNP 500 ug/ml with P218R, showing increased MNP concentration within vesicles (arrowhead); scale bar 2 μm.

**Figure 43** Percentage of P21mR8R (dots) and RFP (squares) adsorbed onto MNP. n=3

**Figure 44** Zeta potential of MNP (black bars) and MNP conjugated with P218R (gray bars) in water at increasing concentrations of FCS. N=3, (p<0.001)

**Figure 45** Zeta potential of MNP (black bars) and MNP conjugated with P218R (gray bars) in water at increasing concentrations of FCS. N=3, (p<0.001)

**Figure 46** MNP delivery to cell monolayer in CO2 independent media. Representative bright field images of Prussian

blue stained NIH 3t3 cells. Columns from left to right: pH 6, 6.5, 7 and 7.5. From top to bottom the proteins were: P218R, P21a10H, P21aMix5K, P21aMix3K, P21a7H3K, P21a7H3K Mix and P21a5H5K. The order of histidines (H) and lysines (L) is displayed for each peptide. scale bar 100 um. n=1

**Figure 47** Percentage of pH-inducible peptide (P21a10H) adsorbed onto MNPs in the range of pH 6-7.5 (grey bars) and percentage of peptide remaining on MNP after pre-conjugation at pH 6-7.5 and wash at pH 7.5. N=3, ($p<0.001$)

**Figure 48** MNP -P218R delivered to NIH 3t3s: for a & b MNP were conjugated with GET and delivered at the same pH, c to f MNP were pre-conjugated with GET at either pH 6 or 7.5, washed at pH 7.5 and delivered at pH 6 or 7.5. Scale bar 100 $\mu$m.)

**Figure 49** MNP -P21a10H delivered to NIH 3t3s : for a & b MNP were conjugated with GETpH1 and delivered at the same pH, c to f MNP were pre-conjugated with GETpH1 at either pH 6 or 7.5, washed at pH 7.5 and delivered at pH 6 or 7.5. Scale bar 100 $\mu$m.

**Figure 50** Adsorption isotherms of RFP GET on dextran coated 250 nm MNP in PBS. Adsorption isotherms of RFP GET on dextran coated 250 nm MNP in FCS. Adsorption isotherms of RFP GET on dextran coated 250 nm MNP in heparin.

## EXAMPLE 1

### Summary

Hyperthermia is a potentially powerful treatment for the ablation of cancer. Biocompatible iron oxide magnetic nanoparticles (MNPs) facilitate delivery of the treatment at very low concentrations. To achieve maximum therapeutic efficiency, a targeted delivery of MNPs directly to the tumour is of prime importance. Tumour tissues possess more acidic microenvironment than physiological normal tissues. In this report we have investigated a pH specific cell penetrating peptide (CPP) P21a10H for hyperthermia applications. P21a10H associate to MNPs only at low pH (pH <6.6), therefore its cell transducing ability is pH dependent. The peptide was tested against a non-pH dependent CPP (P218R) for MNP delivery to Human Mammalian tumour cells (MCF7). When delivered to MCF7 in vitro P21a10H showed inducible MNP delivery at lower pH, however, P218R always showed high delivery pH-independently. MNP delivery in MCF7 was assessed to mimic the dense tissue formed in tumours. P21a10H and P218R, showed similar particle uptake.

CPP-mediated delivery of MNPs can be a viable route to improve the application of hyperthermia to cancer treatment.

### Results

*CO2-independent media can stabilise pH in MCF7 cultures*

In order to optimize the *in vitro* model for pH responsive delivery, pH stability was tested in different media and culture conditions. Cell viability and metabolism was also evaluated when altering media and pH conditions.

The three different media used in this experiment were: buffered media (Dulbecco's modified Eagle's media, DMEM, with sodium bicarbonate as buffering system), non-buffered media (DMEM with sodium bicarbonate removed to allow pH variability) and $CO_2$ independent media (Invitrogen).

The pH of each media was adjusted between 7.6 and 6.6 (average estimated pH of tumour tissues (Griffiths, 1991)) in 0.2 intervals. The pH of the samples was evaluated after incubation overnight with MCF7 (at a seeding density of 0.2 million cells per well) at 37°C with and without 5% $CO_2$ atmosphere which affected the DMEM buffering system.

The standard deviation between the pH values measured before and after incubation was used to determine pH stability of the media under the culture conditions. The variability was plotted at each pH for each different media. (Figure 1 (B, C))

It was demonstrated that through the range of pH (6.6 to 7.6) the smallest variability is obtained with the $CO_2$ independent media. $CO_2$ independent media also showed similar variability through all the pH.

Importantly cell viability and metabolic studies showed normal metabolic activity and viability from pH 7.6 to 6.6 with a reduction in metabolic activity of approximately 15% down to pH 6.0 when the cells were incubated in $CO_2$ independent media in a non-$CO_2$ atmosphere.

*P218R and P21a10H pH responsive delivery in MCF7 monolayer*

Once the best media system had been optimised to maintain the stability of the pH during incubation with cells, the next step was to test the efficiency of the pH specific delivery system on human cancer cells. 250 MNPs were delivered to MCF7 cells. The iron oxide present in the Prussian blue produces a blue precipitate in the presence of iron (MNPs), which was used to determine particle uptake of MNPs.

Cells incubated with MNPs (Figure 2,3 and 4 left panel) presented some staining independent of the pH of the media, suggesting low quantities of MNPs can be passively uptaken by the MCF7. MNPs delivered with P218R (Figure 2, 3 and 4 central panel) peptide showed a significant enhancement in the staining of MCF7 in all three media. Staining was in general more intense at higher pH than at lower pH, this effect more pronounced in $CO_2$ independent media.

When the nanoparticles were delivered together with peptide P21a10H (Figure 2, 3 and 4 right panel), the media used significantly affected MNPs uptake. In buffered media, at higher pH the staining was minimum, similar to the results obtained with the passive delivery of MNP, however at lower pH the staining became slightly more intense (although never at P218R peptide levels). In the non-buffered media, the staining seems to be consistently intense through all the different pHs tested. Finally in $CO_2$ independent media, the staining becomes progressively more intense as the pH decreases. This is consistent with the pH stability data; with the most stable media ($CO_2$-independent) yielding a pH responsive result.

A quantitative colorimetric assay was carried out based on Prussian blue intensity after stained cells were dissolved in concentrated hydrochloric acid. The intensity of the signal measured was proportional to the amount of MNPs uptaken by the cells.

*Binding of protein to MNPs is pH dependent*

In order to complete pH inducibility of the delivery system, a protein binding experiment was carried out to study the effect of the pH on the protein-MNP association (Figure 5).

P21acidmR10H shows 100 % binding to MNPs at pH lover than 6.2, meaning all the protein in the media is associated to MNPs at that pH. Binding becomes worse as pH increases. The opposite effect was observed for P21mR8R which achieves maximum binding efficiency at around pH 7.2 (close to physiological pH).

## Discussion

Since the aim of this study is to demonstrate the efficiency of a pH responsive delivery system for nanoparticles, the first step was to evaluate optimal conditions for the *in vitro* study of the delivery system. The first goal was to find a cell culture media capable of maintaining the pH stable during the course of the experiments and maintain cell viability and metabolism. Conventional buffered media achieves its buffering ability when exposed to $CO_2$, therefore, in a $CO_2$ atmosphere media with this system will equilibrate back to physiological pH independent of the initial pH. This agrees with the results obtained in Figure 1, where pH variability decreases as the samples were originally closer to physiological pH.

The non-buffered media has no regulatory action on the pH so every external or internal change will affect the pH. Cell metabolism lowers the pH of the media without buffering, therefore overtime the pH of non-buffered media decreases towards pH 7 (minimum variability). This trend can be observed in both culture conditions.

Finally $CO_2$ independent media in a non-$CO_2$ atmosphere shows the smallest variability throughout the different pH, which means the pH of the samples after overnight incubation was similar to the initial pH, showing this media is capable of maintaining pH stability under experimental conditions. This data together with metabolic activity and viability (not shown) justify the choice of $CO_2$ independent media in a non-$CO_2$ environment for the *in vitro* study of a pH delivery system.

The pH specific delivery system was first tested in MCF7 cell monolayers. Prussian blue staining of cells treated with just MNP suggests that MCF7 are capable of uptaking MNP without any delivery system, however this process doesn't seem to be consistent throughout the cell population. Most cells are not fully stained suggesting an incomplete uptake process that would not be adequate for the use on therapy. A delivery system of MNP to the cells is therefore needed to achieve an efficient uptake of MNPs.

In buffered media the quantitative analysis shows poor enhancement of delivery using the pH specific peptide P21a10H. More acidic environments show slightly higher MNP uptake, however, this effect is not as obvious in the qualitative analysis in which uptake of MNP seems to be very similar throughout the different pH. These results match with the pH assay. A possible explanation to the slightly enhanced intensity of delivery is that at lower pH only existed at the start of the culture; based on the protein-MNP binding assay this would only allow MNP-peptide association and deliver for a short period of time.

When MNPs were delivered in a non-buffered media, the environment progressively becomes more acidic.

This masking the initial differences between the media. At lower pH association MNP-peptide association is less efficient for P218R (conjugation experiment) which explains the lower delivery efficiency of P218R compared to the other media (uptake fold increase always below 4). Cell uptake could also be affected by the pH of the media; however this has not been evaluated during this report.

In $CO_2$-independent media, qualitative and quantitative data show a progressive increase in MNPs uptake towards lower pH with P21a10H peptide and a slight decrease of MNP uptake towards lower pH when MNPs were delivered with P21a8R. $CO_2$-independent media is capable of maintaining pH stability after an overnight incubation with cells and therefore provides the most accurate results. Even if P218R is still capable of delivering nanoparticles better at any pH, P21a10H has demonstrated to alter its delivery efficiency based on the pH of the media.

Previous studies on peptide-MNP conjugation suggest that delivery of MNP with P218R peptide would be optimum at higher pH and would progressively get worse as the pH decreases, an opposite effect would be expected in delivery of MNP with P21a10H peptide, where optimum delivery would be expected at lower pH. P218R still shows higher delivery efficiency than P21a10H at lower pH. Future studies should include assays such as flow cytometry (to assess delivery to individual cells as such) or ICP (for a most accurate quantification of iron in cells) to more accurately measure the delivery efficiency of both peptides.

The aim of the experiment was to deliver MNP to develop a pH responsive delivery system capable of delivering MNP to the inside of tumours which is known to have a lower pH than the outside. Specific delivery to the inside of the tumour is one of the main obstacles in hyperthermia therapy (Hergt et al., 2006); a pH specific delivery system would deliver MNP to the more acidic tissue improving the efficiency of the therapy.

In conclusion, it has been demonstrated that a MNP pH responsive delivery system is attainable by modification of CPP peptides. This study examined a pH range between 6.0 and 7.0, trying to mimic a broad range of tumorigenic tissues (Griffiths, 1991). It was demonstrated that P21a10H was pH inducible in this range of pH, enhancing MNP uptake on MCF7 *in vitro* compared to passive MCF7 uptake of MNPs. These findings suggest that, the application of a combined CPP-MNP system could make the tumour ablation by hyperthermia more efficient.

**Materials and Methods**

***Cell culture***

NIH 3t3 fibroblast cells, Hela cells and MCF7 cells were cultured in Dulbecco's modified Eagle's media (DMEM; Sigma), supplemented with 10% (v/v) Fetal Calf

Serum (FCS, Sigma), 2mM L-glutamine and 100units/ml penicillin and 100units/ml streptomycin (Invitrogen). All cell lines were cultured at 37oC and 5% CO2.

Cell passage was carried out using 0.05% trypsin (Invitrogen).

***Aggregate formation: hanging drops***

To generate MCF7 aggregates, 15 μL drops containing 3000 cells each were added onto the cover lid of a non-tissue culture treated dish (Scientific Laboratory Supplies). The lids were inverted and placed on top of the dish, which contained approximately 10 mL of PBS to prevent evaporation of the hanging drops. The hanging drops were cultured for 48 hours at 37°C in a 5% $CO_2$ atmosphere.

***Nanoparticle delivery***

All particles used during the experiments were dextran coated Nanomag®-D MNPs (Fe3O4 core; 250nm; Micromod). P21-8R and P21a-10 H were recombinantly made from expressing genes in bacteria and then purified.

*Cell monolayer.* Cells were seeded into falcon 12 well tissue culture treated plates (200,000 cells/well, 500 μL/well; Scientific Laboratory Supplies) and incubated for 24 hours.

After incubation cells were treated, with nothing (media exchange), 100 μg/mL MNPs, 100 μg/mL dMNPs + 1μM peptide (P21-8R, P21a-10H or P21a-CPP27). Cells were incubated overnight.

*Aggregates.* Aggregates were collected into eppendorfs (200 aggregates per tube, Scientific Laboratory Supplies). The aggregates were treated with nothing (media exchange), 200 μg/mL dMNPs, 200 μg/mL dMNPs + 1μM peptide (P21-8R or P21a-10H). Aggregates were incubated overnight (non-static conditions to allow homogenization).

***Protein binding assay***

50μg of MNPs were incubated with 20ug/ml of red fluorescent protein, p21mR8R and P21acidmR10H (recombinantly made from expressing genes in bacteria and then purified) in PBS of different pHs (6.0 to 7.6 in 0.2 intervals).

Samples were incubated during one hour at room temperature and then centrifuged at 15,000xg. The supernatant was collected onto a black 96 well-plate and analysed in the plate reader at excitation of 584nm and emission of 620nm (Infinite® 200 PRO, TECAN).

For each protein, the fluorescent signal obtained in the absence of protein was taken as the control and subtracted from the rest of the values. The percentage of bound protein was calculated taken the fluorescence obtained in the absence of MNP at pH 7.4 as 0% of bound protein.

***Prussian Blue***

*Staining.* Cells and aggregates were fixed for 20 mins with 4% paraformaldehyde (PFA; Sigma) at room temperature. Prussian blue staining solution (potassium ferrocyanide in 2.5% hydrochloric acid, 25 mg/mL; Scientific Laboratory Supplies) was added to the cells and incubated for one hour. Stained cells were imaged using a Nikon Eclipse TS1000 light microscope.

*Quantification assay.* After Prussian blue staining the cells were washed with deuterated water and then let dry at room temperature. Once the cells were dried a hydrochloric acid solution was added (36% hydrochloric acid; Fisher Scientific). The cells were stored in acidic conditions for one hour. The hydrochloric acid solution was recovered into eppendorfs and centrifuged at 15000xg. The supernatant was then collected on 96 well-plate and analysed in the plate reader at an absorbance of 450 nm (Infinite® 200 PRO, TECAN).

For each media, the signal obtained in the absence of nanoparticles was taken as the control and subtracted from the rest of the values. The uptake fold increase for each pH was calculated by normalizing the results to the signal obtained by passive delivery of MNP.

***pH media***

Buffered Dulbecco's modified Eagle's media (DMEM; Sigma); Buffered Dulbecco's modified Eagle's media without sodium bicarbonate (DMEM; Sigma) and $CO_2$ independent media (Invitrogen), all of them supplemented with 10% (v/v) Fetal Calf Serum (FCS, Sigma), 2mM L-glutamine and 100units/ml penicillin and 100units/ml streptomycin (Invitrogen) were adjusted to different pH by adding NaOH 0.1 M or HCl 0.1 M (Scientific supplies).

***Cryosectioning***

Once fixed the aggregates were covered in 30% sucrose solution and stored overnight at 4°C. The aggregates were then seeded on the mould and covered with compound mounting media for cryotomy (OCT, VWR Chemicals), the mould was then submerged on a bath of isopentane (that had previously been cooled in liquid $N_2$) until the whole block became white. The samples were then stored at -80°C for 1 hour before sectioning. The sectioning was carried out at -20°C using a Leica CM 1100 cryostat.

**References**

Beerens, a., Al Hadithy, a., Rots, M., & Haisma, H. (2003). Protein Transduction Domains and their Utility in Gene Therapy. Current Gene Therapy, 3(5), 486-494.

Byrne, J. M., Coker, V. S., Cespedes, E., Wincott, P. L., Vaughan, D. J., Pattrick, R. a. D., ... Telling, N. D. (2014). Biosynthesis of Zinc Substituted Magnetite Nanoparticles with Enhanced Magnetic Properties. Advanced Functional Materials, 24(17), 2518-2529.

Dixon, J. E., Morris, G., Lane, N., Denning, C. et al. (2014). Highly efficient delivery of functional 545 proteins by the synergistic effect of GAG binding motifs and cell-penetrating peptides. [Unpublished].

Falk, M. H. & Issels, R.D. (2001). Hyperthermia in Oncology. International Journal of Hyperthermia, 17(1), 1-18.

Griffiths, J. R. (1991). Are cancer cells acidic British Journal of Cancer, 64(April), 425-427.

Hergt, R., Dutz, S., Müller, R., & Zeisberger, M. (2006). Magnetic particle hyperthermia: nanoparticle magnetism and materials development for cancer therapy. Journal of Physics: Condensed Matter, 18(38), S2919-S2934.

Kumar, C. S. S. R., & Mohammad, F. (2011). Magnetic nanomaterials for hyperthermia-based therapy and controlled drug delivery. Advanced Drug Delivery Reviews, 63(9), 789-808.

Vaupel, P. (2004). Tumor microenvironmental physiology and its implications for radiation oncology. Seminars in Radiation Oncology, 14(3), 198-206.

Wang, Z., & Cuschieri, A. (2013). Tumour cell labelling by magnetic nanoparticles with determination of intracellular iron content and spatial distribution of the intracellular iron. International Journal of Molecular Sciences, 14(5), 9111-25.

**EXAMPLE 2**

**Example of the GET system**

**Results**

**Isolation of P21, a HBD that enhances PTD function through HS-GAG interaction**

Several short peptides were screened which have been reported to interact with molecules known to be present on mESC, HESC or HiPSC membranes including integrins, CD markers and GAGs. Peptides were fused *N*-terminally to mRFP1, expressed, affinity purified and incubated with the three cell-types. Screening of 12 variants yielded one which clearly increased localisation of mRFP1-fluorescence (mR) to cells and their membranes, termed P21 (KRKKKGKGLGKKRDPCLRKYK (SEQ ID NO: 1)) (Fig 16). Interestingly this peptide also demonstrated transduction activity as evidenced by punctate intracellular fluorescence indicative of endosomal localisation (Fig 16b,c). P21 was derived from HB-EGF, which belongs to the EGF family of cytokines. HB-EGF shows a strong affinity to heparin and binds to the same receptor as EGF and TGF-$\alpha$ {Sakuma, 1997 #76}. The interaction of HB-EGF with cell surface HS-GAG is essential for its optimal binding to EGFR and for promoting its growth/migratory activity toward vascular smooth muscle cells {Higashiyama, 1993 #90}. Mutagenesis and protease digestion of recombinant HB-EGF, coupled with analyses using synthetic peptides and heparin, revealed the P21 sequence in the amino-terminal region of the soluble HB-EGF is responsible for its binding to heparin so is considered a typical HBD {Thompson, 1994 #77}. In addition, P21 also interacts with cell surface HS-GAG but not the EGFR which is mediated by other sequences in HB-EGF. Therefore, a short 21-residue peptide, P21, was isolated, which enhances the association of a fluorescent reporter to both mouse and human pluripotent stem cells.

P21-mediated binding of cell membranes was tested to determine if it could enhance PTD-mediated transduction of mR by combining both moieties in one molecule (Fig 16a). P21-mR-8R was cloned, expressed and purified, and its activity compared to P21-only (termed P21-mR) or 8R-only (mR-8R) proteins (Fig. 16a). Data demonstrated that the inclusion of both P21- and 8R- moieties synergized in the same protein to significantly enhance the fluorescence of all cell-lines tested. (Fig. 16b). Importantly mouse and human pluripotent stem cells (CGR-8, HUES7 and IPS2) and cardiomyocytes (HL1) only transduced efficiently with the inclusion of P21-tag, and both tags synergized to produce the high-levels of transduction similar to that seen in other cell lines (Fig. 16c). These motifs were also placed in tandem at N- and C-terminal of mRFP (P21 or 8R first; P21-8R-mR, 8R-P21-mR or mR-P21-8R, mR-8R-P21) or switched their termini (i.e. 8R-mR-P21) with all variants demonstrating similar synergy and cell transducing behaviour (Fig 19) as seen for P21-mR-8R. Furthermore 8R was swapped for alternative well characterised PTDs (TAT, 8K and 8RQ; {El-Andaloussi, 2005 #36}) and showed that these also synergized with P21 (Fig 20).

By incubating cells with protein for different timings and including a post-culture period it could be efficiently distinguished between fluorescence signal produced at the cell surface with that internalised (Fig. 16d). Cells demonstrated membrane localisation of fluorescence with short incubation times (1 hour), termed 1h. With this short incubation and a subsequent culture period (1 hour with a 5 hour post-culture), termed 1h-5h we observed near exclusive intracellular fluorescence indicating transduction. Using longer incubation times (6 hours), termed 6h, cells exhibited strong punctate peri-nuclear fluorescence indicative of endosomal-mediated transduction. This synergistic delivery mechanism is described as GAG-binding enhanced transduction (GET) or otherwise Heparan-sulfate enhanced transduction domain (HETD)-mediated delivery.

**GET requires the presence of trypsin-sensitive and detergent-soluble cell membrane molecules**

To evaluate the mechanism of GET interaction and uptake by cells, a series of experiments were performed which were previously used to assess PTD. To assess which cell membrane components are required for both initial cell association and transduction by HETDs it was determined whether similar transduction would be obtained by enzymatic depletion of cell membrane before transduction. Cells were pre-treated with proteolytic enzyme trypsin and tested cell transduction using the 1h-5h regime protocol. Enzymatic removal of cell-surface proteins potently inhibited GET / HETD-mediated transduction (~8.4-fold; $p<0.05$) (Fig 16e). In contrast, non-enzymatic release of cells from the culture plastic using ionic cell dissociation solution (CDS) did not alter HETD-mediated uptake.

Next it was tested if depletion of detergent-soluble cell-membrane molecules would also have a similar effect on transduction. Cells were pre-incubated in 0.1% (v/v) Triton X-100 and using the 1h-5h protocol a decrease (~2.2-fold; $p<0.05$) was observed in GET (Fig 16f) without a decrease in viability. Therefore, it was demonstrated that both protein and detergent soluble moieties on the cell membrane affect the efficacy of protein transduction through P21- and PTD- synergy in GET / HETD-transduction.

**GET generates higher intracellular protein levels than Lentiviral transgenesis**

Several studies have concluded that PTD-mediated transduction is sufficiently refined to allow the transport of biologically active cargos for clinical studies. These now include trials of cancer therapies {Gump, 2007 #39}, siRNAs {Meade, 2007 #37} and *in vivo* imaging technologies {Bullok, 2006 #79}. As well as the benefits of avoiding genomic modification, if PTD-mediated transduction is to be preferential to gene-therapy approaches it must achieve the delivery of high-levels of molecule, be amenable to control of protein levels over short time-frames and also allow cell-type specific delivery. The levels achieved in cells by PTD- or GET / HETD-delivery were compared to those achieved by efficient lentiviral transduction {Dick, 2011 #10} and exogenous expression of mRFP1 (with stable EF1$\alpha$-promoter driven) (Fig 21). To achieve this soluble protein was extracted from transduced cells and measured amounts by fluorometry (Fig 21a), or flow cytometry was used (Fig 21b). Using 6 hour incubations, mR-8R levels were several times lower (~3-fold; $p<0.05$) than that achieved by viral transgenesis even at the highest tested doses (200$\mu$g/ml). However P21-mR-8R levels under the same conditions were ~16-fold higher ($p<0.001$) than transgenic cells. Importantly of the amount of P21-mR-8R protein incubated a significant proportion was recovered as soluble intracellular protein (~46$\pm$3.5$\mu$g/200$\mu$g used; ~23$\pm$1.7% recovery). It is important to note that under these conditions transduced cells appear red/purple in colour under normal light, demonstrating the efficient enrichment of large quantities of HETD-tagged / GET proteins in cells.

The rate at which these proteins were concentrated in cells was investigated by measuring the depletion of fluorescence in media over the incubation period. Proteins were diluted (20$\mu$g/ml) and incubated with cells for 12 hours in serum-free conditions. 8R-tagged proteins were depleted by ~12% in NIH3t3- and ~3.5% in HUES7-incubations. This precisely mirrors flow cytometric data with 8R-proteins poorly transduced into HUES7 cells but at moderate levels in NIH3t3 cells (Fig 16). P21-tagged proteins were significantly depleted in both cell types (~37% and ~25% in NIH3t3 and HUES7 cells, respectively; $p<0.05$) with HETD-proteins depleted from media to the highest levels and majority of protein removed (~72 and ~66% in NIH3t3 and HUES7 cells, respectively; $p<0.01$).

The time required to deplete half of the fluorescence (T½) was determined, with P21-mR-8R requiring only ~9.4 hours, in comparison to mR-8R which required ~62 hours and untagged protein never achieving half-depletion even after 7 days. These data are corroborative with the cytometric data proving a rapid and efficient enrichment of exogenous GET-protein/HETD-protein into cells. Therefore, it was demonstrated that within a relatively short incubation period (6 hours) that a significant protein concentration can be achieved within cells. In less than a day, the majority of extracellular protein has been effectively internalised using GET delivery. This system will be amenable to precise regulation of protein stoichiometry, while avoiding the stochastic transgene expression variation and silencing of integrating vectors used in gene-therapy approaches.

**GET enhances Cre-mediated genome modification**

It was determined that HETDs bind rapidly to cell membranes through HS-GAGs and transduce efficiently into cells, but it was yet to be confirmed if the mode of uptake was through macropinocytosis as for PTDs. Also, what proportion of this protein escaped endosomes and may be considered successfully delivered was not assessed. Previous studies have the avoided issues associated with direct measurement of fluorescent-tagged proteins (such as being unable to distinguish membrane, vesicle or functional cytosolic/nuclear protein) by assaying for the successful nuclear activity of Cre recombinase {Gump, 2010 #2}. This system was used to measure Cre-mediated recombination of a *lox*P-STOP-*lox*P (LSL) enhanced green fluorescent protein (eGFP) reporter gene in live NIH3t3 mouse fibroblast cells (NIH3t3: LSL-eGFP cells) as an indicator of cellular uptake (Fig 18a). This system is rigorous as activation of green fluorescence requires exogenous Cre protein to enter the cell, undergo nuclear-translocation and excise the LSL fragment of the transgene. This must occur in live cells and be non-toxic for the subsequent expression of eGFP. However, this process requires only one functional Cre recombinase molecule to be delivered to activate eGFP so does not allow the determination of the precise amount of cargo delivered. To overcome this issue Cre proteins were delivered at limiting dilutions for a short exposure time (1 hour) and the minimum dose required to activate green fluorescence after 48 hours (Fig

18c) was determined.

Transduction of NIH3t3: LSL-eGFP cells with SIN Cre lentiviruses to overexpress *Cre* transgenically led to near complete (92±6%; $p<0.001$) activation of eGFP-expression in all cells confirming the utility of this system (Fig 18b). The benefits of the fluorescence system and delivered fluorescent-versions of Cre-recombinase protein were retained by purifying proteins with *mRFP1* cloned to the *N*-terminal of *Cre* cDNA. Treatment of NIH3t3: LSL-eGFP cells with mR-Cre (mRFP fused to Cre) resulted in recombination and eGFP activation (22.1±6.7%; $p<0.05$) at the highest doses (500μg/ml) (Fig 18d). eGFP activation was inhibited at 4°C and negatively affected by serum concentration-dependently. mR-Cre-8R demonstrated that the 8R PTD enhanced functional delivery of Cre (~22-fold; $p<0.01$).

The GET-protein / HETD-protein, P21-mR-Cre-8R, required as little as one minute incubation with cells at a low dose (1μg/ml) to elicit recombination (4.3±2.5%; $p<0.05$) confirming that binding and internalization is an efficient and rapid process. For a moderate dose (10μg/ml) GET/ HETD-transduction achieved a functional delivery ~15-fold ($p<0.01$) above PTD only levels and completely recombined all NIH3t3: LSL-eGFP cells (Fig 18d,e). Importantly this activity was ~340-fold better than mR-Cre (p<0.001). Heparinase III, free-heparin and serum-free experiments were repeated using the Cre recombination system. It was confirmed that heparinase III pretreatment reduced recombination to basal-levels and that media serum plays a role in replenishing cell membrane GAGs depleted by heparinase. Overall these data correlate well with the fluorescence delivery conclusions and show synergy between P21- and PTD- moieties to achieve significant increases in functional transduction of protein cargo.

**GET protein enters cells by lipid raft macropinocytosis**

Previously it has been shown that PTD-mediated internalization is via macropinocytosis rather than other endocytotic pathways {Wadia, 2004 #25}. It was next determined whether the cellular uptake of GET-proteins / HETD-proteins occurs through a specific endocytotic pathway employing the Cre assay system. Removal of cholesterol from the cell plasma membrane disrupts several lipid raft-mediated endocytotic pathways, including caveolae and macropinocytosis {Anderson, 1998 #29; Nichols, 2001 #30; Liu, 2002 #28}. NIH3t3: LSL-eGFP cells treated with methyl-β-cyclodextrin and nystatin were used to deplete or sequester cholesterol, respectively, then transduced HETD-tagged proteins. Both methyl-β-cyclodextrin (Fig 22a) and nystatin (Fig 22b) disruption of lipid rafts resulted in a dose-dependent inhibition of functional delivery. These data demonstrates that GET / HETD-mediated transduction specifically requires lipid raft-mediated endocytosis.

Macropinocytosis is a rapid, lipid raft-dependent and receptor-independent form of endocytosis which requires actin membrane protrusions that envelope into vesicles termed macropinosomes {Nichols, 2001 #30; Liu, 2002 #28; Conner, 2003 #22}. To confirm macropinocytosis was indeed the endocytotic mechanism of HETD-mediated transduction cells were pre-treated with macropinocytosis-inhibiting compounds (Fig. 20a,b). Amiloride is a specific inhibitor of the $Na^+/H^+$ exchange required for macropinocytosis {West, 1989 #31}. Cytochalasin D is an inhibitor of F-actin elongation which is required for macropinosome-linked membrane protrusions {Sampath, 1991 #32}. Amiloride and Cytochalasin D did not disrupt cell binding of HETD-proteins but resulted in a dose-dependent reduction of functional transduction into cells (Fig 22c and 22d, respectively). These data confirm that P21 enhances the macropinocytotic pathway used by PTD to internalize cargo molecules.

**GET-delivery promotes general macropinocytosis**

The effects of GET-binding / HETD-binding on the induction of macropinocytosis was investigated. PTD-mediated transduction has previously been shown to promote the uptake of other proteins by an increase in the overall level of macropinocytosis {Wadia, 2004 #25}. Cells were incubated with a fluorescent fluid-phase macropinocytotic maker, FITC-labeled 70kDa neutral dextran, in combination with GET / HETD protein, P21-mR-8R (Fig. 23). Other studies {Oliver, 1984 #24; Araki, 1996 #23; Wadia, 2004 #25} have demonstrated that neutral dextrans are taken up by amiloride-sensitive macropinocytosis. P21-mR-8R induced a significant dose dependant increase in fluid-phase dextran uptake over steady-state control levels. PTD-tagged versus GET / HETD-tagged activity to stimulate this macropinocytosis was compared. P21-mR-8R enhanced FITC-dextran uptake ~2.5-fold ($p<0.05$) over the stimulation achieved by the same concentration of mR-8R demonstrating that engagement with HS-GAG through P21 and its subsequent effect on PTD-mediated transduction stimulates macropinocytotic uptake.

**Significant amounts of GET-delivered protein is trapped in Endosomes which can be efficiently released with Chloroquine**

The majority of PTD-delivered molecules remain trapped in macropinosomes even after further incubation indicating that release from these vesicles is inefficient. If fine-tuned and graded amounts of delivery are to be controlled then it would be beneficial if the majority of internalized protein could be considered as functional. Cells were treated

with chloroquine, an ion-transporting ATPase inhibitor that disrupts endosomes by preventing their acidification {Seglen, 1979 #33} (Fig. 22e). Similar doses have been demonstrated to significantly improve the functional delivery of PTD-delivered proteins {Wadia, 2004 #25}. Sub-cytotoxic doses of Chloroquine (100$\mu$M) resulted in a marked increase (95.3$\pm$4.8-fold; $p$<0.001) in functional HETD-tagged / GET protein delivery at a sub-threshold dose (0.1$\mu$g/ml) indicating that this is point in the pathway is still a major issue to resolve for GET/HETD medicinal application. Nevertheless the GET-delivery / HETD-delivery system was so efficient that with chloroquine treatment we achieved significant and measureable levels of recombination (4.8$\pm$2.9%; $p$<0.05) with short (1 hour) incubations of >10pg/ml (Fig 22f). Combination of GET / HETD-delivery efficiency with endosomal-escape technologies may therefore allow precise and temporally controlled amounts of cargo function in cells.

**GET-mediated internalisation is efficient after cell membrane association**

Even for incubations using low amounts of GET-protein functional quantities of protein activity were observed within cells. However, to categorically and stringently prove that most GET protein was indeed efficiently internalising a series of analyses was conducted using reporters that are responsive to their cellular or extracellular localisation. HALO (Halo$^{Tag}$) was used, which is a self-labelling protein derived from DhaA[29]. HALO rapidly forms a covalent attachment to synthetic chloroalkane-based ligands; with cell permeant and impermeant ligands available. Intra- versus extracellular labelling of HALO was confirmed using transgenic over-expression of untagged HALO (for intracellular) and LAMP2b-HALO which is presented on the external cell membrane (for extracellular) and labeling with cell permeant (HALO$^{TAG}$ Oregon Green) or impermeant (HALO$^{TAG}$ Alexafluor$^{488}$) ligands (Fig 25). GET-HALO proteins were constructed and recombinantly expressed (Fig 26a) and delivered them to cells testing the the internalisation by sensitivity to labelling with the cell impermeant ligand. One hour incubation demonstrated that GET-proteins remained mainly extracellularly localised and attached to the cell membrane (Fig 26b). However, with further incubation (1h exposure with 5h further incubation; 1h-5h) GET-protein (P21-HALO-8R) is effectively internalised (remaining cell permeant ligand-sensitive but impermeant ligand-insensitive) (Fig 26c). These experiments were repeated using the mNectarine (mNect) variant of RFP which is pH-sensitive and loses almost all fluorescence in environments <pH6[30] (Fig 27). In agreement with HALO transduction, mNect remained mostly membrane localised after 1 h and its fluorescence sensitive to acidic pH media incubation (pH5.5) (Fig 27 d). After further incubation post-delivery (1h-5h) GET-mNect fluorescence was no longer sensitive to extracellular pH (Fig 27e); however interestingly the absolute fluorescence levels were significantly decreased when compared to GET-mR, presumably due to the internal pH change the protein is experiencing during endosomal acidification[1]. These data demonstrates that GET protein membranes association is rapid and transduction is efficient post-cell binding. It was hypothesised that membrane clearance of GET-protein could be a rate-limiting step in the delivery process and this was tested by undertaking multiple transductions (1h) of GET-mR varying the time between transductions (Fig 28a). Indeed re-transduction directly after the initial transduction decreases the effectiveness of the second transduction however as little as 1 h between new transductions is required to obtain a maximal efficiency of re-transduction (Fig 28b).

**Experimental Procedures**

**Expression and Purification of Recombinant Proteins**

cDNA was obtained for *mRFP1* (*mR*) as a kind gift from Prof. R. Y. Tsien (University of California, USA) {Campbell, 2002 #12}. *8R, TAT, 8K, 8RQ, P21, Cre, NANOG, MYOD* and *NEO* cDNAs were synthesized *de novo* (Eurofins MWG Operon). cDNAs were cloned into the pGEX6-P1 expression vector (Novagen) to create in-frame fusions and expressed proteins in BL21 (DE21) pLysS *Escherichia coli* (Novagen). Exponentially growing LB cultures ($OD_{600}$ = 0.4) shaken at 220rpm at 37°C were induced using 1 mM IPTG for 24 hours at 25°C. Bacterial pellets were lysed and sonicated (7 amplitudes, 1 minute, 5 times) in 1X STE extraction buffer (50 mM Tris, pH 7.5, 150 mM NaCl, 1 mM EDTA containing 1mM DTT, 0.2 mg/ml lysozyme, and 1X protease inhibitor cocktail). Insoluble protein was retrieved using the Rapid GST inclusion body solubilisation and renaturation kit (AKR-110; Cell Biolabs, Inc., San Diego, CA). Recombinant proteins were purified by affinity chromatography using Glutathione-Sepharose resin (GE Healthcare). GST-tags were removed and eluted from resin by PreScission™ Protease cleavage (GE healthcare) in 1X cleavage buffer (50 mM Tris-HCl pH 7.0, 150 mM NaCl, 1 mM EDTA and 1 mM DTT). Protein concentration was determined using a BCA-based protein assay (BioRad) with absorbance measured at 595nm using recombinant mR protein as a standard. Integrity and full-length protein expression was confirmed by SDS-PAGE. The fluorescence of recombinant proteins (excitation: 584 nm; emission: 607 nm) was determined with all preparations <10% intensity difference between samples (fluorescence/$\mu$g). Standards and samples were analysed using the TECAN infinite 200PRO multimode reader. Aliquots were stored at -80°C.

**Cell Culture**

NIH3T3 mouse fibroblast cells, HEK293T human embryonic kidney cells, C2C12 mouse myoblast cells, iHMSC immortalised human mesenchymal stem cells (created as described {Okamoto, 2002 #8}) and MEF murine embryonic fibroblasts (harvested as described {Anderson, 2007 #9}) were maintained in DMEM with 10% (v/v) fetal calf serum (FCS; Sigma) media supplemented with 2mM L-glutamine, 100units/ml penicillin and 100μg/ml streptomycin). CGR-8 mouse embryonic stem cells (mESCs) and *EXT1*-/- mESCs (a kind gift from Dr. D. E. Wells, University of Houston, USA; {Lin, 2000 #78}) were maintained in DMEM, 20% (v/v) FCS, 1000units/ml leukaemia inhibitory factor (LIF), non-essential amino acids, 100μM β-mecaptoethanol (Sigma) 2mM L-glutamine, 100units/ml penicillin and 100μg/ml streptomycin). HL1 mouse cardiomyocyte cells were maintained as described {Claycomb, 1998 #7}. HUES7 human embryonic and IPS2 induced pluripotent stem cells were cultured as previously described {Dick, 2011 #10}. HUES7fib human fibroblasts derived from HUES7 cells were generated and cultured as previously described {Dick, 2011 #10}. All cells were cultured at 37°C under 5% $CO_2$.

**Flow Cytometry and Microscopy**

For flow cytometry, cells were trypsinized (unless otherwise stated), fixed in 4% (w/v) PFA, resuspended in PBS (pH7.5) and analysed on a MoFlo™ DP (DAKO) Flow Cytometer using a 488nm green laser. (50,000 cells; gated on live cells by forward/side scatter). Median fluorescence was used for statistical analyses with background from unlabelled/transduced cells subtracted and values taken as ratios to the experimental control. Data shown are three experiments of triplicate samples. For microscopy, cultures were rinsed twice with PBS and imaged with inverted fluorescence microscope (Nikon Eclipse TS100).

**Fluorescence Delivery Assay**

For testing multiple cell lines we plated 2x $10^5$ cells/well (in 12-well plates) onto the surface relevant to the tested cell line, attached cells for 2 hours and transduced with recombinant proteins in cell-type specific growth media. After transduction cells were washed with PBS, trypsinized and fixed in 4% PFA for flow cytometry. For membrane localization, intracellular localisation or both we plated cells as above, but cultures pre-incubated in serum-free media for 1 hour before transduction. Membrane localization to assess cell interaction was achieved by a short transduction of 1 hour in serum-free media. Intracellular localization to assess transduction efficiency was achieved by a short transduction of 1 hour followed by 5 hour incubation in serum-free media only. Cell association (membrane and intracellular levels) were assessed by transducing cells for 6 hours in serum-free medium. For flow cytometry cells were trypsinized, washed and fixed in 4% PFA and for microscopy cells were imaged live after washing in PBS. For trypsin depletion of cell-surface proteins, cells were treated with trypsin/EDTA (Invitrogen) or EDTA-based cell dissociation solution (CDS) (Sigma) for 15 minutes at 37 °C, followed by washes with PBS and 1X soybean trypsin inhibitor (10 mg/ml in PBS; Sigma). Cells were then treated with proteins for 1 hour at 37 °C in serum-free medium. For detergent depletion of cell membranes, cells were treated with PBS (pH7.5) containing 0.1 % (v/v) Triton-X100 (Tx100) for 10 minutes at 37 °C, followed by washes with PBS. Cells were then treated with proteins for 1 hour at 37 °C in serum-free medium. For GAG-treatment cells were pre-treated with GAGs in DMEM without serum before transduction and were included in the transduction media. This included heparin and chondroitin sulphate A, B and C (0-50 μg/ml).

**Total delivered Protein Analyses**

5 x $10^6$ NIH3t3 cells were plated (in T25 flasks), pre-incubated cells in serum-free DMEM for 1 hour, and transduced them with mR-8R or P21-mR-8R (0-200μg/ml; 1 ml volume) in serum-free DMEM for 6 hours. NIH3t3 cells transduced with SIN-mR lentiviruses were used as a control for the levels achieved by transgenic systems {Dixon, 2011 #15; Dick, 2011 #10}. Cells were harvested by trypsinization, fixed in 4% PFA for flow cytometry or washed several times in cold PBS with soluble protein extracted in cold HKM buffer (20mM HEPES, pH 7.5, 5mM KCl, 0.5 mM $MgCl_2$ and 0.5 mM DTT with 1X complete EDTA-free protease inhibitor cocktail) for fluorometry {Medina, 2000 #14}. Extracts were sonicated, centrifuged and NaCl added to yield a final concentration of 100mM prior to analyses. Fluorometry was used to compare soluble extracts with purified mRFP protein diluted in HKM buffer with 100mM NaCl as standards. Flow cytometry was used to assess total delivered protein in intact cells.

**Media depletion Assessment**

2 x $10^6$ NIH3t3 cells or HUES7 HESCs were plated (in 6-well plates), pre-incubated cells in serum-free DMEM for 1 hour, and transduced with recombinant proteins (20μg/ml; 1 ml volume) in serum-free DMEM for 12 hours. Media

was harvested and fluorometry was used to compare the remaining fluorescence in media verses that before cell-incubation. Fluorescence of media pre-incubation was assigned as 100% fluorescence units and background of serum-free media subtracted.

**Heparin-binding assay, Heparinase treatment and depletion of P21-binding molecules from Serum**

For Heparin binding activity we incubated 1ml of recombinant proteins (20μg/ml) in DMEM with 50μl of PBS-washed Heparin-sepharose beads (Sigma) for 1 hour at 37°C shaking at 100rpm. Media pre- and post-incubation was compared by fluorometry. For Heparinase treatment, we plated NIH3t3 cells at 2 x $10^5$/well (in 12-well plates) and were pre-incubated in serum-free media for 1 hour with Heparinase III (0-1 U/ml) or Heparin (0-50μg/ml). Cells were then washed and transduced with mR or P21-mR-8R (20μg/ml in serum-free media or media with different FCS concentrations) containing Heparinase III or Heparin for 12 hours. FCS was depleted of P21-binding material by affinity chromatography. This was achieved by incubating 50 ml FCS with 2 ml Glutathione-Sepharose resin (GE Healthcare) pre-absorbed with GST-P21 protein expressed in *Escherichia coli.*

**Macropinocytosis Assessment**

To measure the effects of protein transduction on general macropinocytosis, cells were incubated with 100μg/ml FITC-70kDa neutral dextran (Sigma), along with different recombinant proteins (0-10μg/ml) for 1 hour at 4°C or 37°C. Cells were trypsinized and washed in PBS before analyses by flow cytometry.

**Cre Recombination Assay**

To measure Cre Recombinase activity the NIH3t3: LSL-eGFP cell line was created using the pZ/EG plasmid transfection and G-418 selection {Novak, 2000 #6}. To confirm Cre activity efficiently led to recombination and eGFP activation cells were transduced with SIN-Cre lentiviruses (as described in Dixon et al. 2011) and >95% of cells were confirmed eGFP-positive 48 hours post-transduction. 2 x$10^5$ cells/well were plated (in 12-well plates), pre-incubated them in DMEM without serum for 1 hour and treated with Cre proteins (0-500 μg/ml) in DMEM without serum. After the Cre incubation cells were trypsinized, replated into complete media and incubated for 2 days. Cells were pre-treated with drugs for the stated time-period in DMEM without serum, were included in Cre-transduction medium and were added after replating. Pretreatments included: heparin (0-50 μg/ml), chondroitin sulphate A, B and C (0-50 μg/ml), chloroquine (0-100μM), cytochalasin-D (0-10 μM), amiloride (0-5 mM), methyl-β-cyclodextrin (0-5mM), and nystatin (0-50μg/ml). After incubations cell were trypsinized, washed, fixed in 4% PFA and % recombined cells was determined by flow cytometry. For mR-Cre-8R and P21-mR-Cre-8R comparisons concentrations of 100μg/ml and 10μg/ml were used, respectively and data was expressed as % maximum recombination (i.e. the % relative to the maximum recombination achieved at the stared dose of Cre).

**Antibody, Nucleic acid and Nanoparticle delivery**

Biotinylated-Goat anti-Rabbit and FITC-Rabbit anti-mouse antibodies (Sigma), pSIN-GFP (Dixon et al. 2014), modified nucleotide RNA (modRNA) for GFP (Miltenyi Biotech) and FAM-labelled siRNA against GAPDH (Sigma), and nanomag-D (250nm) (MircoMod) were complexed with GET-proteins or -peptides and added to cells. For antibodies complexes were allowed to form in growth media for 20mins before cell addition. For nucleic acids a 2:1 peptide:nucleic acid charge ratio was used for complexation. GET- or LIPO2000 (lipofectamine 2000; Invitrogen) transfection used 10μg or 1 μg nucleic acid per transfection of 100,000 hMSCs in 12 well plates. GET-peptide substituted LIPO2000 following the exact manufacturer's instructions. For MNPs, a final concentration of 25μM peptide was used in an EDAC/NHS reaction using 2mg MNPs according to manufacturer's instructions. Prussian blue was carried out using potassium ferrocyanide (2.5% w/v) in 2.5% w/v HCl.

**Statistical Analysis**

Statistical comparisons were carried out using the GraphPad Prism software package. Comparisons were made using Tukey-Kramer analysis of variance (ANOVA). Results were considered significant if $p<0.05$.

**References:**

1. Gump JM & Dowdy SF (2007) TAT transduction: the molecular mechanism and therapeutic prospects. Trends in molecular medicine 13(10):443-448.

2. El-Andaloussi S, Holm T, & Langel U (2005) Cell-penetrating peptides: Mechanisms and applications. Curr Pharm Design 11(28):3597-3611.

3. Goun EA, Pillow TH, Jones LR, Rothbard JB, & Wender PA (2006) Molecular transporters: Synthesis of oligoguanidinium transporters and their application to drug delivery and real-time imaging. Chembiochem 7(10):1497-1515.

4. Meade BR & Dowdy SF (2007) Exogenous siRNA delivery using peptide transduction domains/cell penetrating peptides. Adv Drug Deliver Rev 59(2-3):134-140.

5. Fischer R, Fotin-Mleczek M, Hufnagel H, & Brock R (2005) Break on through to the other side - Biophysics and cell biology shed light on cell-penetrating peptides. Chembiochem 6(12):2126-2142.

6. Nakase I, Takeuchi T, Tanaka G, & Futaki S (2008) Methodological and cellular aspects that govern the internalization mechanisms of arginine-rich cell-penetrating peptides. Adv Drug Deliver Rev 60(4-5):598-607.

7. Heitz F, Morris MC, & Divita G (2009) Twenty years of cell-penetrating peptides: from molecular mechanisms to therapeutics. Brit J Pharmacol 157(2):195-206.

8. Gump JM, June RK, & Dowdy SF (2010) Revised Role of Glycosaminoglycans in TAT Protein Transduction Domain-mediated Cellular Transduction. J Biol Chem 285(2):1500-1507.

9. Norbury CC, Hewlett LJ, Prescott AR, Shastri N, & Watts C (1995) Class I MHC presentation of exogenous soluble antigen via macropinocytosis in bone marrow macrophages. Immunity 3(6):783-791.

10. Meier O, et al. (2002) Adenovirus triggers macropinocytosis and endosomal leakage together with its clathrin-mediated uptake. J Cell Biol 158(6):1119-1131.

11. Conner SD & Schmid SL (2003) Regulated portals of entry into the cell. Nature 422(6927):37-44.

12. Wadia JS, Stan RV, & Dowdy SF (2004) Transducible TAT-HA fusogenic peptide enhances escape of TAT-fusion proteins after lipid raft macropinocytosis. Nat Med 10(3):310-315.

13. Skehel JJ, Cross K, Steinhauer D, & Wiley DC (2001) Influenza fusion peptides. Biochem Soc T 29:623-626.

14. Han X, Bushweller JH, Cafiso DS, & Tamm LK (2001) Membrane structure and fusion-triggering conformational change of the fusion domain from influenza hemagglutinin. Nat Struct Biol 8(8):715-720.

15. Sakuma T, Higashiyama S, Hosoe S, Hayashi S, & Taniguchi N (1997) CD9 antigen interacts with heparin-binding EGF-like growth factor through its heparin-binding domain. Journal of biochemistry 122(2):474-480.

16. Higashiyama S, Abraham JA, & Klagsbrun M (1993) Heparin-Binding Egf-Like Growth-Factor Stimulation of Smooth-Muscle Cell-Migration - Dependence on Interactions with Cell-Surface Heparan-Sulfate. J Cell Biol 122(4):933-940.

17. Thompson SA, et al. (1994) Characterization of Sequences within Heparin-Binding Egf-Like Growth-Factor That Mediate Interaction with Heparin. J Biol Chem 269(4):2541-2549.

18. Kaplan IM, Wadia JS, & Dowdy SF (2005) Cationic TAT peptide transduction domain enters cells by macropinocytosis (vol 102, pg 247, 2005). J Control Release 107(3):571-572.

19. Lawrence R, Lu H, Rosenberg RD, Esko JD, & Zhang LJ (2008) Disaccharide structure code for the easy representation of constituent oligosaccharides from glycosaminoglycans. Nat Methods 5(4):291-292.

20. Lin X, et al. (2000) Disruption of gastrulation and heparan sulfate biosynthesis in EXT1-deficient mice. Dev Biol 224(2):299-311.

21. Dick E, Matsa E, Young LE, Darling D, & Denning C (2011) Faster generation of hiPSCs by coupling high-titer lentivirus and column-based positive selection. Nat Protoc 6(6):701-714.

22. Anderson RGW (1998) The caveolae membrane system. Annu Rev Biochem 67:199-225.

23. Nichols BJ & Lippincott-Schwartz J (2001) Endocytosis without clathrin coats. Trends Cell Biol 11(10):406-412.

24. Liu NQ, et al. (2002) Human immunodeficiency virus type 1 enters brain microvascular endothelia by macropinocytosis dependent on lipid rafts and the mitogen-activated protein kinase signaling pathway. J Virol 76(13):6689-6700.

25. West MA, Bretscher MS, & Watts C (1989) Distinct Endocytotic Pathways in Epidermal Growth Factor-Stimulated Human Carcinoma A431 Cells. J Cell Biol 109(6):2731-2739.

26. Sampath P & Pollard TD (1991) Effects of Cytochalasin, Phalloidin, and Ph on the Elongation of Actin-Filaments. Biochemistry-Us 30(7):1973-1980.

27. Oliver JM, Berlin RD, & Davis BH (1984) Use of Horseradish-Peroxidase and Fluorescent Dextrans to Study Fluid Pinocytosis in Leukocytes. Method Enzymol 108:336-347.

28. Araki N, Johnson MT, & Swanson JA (1996) A role for phosphoinositide 3-kinase in the completion of macropinocytosis and phagocytosis by macrophages. J Cell Biol 135(5):1249-1260.

29. Seglen PO, Grinde B, & Solheim AE (1979) Inhibition of the Lysosomal Pathway of Protein-Degradation in Isolated Rat Hepatocytes by Ammonia, Methylamine, Chloroquine and Leupeptin. Eur JBiochem 95(2):215-225.

30. Eustice DC & Wilhelm JM (1984) Mechanisms of Action of Aminoglycoside Antibiotics in Eukaryotic Protein-Synthesis. Antimicrob Agents Ch 26(1):53-60.
31. Yu JY, Chau KF, Vodyanik MA, Jiang JL, & Jiang Y (2011) Efficient Feeder-Free Episomal Reprogramming with Small Molecules. Plos One 6(3).
32. Warren L, et al. (2010) Highly Efficient Reprogramming to Pluripotency and Directed Differentiation of Human Cells with Synthetic Modified mRNA. Cell Stem Cell 7(5):618-630.
33. Kim D, et al. (2009) Generation of human induced pluripotent stem cells by direct delivery of reprogramming proteins. Cell Stem Cell 4(6):472-476.
34. Zhou HY, et al. (2009) Generation of Induced Pluripotent Stem Cells Using Recombinant Proteins (vol 4, pg 381, 2009). Cell Stem Cell 4(6):581-581.
35. Takahashi K, et al. (2007) Induction of pluripotent stem cells from adult human fibroblasts by defined factors. Cell 131(5):861-872.
36. Dixon JE, et al. (2010) Axolotl Nanog activity in mouse embryonic stem cells demonstrates that ground state pluripotency is conserved from urodele amphibians to mammals. Development 137(18):2973-2980.
37. Chambers I, et al. (2003) Functional expression cloning of Nanog, a pluripotency sustaining factor in embryonic stem cells. Cell 113(5):643-655.
38. Robinton DA & Daley GQ (2012) The promise of induced pluripotent stem cells in research and therapy. Nature 481(7381):295-305.
39. Do Kwon Y, et al. (2005) Cellular manipulation of human embryonic stem cells by TAT-PDX1 protein transduction. Mol Ther 12(1):28-32.
40. Hidema S, Tonomura Y, Date S, & Nishimori K (2012) Effects of protein transduction with intact myogenic transcription factors tagged with HIV-1 Tat-PTD (T-PTD) on myogenic differentiation of mouse primary cells. J Biosci Bioeng 113(1):5-11.
41. Liang QL, Mo ZY, Li XF, Wang XX, & Li RM (2013) Pdx1 protein induces human embryonic stem cells into the pancreatic endocrine lineage. Cell Biol Int 37(1):2-10.
42. Bichsel C, et al. (2013) Direct Reprogramming of Fibroblasts to Myocytes via Bacterial Injection of MyoD Protein. Cell Reprogram 15(2):117-125.
43. Chan EM, et al. (2009) Live cell imaging distinguishes bona fide human iPS cells from partially reprogrammed cells. Nat Biotechnol 27(11):1033-U1100.
44. Smith KP, Luong MX, & Stein GS (2009) Pluripotency: Toward a Gold Standard for Human ES and iPS Cells. J Cell Physiol 220(1):21-29.
45. Burridge PW, et al. (2011) A Universal System for Highly Efficient Cardiac Differentiation of Human Induced Pluripotent Stem Cells That Eliminates Interline Variability. Plos One 6(4).
46. Campbell RE, et al. (2002) A monomeric red fluorescent protein. Proceedings of the National Academy of Sciences of the United States of America 99(12):7877-7882.
47. Okamoto T, et al. (2002) Clonal heterogeneity in differentiation potential of immortalized human mesenchymal stem cells. Biochem Bioph Res Co 295(2):354-361.
48. Anderson D, et al. (2007) Transgenic enrichment of cardiomyocytes from human embryonic stem cells. Mol Ther 15(11):2027-2036.
49. Claycomb WC, et al. (1998) HL-1 cells: A cardiac muscle cell line that contracts and retains phenotypic characteristics of the adult cardiomyocyte. Proceedings of the National Academy of Sciences of the United States of America 95(6):2979-2984.
50. Dixon JE, Dick E, Rajamohan D, Shakesheff KM, & Denning C (2011) Directed differentiation of human embryonic stem cells to interrogate the cardiac gene regulatory network. Mol Ther 19(9): 1695-1703.
51. Medina D, Moskowitz N, Khan S, Christopher S, & Germino J (2000) Rapid purification of protein complexes from mammalian cells. Nucleic Acids Res 28(12).
52. Novak A, Guo CY, Yang WY, Nagy A, & Lobe CG (2000) Z/EG, a double reporter mouse line that expresses enhanced green fluorescent protein upon Cre-mediated excision. Genesis 28(3-4): 147-155.
53. Bayoussef Z, Dixon JE, Stolnik S, & Shakesheff KM (2012) Aggregation promotes cell viability, proliferation, and differentiation in an in vitro model of injection cell therapy. J Tissue Eng Regen M 6(10):e61-e73.

**Improving the Efficiency of Iron Oxide Nanoparticle Uptake Using Cell Penetrating Peptides**

**Background**

Superparamagnetic Iron Oxide nanoparticles (SPIONS) are small highly magnetised particles consisting of an iron oxide core and surface coating. SPIONS have been clinically approved for use in MRI contrast agents[1], and are currently being researched for use in targeted drug delivery[2], hyperthermia treatment and cell labelling[3]. SPIONS have been approved for uses in MRI contrast agents and commercially available products include Lumiren, Resivist and

Feridex.[1]

Applications of SPIONS require an adequate concentration being internalised into cells and without the required targeting of nanoparticles it can lead to an inefficient outcome. The efficiency of cell internalisation can depend on the size, coating and additional ligands to name a few[4]. Literature shows that without the attachment of internalisation agents researchers are achieving a range of 15-30 pg of iron per cell[5,6]. The functional groups on nanoparticle coatings can be exploited to target increase cell internalisation by attaching monoclonal antibodies, cell penetrating peptides and small molecules as internalisation agents.[7]

A currently researched cell penetrating peptide is Arg-Gly-Asp (RGD). RGD was designed to target the $\alpha$**v**$\beta$**3** intergrin[8]. The intergrin can be found predominantly on cancer cells, so can also be used as a targeting peptide. Research found that the RGD peptide increased nanoparticle uptake by 50%.[9]

The following study focuses on a cell penetrating peptide of the invention herein, in particular P218R. The peptide has two domains, P21 binds to the heparan sulphate (HS) on the cell membrane and the 8R aids in the transduction. The aim of the study was to identify the efficiency of the P218R and to investigate its mechanism.

## Materials and Methods

### Nanoparticle labelling

A 31 mM EDAC with 0.1 M NHS dissolved in 0.5M MES buffer was added to Nanomag-D (250 nm) particles in a 1:5 ratio respectively and mixed for 1 hour. The particles are then washed in a 0.1M MES buffer and 0.2 $\mu$g/$\mu$l of the required labelling agent dissolved in the same buffer was added to give a 1:1 ratio of labelling solution and nanoparticles, an aliquot of the labelling solution was kept for testing labelling efficiency. The solution is then continuously mixed at room temperature for 3 hours. Once the particles are labelled a 25 mM glycine solution is added to the particles then further incubated for 30 minutes. An aliquot of the labelling solution is kept for comparison with the earlier aliquot and the particles washed in 0.1% BSA in PBS. Particles are finally diluted in 0.1% BSA in PBS to give a 1mg/ml solution. Both aliquots of labelling solution and some of the labelled nanoparticles were assessed for fluorescence.

### Cell Culture

NIH 3t3 fibroblast cells were cultured in Dulbecco's modified Eagle's media (DMEM; Gibeco), supplemented with 10% (v/v) Fetal Calf Serum (FCS, Sigma), 2mM L-glutamine and (PS) at 37oC and 5% CO2. The cells were then cultured until confluent.

### Cell Labelling

Confluent cells were split into 12 well plates at 200,000 cells/well and incubated for 24 hours at 37oC. After 24 hours 50 $\mu$g of Nanomag-D iron oxide nanoparticles (250 nm) and either 0, 0.01, 0.05, 0.1, 0.5, 2, 1, 5 and 10 $\mu$M of cell penetrating peptide were added to the cells with either 10% FCS DMEM or serum free DMEM media and left for 24 hours for iron nanoparticles to be internalised. After incubation cells were washed in PBS to remove excess nanoparticles then harvested for qualitative Prussian blue staining, quantitative colorimetric iron assay or fluorescence activated flow cytometry.

### Prussian Blue Staining

Cells were labelled then fixed in 4% (w/v) PFA for 15-20 minutes at 4oC. A staining solution of 2.5% potassium ferrocyanide in 2.5% HCL was added to cells and incubated for an hour at room temperature. If nanoparticles were present a blue stain appeared which is proportional to the concentration of iron.

Quantitative Colorimetric Iron Assay Cells were labelled, trypsinised and pelleted then all media removed. 40$\mu$l of 37% HCL was added to the cells and heated at 70°C until dissolved, then neutralised with 50$\mu$l of NaOH. Those samples containing a high concentration of iron were diluted 1:10 then 40 $\mu$l of Quantichrom working reagent was added and the instructions in the Quantichrom iron assay followed.

### Flow Cytometry

Cells were labelled with 50 $\mu$g of Nanomag-D particles, 1 $\mu$M P218R and either 0, 0.1, 1 and 5 $\mu$g/ml of FitC-BSA. Cells were then fixed cells and run through a Coulter Altra flow cytometer to assess the green fluorescence. Findings were then statistically analysed by Wesal software.

**Results**

**Nanoparticle and Cell labelling**

Nanomag-D (250 nm) particles were successfully labelled with mR, P21mR, 8RmR and P21mR8R.

**Quantitative Assessment of Nanoparticle Uptake**

**Optimisation of Protein Concentration**

As shown in figure 30, Prussian blue staining proves that the P218R is the most effective peptide for particle uptake compared to P21 and 8R alone. The iron assay results showed that when cells are incubated for 24 hours with 1μM of P218R and 50μg of Nanomag-D particles 100% of the particles become associated with the cells and 63 pg/cell. Therefore it was concluded that only 1 μM of P218R is needed for 100% uptake of particles.

**Discussion**

The results show that the addition of a small amount of P218R leads to 100% uptake of iron oxide nanoparticles. Microscopy and the trypsinisation of the cells indicate that the particles are being internalised. Experiments were also conducted using mesenchymal stem cells showing a 90% association of particles. The mechanism behind the uptake is dependent on the symbiotic action of the two domains of the peptide, The hypothesis is that the P21 can bind to both the HS on the cell membrane and the dextran in the coating of the nanoparticles, the peptide either has multiple binding points by which both nanoparticle and cell can both be attached to the same P21. Therefore the pre bound protein to the particle can also bind to the membrane keeping the particle in close proximity to the cell. The 8R can then aid in the transduction of the nanoparticle by endocytosis. Or the other mechanism could involve the peptide pre binding to the nanoparticle then when in close proximity to a cell membrane the HS has a higher binding efficiency so the P21 then binds to the cell. This may then lead to the particle being internalised. The advantages of using the P218R peptide is its efficiency in serum media which is more relatable to the in vivo environment and that the system does not require the use of the functional group on the nanoparticles surface coating. The free functional group means that targeting molecules or drugs can be covalently attached to the particle.

**Conclusion**

The peptide P218R has been found to cause 100% cell association of nanoparticles. This has been found to be due to a dextran binding mechanism which can be utilised for many applications for example targeting of nanoparticles for specific tissues by attaching antibodies, or drug delivery.

**References**

1. Singh, A & Sahoo, S, (2013), Magnetic Nanoparticles: a novel platform for theranostics, Drug Disc Today,
2. Arruebo, M et al, (2007) Magnetic Nanoparticles for drug delivery, Nanotoday, 3, 22
3. Wang, Z and Cuschieri A, (2013) Tumor cell labelling by magnetic nanoparticles with determination of intracellular iron content and spatial distribution of the intracellular iron, Int. J. Mol. Sci, 14, 9111
4. Sun, C, Lee, J and Zhang, M, (2008), Magnetic Nanoparticles in MR Imaging and drug delivery, Adv Drug Deli Rev, 60, 1253
5. Schlorf, T et al, (2011), Biological properties of iron oxide nanoparticles for cellular and molecular magnetic resonance imaging, Int. J. Mol. Sci. 12, 12
6. Markides, H et al, (2013) Whole body tracking of superparamagnetic iron oxide nanoparticle labelled cells - a rheumatoid arthritis mouse model, Stem cell Res & ther, 4, 126
7. Peng et al, (2008), targeted magnetic iron oxide nanoparticles for tumour imaging and therapy, Int. J. of Nanomed. 3, 311
8. Zhang, C et al, (2007), Specific Targeting of Tumor Angiogenesis by RGD-Conjugated Ultrasmall Superparamagnetic Iron Oxide Particles Using a Clinical 1.5-T Magnetic Resonance Scanner, Can. Res. 67, 1555
9. Ji, S et al, (2012), RGD-conjugated albumin nanoparticles as a novel delivery vehicle in pancreatic cancer therapy, Cancer Biology & Therapy 13:4, 206.

**Modified CPPs for Efficient Cell Type Specific Delivery of Therapeutic Molecules via GET (Glycosaminoglycan (GAG)-binding Enhanced Transduction)**

**Introduction**

The first aim of this study was to investigate whether the GET-mediated synergistic increase in delivery of mRFP into cells with P21 8R could be observed when P21 was replaced by growth factor derived HS-GAG binding domains. The second aim of this study was to show cell type specific delivery in a heterogeneous population of cells by targeting a specific cell surface HS-epitope. Merry et al have demonstrated the utility of a HS-epitope binding antibody in targeting a subpopulation of cells during mesodermal differentiation [13]. The variable region of this antibody was conjugated to 8R to show an example of cell type specific delivery. The third aim of this study was to demonstrate the GET-mediated delivery of therapeutic biomolecules. The transfection of reporter gene (pSIN GFP) was optimized with P21 LK15 8R peptide and compared to a 'gold standard' commercial lipid based transfection reagent Lipofectamine2000.

**Cell Type Specific Delivery via GET**

***Experimental Procedures***

**Preparation of Peptides**

Peptides, mRFP, mRFP 8R, P21 mRFP 8R, FGF1A mRFP, FGF1A mRFP 8R, FGF2A mRFP, FGF2A mRFP 8R, FGF4A mRFP, FGF4A mRFP 8R, FGF7A mRFP, FGF7A mRFP 8R, FGF1B mRFP, FGF1B mRFP 8R, FGF2B mRFP, FGF2B mRFP 8R, FGF4B mRFP, FGF4B mRFP 8R, FGF7B mRFP, FGF7B mRFP 8R, FGF1C mRFP, FGF1C mRFP 8R, FGF2C mRFP, FGF2C mRFP 8R, FGF4C mRFP, FGF4C mRFP 8R, FGF7C mRFP, FGF7C mRFP 8R, ATIII mRFP, ATIII mRFP 8R, PDGF mRFP, PDGF mRFP 8R, VEGF mRFP, VEGF mRFP 8R, HS4C3 mRFP and HS4C3 mRFP 8R, were cloned as cDNAs into pGEX6-PI vector (Novagen), expressed in BL21 (DE21) pLysS *Escherichia coli* (Novagen) and purified as previously described [12]. Integrity and full length peptide expression was confirmed by SDS-PAGE. Fluorescence of the recombinant peptides was confirmed using the TECAN infinite 200PRO multimode reader, the difference in fluorescence intensity measurements between samples was <10%.

**Peptide Assay**

Bradford assay was used to quantify protein concentration [14]. Absorbance was measured at 595nm using recombinant mRFP protein as a standard [12]. Samples were analysed using the TECAN infinite 200PRO multimode reader.

**Cell Culture of NIH3T3, CGR-8 and HUES7 Cells**

NIH3T3, CGR-8 and HUES7 cells were grown and maintained as previously described [12]. NIH3T3 mouse fibroblast cells were maintained in Dulbecco's modified Eagle's medium (DMEM) with 10% (v/v) fetal calf serum (FCS) supplemented with 2mM L-glutamine and 100 ug/ml streptomycin. CGR-8 mouse embryonic stem cells were maintained in DMEM with 20% (v/v) FCS supplemented with 1000units/ml *l*eukaemia inhibitory factor (LIF), 100μM β-mecaptoethanol, 2mM L-glutamine and 100ug/ml streptomycin. HUES7 human embryonic stem cells were cultured on gelatin coated tissue culture flask. The cells were maintained in DMEM with 20% (v/v) FCS supplemented with 1000units/ml LIF, 100μM β-mecaptoethanol, 2mM L-glutamine and 100ug/ml streptomycin. All cells were incubated at 37°C under humidified 5% $CO_2$ conditions.

**Peptide Delivery to Cells**

Cells were seeded at 2 x $10^5$cells/well in 12-well plates and incubated for 2h in 1mL of relevant growth media (GM) at 37°C in a 5% $CO_2$ humidified incubator. The peptide was diluted to 20ug/ml in 500ul of GM. Each well of cells was aspirated, washed with *p*hosphate *b*uffered *s*aline (PBS) and replaced with 500ul of peptide solution. The cells were incubated with the peptide at 37°C in a 5% $CO_2$ humidified atmosphere for 20h. Following incubation, each well of cells was washed with PBS, trypsinized and fixed with 3.7% *p*ara*f*orm*a*ldehyde (PFA) in preparation for flow cytometry. Each experiment was done in duplicate and repeated 3 times, n=3.

**Maintenance and Differentiation of Bry-GFP ES Cells, Generation of EBs**

Bry-GFP murine embryonic stem cell line was maintained and differentiated as previously described [13]. Bry-GFP cells were maintained on feeders in DMEM-ES (DMEM with 15% FCS supplemented with 1.5x10$^5$ M monothioglycerol (MTG), 10ng/ml LIF and 2mM L-glutamine).

Bry-GFP cells were differentiated as EBs. Prior to differentiation the cells were passaged twice, first onto a gelatin coated flask in DMEM-ES and second into a flask in Iscove's modified Dulbecco's medium (IMDM)-ES (IMDM with 15% FCS supplemented with 1.5x10$^4$ M monothioglycerol (MTG), 10ng/ml LIF and 2mM L-glutamine). Cells were then differentiated as EBs for 2.8 days in IMDM with 15% FCS supplemented with 4x10$^4$ M MTG, 300ug/ml transferrin, 25ug/ml ascorbic acid and 2mM L-glutamine in Petri-grade dishes. 3h before dissociation, EBs were treated with 50ug/ml of HS4C3 mRFP or HS4C3 mRFP 8R. Following differentiation EBs were separated into single cells by 10min incubation and agitation in cell dissociation buffer and fixed in PFA.

**Flow Cytometry Analysis**

Cells were analysed on a MoFlo™ DP (DAKO) Flow Cytometer using a 488nm green laser and/or 633nm red laser. (40,000 cells; gated on live cells by forward/side scatter). Median fluorescence was used for statistical analyses.

***Results and Discussion***

**CPPs Modified to Include GET**

In this study the HS-GAG binding domains of fibroblast growth factor (FGF)-1, FGF-2, FGF-4, FGF-7, platelet derived growth factor (PDGF) and antithrombin-III (ATIII) were coupled to 8R. These growth factors play important biological roles in embryonic development and angiogenesis. They have also been shown to interact with cell surface HS-GAGs, similarly to P21. NIH 3T3 murine fibroblasts, CGR8 murine embryonic stem cells and HUES-7 human embryonic stem cells were treated with these modified CPPs to investigate whether any of the peptides demonstrated a GET-mediated increase in delivery of mRFP (Figure 31). It was also important to explore whether any of the modified peptides would preferentially target HS epitopes that were more abundantly expressed in any of the different cell types.

A panel of four modified CPPs that showed GET-mediated enhanced transduction into cells have been identified. P21 8R, FGF2B 8R, FGF7B 8R and PDGF 8R have demonstrated 30-100 fold increase in transduction of mRFP into cells over using an 8R alone (Figure 32 and 33). P21 8R, FGF7B 8R and PDGF 8R showed preferential transduction into HUES-7 embryonic stem cells. This demonstrates preferential transduction of CPPs into a cell-type that is considered difficult to transduce in to. FGF2B 8R showed pluripotency specific transduction into CGR8 and HUES7 embryonic stem cells. The diverse delivery profiles of the modified CPPs into the three cell types suggests the HS-GAG binding domains of different growth factors target and bind different cell surface HS-epitopes. Targeting HS-epitopes that are expressed more abundantly by specific cell types can be utilized for the selection of CPPs that are more suitable for their application.

**GET Mediated Delivery of Plasmid DNA**

***Experimental Procedures***

**Preparation of Peptides**

P21-LK15-8R peptide was synthesised using solid phase t-Boc chemistry (Novabiochem (Beeston, Nottinghamshire, UK)).

**Cell culture**

NIH3T3 mouse fibroblast cells were maintained in DMEM with 10% (v/v) fetal calf serum (FCS) media supplemented with 2mM L-glutamine and 100ug/ml streptomycin. The cells were incubated at 37°C under humidified 5% $CO_2$ conditions.

**Preparation of Plasmid DNA**

DNA (pSIN GFP) was amplified in E. coli. The DNA was extracted and purified using a QIAGEN Plasmid Maxi kit (Qiagen). DNA was precipitated in 100% ethanol and rehydrated in d$H_2O$. Plasmid purity was confirmed using the nanodrop.

**Peptide-DNA Complexation Assay**

10ug DNA was diluted in 60ul 4-(2-hydroxyethyl)-1-piperazineethanesulfonic acid (HEPES)-buffered saline (10mM HEPES, 150mM sodium chloride (NaCl) solution, pH 7.4). 1mM YO-PRO-1 stock solution was diluted to 0.1mM in dimethyl sulfoxide (DMSO). 2.7ul of the diluted YO-PRO-1 solution was made up to 60ul in HEPES-buffered saline and added dropwise to the diluted DNA. The DNA/YO-PRO-1 solution was mixed, wrapped in foil and incubated for 5h at room temperature. After 5h, the DNA/YO-PRO-1 solution was made up to 1ml in HEPES-buffered saline and 100ul aliquots were pipetted into eppindorf tubes per treatment condition. Peptide amounts corresponding to the desired (+/-) charge ratios were added to each eppindorf (Appendix 1). Peptide/DNA/YO-PRO-1 solutions were mixed and incubated at room temperature for 10min. Fluorescence measurements were then analysed using the TECAN infinite 200PRO multimode reader. Similarly, a no DNA control was made by diluting 2.7ul of the diluted YO-PRO-1 solution in 120ul HEPES-buffered saline and following the procedure above.

**Design and Optimization of Transfection Experiment**

Cells were seeded at 80,000 cells per well on 12-well plates and incubated overnight in 1mL of 10% GM at 37°C in a 5% $CO_2$ humidified incubator. For each well of cells to be transfected, DNA was diluted in 100ul Opti-MEM® and mixed. The peptide was added directly to the diluted DNA at the optimal (+/-) charge ratio. The solution was then mixed and incubated for 25min at room temperature. The cells were aspirated, washed with PBS and replaced with 400ul Opti-MEM®. Each well of cells was treated with 100ul of Peptide/DNA complex and incubated 37°C in a 5% $CO_2$ humidified atmosphere. Appendix 2 shows the different treatment conditions used for each well. Following incubation, the cells were washed with PBS and replaced with 1ml GM. After 48h, each well of cells was washed with PBS, trypsinized and fixed with 3.7% PFA. Experiments were repeated 3 times.

Lipofectamine2000 transfection optimization was carried out as described in the manufacturers guide (Invitrogen). Cells were seeded at 80,000 cells per well on 12-well plates and incubated overnight in 1mL of GM at 37°C in a 5% $CO_2$ humidified incubator. For each well of cells to be transfected, DNA was diluted in 100ul Opti-MEM® and mixed. 1.5ul of Lipofectamine2000 was added directly to the diluted DNA. The mixture was then mixed and incubated for 25mins at room temperature. The cells were aspirated, washed with PBS and replaced with 400ul Opti-MEM®. Each well of cells was treated with 100ul of Lipofectamine2000/DNA complex and incubated 37°C in a 5% $CO_2$ humidified atmosphere. After 6h, the cells was washed with PBS, trypsinized and fixed with 3.7% PFA. This was repeated with varying volumes of Lipofectamine2000 (3ul and 4.5ul) to find the optimal ratio of Lipofectamine2000 to DNA. Experiments were repeated 3 times.

**Flow Cytometry Analysis**

Cells were analysed on a MoFlo™ DP (DAKO) Flow Cytometer using a 633nm red laser. (40,000 cells; gated on live cells by forward/side scatter). Median fluorescence was used for statistical analyses.

***Results and Discussion***

**Peptide to DNA Binding**

YO-PRO-1 assay can be used to investigate the DNA condensation ability of a DNA binding peptide. YO-PRO-1 is a cyanine dye that binds DNA to form a fluorescent DNA/dye complex. Different (+/-) charge ratios of peptide can be added to the fluorescent DNA/dye complex. As the peptide out competes the dye by binding the DNA a reduction in fluorescence intensity is observed. In this study, LK15 was fused to P21 8R transduction protein to improve the DNA binding ability of the modified cell penetrating peptide. Fusion of LK15 peptide to TAT has been shown to significantly improve transfection of pDNA into HT29 and HT1080 cultured cells [19]. Enhanced transduction efficiency of Tat-LK15 over Tat is thought to be due to the improved condensation ability of the peptide and DNA, and better transduction of the DNA across the cell membrane [20].

A graph of (+/-) charge ratio was plotted against % fluorescence to investigate the optimum ratio of P21 LK15 8R to pSIN GFP (Figure 24). Results showed that the optimal (+/-) charge ratio of P21 LK15 8R to pSIN GFP was 2:1, respectively. This ratio was used in the transfection experiments.

**Transfection of pDNA Reporter Gene via P21 LK15 8R**

The phospholipid bilayer of the cell membrane acts as an impenetrable barrier to nucleic acids and thus pDNA will be conjugated to a modified CPP to facilitate its transport into the cell [2]. In this study the GET-mediated transfection

of the reporter gene pSIN GFP into NIH 3T3 murine fibroblast cells was optimized in terms of transfection time (3, 6 or 24h), transfection media (with or without serum) and amount of DNA (1, 4 or 10ug). The reporter gene pSIN GFP was transfected into cells using P21 LK15 8R where P21 targets and binds cell surface HS-GAGs, LK15 complexes pSIN GFP and 8R transduces pSIN GFP across the cell membrane. The transfection efficiency of pSIN GFP with P21 LK15 8R was compared to the transfection efficiency of commercially used lipid-based transfection reagent lipofectamine2000. Cells were fixed at 48h following transfection to allow time for the transient expression of GFP to be captured and transfection efficiencies were quantified by flow cytometry (Figure 35).

Gene carrier systems must be serum resistant for efficacious in-vivo applications, however most gene carries, including lipofectamine2000, have demonstrated steep decreases in transfection efficiency in serum containing media [21]. This is believed to be because serum molecules competitively bind the gene carrier, therefore decreasing free gene carriers available to bind the DNA [22]. The transfection efficiency of P21 LK15 8R was characterised in serum and serum free transfection media. The optimal transfection conditions were when cells were transfected with 10ug DNA for 24h in serum conditions where transfection efficiency reached 17.9 $\pm$ 4.8%. (Figure 36). This is 3 fold lower than the optimized transfection efficiency observed for lipofectamine2000 in serum free conditions (54.7 $\pm$ 10.3%, Appendix 3) however, the serum-resistance of P21 LK15 8R transfection is advantageous for any sort of clinical/ in-vivo delivery of therapeutic biomolecules. In addition, it is well documented that endosomal escape strategies greatly increase the efficiencies of CPP-mediated transfections.

**Conclusions**

A panel of CPPs that have been modified to include growth factor derived cell surface HS-GAG binding domains have shown 30-100 fold increase in transduction into cells, compared to unmodified CPPs. The hypothesis is that the GET-mediated delivery of these peptides is due to the dual functionality of the peptide in i) increasing interaction with the cell membrane via the HS-GAG binding domain, and ii) transducing protein across cell membrane via 8R. The modified CPPs showed preferential delivery profiles of mRFP into different cell types, this is due the HS-GAG binding domains targeting specific HS-epitopes that are more abundantly expressed in different cell types. Future work is to modify CPPs to include specific antibody-derived HS-epitope binding domains. HS-epitope binding libraries of antibodies can be utilized for the cell type specific delivery of therapeutic molecules via GET.

To demonstrate the utility of these peptides for the delivery of therapeutic molecules P21 LK15 8R was used to deliver the reporter gene pSIN GFP into cells. Results showed GET-mediated transfection efficiencies of up to 17.9 $\pm$ 4.8% in serum conditions, without any endosomal escape strategy. CPPs modified to include HS-GAG binding domains show great promise as alternatives to using viral and lipid based delivery vehicles for the in-vivo delivery of therapeutic biomolecules.

**References**

[1] Bechara C, Sagan S. Cell-penetrating peptides: 20 years later, where do we stand? Febs Letters 2013;587:1693-702.

[2] Tanaka K, Kanazawa T, Ogawa T, Suda Y, Takashima Y, Fukuda T, et al. A Novel, Bio-Reducible Gene Vector Containing Arginine and Histidine Enhances Gene Transfection and Expression of Plasmid DNA. Chemical & Pharmaceutical Bulletin 2011;59:202-7.

[3] Mitchell DJ, Kim DT, Steinman L, Fathman CG, Rothbard JB. Polyarginine enters cells more efficiently than other polycationic homopolymers. Journal of Peptide Research 2000;56:318-25.

[4] Nakase I, Niwa M, Takeuchi T, Sonomura K, Kawabata N, Koike Y, et al. Cellular uptake of arginine-rich peptides: Roles for macropinocytosis and actin rearrangement. Molecular Therapy 2004;10:1011-22.

[5] Ma DX, Shi NQ, Qi XR. Distinct transduction modes of arginine-rich cell-penetrating peptides for cargo delivery into tumor cells. International Journal of Pharmaceutics 2011;419:200-8.

[6] El-Sayed A, Futaki S, Harashima H. Delivery of Macromolecules Using Arginine-Rich Cell-Penetrating Peptides: Ways to Overcome Endosomal Entrapment. Aaps Journal 2009;11:13-22.

[7] Shiraishi T, Nielsen PE. Enhanced delivery of cell-penetrating peptide-peptide nucleic acid conjugates by endosomal disruption. Nature Protocols 2006;1:633-6.

[8] Matsubara Y, Chiba T, Kashimada K, Morio T, Takada S, Mizutani S, et al. Transcription activator-like effector nuclease-mediated transduction of exogenous gene into IL2RG locus. Scientific Reports 2014;4.

[9] Parelkar SS, Letteri R, Chan-Seng D, Zolochevska O, Ellis J, Figueiredo M, et al. Polymer-Peptide Delivery Platforms: Effect of Oligopeptide Orientation on Polymer-Based DNA Delivery. Biomacromolecules 2014;15:1328-36.

[10] Yang HY, Vonk LA, Licht R, van Boxtel AMG, Bekkers JEJ, Kragten AHM, et al. Cell type and transfection reagent-dependent effects on viability, cell content, cell cycle and inflammation of RNAi in human primary mesenchymal cells. European Journal of Pharmaceutical Sciences 2014;53:35-44.
[11] Ma Y, Gong C, Ma YL, Fan FK, Luo MJ, Yang F, et al. Direct cytosolic delivery of cargoes in vivo by a chimera consisting of D- and L-arginine residues. Journal of Controlled Release 2012;162:286-94.
[12] James E. Dixon GM, Nina Lane, Chris Denning and Kevin M. Shakesheff Highly Efficient Delivery of Functional Proteins by the Synergistic Effect of GAG Binding Motifs and Cell-Penetrating Peptides. Unpublished 2014.
[13] Baldwin RJ, ten Dam GB, van Kuppevelt TH, Lacaud G, Gallagher JT, Kouskoff V, et al. A Developmentally Regulated Heparan Sulfate Epitope Defines a Subpopulation with Increased Blood Potential During Mesodermal Differentiation. Stem Cells 2008;26:3108-18.
[14] Bradford MM. RAPID AND SENSITIVE METHOD FOR QUANTITATION OF MICROGRAM QUANTITIES OF PROTEIN UTILIZING PRINCIPLE OF PROTEIN-DYE BINDING. Analytical Biochemistry 1976;72:248-54.
[15] Schamhart DHJ, Kurth KH. Role of proteoglycans in cell adhesion of prostate cancer cells: From review to experiment. Urological Research 1997;25:S89-S96.
[16] Delehedde M, Deudon E, Boilly B, Hondermarck H. Proteoglycans in breast cancer. Pathologie Biologie 1997;45:305-11.
[17] Shao C, Shi XF, Phillips JJ, Zaia J. Mass Spectral Profiling of Glycosaminoglycans from Histological Tissue Surfaces. Analytical Chemistry 2013;85:10984-91.
[18] Thompson KE, Bashor CJ, Lim WA, Keating AE. SYNZIP Protein Interaction Toolbox: in Vitro and in Vivo Specifications of Heterospecific Coiled-Coil Interaction Domains. Acs Synthetic Biology 2012;1:118-29.
[19] Saleh AF, Aojula H, Arthanari Y, Offerman S, Alkotaji M, Pluen A. Improved Tat-mediated plasmid DNA transfer by fusion to LK15 peptide. Journal of Controlled Release 2010;143:233-42.
[20] Dufourcq J, Neri W, Henry-Toulme N. Molecular assembling of DNA with amphipathic peptides. Febs Letters 1998;421:7-11.
[21] Zhang X, Hu HM, Liu TB, Yang YY, Peng YF, Cai QQ, et al. Multi-armed poly(L-glutamic acid)-graft-polypropyleneinime as effective and serum resistant gene delivery vectors. International Journal of Pharmaceutics 2014;465:444-54.
[22] Wu HM, Pan SR, Chen MW, Wu Y, Wang C, Wen YT, et al. A serum-resistant polyamidoamine-based polypeptide dendrimer for gene transfection. Biomaterials 2011;32:1619-34.

## EXAMPLE 3

As described by The European Science Foundation, Nanomedicine uses nano-sized tools for the diagnosis, prevention and treatment of disease and to gain increased understanding of the complex underlying patho-physiology of disease. The ultimate goal is improved quality-of-life. As part of nanomedicine, nanoparticles for medical applications are defined as particles with a size between 1 and 1000 nm, their small size makes them suitable for penetrating biological barriers, concentrating in tumours and mediating intracellular delivery.In the past few decades a wide range of nanoparticles have been developed for their use in medicine, special interest has been paid to magnetic nanoparticles (MNP), due to their magnetic properties they can be directly targeted to the therapeutic site tissue using magnetic field. MNPs have four main biological applications: molecular detection, imaging (including a focus on regenerative medicine), targeted delivery and hyperthermia.

Most of the nanomedicine developing products currently in clinical trials are aimed at the treatment or imaging of cancer. Most chemotherapeutic substances are very cytotoxic but very unspecific, leading to undesired side effects on healthy tissue, which means the optimal dose for tumor eradication can never be employed. Specific targeting of anticancer drugs to the tumour is key to maximize the drug effects and limit its off-target toxicity. Many attempts have been carried out in the past decades to develop targeted therapies, either targeted drugs or targeted platforms for drug delivery. Understanding the nature of the tumours is essential in order to develop specifically targeted therapies. Tumours present certain characteristics that can facilitate specific delivery. Their physiology is characterized by $O_2$ depletion (hypoxia and anoxia), extracellular acidosis and high lactate levels creating an acidic microenvironment. Furthermore tumours also possess glucose deprivation, energy impoverishment, significant interstitial fluid flow and interstitial hypertension.

Use of hyperthermia involves the application of an external alternating magnetic field that induces heating of the local environment of the tumour and aims at changing the physiology of the diseased cells, eventually leading to apoptosis. The current aim of hyperthermia therapy research is to achieve the desired temperature enhancement for a particular application with the lowest concentration of MNPs possible.

For imaging purposes, MNP has already been approved by the FDA as contrast agents for MRI scanning, with several products already commercially available such Feridex, Resovist, Endorem, Lumirem, Sirenem, etc (Duncan & Gaspar, 2011) although these products haven't been as successful as expected and are no longer clinically used,

however, in the last couple of years, the fast development of regenerative medicine has found new applications for MNPs as imaging agents. MNP based contrast agents have successfully been used in preclinical trials for the delivery of stem cells (cardiovascular and neurodegenerative diseases) and there are several clinical trials that have been approved by the FDA, however, there are a number of limitations that haven't been overcome yet. There is a general lack of understanding on the fate of MNPs in magnetically labelled cells after their transplantation in an organism. So far, it has been observed that MNP-induced signal has a maximum shortly after transplantation and then completely declines. There are several theories that aim to justify this phenomenon: on one hand, stem cell proliferating nature could potentially lead to a dilution in the concentration of MNPs, decreasing the intensity of the signal; on the other hand cells that actively take up MNPs by endocytosis store them in lysosomes where MNPs can either undergo metabolic degradation or may leave the cell via exocytosis and then be degraded by macrophages.

So far, passive delivery of MNPs to cells has always been characterized by different levels of uptake and endosomal localization of the particles. For the general use of MNPs as contrasting agents it is important to develop robust and relatively easy protocols that could be implemented at a clinical level, these protocols should ideally ensure consistent cell uptake at therapeutically relevant quantities.

A delivery system has been developed based on the fusion of a Protein Transduction Domain (PTD) and a membrane docking peptide to heparan sulfate glycosaminoglycans (GAGs), the resulting protein was called GET (GAG-binding enhanced transduction). PTDs are capable to intracellularly delivery a series of cargoes, but their inability to deliver to the cytosol or the nucleus has so far hampered their application in biomedicine. In order to overcome this limitation a membrane docking peptide was fused with the PTD. This new delivery platform has showed much enhanced delivery compared to previously reported.

Free protein gets delivered to the cells in solution where proteins can rotate freely, however when on a surface each protein will adapt a certain orientation which determines which part of the molecule interacts with the surface and which part is exposed to the bulk solution. The orientation of the protein when adsorbed to the particles is certainly important since for the delivery of the cargo into the cell it is paramount that the CPP is able to interact with the cell membrane. Peptide-MNP interactions could potentially disturb the bioactivity of our delivering peptide. In this study different methods of characterisation have been used including Langmuir isotherms, zeta potential, ICP and FTIR to characterize GET and GET-pH.

The aim of my work is to develop and optimize a GET based technology for MNPs delivery to cells for further use in biomedicine focusing on regenerative medicine and cancer treatment. As part of the cancer treatment application the standard GET system was modified to be pH responsive such that it can deliver at tumour environment pHs but remain inert at physiological pHs.

**Methods**

**Cell culture**

NIH 3t3 fibroblast cells and Hela cells were cultured in Dulbecco's modified Eagle's media (DMEM; Sigma), supplemented with 10% (v/v) Fetal Calf Serum (FCS, Sigma), 2mM L-glutamine and 100units/ml penicillin and 100units/ml streptomycin (Invitrogen). Glioblastoma cell lines were provided by Dr Ruman Rahman from Children's Brain Tumour Research Center, University of Nottingham, U87 and GIN 8 cells were cultured in Dulbecco's modified Eagle's media (DMEM; Sigma) 1g/L D-Glucose and L-Glutamine supplemented with 10% v/v FCS (Sigma) and 100units/ml penicillin and 100units/ml streptomycin (Invitrogen) (GIN 8 were supplemented with 15 % v/v FCS). KNS-42 cells were cultured in DMEM/F-12 (1:1) (Sigma) supplemented with 10% (v/v) Fetal Calf Serum (FCS, Sigma), 2mM L-glutamine and 100units/ml penicillin and 100units/ml streptomycin (Invitrogen). All cell lines were cultured at 37oC and 5% CO2. Cell passage was carried out using 0.05% trypsin (Invitrogen).

**Nanoparticle delivery**

All particles used during the experiments were dextran coated Nanomag®-D MNPs (Fe3O4 core; 250nm; Micromod), unless differently stated MNPs used were COOH functionalized dextran coated Nanomag®-D MNPs. P21-8R and P21a-10 H were recombinantly made from expressing genes in bacteria and then purified. Cells were seeded into falcon 12 well tissue culture treated plates (200,000 cells/well, 500 μL/well; Scientific Laboratory Supplies) and incubated for 24 hours. After incubation cells were treated, with nothing (media exchange), 100 μg/mL MNPs, 100 μg/mL dMNPs + 1μM peptide (P21-8R or P21a-10H). Cells were incubated overnight.

**Prussian Blue Staining**

Cells were fixed for 20 mins with 4% paraformaldehyde (PFA; Sigma) at room temperature. Prussian blue

staining solution (potassium ferrocyanide in 2.5% hydrochloric acid, 25 mg/mL; Scientific Laboratory Supplies) was added to the cells and incubated for one hour. Stained cells were imaged using a Nikon Eclipse TS1000 light microscope.

**pH media**

Buffered Dulbecco's modified Eagle's media (DMEM; Sigma); Buffered Dulbecco's modified Eagle's media without sodium bicarbonate (DMEM; Sigma) and CO2 independent media (Invitrogen), all of them supplemented with 10% (v/v) Fetal Calf Serum (FCS, Sigma), 2mM L-glutamine and 100units/ml penicillin and 100units/ml streptomycin (Invitrogen) were adjusted to different pH by adding NaOH 0.1 M or HCl 0.1 M (Scientific supplies).

**Transmission electron microscopy (TEM)**

To confirm the cellular localization of the particles, samples were fixed in 3% glutaraldehyde in 0.1 M cacodylate buffer for one hour and post-fixed in 1% aqueous osmium tetroxide for 30 min. The samples were then dehydrated in a graded ethanol series and infiltrated with Transmit resin (TAAB, UK), then allowed to polymerize for 48h at 70oC. Semi-thin sections were cut (0.5 μm), using a Reichert-Jung ultramicrotome, and stained with 2% toluidine blue. Ultra-thin sections were cut (100 μm) using the same equipment and collected on copper grids, which were then contrasted using 50% methanol uranyl acetate and Reynolds lead citrate. Imaging was performed on a Tecnai 12 Biotwin TEM (FEI, USA) run at 100Kv.

**Inductively Coupled Plasma**

Inductively coupled plasma (ICP) was performed to quantify the uptake of particles by NIH 3t3s. MNP were delivered as described above. After incubation overnight, the supernatant was removed and cells were washed twice with PBS. Cells were trypsinized and collected in water. The cell suspension was incubated in 3.7% HCl for 2h at 70°C for the degradation of the particles in order to release the Fe content. Samples were then diluted in water in order to achieve a final acid concentration of less than 2%. A calibration curve was also produced at MNPs concentrations up to 50ug/ml to account for possible matrix effects.

Diluted solutions were analysed by ICP-MS (Thermo-Fisher Scientific iCAP-Q; Thermo Fisher Scientific, Bremen, Germany). Samples were introduced from an autosampler (Cetac ASX-520) incorporating an ASXpress™ rapid uptake module through a PEEK nebulizer (Burgener Mira Mist). Internal standards were introduced to the sample stream on a separate line via the ASXpress unit and included Ge (10 μg L-1), Rh (10 μg L-1) and Ir (5 μg L-1) in 2% trace analysis grade (Fisher Scientific, UK) HNO3. Fe External calibration standard (Claritas-PPT grade CLMS-2 from SPEX Certiprep Inc., Metuchen, NJ, USA), in the range 0 - 100 μg L-1 (0, 20, 40, 100 μg L-1). Phosphorus, boron and sulphur calibration utilized in-house standard solutions (KH2PO4, K2SO4 and H3BO3). A collision-cell (Q cell) using He with kinetic energy discrimination (He-cell) to remove polyatomic interferences was used to measure Fe. Sample processing was undertaken using Qtegra™ software (Thermo-Fisher Scientific). Results were reported back in ppb (ug/L). The concentration of Fe per cell was calculated for an estimated final cell density of 800 K cells per well which was based on the standard doubling time for NIH 3t3s of 24h.

**Adsorption and Adsorption isotherms**

Adsorption isotherms represent the adsorbed protein against the proteins concentration in solution. Samples with protein concentrations ranging from 0.04 to 40 mg/mL in PBS or FCS were divided into two sets: one with proteins only for reference and a second group with MNPs at a constant concentration of 50 ug/mL. Both sets were incubated in a rotar for 30 mins at 37oC. (Labarre, Pin, Renault, & Paris-sud, 2016) Kinetic data and isotherms were adjusted using non-linear least squares fitting on Excel.

**Zetasizer**

To study zeta potential and size of the MNPs and MNP-peptide complexes we used a Malvern zetasizer Nano ZS. Measurements consisted of 3 repeats (12-15 subruns per repeat) of the same sample to estimate the error in the measurements. The measurements were recorded at room temperature. Because zeta potential measurements were performed in an aqueous solution, the Smoluchowski approximation was used to calculate the zeta potentials from the measured electrophoretic motilities.

**Results and discussion**

***P218R enhances delivery in vitro***

In order to demonstrate that the intracellular delivery system enhanced MNP delivery, MNP was delivered to NIH 3t3s. Three different methods were used to determine delivery efficiency, Prussian Blue stain and Transmission Electron Microscopy (TEM) for qualitative study and Induced Coupled Plasma (ICP) for quantitative analysis. The iron oxide present in MNPs produces a blue precipitate in the Prussian blue reagent which was used to determine particle uptake. NIH 3t3 cells were incubated with 250 nm MNPs at 50 ug/mL (Figures 38 & 39) Cells incubated with MNP only presented some staining; suggesting low quantities of MNPs can be passively uptaken by the NIH 3t3s. MNPs delivered with P218R (Figures 38 & 39) peptide showed a significant enhancement in the staining of NIH 3t3s.

To further proof the efficiency of our system we repeated the same experiment in other cell lines. As discussed on the introduction magnetic nanoparticles are widely used in biomedicine and more specifically for the treatment and diagnostics of cancer, in order to focus on a particular application of MNPs, we investigated particle uptake on different cancer cell lines, more specifically we tested glioblastoma cell lines, GIN 8 were derived from a glioblastoma patient (primary cell line), KNS-42 a paediatric cell line and U87, an adult glioblastoma cell line. Based on Prussian Blue staining, all cell lines showed significantly increased delivery of MNPs when delivered with P218R compared to passive uptake (Figure 40).

TEM confirms internalization of 250 nm MNPs in vesicles on NIH 3t3s both with and without P218R (Figure 41). Consistently with Prussian blue, passive delivery of MNP seems to be significantly lower than enhanced delivery with P218R. In order to demonstrate the broad efficiency of our system we delivered 20 nm MNP (Figure 42). TEM results confirms uptake of different concentrations of particles. As with the 250 nm particles, these were localized within endosomes in the cytoplasm. TEM images show higher concentration of particles in the endosomes at the highest delivery concentration.

Previous literature has reported passive uptake of MNPs by different types of cell lines, most of these deliveries have been performed in serum free media. Other groups report poorer efficiency of their delivery systems in the presence of biological fluids. To investigate the effect of FCS on our delivery system we repeated the delivery experiments in the presence or absence of FCS and analysed iron uptake by Induced Coupled Plasma (ICP).

When delivery was carried out in the presence of FCS, ICP analysis confirmed Prussian Blue observations, when NIH 3t3s are incubated with MNP they passively uptake around 6% of the total amount of MNP delivered (4 pg Fe per cell), whereas when MNP are delivered with GET peptide, cell internalization of MNP increases to 51% (32 pg Fe per cell).

In the absence of FCS, passive uptake was significantly increased in comparison to FCS up to 22% (14 pg Fe per cell), uptake of MNP with P218R was similar to the previous experiment 45% (28 pg Fe per cell).

In order to demonstrate that our delivery system works on different substrates, we tested MNP coated with different functional groups such as $NH_2$, COOH-PEG and plain (no functional groups on the surface). Significantly improved delivery was observed when particles were delivered with P218R compared to passive uptake (Figure 39).

We have demonstrated that our delivery system is capable of efficiently deliver MNPs to a variety of cell types. TEM images show that up-taken MNPs are localized in endosomes suggesting that the internalization mechanism occurs through endocytosis. We have also demonstrated that irrespective on the functional groups present on the particle surface, P218R is capable of binding and enhancing dextran coated MNP intracellular delivery.

***P21a10OH pH responsive delivery in vitro***

This project is based on the enhanced delivery of MNP through the use of the GET system, so far we have demonstrated that we can efficiently deliver MNP in vitro, however, we wanted to tailor the system so we could make it more applicable to current biomedical issues. MNPs are excellent candidates for directly targeting tumoral tissues either for imaging or treatment purposes. Cancer tissues possess specific characteristics that make them distinguishable from healthy tissues, they present a more acidic environment than physiological tissues. We exploit this specific feature of cancer tissues to add specificity to the delivery system.

A new series of proteins were engineered starting from P218R to make it pH responsive, this means, the new peptides would only deliver at specific pH. Different domains of P218R were altered, arginine residues were replaced by histidines, when protonated histidines behave like lysines, this means that when protonated the pH inducible peptides should resemble the original P218R and present similar intracellular delivery activity.

Red fluorescent versions of different proteins presenting different permutations of lysines and histidines were screened for cell uptake at different pHs using flow cytometry. The most successful candidates were tested for in vitro delivery of MNP using a media that maintains the pH stable during the delivery (CO2-independent media culture; Invitrogen).

Overall, it can be observed that the pH inducible peptides show poor delivery at pH 7.5 and then progressively improve as pH decreases. Comparing Prussian Blue results with the sequence of histidines and lysines, there seems to be a trend that as a general rule, in proteins where histidines are grouped together or subunits of histidines are close together, delivery starts occurring at a higher pH, whereas when the histidines are isolated or far apart delivery occurs at a lower pH. This could be explained by a protonation theory, if we consider that we need a set of histidines together to start the delivery process it would make sense to assume that when histidines are together as the pH decreases, longer chains of amino acids become protonated, and delivery gets initiated. However when the histidines are separated it takes a higher concentration of protons to reach enough histidines to form a sufficient amount of lysines so that the protein becomes active.

After qualitatively demonstrating that we could deliver pH specifically in vitro we carried out some characterization work. In this next section, we wanted to investigate if the peptide inducibility affected not only the delivery but also the binding to the MNPs, in order to do so we conjugated one of the pH inducible versions of the peptide, P21a10H, to MNPs at different pHs ranging from 6 to 7.5 (same pHs as delivery experiments) (Figure 43).

Almost 100% of P21a10H was bound to MNPs at pH 6, whereas the percentage of bound peptide at pH 7.5 was lower than 40%. After washing the complex MNP-peptide with water at pH 7.5 the percentage of peptide that remained bound to the MNPs was similar through the whole pH range. Proteins 3D structure depends greatly on the different charges of the different domains, the spatial distribution of the different subunits of the protein will always look to accommodate all the different charges in the most stable configuration, so the whole protonation and deprotonation process is more complex than described above and does takes place over time. If the pH of the solution changes while a protein is still binding to the particle surface the different domains change their charge and their affinity for the particle surface can change. After being washed with PBS, there is almost not a change in the amount of protein bound at pH 7.5 but there is a significant decrease on protein adsorbed at all the other pHs (figure 44).

As said before a protein is not a uniform molecule and not all its domains present the same charge, so even if the overall charge of the protein might be positive or negative there are still going to be subunits with individual charges. These charges are going to be responsible for an initial protein adsorption onto any surface just based on local electrostatic interactions, the strength and kinetics of that binding will then be determined by the affinity between surface and protein. This explains why even if P21a10H should not be "active" at physiological pH, it still binds to the MNPs, although on a much lesser extent than its "activated" form at pH 6.

After conjugating MNP and peptide, and washing with PBS we delivered the complex to 3t3s at different pHs in order to test whether the preconjugated complex maintained pH inducibility (Figures 44, 45). We compared the results to MNP delivered with P21a10H without preconjugation and wash steps. MNPs were conjugated at pH 6 (acidic conditions) and pH 7.5 (physiological pH), then the particles were washed and delivered at pH 6 and pH 7.5.We also used P218R as a control. Prussian Blue stained images show that P218R is capable of efficiently delivering MNPs to the cells in vitro independent on the preconjugation pH (Figure 46) P21a10H was able to deliver at pH 6 irrespective of preconjugation pH or wash step (Figure 46), it is worth noting that the washing step seems to have an effect on efficiency which corresponds with the binding results obtained on figure 44, in which the amount of peptide bound to the MNP significantly decreased after washing. Finally P21a10H did not deliver at pH 7.5 independently of the conjugation and washing steps. These results suggest that temporary binding to MNPs does not affect the protein structure enough to hamper its delivery efficiency, as long as the protein is exposed to the right pH.

***P218R interaction with MNP***

Once the delivery system was demonstrated to improve MNP uptake, the interactions between MNP and peptide were studied in order to gain further understanding on mechanism of action and potential improvement of the delivery system. Our initial hypothesis was that GET peptide binds to the MNP and when the complex MNP-peptide reaches the cell membrane, then GET stimulates the uptake of the cargo, in this case the MNP. We wanted to test whether the same domain responsible for binding the MNPs surface was also playing a role in the internalization of the cargo, and whether the interaction with the MNPs long term could affect protein configuration to the point where it wouldn't be effective.

In order to test this hypothesis we first studied peptide adsorption onto MNPs, we compared a fluorescently labelled version of P218R (P21mR8R) and Red Fluorescent Protein (RFP) adsorption, to prove that adsorption was specific to the peptide and not due to the fluorescent tag. As observed on figure 47 the percentage of free P21mR8R decreases as MNP concentration increases, suggesting an interaction between the protein and the particles. The percentage of free RFP doesn't follow any particular pattern.

***-Zeta sizer results***

It could be argued that our P218R mechanism for cell delivery is based on its positive charge and its interaction with the negatively charged cell membrane and not so much on its interaction with the cell membrane as we hypothesized.

In order to further investigate this theory we analysed the zeta potential of the peptide-MNP complex. We also investigated the size of the complex. Because of MNP delivery was carried out in the presence of 10% FCS, zeta potential and size were studied in water, and increasing concentrations of FCS up to 10% (Figure 48).

MNPs are negatively charged at all concentrations of FCS (as expected from manufacturer specifications). We didn't investigate in depth why the negative charge of MNP became less negative as the concentration of FCS increased but this could be due to the adsorption of positively charged proteins present in serum to the particle surface. Interestingly, when MNP are conjugated with P218R in water, the zeta potential of the overall complex becomes positive, this again, provides evidence that the peptide does bind to the MNP, however when the conjugation takes place in the presence of serum, the charge of the complex becomes negative again. We hypothesized that this was due to the interaction of P218R with negatively charged proteins in FCS, such as Bovine Serum Albumin (BSA) (Figure 49).

In terms of size, there were not significant differences between the MNP on their own or MNP conjugated with P218R, except at 0.01% FCS.

- ***Langmuir isotherm***

One of the most common ways of characterizing protein behaviour is through the generation of adsorption isotherms, which represent the amount of protein adsorbed onto a surface at a given concentration of the free protein in the solvent. Several models have been used to represent protein adsorption processes, in the literature the most commonly used are the Langmuir model, together with Freundlich and Brunnauer-Emmet (BET) (Latour, 2014). The Langmuir model is based on the assumption that only a mononayer of non-interacting molecules is formed and that all the adsorption sites are equivalent. Freundlich model, describes monolayer adsorption on heterogeneous surfaces where different adsorption sites have different adsorption energies and adsorption rates. Finally the BET model provides a model for multilayer adsorption on different areas.

In this section, we have used an approximation to the Freundlich model (eq 1) because the fit tests revealed better fitting of the experimental results to this model than to the Langmuir model (figure50).

$$q_e = K_F C_e^{\frac{1}{n}} \qquad \text{(eq 1)}$$

Freundlich equation is an empirical equation where $K_F$ is an indicator of adsorption capacity, the higher $K_F$ the higher the adsorption capacity. 1/n is an indicator of intensity of adsorption, the higher 1/n the more favourable is the adsorption, in other words, the higher n the less favourable is the absorption. Protein adsorption is a complex process that involves various different steps and this makes it very difficult to develop a model that could account for all the different phenomena and are therefore a simple empirical equation such as Freundlich equation is unsuitable for accurately describing the adsorption mechanism, although it is a good starting point and provides us with estimates from which we can infer initial theories on how the system works. The values obtained for the different constants in the different solvents are displayed on table 1.

**Table 1** Parameters of Freundlich Model used for fitting the adsorption isotherms: adsorption capacity ($K_F$) and intensity of adsorption (n) for P21mR8R binding to MNP in each different solvent.

| Solvent | $K_F$ | n |
|---|---|---|
| **PBS** | 37.83 | 3.95 |
| **FCS** | 0.73 | 1.748 |

The results obtained suggest that the maximum adsorption capacity is achieved in PBS; however, surprisingly the most favourable adsorption was obtained FCS, when theoretically we would expect a most favourable adsorption onto the particles in PBS due to the absence of other molecules in the media that could hamper the adsorption process. As already mentioned before, protein adsorption onto MNPs occurs mainly through electrostatic interactions. It is known that the main component in FCS is albumin; this biomolecule has an overall negative charge. The GET peptide has a net positive charge (confirmed by zeta sizer results), whereas MNP are negatively charged. We hypothesized that GET peptide positive charge gets stabilized by its interactions with negatively charged albumin and the negatively charged particles. On a very simple way, this theory would explain why in PBS despite the higher adsorption capacity of the MNP, the intensity if adsorption is lower, due to the lack of stabilization of the complex. If that was the case, then these results would match with other experimental data, such as high efficiency of delivery in FCS, when other intracellular delivery systems report poorer efficiency in the presence of biological fluids.

Proteins are big molecules with multiple domains that present different charges, these domains are going to be responsible for the interactions with the MNP surface. Once the protein is adsorbed onto a surface it tends to undergo surface induced conformational changes to increase the contact area in order to increase its stability, once this occurs, the protein tends to become "irreversibly bound" to the surface. To test whether the protein suffered significant structural changes when adsorbed onto the particles such that it would no longer be able to act as a delivery factor, we carried out an experiment in which we pre-conjugated the protein to the particles for different amounts of time before delivering to cells and then tested iron uptake by ICP. The results in table 2 indicate a significant decrease on delivery efficiency of the peptide after 24 hours of conjugation with MNPs. These could suggest that protein adsorption onto MNPs does lead to a permanent reconfiguration of the protein that then loses its activity, whether the changes in the protein affect only the binding domain and this is the same one that triggers cell uptake or whether structural changes affect the whole protein still remain unclear. Moreover, other factors such as degradation of the protein could account for these results.

| Pre-conjugation time (hr) | Fe uptake (pg/cell) |
|---|---|
| 0.2 | 31.8 |
| 4 | 31.6 |
| 24 | 19.0 |

**Table 2** Fe uptake in NIH 3t3 after delivery of MNPs pre-conjugated with P218R for 0.2, 4 and 24 hours. Pre-conjugation was carried out in 10% FCS under continuous agitation at 37°C

**Conclusion**

The GET intracellular delivery system enhances MNPs uptake in different cell lines in vitro compared to passive uptake. We have characterized the systems interactions with MNPs and demonstrated that unlike other delivery system, this is more stable in biological fluids and hence doesn't lose its activity in the presence of other biomolecules.

We have also demonstrated that a MNP pH responsive delivery system is attainable by modification of P218R. This study examined a pH range between 6.0 and 7.5, trying to mimic a broad range of tumorigenic tissues (Griffiths, 1991). We demonstrated that P21a10H was pH inducible in this range of pH, enhancing MNP uptake on NIH 3t3s in vitro compared to MNPs passive uptake. The characterization of the system revealed that irrespective of the preconjugation steps P21a10H always maintained his specificity.

These findings demonstrate that, the application of a combined GET-MNP system could be used in biomedicine, given its high delivering efficiency, and its stability and activity in a biological like environment.

## Claims

1. A pH mediated cell delivery vehicle comprising:

   - a cargo or a cargo-binding molecule for binding to a cargo;
   - a protein transduction domain; and
   - a glycosaminoglycan (GAG) binding element, which is capable of binding to GAG on the surface of the cell, wherein the GAG binding element is a peptide which is modified to comprise one or more histidine residues which are capable of being protonated in an acidic environment.

2. The pH mediated cell delivery vehicle according to claim 1, wherein the pH mediated cell delivery vehicle comprises a cargo-binding molecule, and wherein the cargo is bound to the cargo-binding molecule.

3. The pH mediated cell delivery vehicle according to any preceding claim, wherein the GAG binding element comprises a heparan sulphate glycosaminoglycan (HS-GAG) binding element, which is capable of binding to HS-GAG on the surface of the cell, and which has been modified such that one or more lysine residues of the wild-type molecule have been substituted with histidine residues.

4. The pH mediated cell delivery vehicle according to any preceding claim, wherein the GAG binding element comprises the amino acid sequence KRKKKGKGLGKKRDPCLRKYK (P21) (SEQ ID NO. 1), or a variant having at least 80% identity with SEQ ID NO: 1, which has been modified such that one or more lysine residues have been substituted with histidine residues.

5. The pH mediated cell delivery vehicle according to any preceding claim, wherein the GAG binding element comprises the amino acid sequence HRHHHGHGLGKKRDPCLRKYK (P21Nacid) (SEQ ID NO: 11); or KRKKKGKGLGHHRDPCLRHYH (P21Cacid) (SEQ ID NO: 12); or KRHKHGKGLGHKRDPCLRHYK (P21Eacid) (SEQ ID NO: 13); or HRKHKGHGLGKHRDPCLRKYH (P21Oacid) (SEQ ID NO: 14); or HRHHHGHGLGHHRDPCLRHYH (P21acid / P21a) (SEQ ID NO: 15);

6. The pH mediated cell delivery vehicle according to any one of claims 1 to 3, wherein the GAG binding element comprises the amino acid sequence G R P R E S G K K R K R K R L K P T (PDGF, SEQ ID NO. 16), or a variant having at least 80% identity with SEQ ID NO: 16, which has been modified such that at least one lysine residue has been substituted with a histidine residue; or wherein the GAG binding element comprises the amino acid sequence T Y A S A K W T H N G G E M F V A L N Q ((FGF7, HBD B) SEQ ID NO. 17), or a variant having at least 80% identity with SEQ ID NO: 17, wherein the lysine residue is substituted with a histidine residue; or wherein the GAG binding element comprises the amino acid sequence Y A S A H W T H N G G E M F V A L N Q ((FGF7, HBD B) SEQ ID NO. 18) or a variant having at least 80% identity with SEQ ID NO: 18; or wherein the GAG binding element comprises the amino acid sequence T Y R S R K Y T S W Y V A L K R (FGF2 HBD B SEQ ID NO. 19); or T Y R S R H Y T S W Y V A L K R (FGF2 HBD B SEQ ID NO. 20); or T Y R S R K Y T S W Y V A L H R (FGF2 HBD B SEQ ID NO. 21); or T Y R S R H Y T S W Y V AL H R (FGF2 HBD B SEQ ID NO. 22); or a variant having at least 80% identity to SEQ ID NO. 19, wherein one or both lysine residues are substituted with a histidine residue.

7. The pH mediated cell delivery vehicle according to any preceding claim, wherein the protein transduction domain is hydrophilic or amphiphilic.

8. The pH mediated cell delivery vehicle according to any preceding claim, wherein the protein transduction domain comprises a poly-arginine.

9. The pH mediated cell delivery vehicle according to any one of claims 1 to 6, wherein the protein transduction domain comprises a poly-histidine.

10. The pH mediated cell delivery vehicle according to any preceding claim, wherein the cargo is an anti-tumour agent.

11. A pH mediated delivery vehicle for use in treating acidic pH tumours, wherein the pH mediated delivery vehicle is arranged to be activated by an acidic environment of the tumour, and wherein the delivery vehicle comprises

- a cargo comprising an anti-tumour therapeutic agent;
- a protein transduction domain; and
- a glycosaminoglycan (GAG) binding element, which is capable of binding to GAG on the surface of the cell, wherein the GAG binding element is a peptide which is modified to comprise one or more histidine residues which are capable of being protonated in an acidic environment.

**12.** A pH mediated delivery vehicle for use in the targeted delivery of a cargo into acidic pH tumours, wherein the pH mediated delivery vehicle is arranged to be activated by an acidic environment of the tumour, and wherein the delivery vehicle comprises

- a cargo;
- a protein transduction domain; and
- a glycosaminoglycan (GAG) binding element, which is capable of binding to GAG on the surface of the cell, wherein the GAG binding element is a peptide which is modified to comprise one or more histidine residues which are capable of being protonated in an acidic environment.

**13.** A method of pH mediated cell delivery *in vitro* comprising:

- providing cells *in vitro;*
- exposing the cells to the pH mediated delivery vehicle according to any one of claims 1 to 10;
- lowering the pH environment of the cells, such that the one or more lysine residues of the pH mediated delivery vehicle are protonated, thereby activating the pH mediated delivery vehicle for intracellular uptake/delivery.

**14.** A nucleic acid encoding the delivery vehicle according to claims 1 to 10.

**15.** A pharmaceutical composition comprising a pH mediated delivery vehicle according to claims 1 to 10, and a pharmaceutically acceptable excipient.

## Patentansprüche

**1.** pH-vermitteltes Zelllabgabevehikel, umfassend:

- eine Fracht oder ein frachtbindendes Molekül zur Bindung an eine Fracht;
- eine Proteintransduktionsdomäne; und
- ein Glykosaminoglykan (GAG) bindendes Element, das an GAG auf der Oberfläche der Zelle binden kann, wobei das GAG bindende Element ein Peptid ist, das so modifiziert ist, dass es einen oder mehrere Histidinreste umfasst, die in einer sauren Umgebung protoniert werden können.

**2.** pH-vermitteltes Zellabgabevehikel nach Anspruch 1, wobei das pH-vermittelte Zellabgabevehikel ein Fracht bindendes Molekül umfasst und wobei die Fracht an das Fracht bindende Molekül gebunden ist.

**3.** pH-vermitteltes Zellabgabevehikel nach einem vorhergehenden Anspruch, wobei das GAG bindende Element ein Heparansulfat-Glykosaminoglykan (HS-GAG) bindendes Element umfasst, das an HS-GAG auf der Oberfläche der Zelle binden kann und das so modifiziert wurde, dass ein oder mehrere Lysinreste des Wildtyp-Moleküls durch Histidinreste ersetzt wurden.

**4.** pH-vermitteltes Zellabgabevehikel nach einem vorhergehenden Anspruch, wobei das GAG bindende Element die Aminosäuresequenz KRKKKGKGLGKKRDPCLRKYK (P21) (SEQ ID NO. 1) oder eine Variante mit mindestens 80 % Identität mit SEQ ID NO: 1 umfasst, die so modifiziert wurde, dass ein oder mehrere Lysinreste durch Histidinreste ersetzt wurden.

**5.** pH-vermitteltes Zellabgabevehikel nach einem vorhergehenden Anspruch, wobei das GAG bindende Element die Aminosäuresequenz HRHHHGHGLGKKRDPCLRKYK (P21NSäure) (SEQ ID NO: 11) umfasst; oder
KRKKKGKGLGHHRDPCLRHYH (P21CSäure) (SEQ ID NO: 12); oder
KRHKHGKGLGHKRDPCLRHYK (P21ESäure) (SEQ ID NO: 13); oder
HRKHKGHGLGKHRDPCLRKYH (P21OSäure) (SEQ ID NO: 14); oder
HRHHHGHGLGHHRDPCLRHYH (P21Säure/P21a) (SEQ ID NO: 15);

6. pH-vermitteltes Zellabgabevehikel nach einem der Ansprüche 1 bis 3, wobei das GAG bindende Element die Aminosäuresequenz GRPRESGKKRKRKRLKPT (PDGF, SEQ ID NO: 16) oder eine Variante mit mindestens 80 % Identität mit SEQ ID NO: 16 umfasst, die so modifiziert wurde, dass mindestens ein Lysinrest durch einen Histidinrest ersetzt wurde; oder
wobei das GAG bindende Element die Aminosäuresequenz TYASAKWTHNGGEMFVALNQ ((FGF7, HBD B) SEQ ID NO. 17) oder eine Variante mit mindestens 80 % Identität mit SEQ ID NO: 17 umfasst, wobei der Lysinrest durch einen Histidinrest ersetzt wurde; oder
wobei das GAG bindende Element die Aminosäuresequenz YASAHWTHNGGEMFVALNQ ((FGF7, HBD B) SEQ ID NO. 18) oder eine Variante mit mindestens 80 % Identität mit SEQ ID NO: 18 umfasst; oder
wobei das GAG bindende Element die Aminosäuresequenz TYRSRKYTSWYVALKR (FGF2 HBD B SEQ ID NO. 19) umfasst; oder
TYRSRHYTSWYVALKR (FGF2 HBD B SEQ ID NO. 20); oder
TYRSRKYTSWYVALHR (FGF2 HBD B SEQ ID NO. 21); oder
TYRSRHYTSWYVALHR (FGF2 HBD B SEQ ID NO. 22) oder eine Variante mit mindestens 80 % Identität mit SEQ ID NO: 19 umfasst, wobei ein oder beide Lysinreste durch einen Histidinrest ersetzt sind.

7. pH-vermitteltes Zellabgabevehikel nach einem vorhergehenden Anspruch, wobei die Proteintransduktionsdomäne hydrophil oder amphiphil ist.

8. pH-vermitteltes Zellabgabevehikel nach einem vorhergehenden Anspruch, wobei die Proteintransduktionsdomäne ein Polyarginin umfasst.

9. pH-vermitteltes Zellabgabevehikel nach einem der Ansprüche 1 bis 6, wobei die Proteintransduktionsdomäne ein Polyhistidin umfasst.

10. pH-vermitteltes Zellabgabevehikel nach einem vorhergehenden Anspruch, wobei die Fracht ein Anti-Tumor-Mittel ist.

11. pH-vermitteltes Abgabevehikel zur Verwendung bei der Behandlung von Tumoren mit saurem pH-Wert, wobei das pH-vermittelte Abgabevehikel so angeordnet ist, dass es durch eine saure Umgebung des Tumors aktiviert wird, und wobei das Abgabevehikel Folgendes umfasst

- eine Fracht, die ein Anti-Tumor-Therapeutikum umfasst;
- eine Proteintransduktionsdomäne; und
- ein Glykosaminoglykan (GAG) bindendes Element, das an GAG auf der Oberfläche der Zelle binden kann, wobei das GAG bindende Element ein Peptid ist, das so modifiziert ist, dass es einen oder mehrere Histidinreste umfasst, die in einer sauren Umgebung protoniert werden können.

12. pH-vermitteltes Abgabevehikel zur Verwendung bei der gezielten Abgabe einer Fracht an Tumore mit saurem pH-Wert, wobei das pH-vermittelte Abgabevehikel so angeordnet ist, dass es durch eine saure Umgebung des Tumors aktiviert wird, und wobei das Abgabevehikel Folgendes umfasst

- eine Fracht;
- eine Proteintransduktionsdomäne; und
- ein Glykosaminoglykan (GAG) bindendes Element, das an GAG auf der Oberfläche der Zelle binden kann, wobei das GAG bindende Element ein Peptid ist, das so modifiziert ist, dass es einen oder mehrere Histidinreste umfasst, die in einer sauren Umgebung protoniert werden können.

13. Verfahren zur pH-vermittelten Zellabgabe *in vitro,* umfassend:

- Bereitstellen von Zellen *in vitro;*
- Aussetzen der Zellen dem pH-vermittelten Abgabevehikel nach einem der Ansprüche 1 bis 10;
- Senken der pH-Umgebung der Zellen, sodass ein oder mehrere Lysinreste des pH-vermittelten Abgabevehikels protoniert werden, wodurch das pH-vermittelte Abgabevehikel für eine intrazelluläre Aufnahme/Abgabe aktiviert wird.

14. Nukleinsäure, die für das Abgabevehikel nach den Ansprüchen 1 bis 10 kodiert.

15. Pharmazeutische Zusammensetzung, umfassend ein pH-vermitteltes Abgabevehikel nach den Ansprüchen 1 bis

10 und einen pharmazeutisch unbedenklichen Hilfsstoff.

**Revendications**

1. Véhicule d'administration de cellules médiée par le pH comprenant :

   - une molécule cargo ou une molécule de liaison à une molécule cargo destinée à se lier à une molécule cargo ;
   - un domaine de transduction de protéine ; et
   - un élément de liaison au glycosaminoglycane (GAG), qui peut se lier au GAG à la surface de la cellule, dans lequel l'élément de liaison au GAG est un peptide qui est modifié pour comprendre un ou plusieurs résidus histidine qui peuvent être protonés dans un environnement acide.

2. Véhicule d'administration de cellules médiée par le pH selon la revendication 1, le véhicule d'administration de cellules médiée par le pH comprenant une molécule de liaison à une molécule cargo et dans lequel la molécule cargo est liée à la molécule de liaison à une molécule cargo.

3. Véhicule d'administration de cellules médiée par le pH selon l'une quelconque des revendications précédentes, dans lequel l'élément de liaison au GAG comprend un élément de liaison au glycosaminoglycane de sulfate d'héparane (HS-GAG), qui peut se lier au HS-GAG à la surface de la cellule, et qui a été modifié de sorte qu'un ou plusieurs résidus lysine de la molécule de type sauvage ont été substitués par des résidus histidine.

4. Véhicule d'administration de cellules médiée par le pH selon l'une quelconque des revendications précédentes, dans lequel l'élément de liaison au GAG comprend la séquence d'acides aminés KRKKKGKGLGKKRDPCLRKYK (P21) (SEQ ID NO. 1), ou une variante ayant au moins 80 % d'identité avec la SEQ ID NO: 1, qui a été modifiée de sorte qu'un ou plusieurs résidus lysine ont été substitués par des résidus histidine.

5. Véhicule d'administration de cellules médiée par le pH selon l'une quelconque des revendications précédentes, dans lequel l'élément de liaison au GAG comprend la séquence d'acides aminés HRHHHGHGLGKKRDPCLRKYK (P21Nacide) (SEQ ID NO: 11); ou KRKKKGKGLGHHRDPCLRHYH (P21Cacide) (SEQ ID NO: 12) ; ou KRHKHGKGLGHKRDPCLRHYK (P21 Eacide) (SEQ ID NO: 13) ; ou HRKHKGHGLGKHRDPCLRKYH (P210acide) (SEQ ID NO: 14) ; ou HRHHHGHGLGHHRDPCLRHYH (P21acide / P21a) (SEQ ID NO: 15).

6. Véhicule d'administration de cellules médiée par le pH selon l'une quelconque des revendications 1 à 3, dans lequel l'élément de liaison au GAG comprend la séquence d'acides aminés G R P R E S G K K R K R K R L K P T (PDGF, SEQ ID NO. 16), ou une variante ayant au moins 80 % d'identité avec la SEQ ID NO: 16, qui a été modifiée de sorte qu'au moins un résidu lysine a été substitué par un résidu histidine ; ou
   dans lequel l'élément de liaison au GAG comprend la séquence d'acides aminés T Y A S A K W T H N G G E M F V A L N Q ((FGF7, HBD B) SEQ ID NO. 17), ou une variante ayant au moins 80 % d'identité avec la SEQ ID NO: 17, dans lequel le résidu lysine est substitué par un résidu histidine ; ou
   dans lequel l'élément de liaison au GAG comprend la séquence d'acides aminés Y A S A H W T H N G G E M F V A L N Q ((FGF7, HBD B) SEQ ID NO. 18) ou une variante ayant au moins 80 % d'identité avec la SEQ ID NO: 18 ; ou
   dans lequel l'élément de liaison au GAG comprend la séquence d'acides aminés T Y R S R K Y T S W Y V A L K R (FGF2 HBD B SEQ ID NO. 19) ; ou
   T Y R S R H Y T S W Y V A L K R (FGF2 HBD B SEQ ID N. 20); ou
   T Y R S R KY T S W Y V A L H R (FGF2 HBD B SEQ ID N. 21) ; ou
   T Y R S R H Y T S W Y V A L H R (FGF2 HBD B SEQ ID N. 22) ; ou une variante ayant au moins 80 % d'identité avec la SEQ ID NO. 19, dans lequel un résidu lysine ou les deux résidus lysine sont substitués par un résidu histidine.

7. Véhicule d'administration de cellules médiée par le pH selon l'une quelconque des revendications précédentes, dans lequel le domaine de transduction de protéine est hydrophile ou amphiphile.

8. Véhicule d'administration de cellules médiée par le pH selon l'une quelconque des revendications précédentes, dans lequel le domaine de transduction de protéine comprend une polyarginine.

9. Véhicule d'administration de cellules médiée par le pH selon l'une quelconque des revendications 1 à 6, dans lequel le domaine de transduction de protéine comprend une polyhistidine.

**10.** Véhicule d'administration de cellules médiée par le pH selon l'une quelconque des revendications précédentes, dans lequel la molécule cargo est un agent antitumoral.

**11.** Véhicule d'administration médiée par le pH destiné à être utilisé dans le traitement de tumeurs à pH acide, le véhicule d'administration médiée par le pH étant conçu pour être activé par un environnement acide de la tumeur, et le véhicule d'administration comprenant

- une molécule cargo comprenant un agent thérapeutique antitumoral ;
- un domaine de transduction de protéine ; et
- un élément de liaison au glycosaminoglycane (GAG), qui peut se lier au GAG à la surface de la cellule, dans lequel l'élément de liaison au GAG est un peptide qui est modifié pour comprendre un ou plusieurs résidus histidine qui peuvent être protonés dans un environnement acide.

**12.** Véhicule d'administration médiée par le pH destiné à être utilisé dans l'administration ciblée d'une molécule cargo dans des tumeurs à pH acide, le véhicule d'administration médiée par le pH étant conçu pour être activé par un environnement acide de la tumeur, et le véhicule d'administration comprenant

- une molécule cargo ;
- un domaine de transduction de protéine ; et
- un élément de liaison au glycosaminoglycane (GAG), qui peut se lier au GAG à la surface de la cellule, dans lequel l'élément de liaison au GAG est un peptide qui est modifié pour comprendre un ou plusieurs résidus histidine qui peuvent être protonés dans un environnement acide.

**13.** Procédé d'administration de cellules médiée par le pH *in vitro* comprenant :

- la fourniture de cellules *in vitro ;*
- l'exposition des cellules au véhicule d'administration médiée par le pH selon l'une quelconque des revendications 1 à 10 ;
- la réduction de l'environnement de pH des cellules, de sorte que le ou les résidus lysine du véhicule d'administration médiée par le pH sont protonés, activant ainsi le véhicule d'administration médiée par le pH pour l'absorption/l'administration intracellulaire.

**14.** Acide nucléique codant pour le véhicule d'administration selon les revendications 1 à 10.

**15.** Composition pharmaceutique comprenant un véhicule d'administration médiée par le pH selon les revendications 1 à 10, et un excipient pharmaceutiquement acceptable.

A

| | CO2 independent media | | Buffered | | Non-buffered | |
|---|---|---|---|---|---|---|
| | non-CO2 | CO2 | non-CO2 | CO2 | non-CO2 | CO2 |
| 7.6 | 7.345 | 7.082 | 8.383 | 7.708 | 7.598 | 7.292 |
| 7.4 | 7.298 | 7.045 | 8.383 | 7.709 | 7.67 | 7.11 |
| 7.2 | 7.134 | 6.971 | 8.175 | 7.607 | 7.083 | 7.022 |
| 7 | 7.049 | 6.972 | 8.161 | 7.584 | 7.144 | 7.052 |
| 6.8 | 6.874 | 6.763 | 8.042 | 7.522 | 7.503 | 7.079 |
| 6.6 | 6.796 | 6.642 | 7.817 | 7.422 | 7.369 | 7.045 |

B

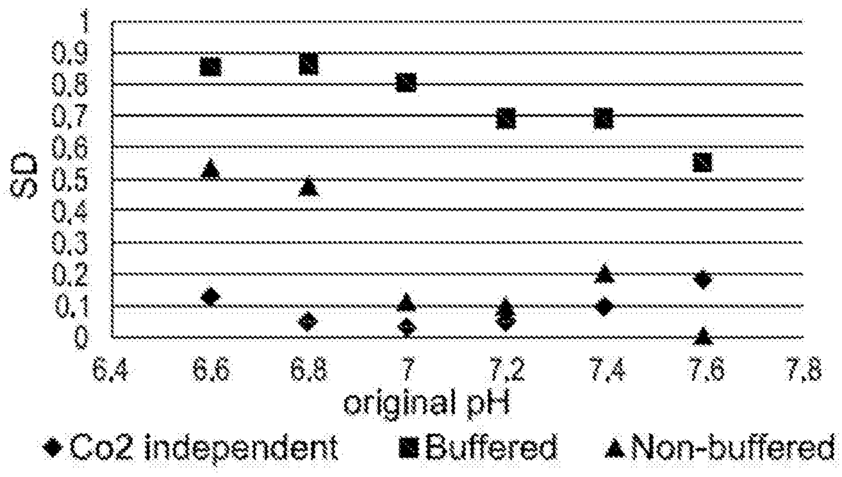


C

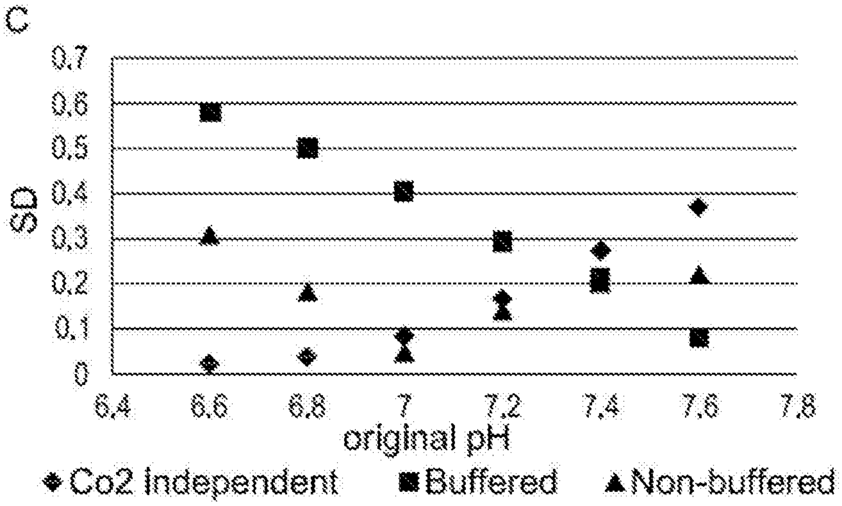


Figure 1

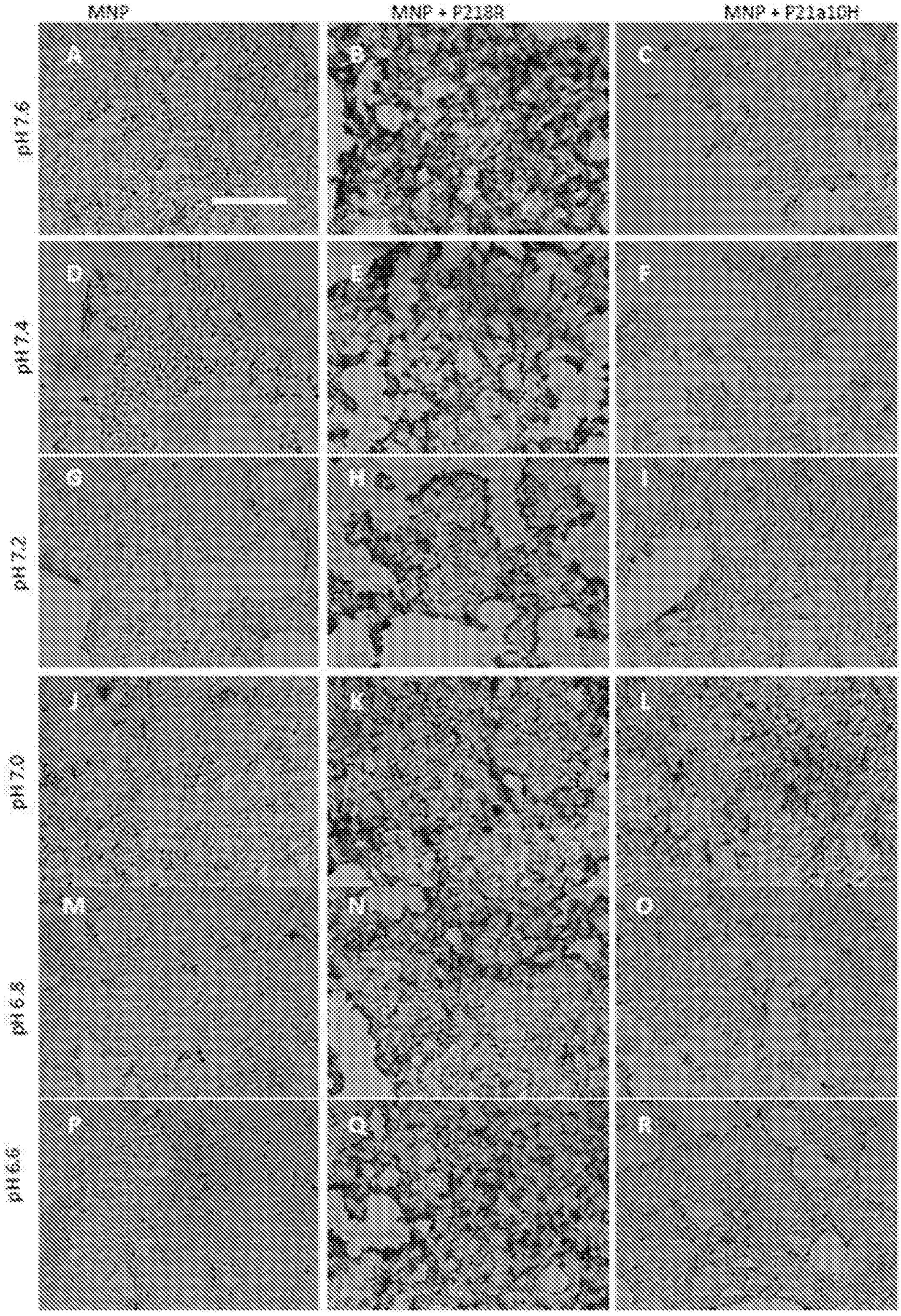
MNP
MNP + P21a10H
pH 7.6
pH 7.4
pH 7.2
pH 7.0
pH 6.8
pH 6.6
A
B
C
D
E
F
G
H
I
J
K
L
M
N
O
P
Q
R

Figure 2

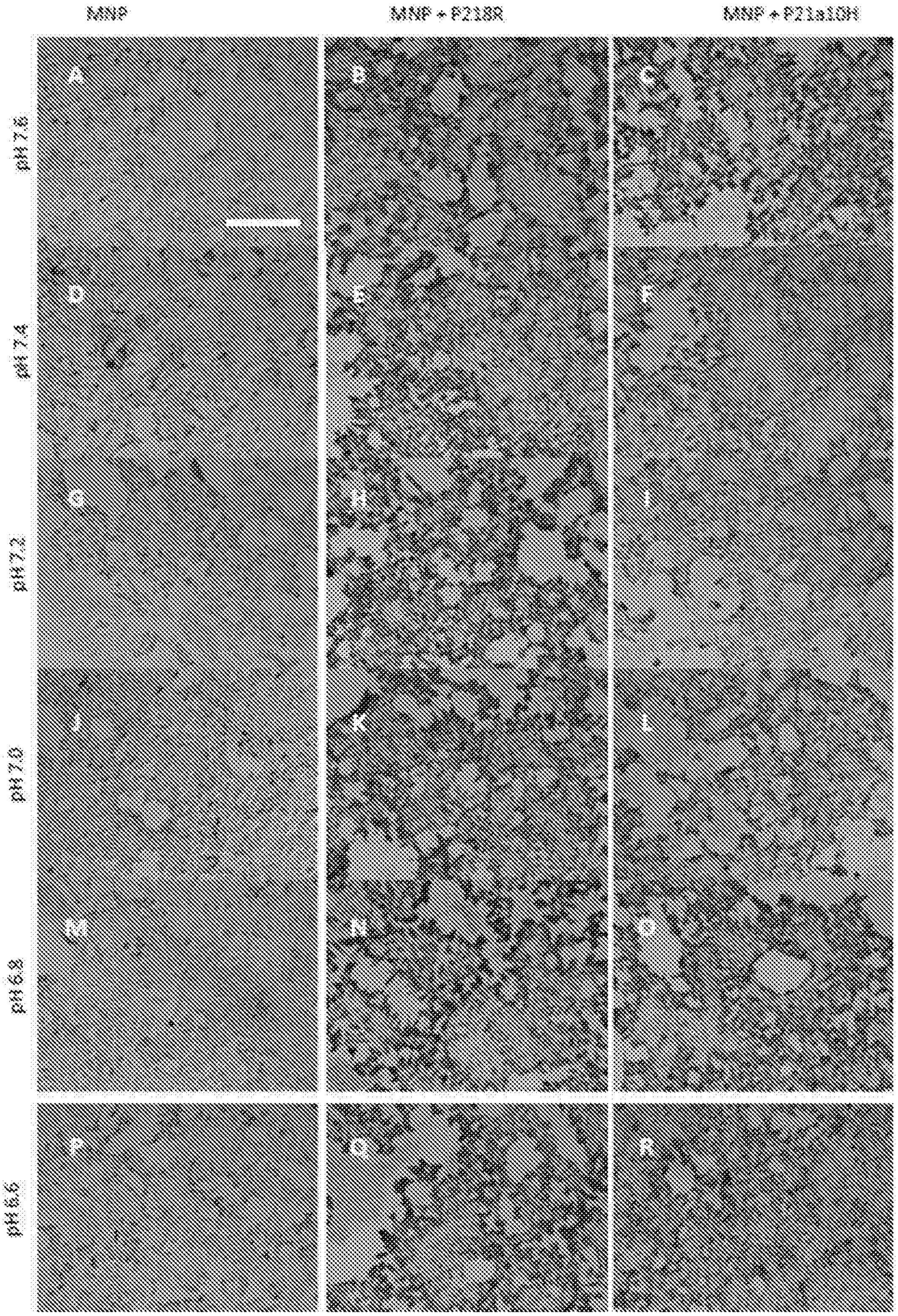
MNP
MNP + P21SR
MNP + P21a10H
pH 7.6
pH 7.4
pH 7.2
pH 7.0
pH 6.8
pH 6.6
A
B
C
D
E
F
G
H
I
J
K
L
M
N
O
P
Q
R

Figure 3

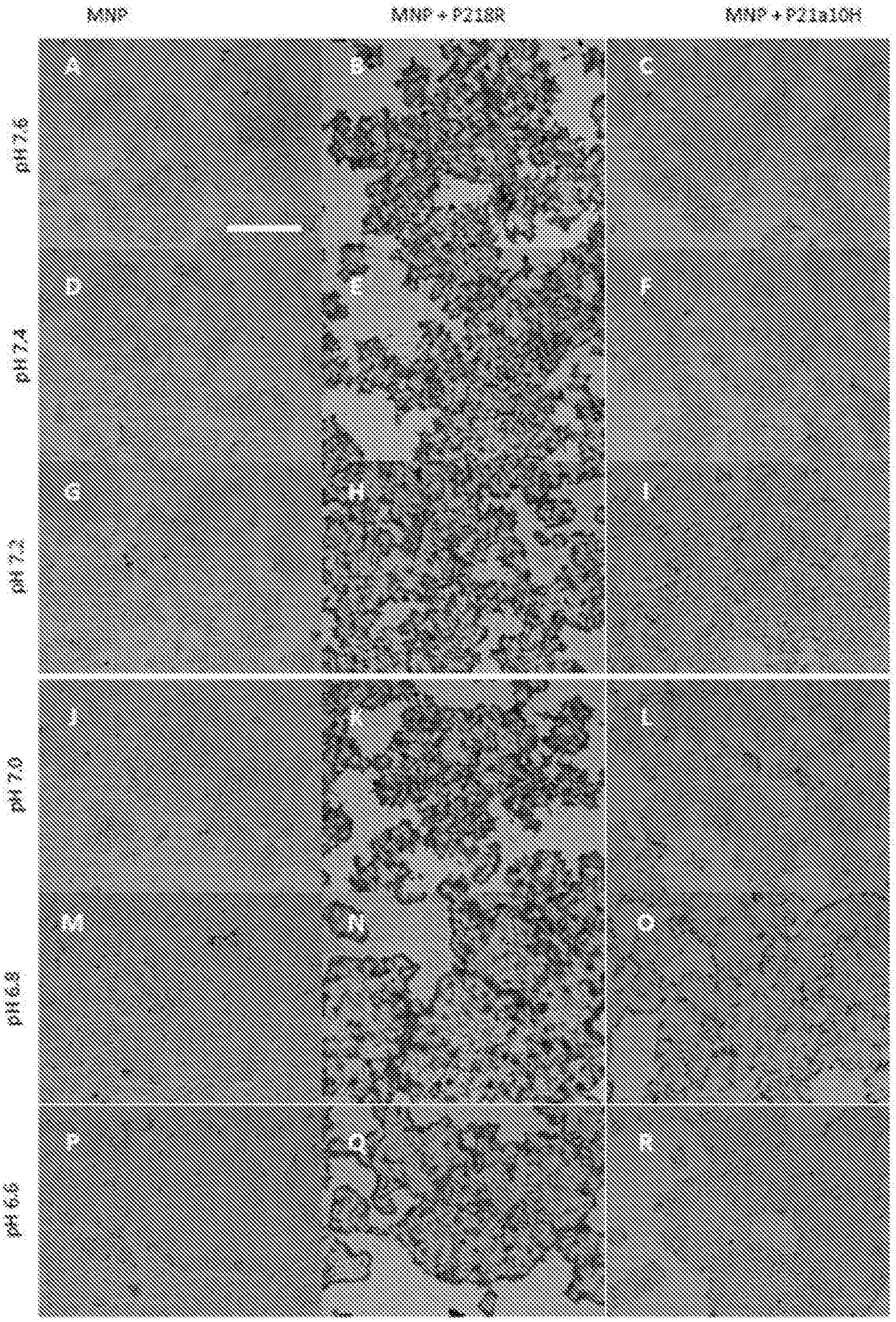
MNP
MNP + P218R
MNP + P21a10H
A
B
C
pH 7.6
D
E
F
pH 7.4
G
H
I
pH 7.3
J
K
L
pH 7.0
M
N
O
pH 6.9
P
Q
R
pH 6.6

Figure 4

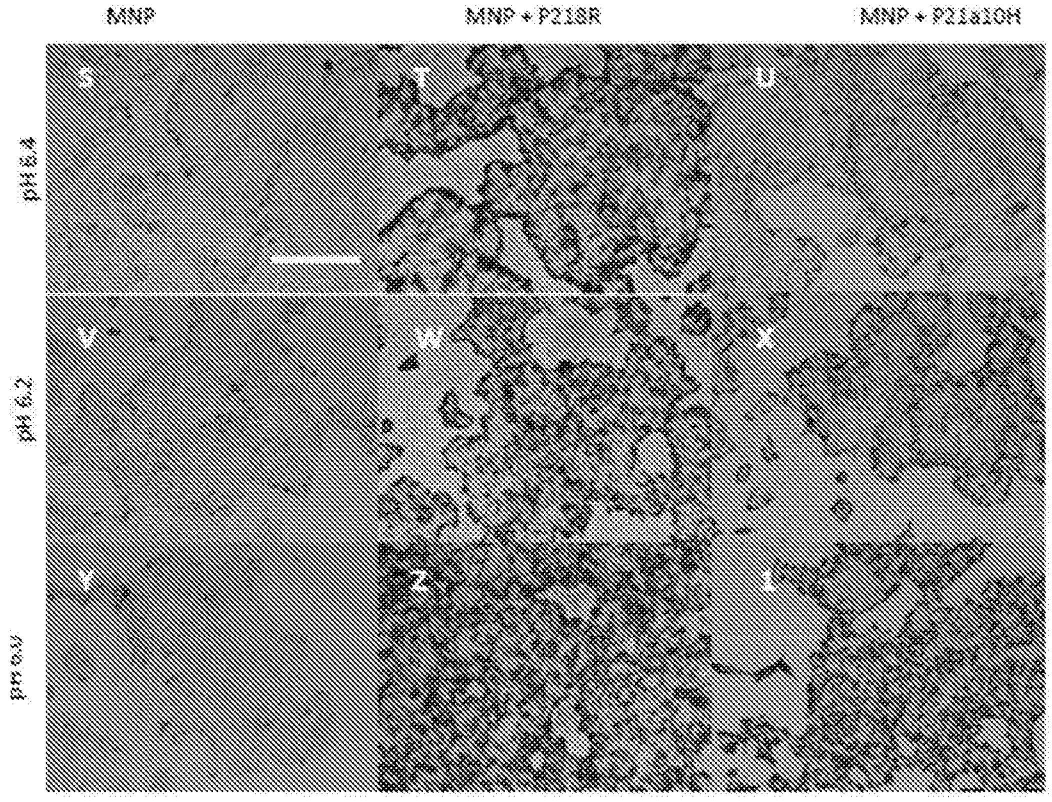


Figure 4 continued

Figure 5

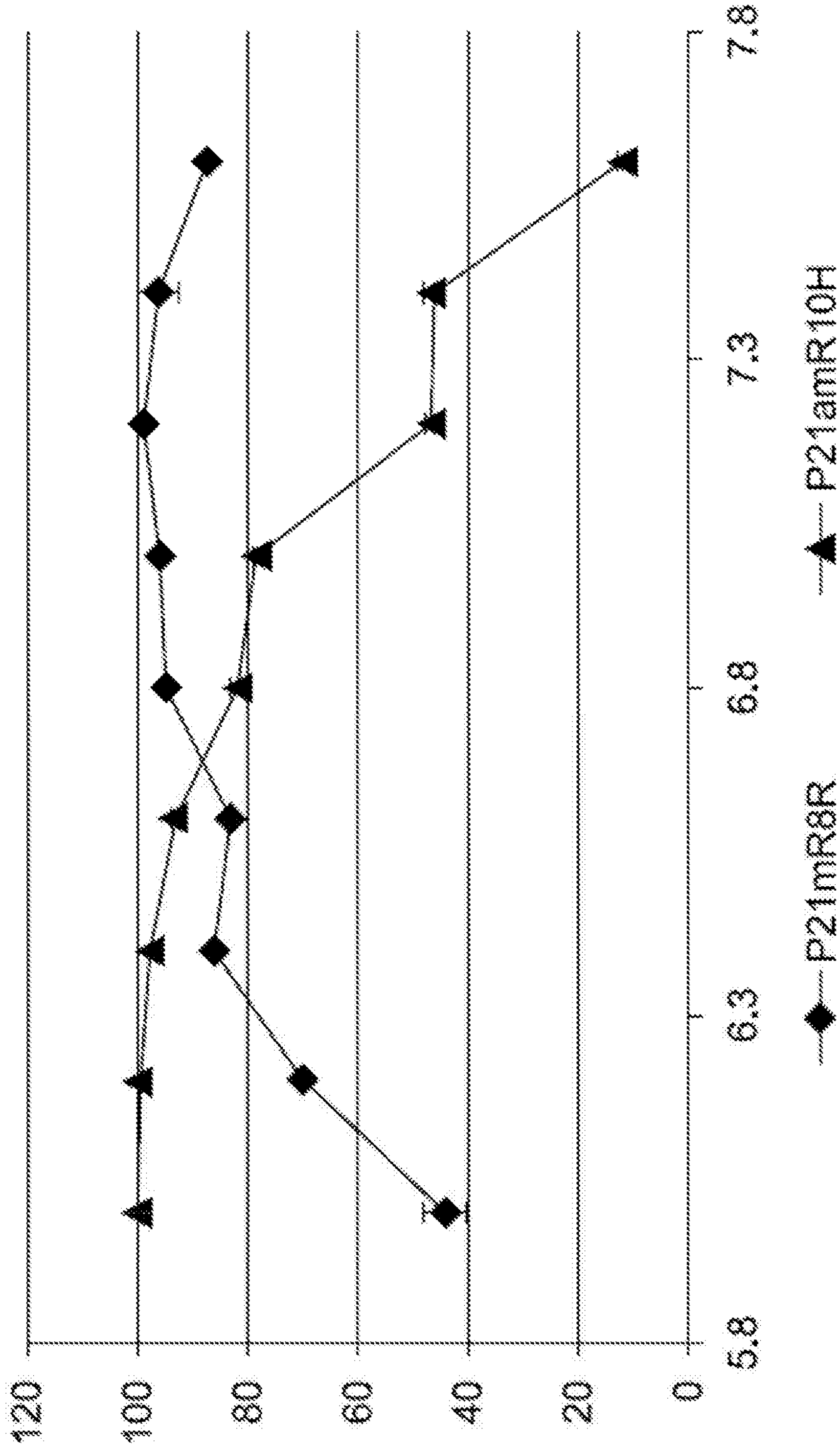

Figure 6

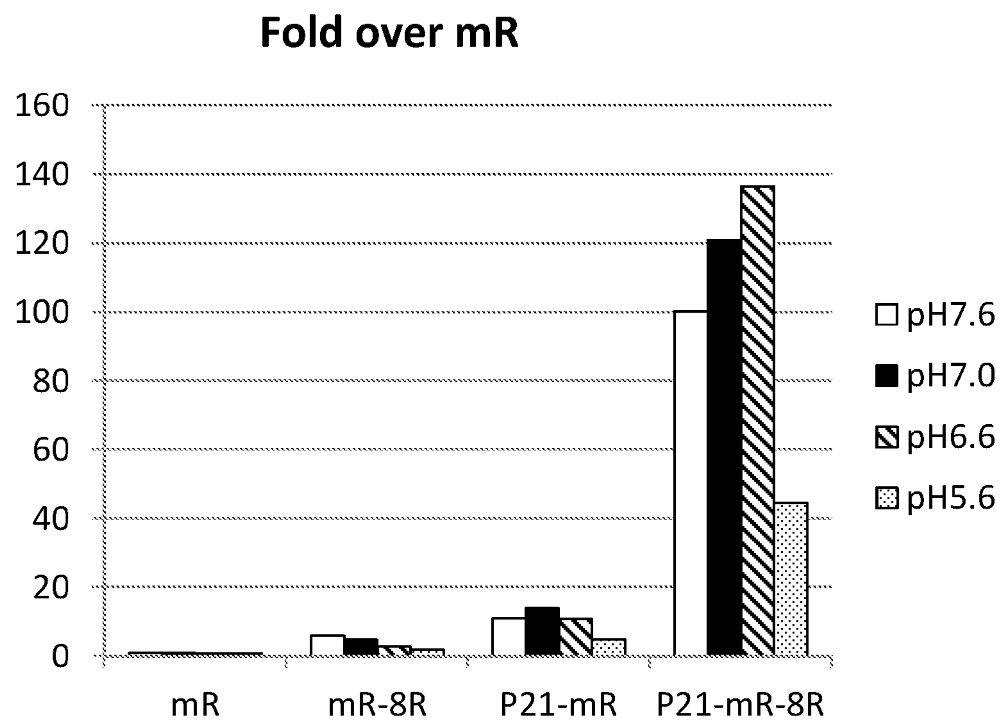
Fold over mR
160
140
120
100
80
60
40
20
0
mR
mR-8R
P21-mR
P21-mR-8R
pH7.6
pH7.0
pH6.6
pH5.6

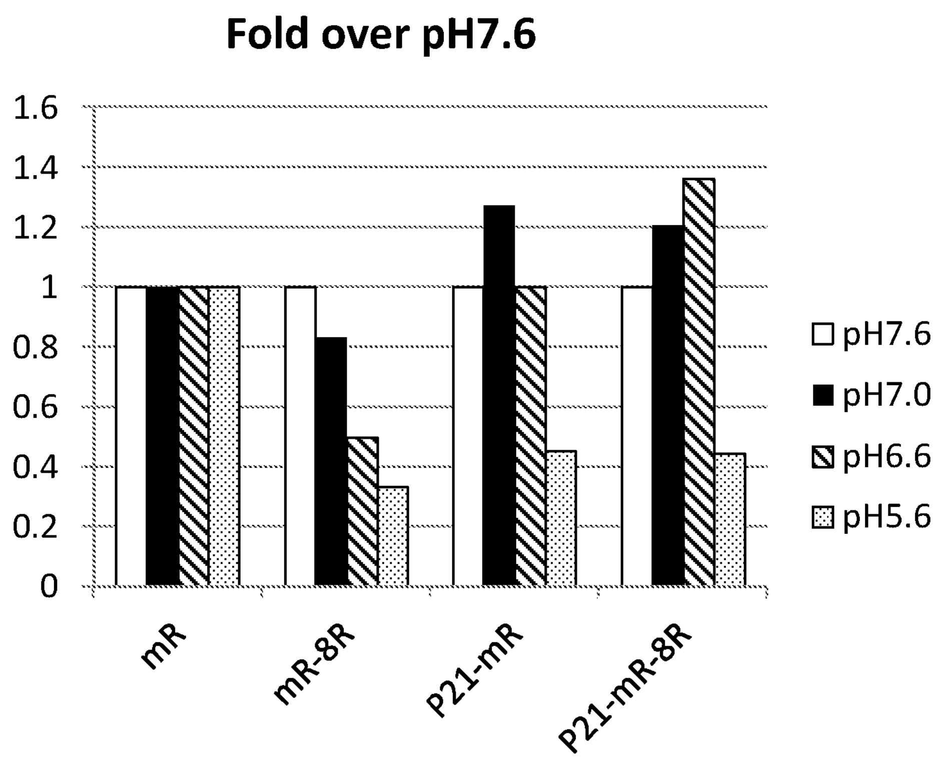
Fold over pH7.6
1.6
1.4
1.2
1
0.8
0.6
0.4
0.2
0
mR
mR-8R
P21-mR
P21-mR-8R
pH7.6
pH7.0
pH6.6
pH5.6

Figure 7

8R: RRRRRRRR
10H: HHHHHHHHHH

CPP26: AGYLLGKINLKKLAKLAKKIL
CPP27: AGYLLGHINLHHLAHLAHHIL

Figure 8

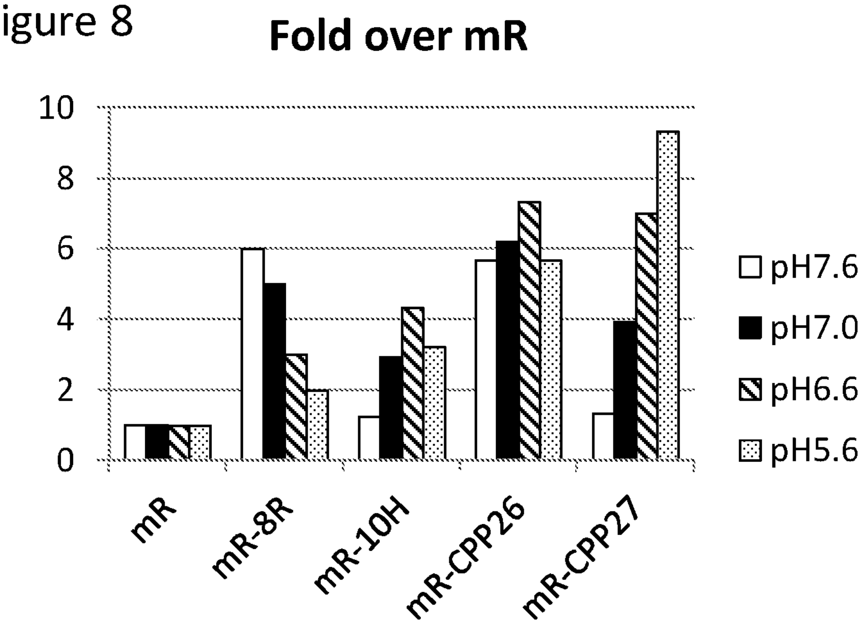
Fold over mR
10
8
6
4
2
0
mR
mR-8R
mR-10H
mR-CPP26
mR-CPP27
pH7.6
pH7.0
pH6.6
pH5.6

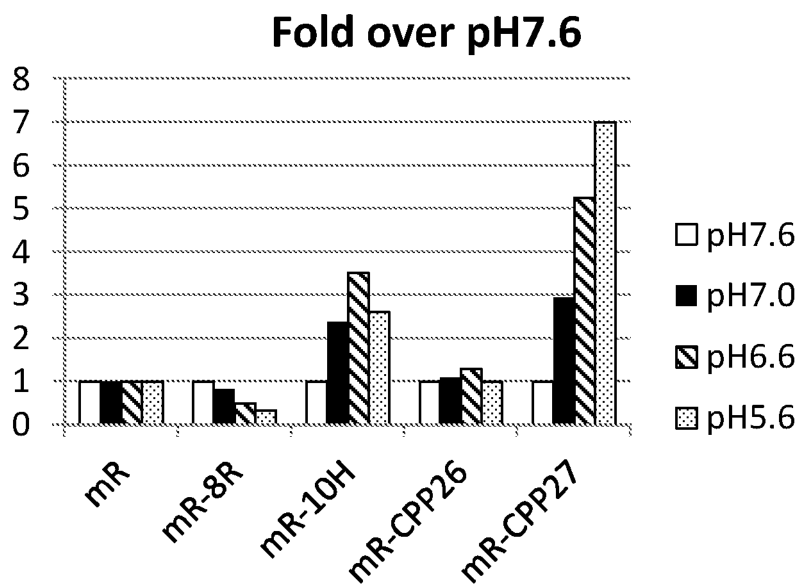
Fold over pH7.6
8
7
6
5
4
3
2
1
0
mR
mR-8R
mR-10H
mR-CPP26
mR-CPP27
pH7.6
pH7.0
pH6.6
pH5.6

Figure 9

```
P21:       KRKKKGKGLGKKRDPCLRKYK
P21Nacid:  HRHHHGHGLGKKRDPCLRKYK
P21Cacid:  KRKKKGKGLGHHRDPCLRHYH
P21Eacid:  KRHKHGKGLGHKRDPCLRHYK
P21Oacid:  HRKHKGHGLGKHRDPCLRKYH
P21acid:   HRHHHGHGLGHHRDPCLRHYH
```

Figure 10

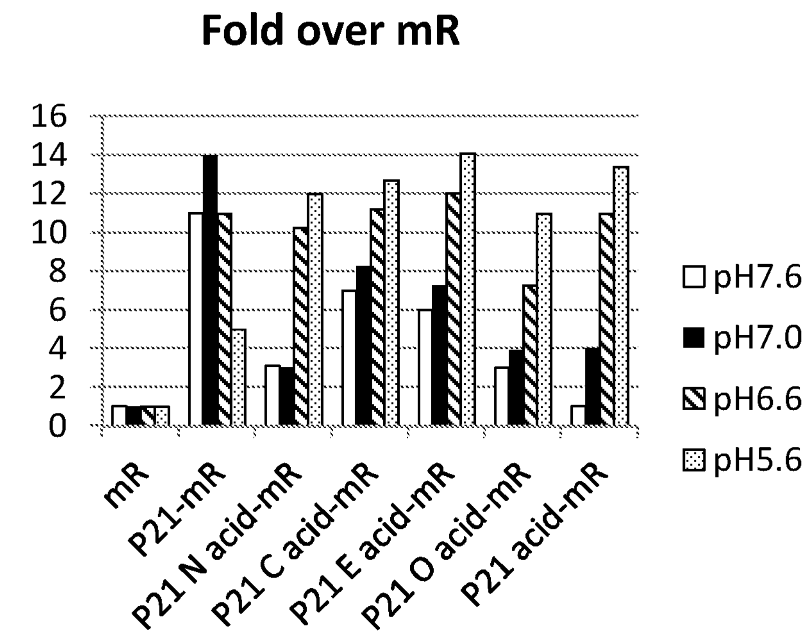
Fold over mR
16
14
12
10
8
6
4
2
0
mR
P21-mR
P21 N acid-mR
P21 C acid-mR
P21 E acid-mR
P21 O acid-mR
P21 acid-mR
pH7.6
pH7.0
pH6.6
pH5.6

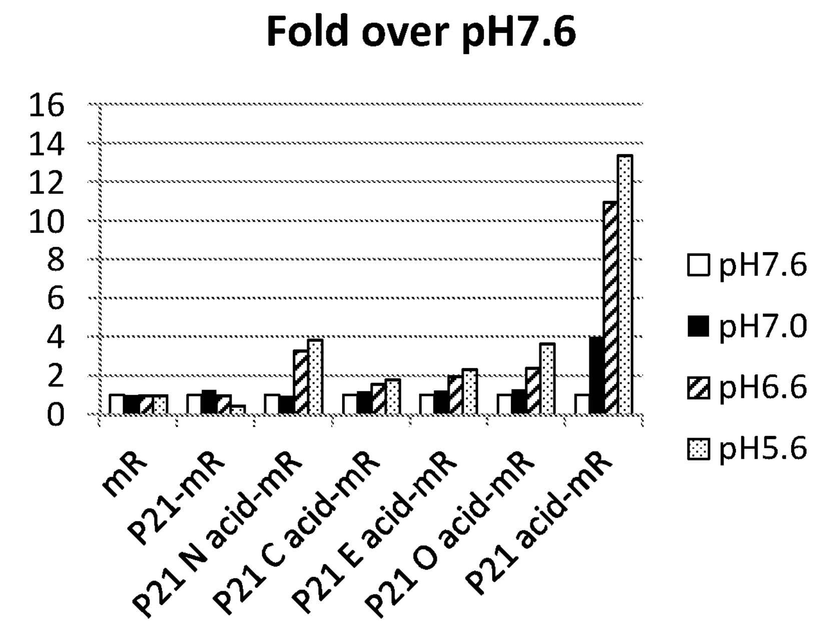
Fold over pH7.6
16
14
12
10
8
6
4
2
0
mR
P21-mR
P21 N acid-mR
P21 C acid-mR
P21 E acid-mR
P21 O acid-mR
P21 acid-mR
pH7.6
pH7.0
pH6.6
pH5.6

Figure 11

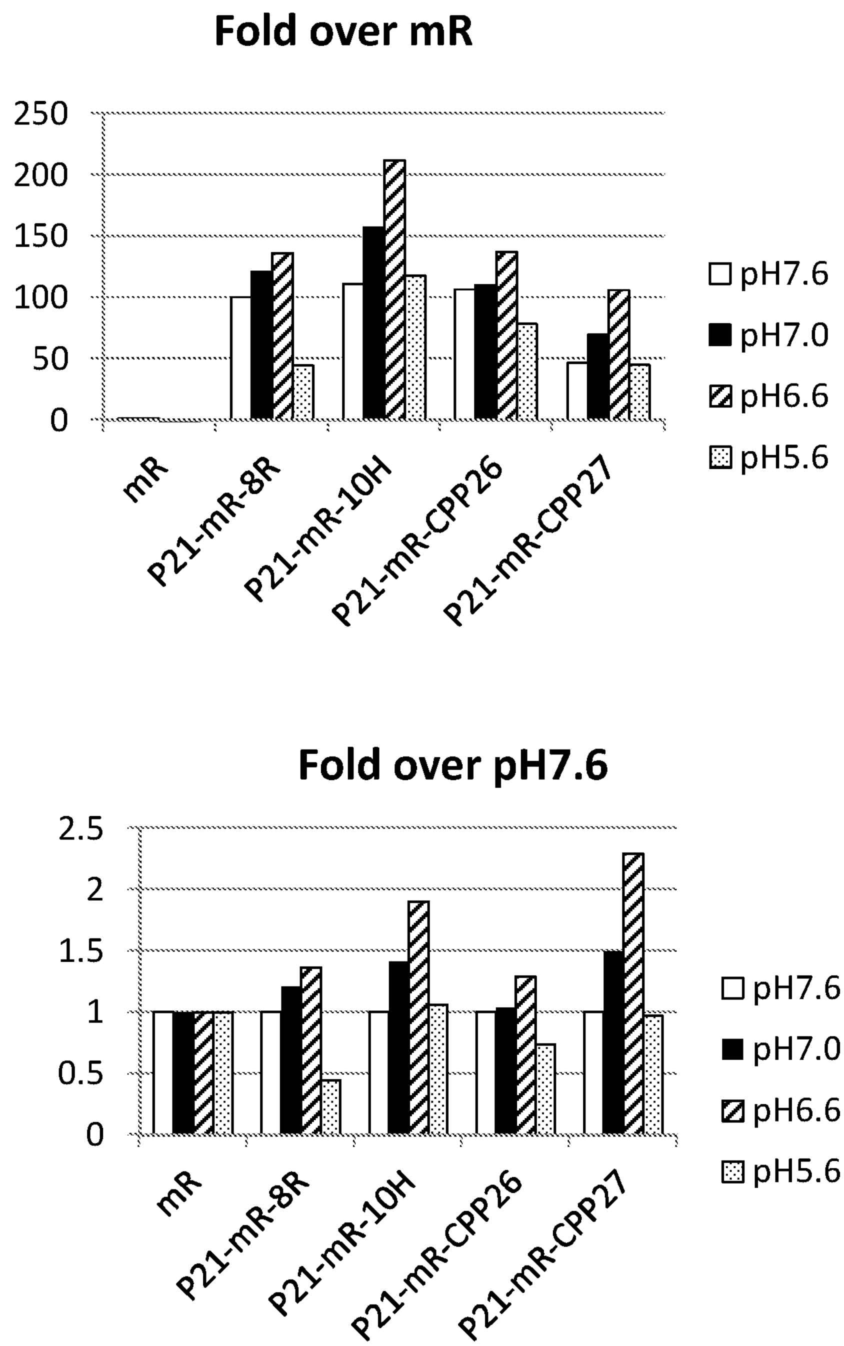
Fold over mR
250
200
150
100
50
0
mR
P21-mR-8R
P21-mR-10H
P21-mR-CPP26
P21-mR-CPP27
pH7.6
pH7.0
pH6.6
pH5.6
Fold over pH7.6
2.5
2
1.5
1
0.5
0
mR
P21-mR-8R
P21-mR-10H
P21-mR-CPP26
P21-mR-CPP27
pH7.6
pH7.0
pH6.6
pH5.6

Figure 12

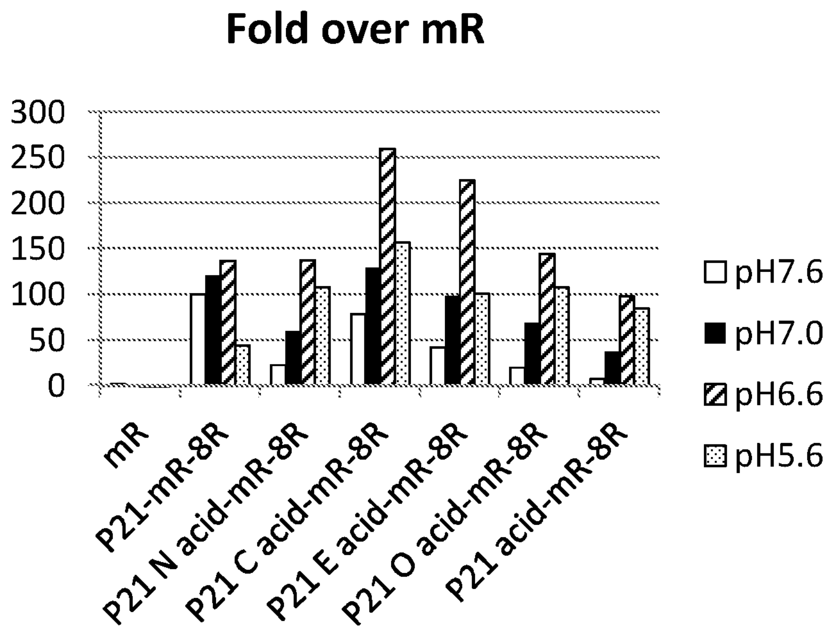
Fold over mR
300
250
200
150
100
50
0
mR
P21-mR-8R
P21 N acid-mR-8R
P21 C acid-mR-8R
P21 E acid-mR-8R
P21 O acid-mR-8R
P21 acid-mR-8R
pH7.6
pH7.0
pH6.6
pH5.6

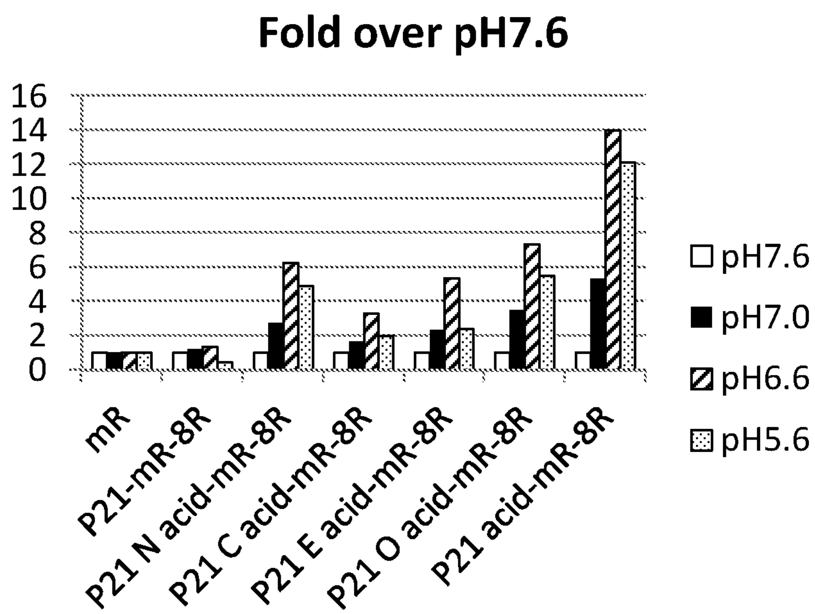
Fold over pH7.6
16
14
12
10
8
6
4
2
0
mR
P21-mR-8R
P21 N acid-mR-8R
P21 C acid-mR-8R
P21 E acid-mR-8R
P21 O acid-mR-8R
P21 acid-mR-8R
pH7.6
pH7.0
pH6.6
pH5.6

Figure 13

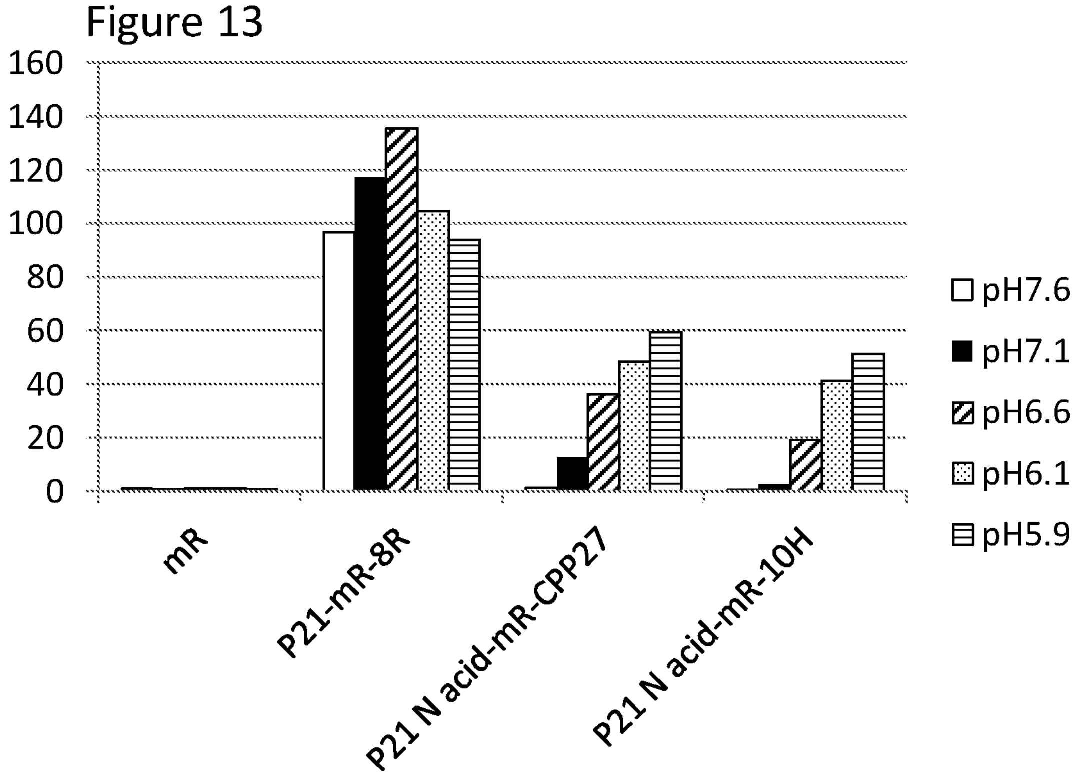
160
140
120
100
80
60
40
20
0
mR
P21-mR-8R
P21 N acid-mR-CPP27
P21 N acid-mR-10H
pH7.6
pH7.1
pH6.6
pH6.1
pH5.9

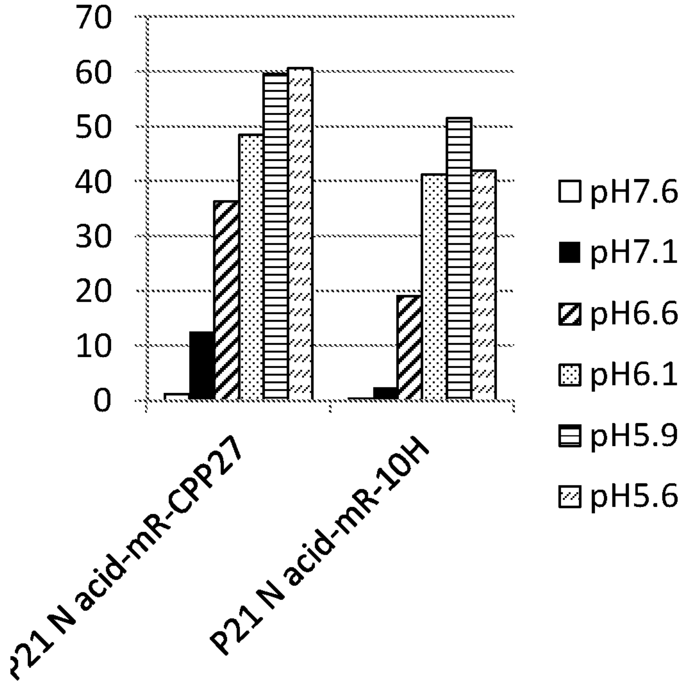
70
60
50
40
30
20
10
0
P21 N acid-mR-CPP27
P21 N acid-mR-10H
pH7.6
pH7.1
pH6.6
pH6.1
pH5.9
pH5.6

# Figure 14

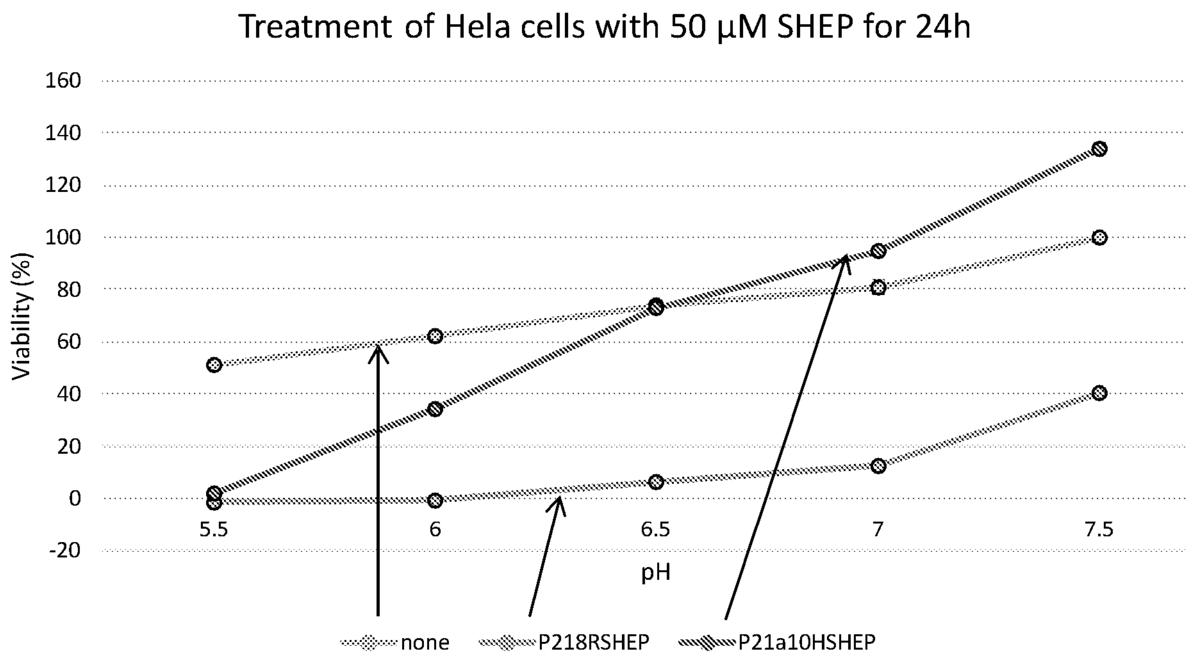
Treatment of Hela cells with 50 µM SHEP for 24h
160
140
120
100
80
60
40
20
0
-20
Viability (%)
5.5
6
6.5
7
7.5
pH
none
P218RSHEP
P21a10HSHEP

Figure 15

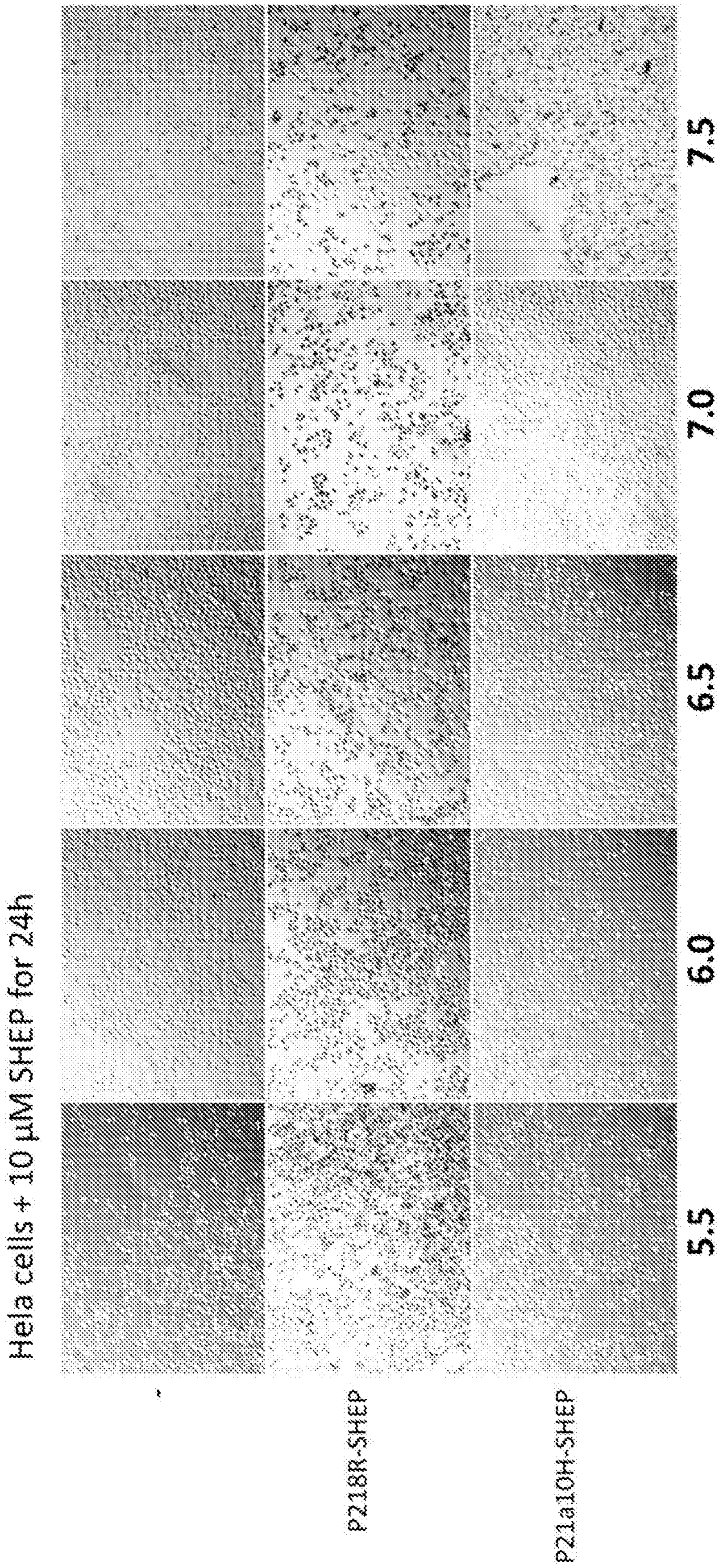
Hela cells + 10 µM SHEP for 24h
-
P218R-SHEP
P21a10H-SHEP
5.5
6.0
6.5
7.0
7.5

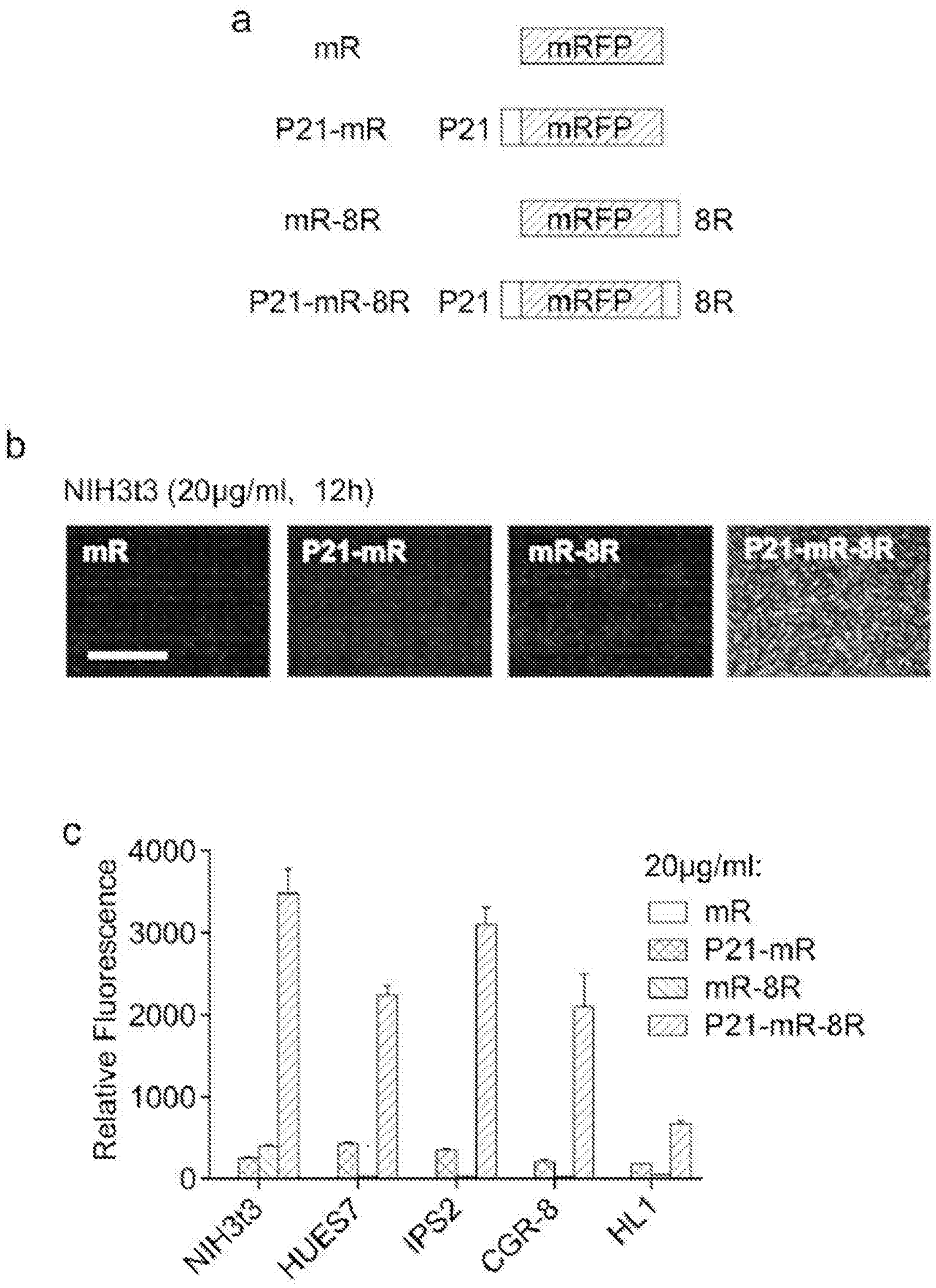
a
mR
mRFP
P21-mR
P21
mRFP
mR-8R
mRFP
8R
P21-mR-8R
P21
mRFP
8R
b
NIH3t3 (20μg/ml, 12h)
mR
P21-mR
mR-8R
P21-mR-8R
c
Relative Fluorescence
4000
3000
2000
1000
0
20μg/ml:
mR
P21-mR
mR-8R
P21-mR-8R
NIH3t3
HUES7
IPS2
CGR-8
HL1

Figure 16

Figure 16 continued

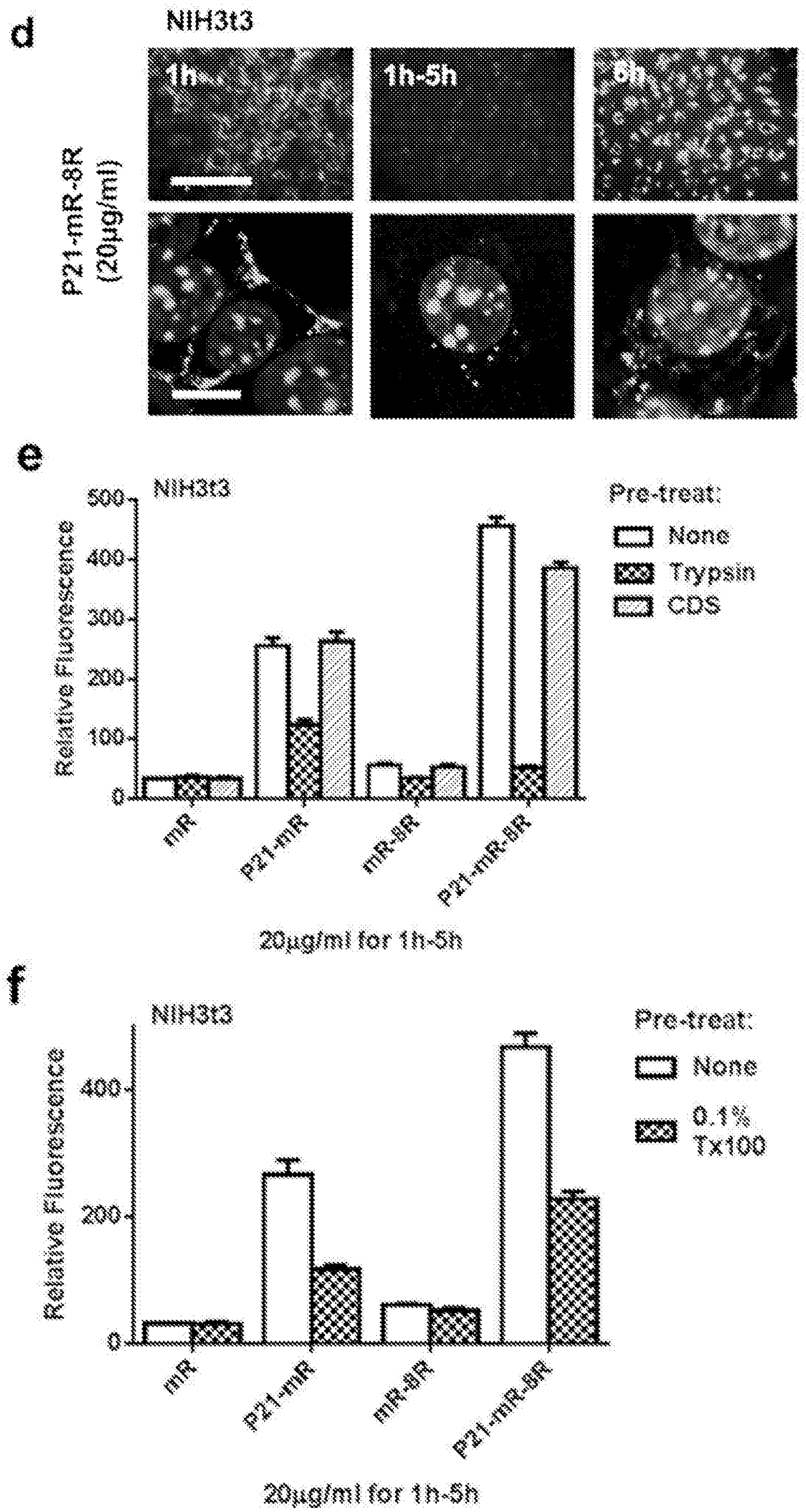

Figure 17

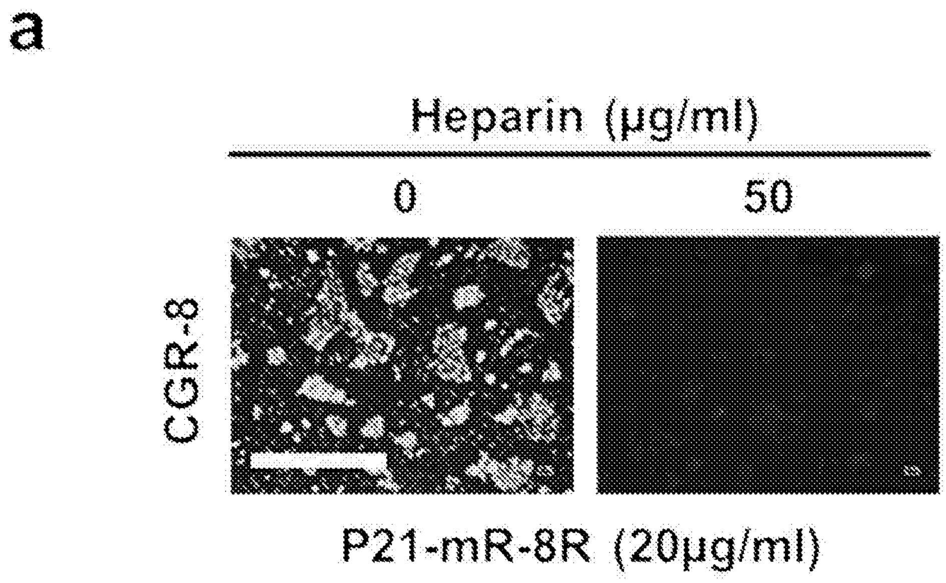
a
Heparin (µg/ml)
0
50
CGR-8
P21-mR-8R (20µg/ml)

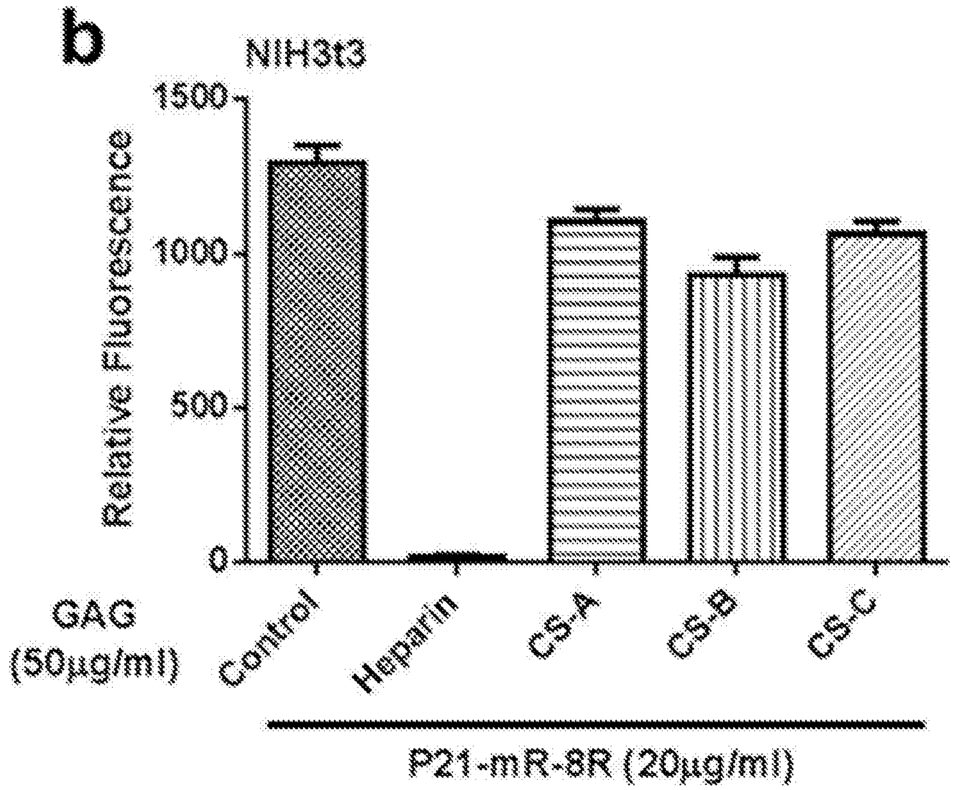
b
NIH3t3
1500
1000
500
0
Relative Fluorescence
GAG (50µg/ml)
Control
Heparin
CS-A
CS-B
CS-C
P21-mR-8R (20µg/ml)

Figure 17 continued

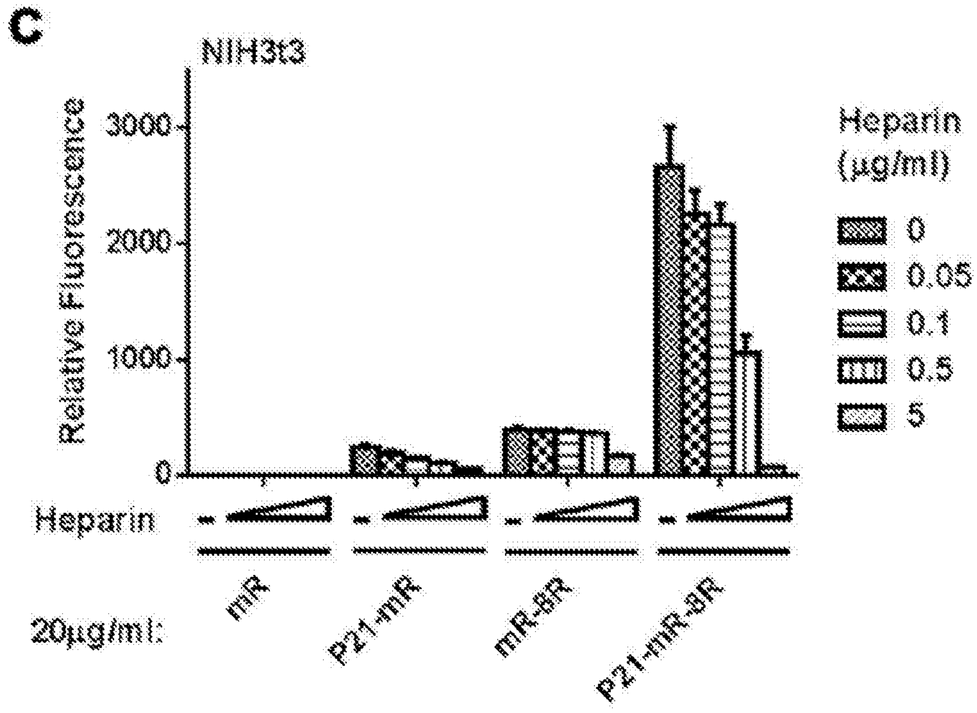


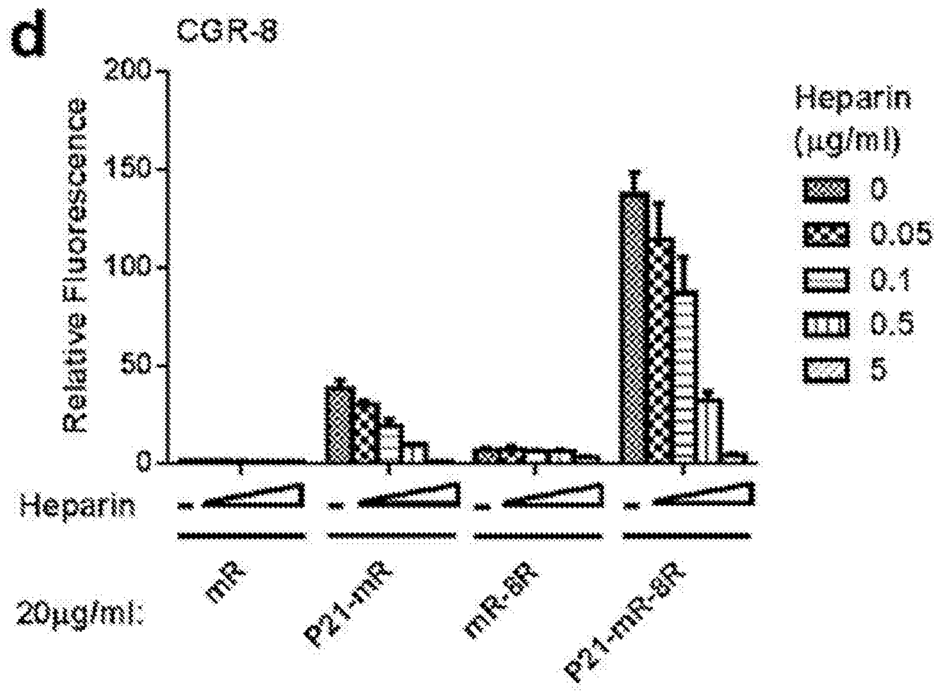

Figure 17 continued

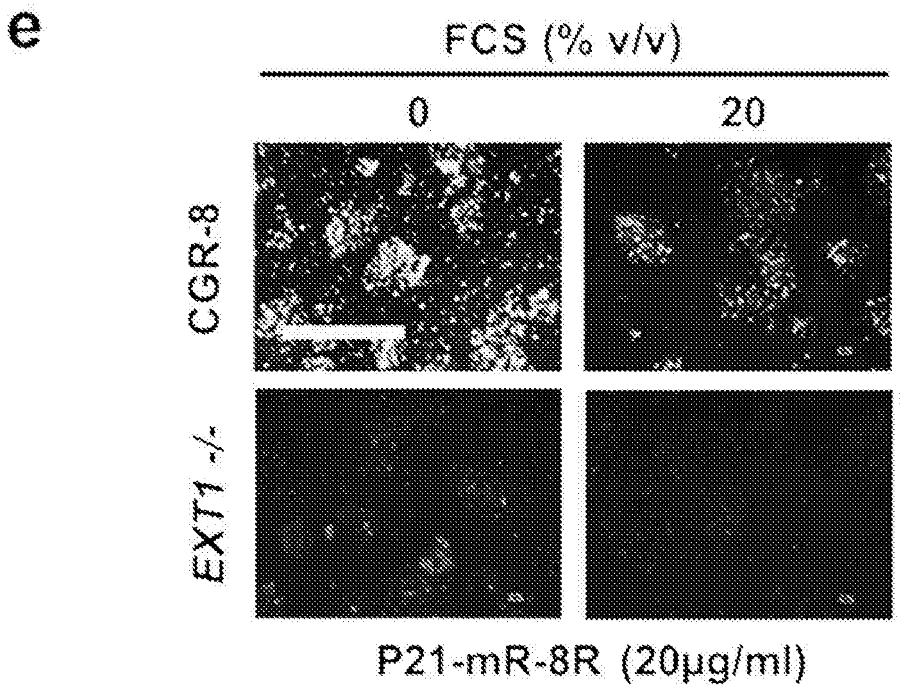


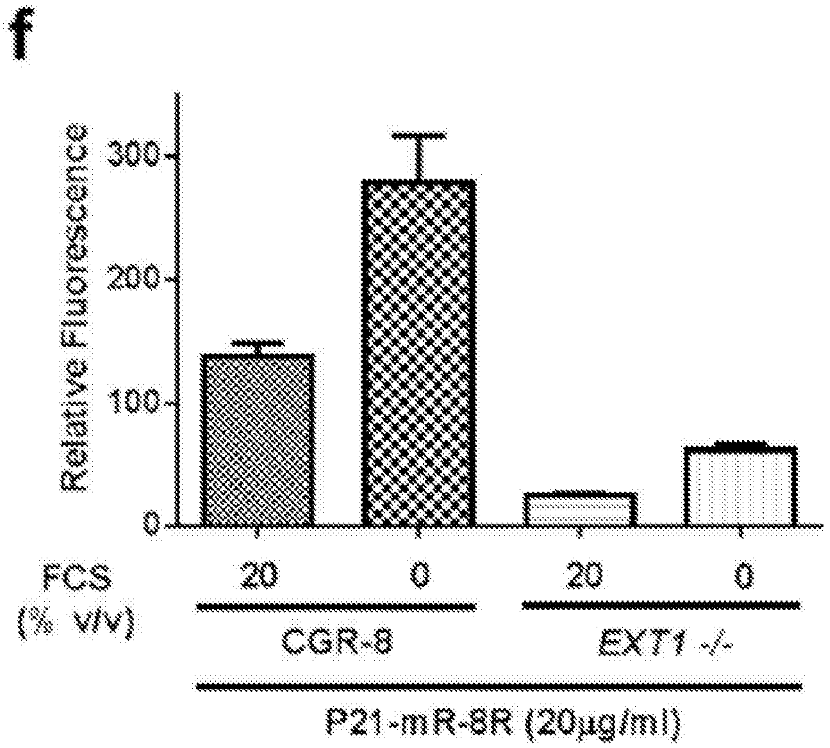

Figure 18

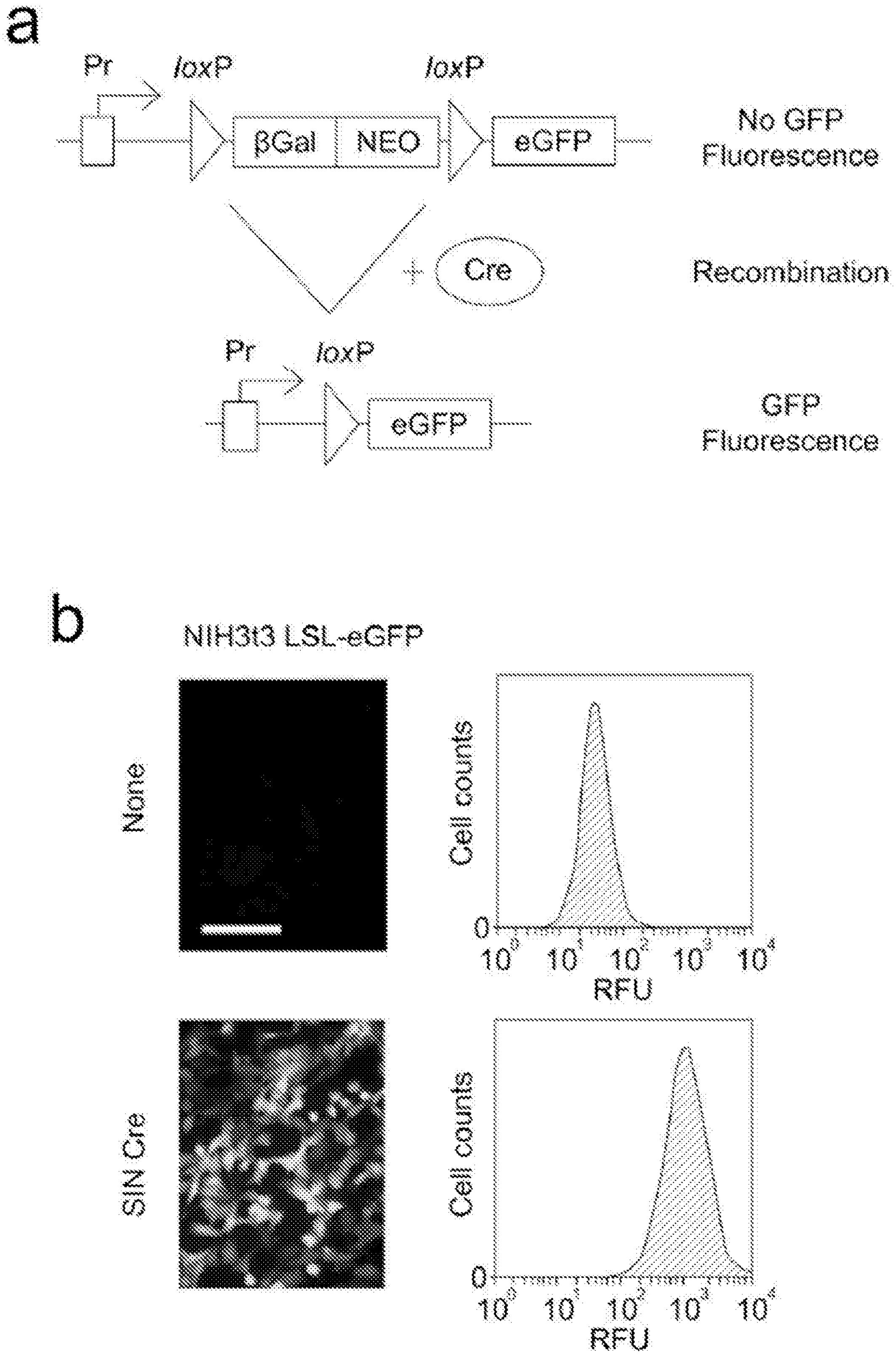
a
Pr
loxP
loxP
βGal
NEO
eGFP
No GFP Fluorescence
Cre
Recombination
Pr
loxP
eGFP
GFP Fluorescence
b
NIH3t3 LSL-eGFP
None
SIN Cre
Cell counts
RFU
0
$10^0$ $10^1$ $10^2$ $10^3$ $10^4$

Figure 18 continued

c

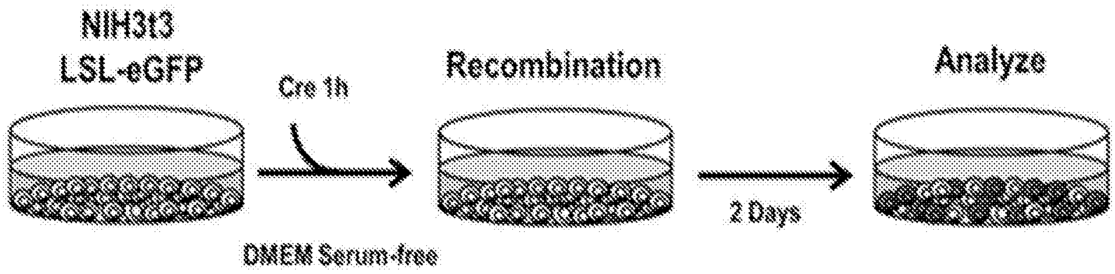


d

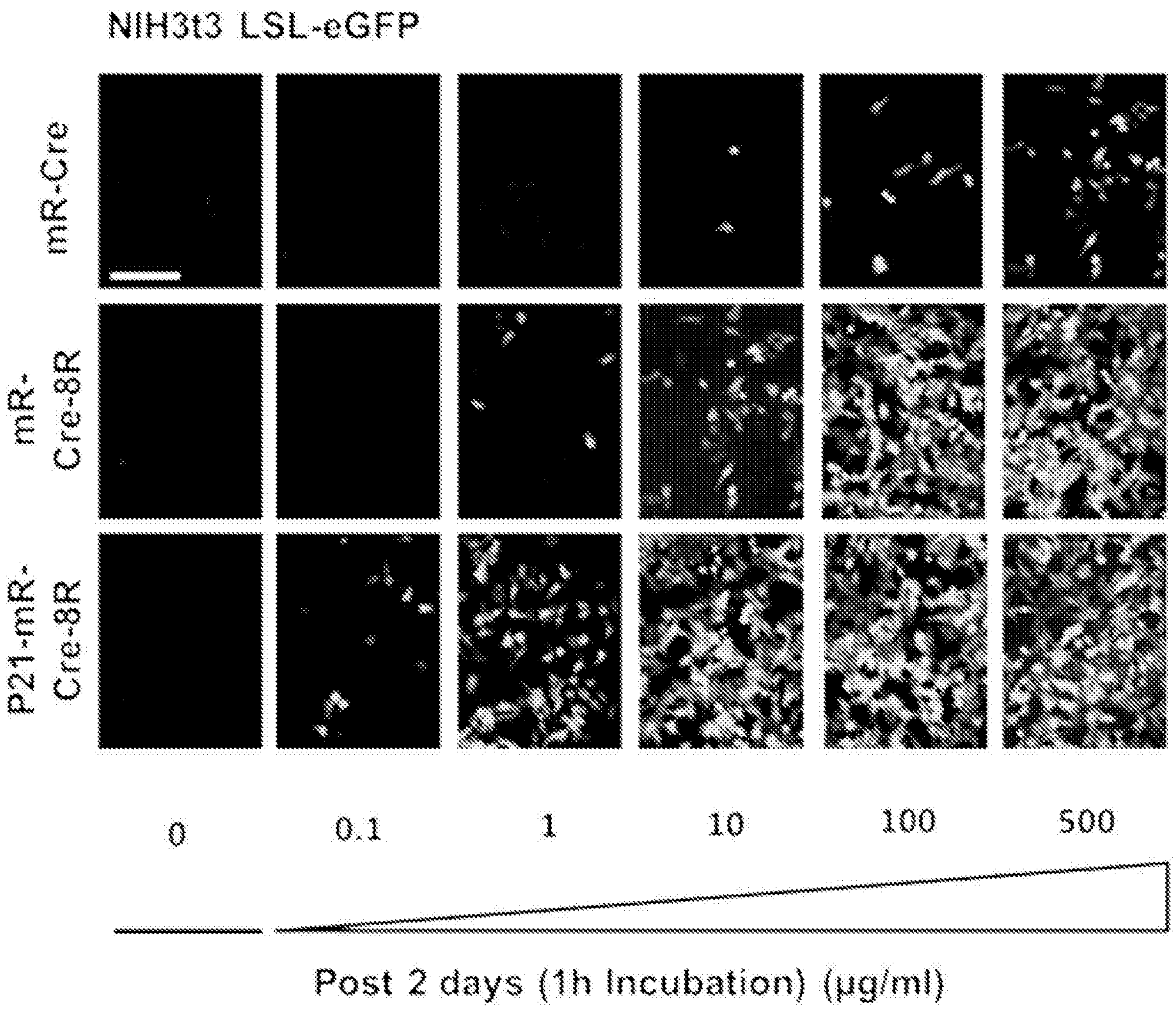

Figure 18 continued

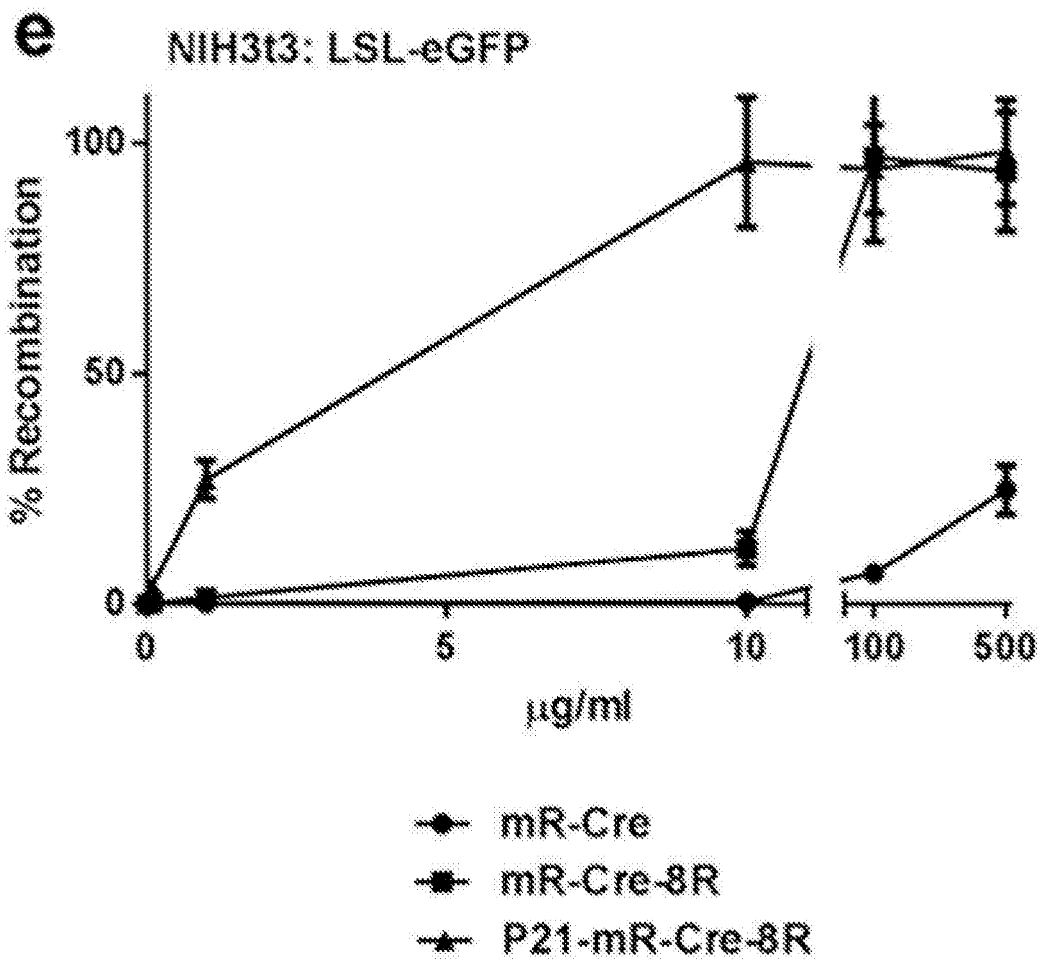

Figure 19

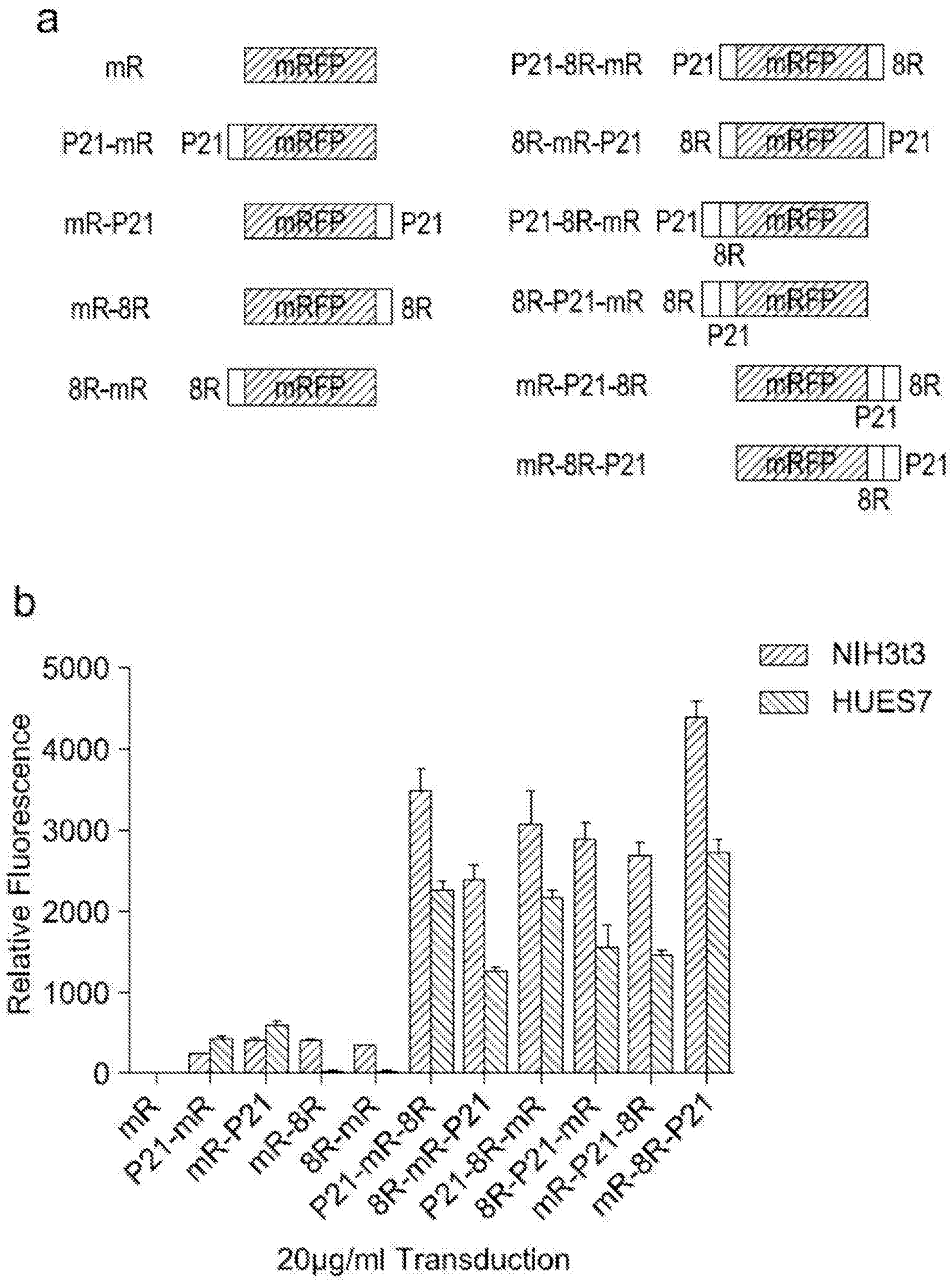
a
mR
mRFP
P21-mR
P21
mR-P21
mR-8R
8R
8R-mR
P21-8R-mR
8R-mR-P21
8R-P21-mR
mR-P21-8R
mR-8R-P21
b
NIH3t3
HUES7
Relative Fluorescence
0
1000
2000
3000
4000
5000
P21-mR-8R
P21-8R-mR
20µg/ml Transduction

Figure 20

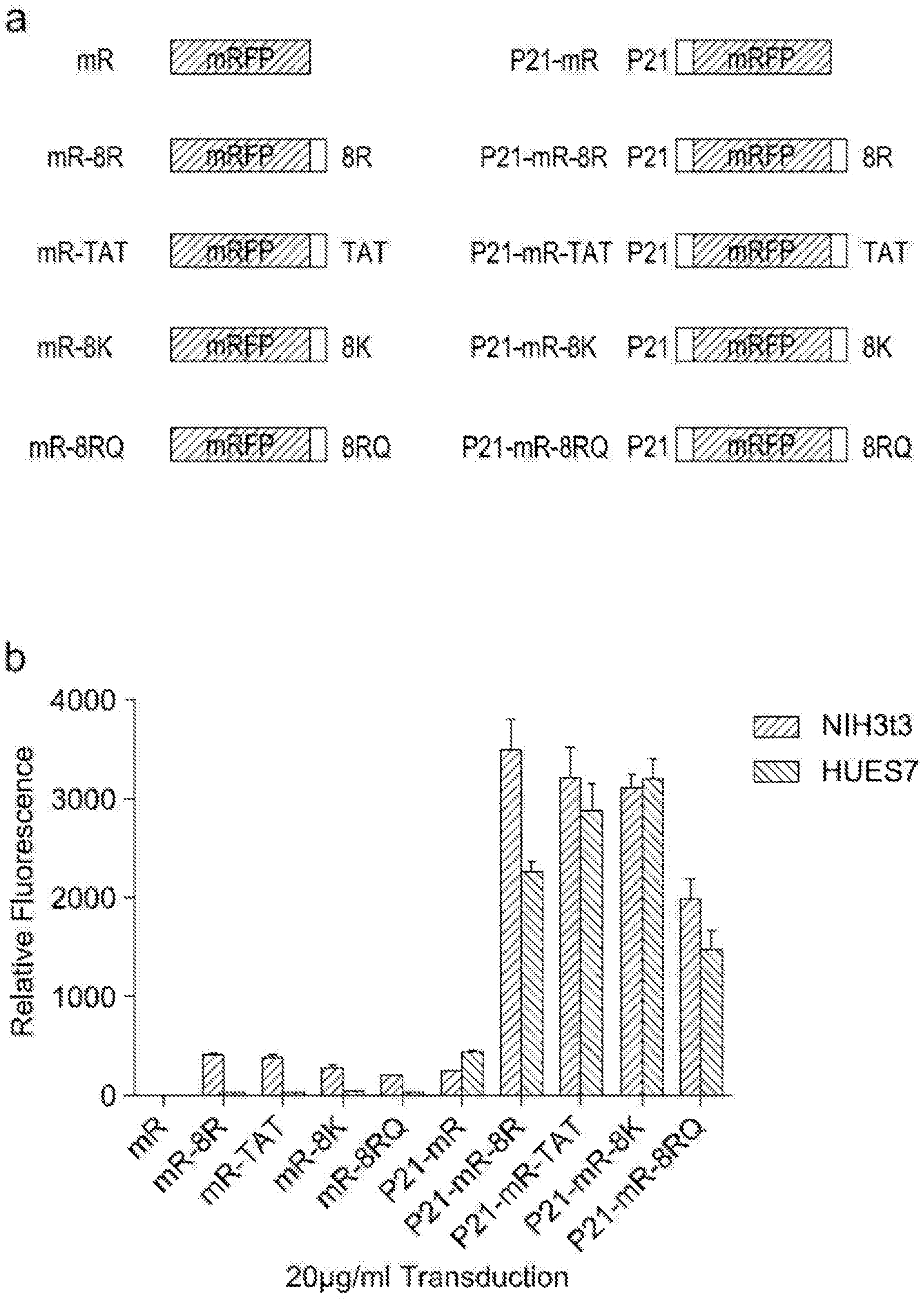
a
mR
mRFP
P21-mR
P21
mRFP
mR-8R
mRFP
8R
P21-mR-8R
P21
mRFP
8R
mR-TAT
mRFP
TAT
P21-mR-TAT
P21
mRFP
TAT
mR-8K
mRFP
8K
P21-mR-8K
P21
mRFP
8K
mR-8RQ
mRFP
8RQ
P21-mR-8RQ
P21
mRFP
8RQ
b
Relative Fluorescence
4000
3000
2000
1000
0
NIH3t3
HUES7
mR
mR-8R
mR-TAT
mR-8K
mR-8RQ
P21-mR
P21-mR-8R
P21-mR-TAT
P21-mR-8K
P21-mR-8RQ
20μg/ml Transduction

Figure 21

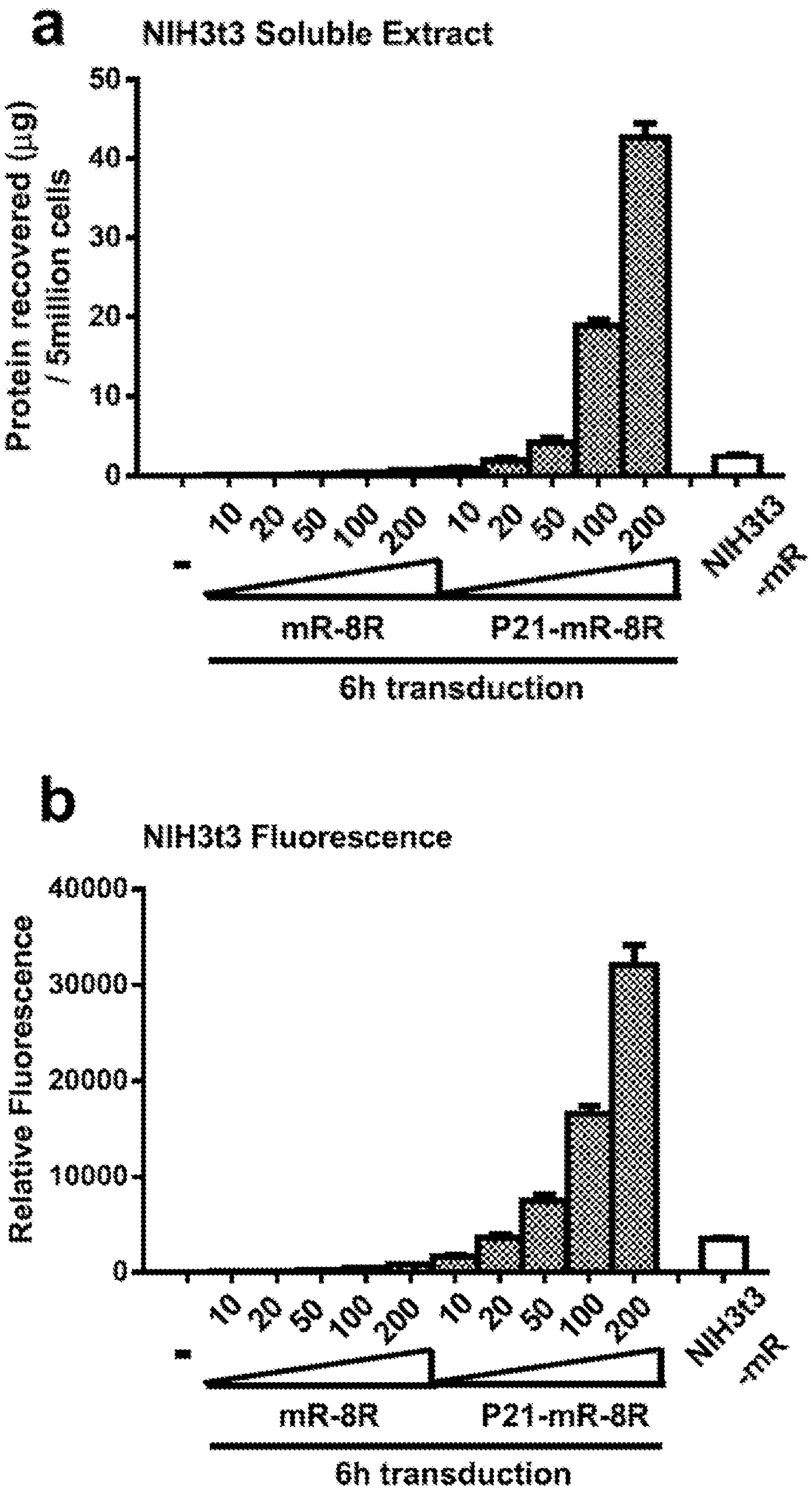
a
NIH3t3 Soluble Extract
Protein recovered (µg) / 5million cells
50
40
30
20
10
0
10
20
50
100
200
10
20
50
100
200
NIH3t3
-mR
-
mR-8R
P21-mR-8R
6h transduction
b
NIH3t3 Fluorescence
Relative Fluorescence
40000
30000
20000
10000
0
10
20
50
100
200
10
20
50
100
200
NIH3t3
-mR
-
mR-8R
P21-mR-8R
6h transduction

Figure 22

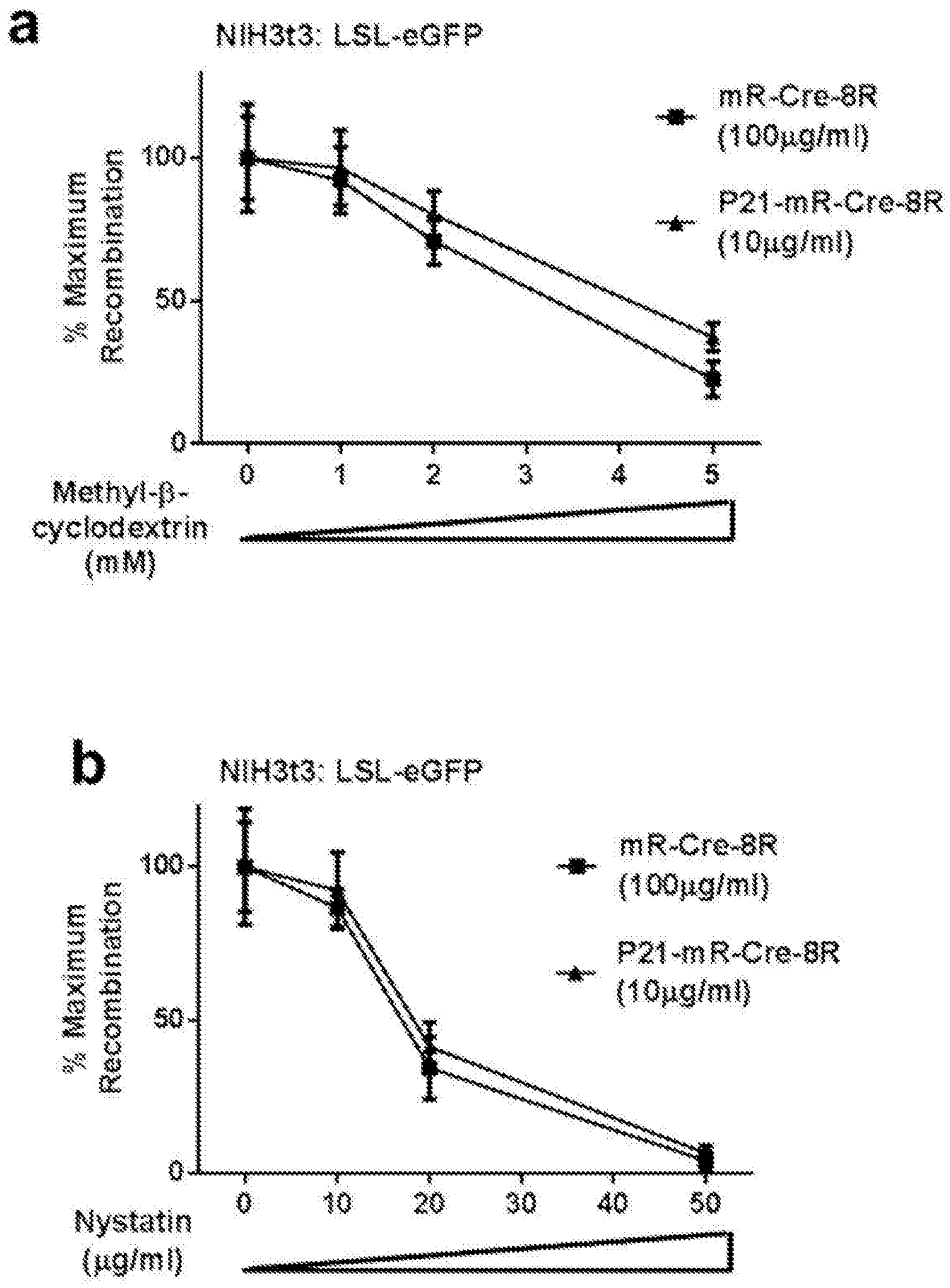
a
NIH3t3: LSL-eGFP
% Maximum Recombination
100
50
0
0
1
2
3
4
5
Methyl-β-cyclodextrin (mM)
mR-Cre-8R (100µg/ml)
P21-mR-Cre-8R (10µg/ml)
b
NIH3t3: LSL-eGFP
% Maximum Recombination
100
50
0
0
10
20
30
40
50
Nystatin (µg/ml)
mR-Cre-8R (100µg/ml)
P21-mR-Cre-8R (10µg/ml)

Figure 22 continued

c

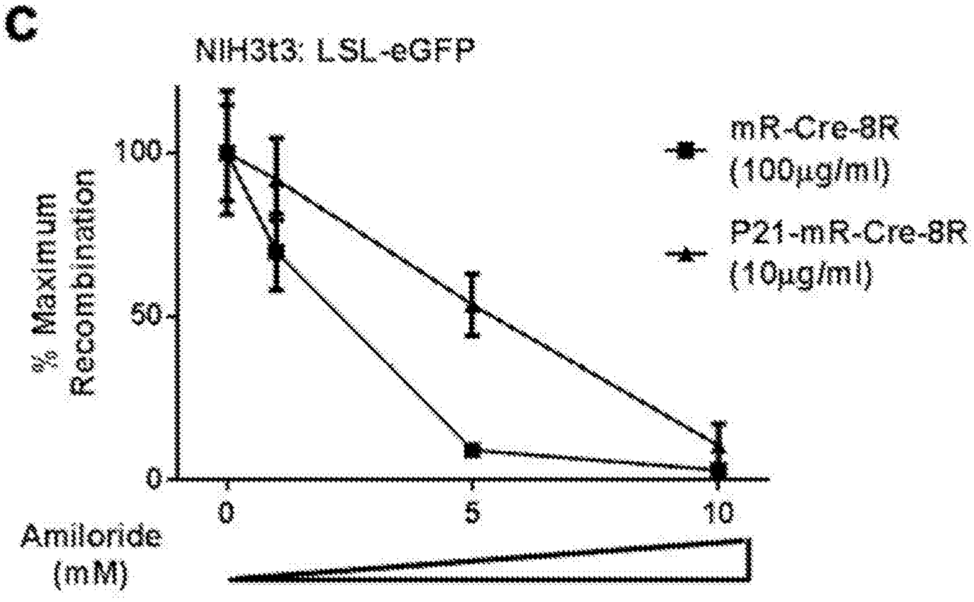


d

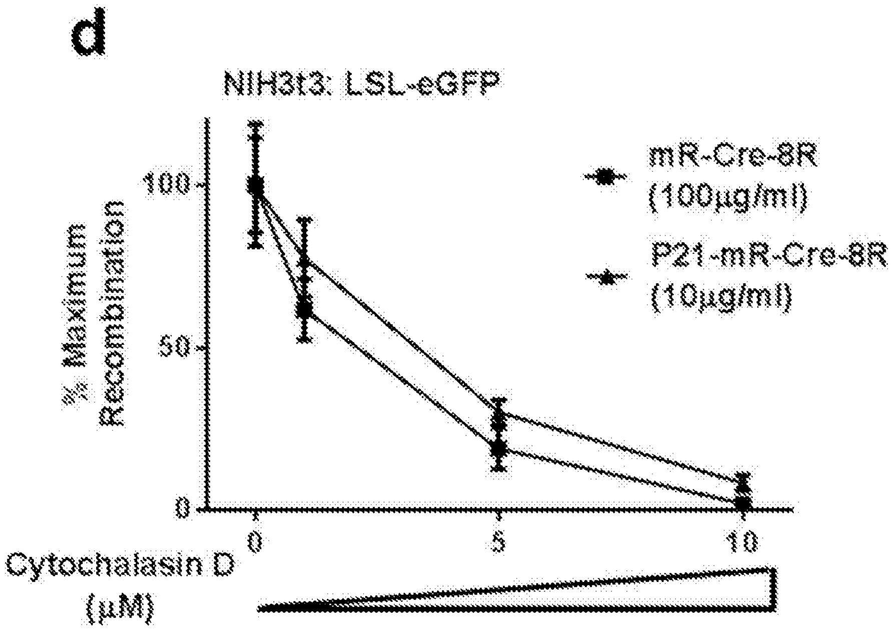

Figure 22 continued

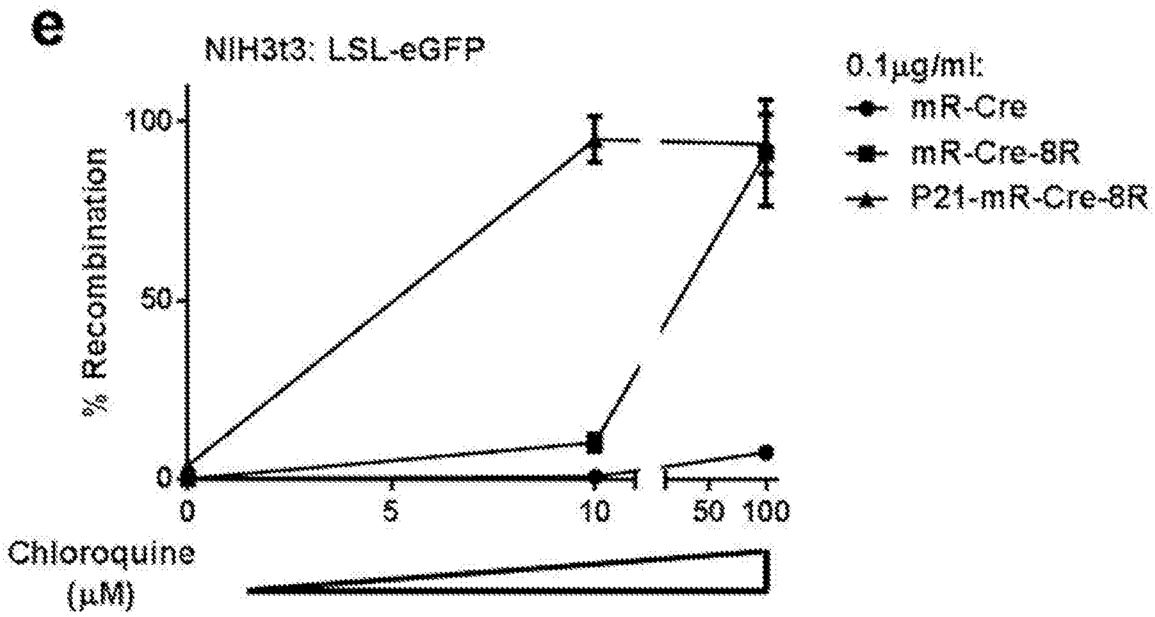


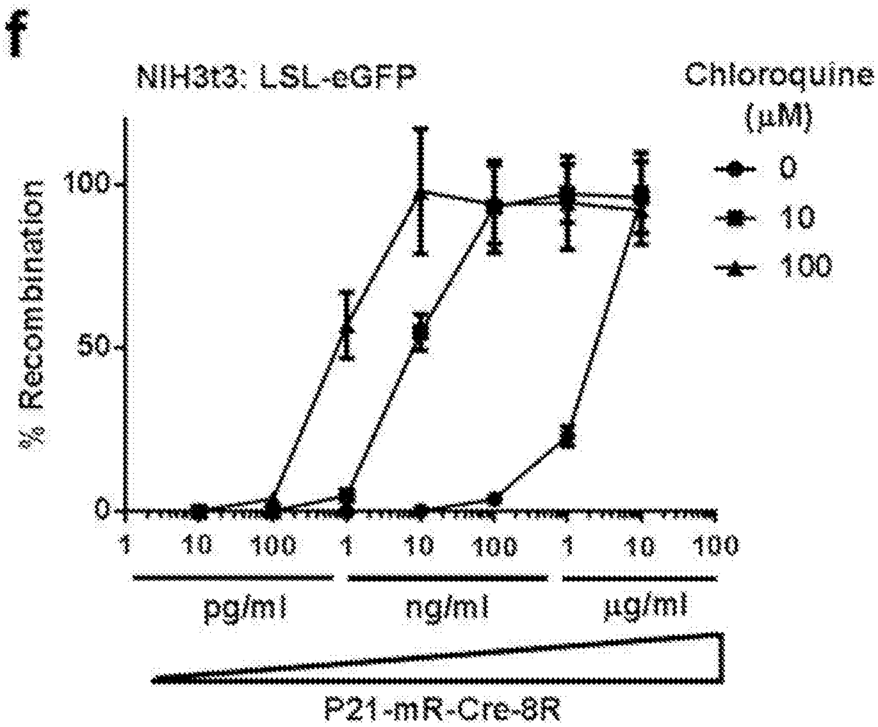

Figure 23

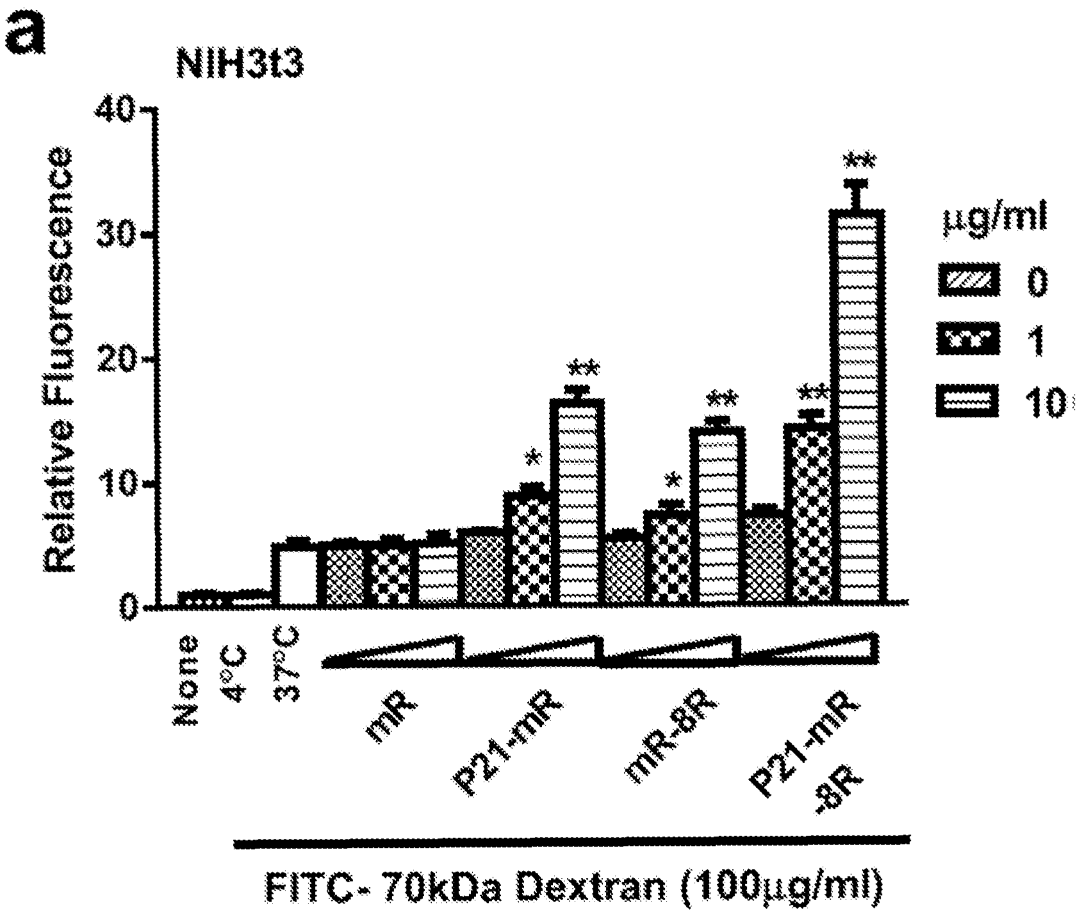
a
NIH3t3
Relative Fluorescence
40
30
20
10
0
μg/ml
0
1
10
None
4°C
37°C
mR
P21-mR
mR-8R
P21-mR -8R
FITC- 70kDa Dextran (100μg/ml)

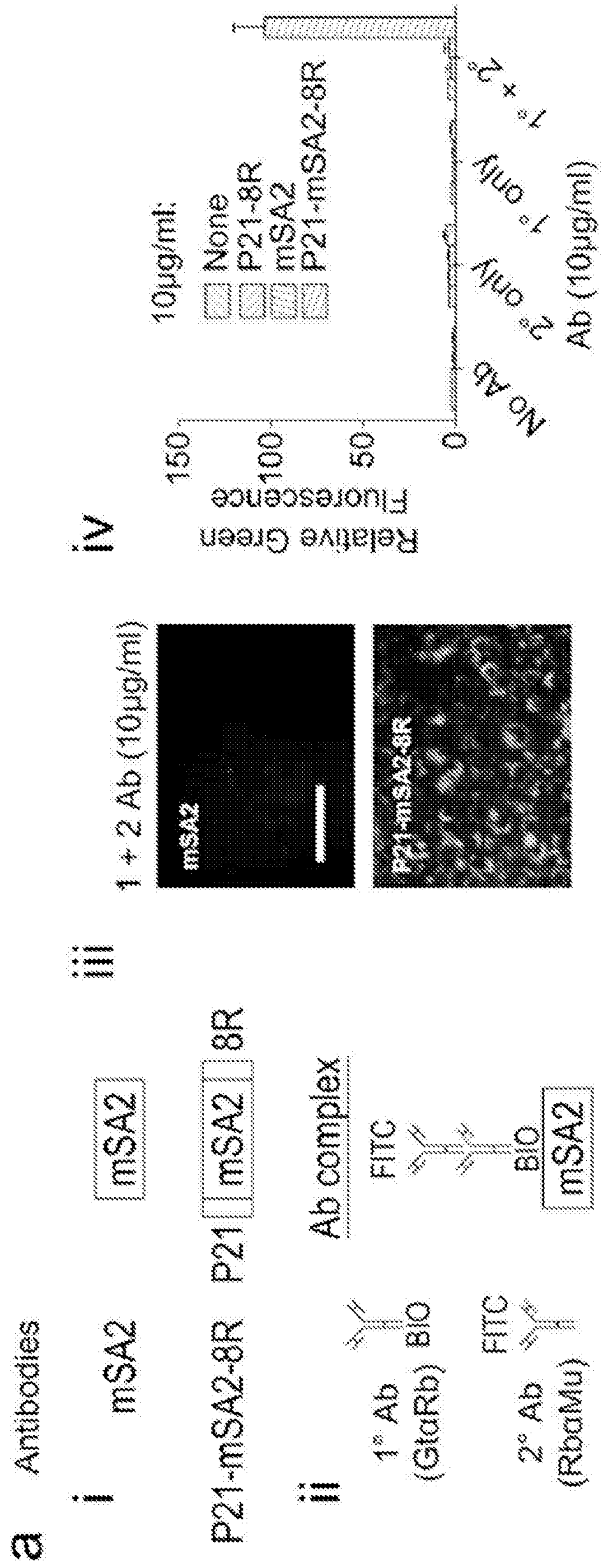
a
Antibodies
i
mSA2
P21-mSA2-8R
mSA2
P21 mSA2 8R
ii
1° Ab (GtαRb) BIO
2° Ab (RbαMu) FITC
Ab complex
FITC
BIO
mSA2
iii
1 + 2 Ab (10µg/ml)
mSA2
P21-mSA2-8R
iv
Relative Green Fluorescence
150
100
50
0
10µg/ml:
None
P21-8R
mSA2
P21-mSA2-8R
No Ab
2° only
1° only
1° + 2°
Ab (10µg/ml)

Figure 24

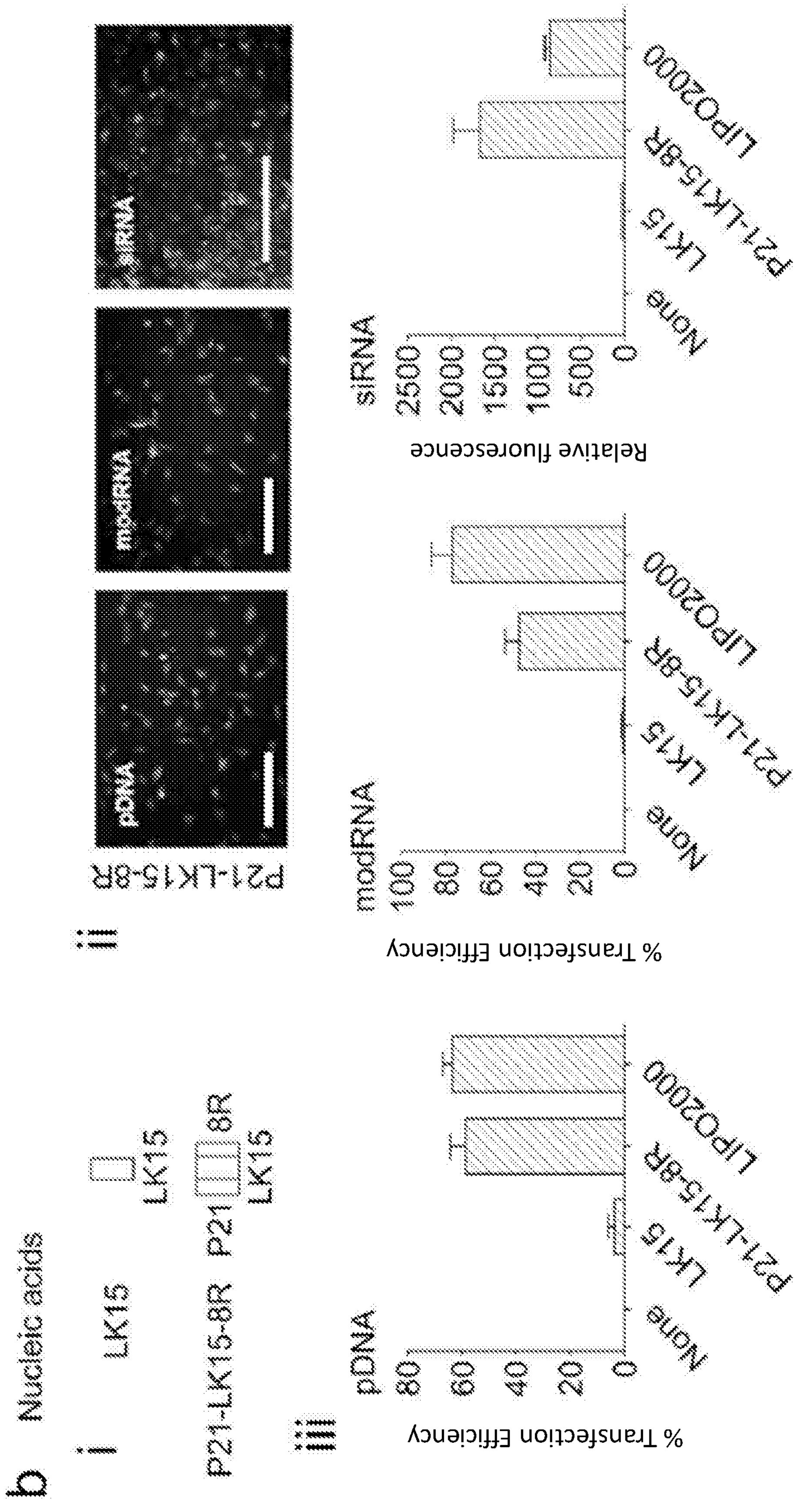


Figure 24 continued

Figure 24 continued

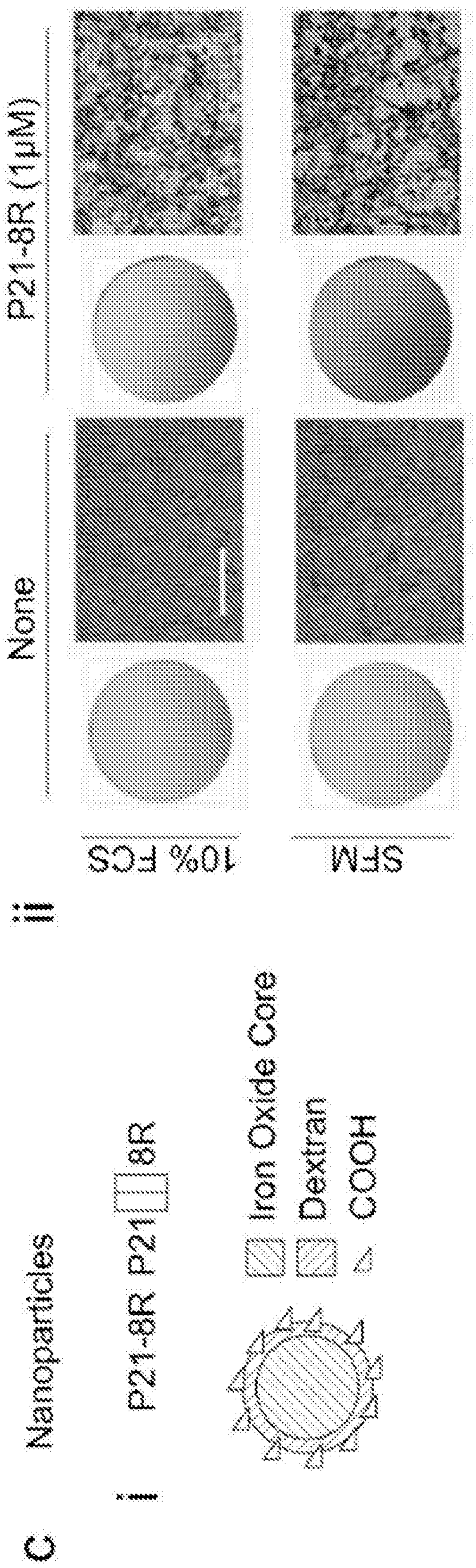


Figure 24 continued

Figure 25

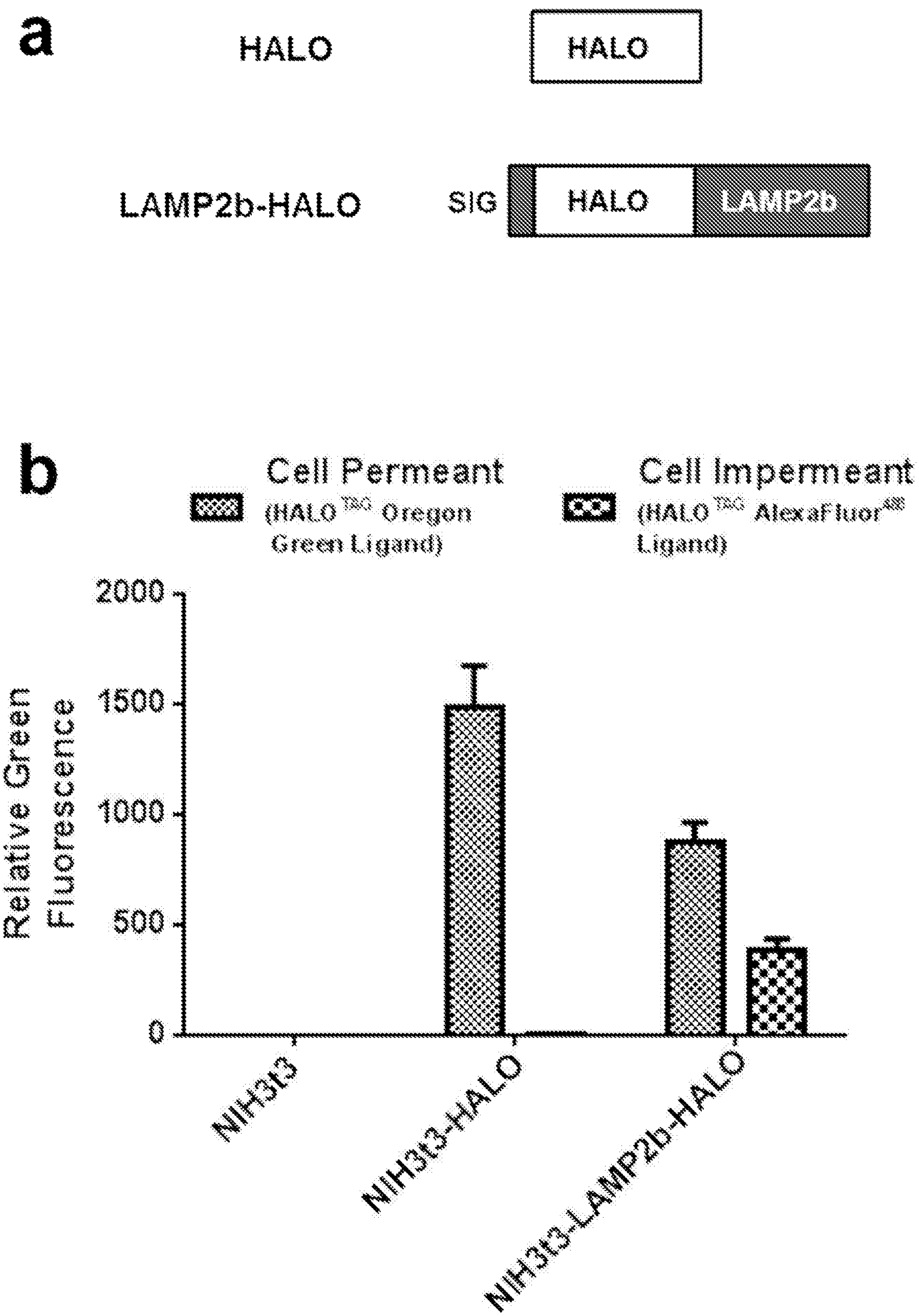
a
HALO
HALO
LAMP2b-HALO
SIG
HALO
LAMP2b
b
Cell Permeant
(HALO TAG Oregon Green Ligand)
Cell Impermeant
(HALO TAG AlexaFluor488 Ligand)
Relative Green Fluorescence
2000
1500
1000
500
0
NIH3t3
NIH3t3-HALO
NIH3t3-LAMP2b-HALO

Figure 26

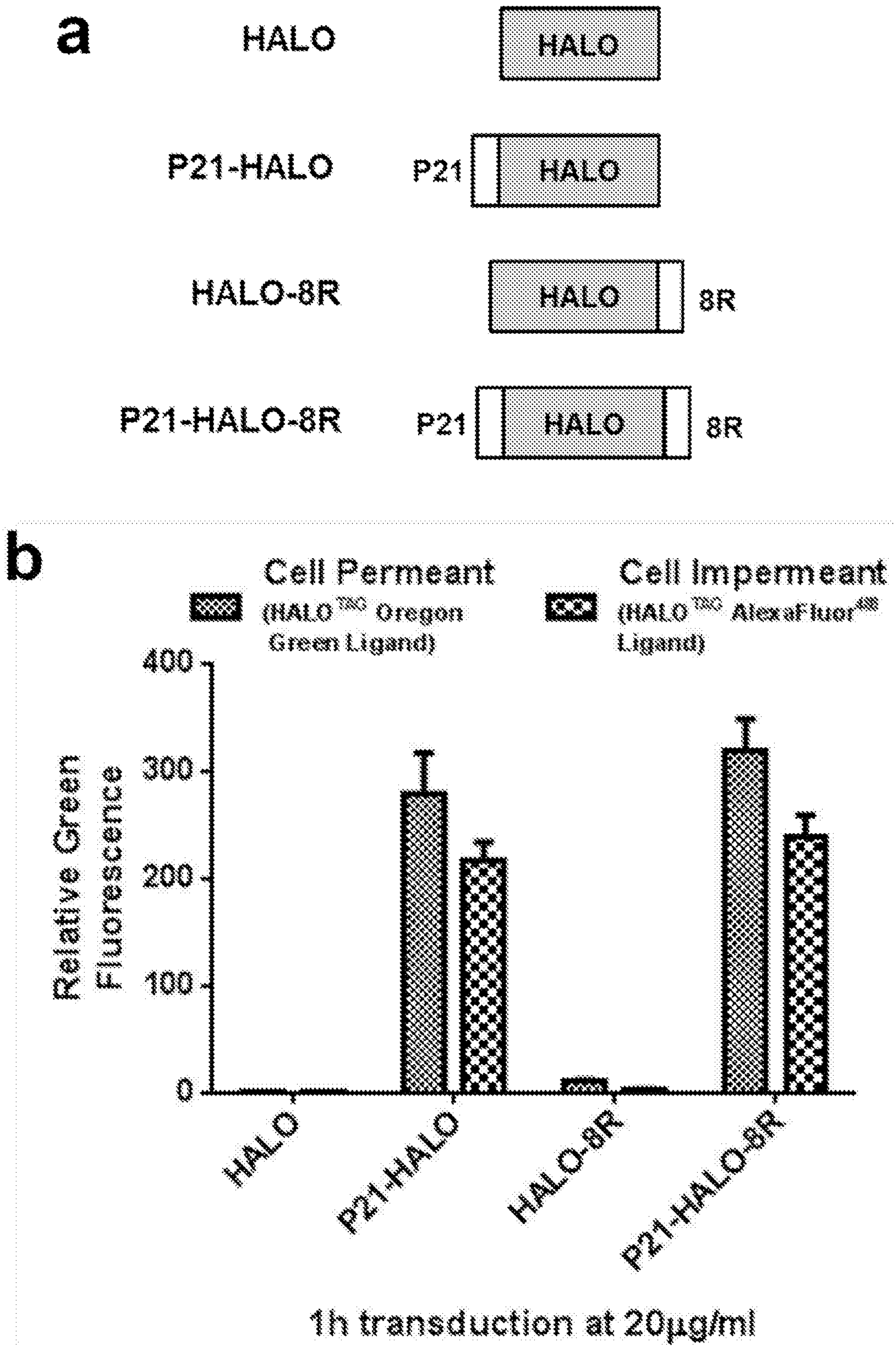
a
HALO
P21-HALO
HALO-8R
P21-HALO-8R
HALO
P21
HALO
HALO
8R
P21
HALO
8R
b
Cell Permeant
(HALO TAG Oregon Green Ligand)
Cell Impermeant
(HALO TAG AlexaFluor488 Ligand)
Relative Green Fluorescence
400
300
200
100
0
HALO
P21-HALO
HALO-8R
P21-HALO-8R
1h transduction at 20µg/ml

Figure 26 continued

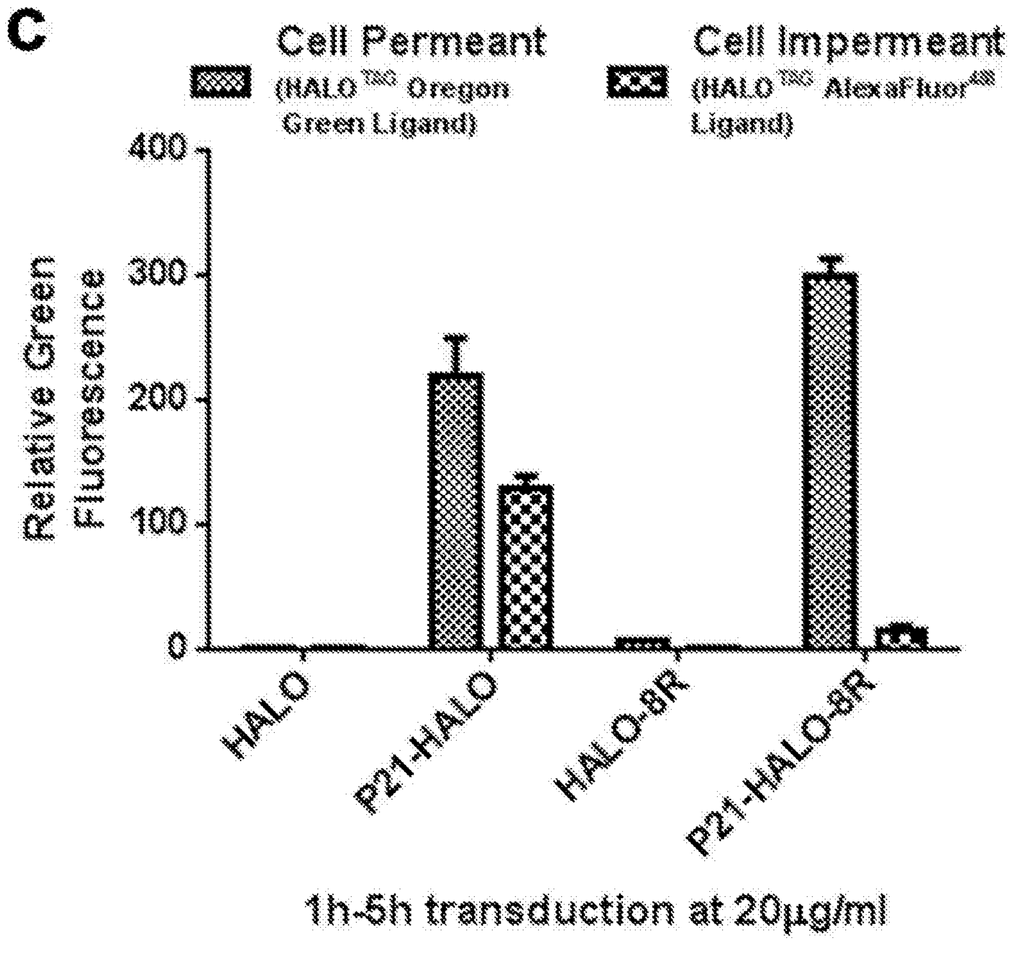

Figure 27

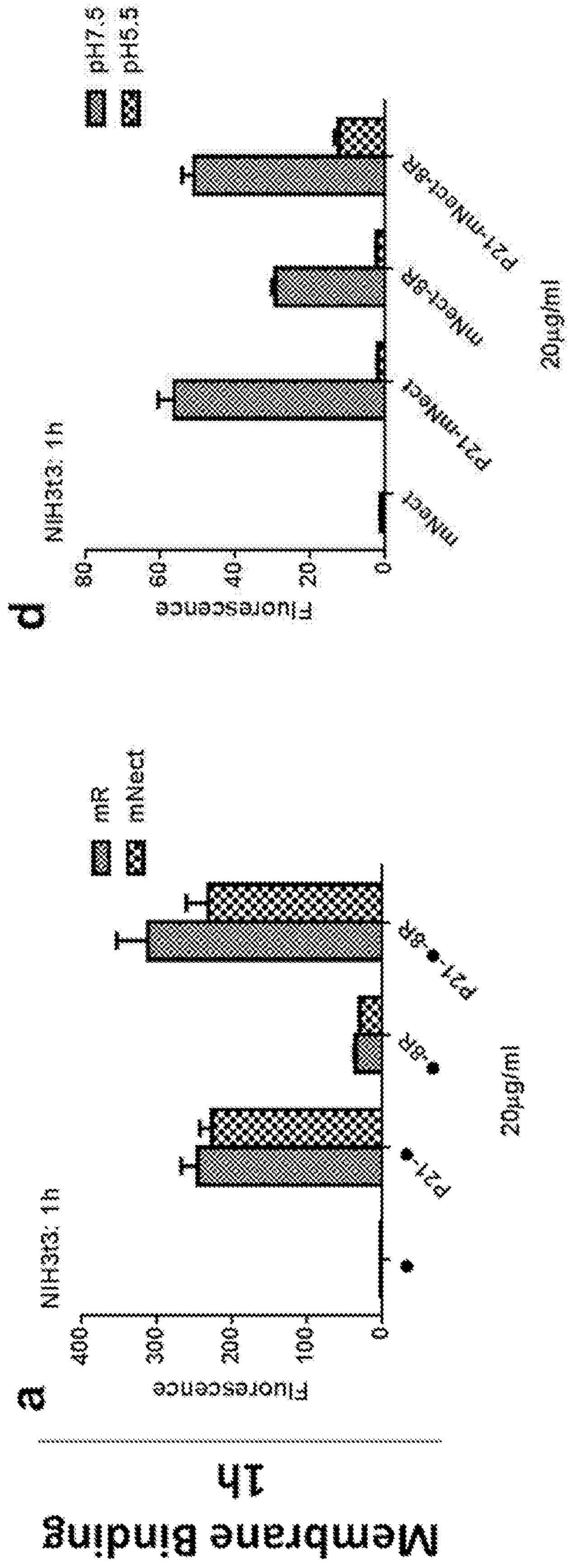
Membrane Binding
1h
a
NIH3t3: 1h
Fluorescence
mR
mNect
20µg/ml
d
NIH3t3: 1h
Fluorescence
pH7.5
pH5.5
mNect
P21-mNect
mNect-8R
P21-mNect-8R
20µg/ml

Figure 27 continued

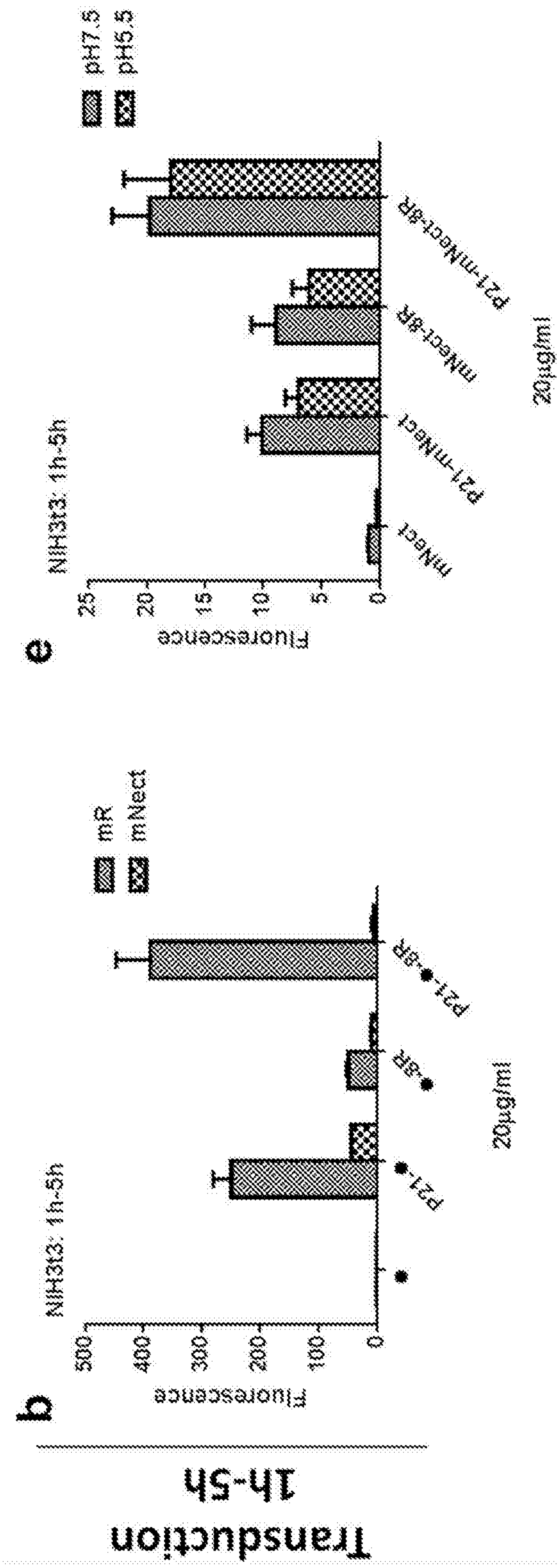

Figure 27 continued

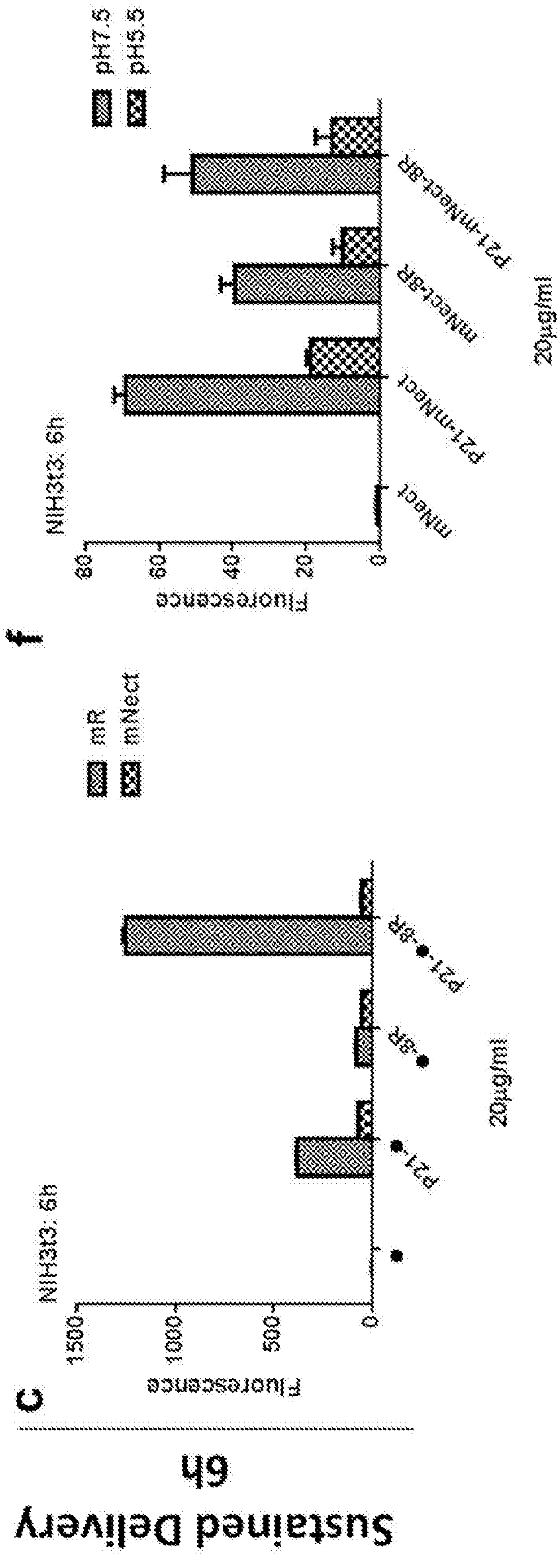

Figure 28

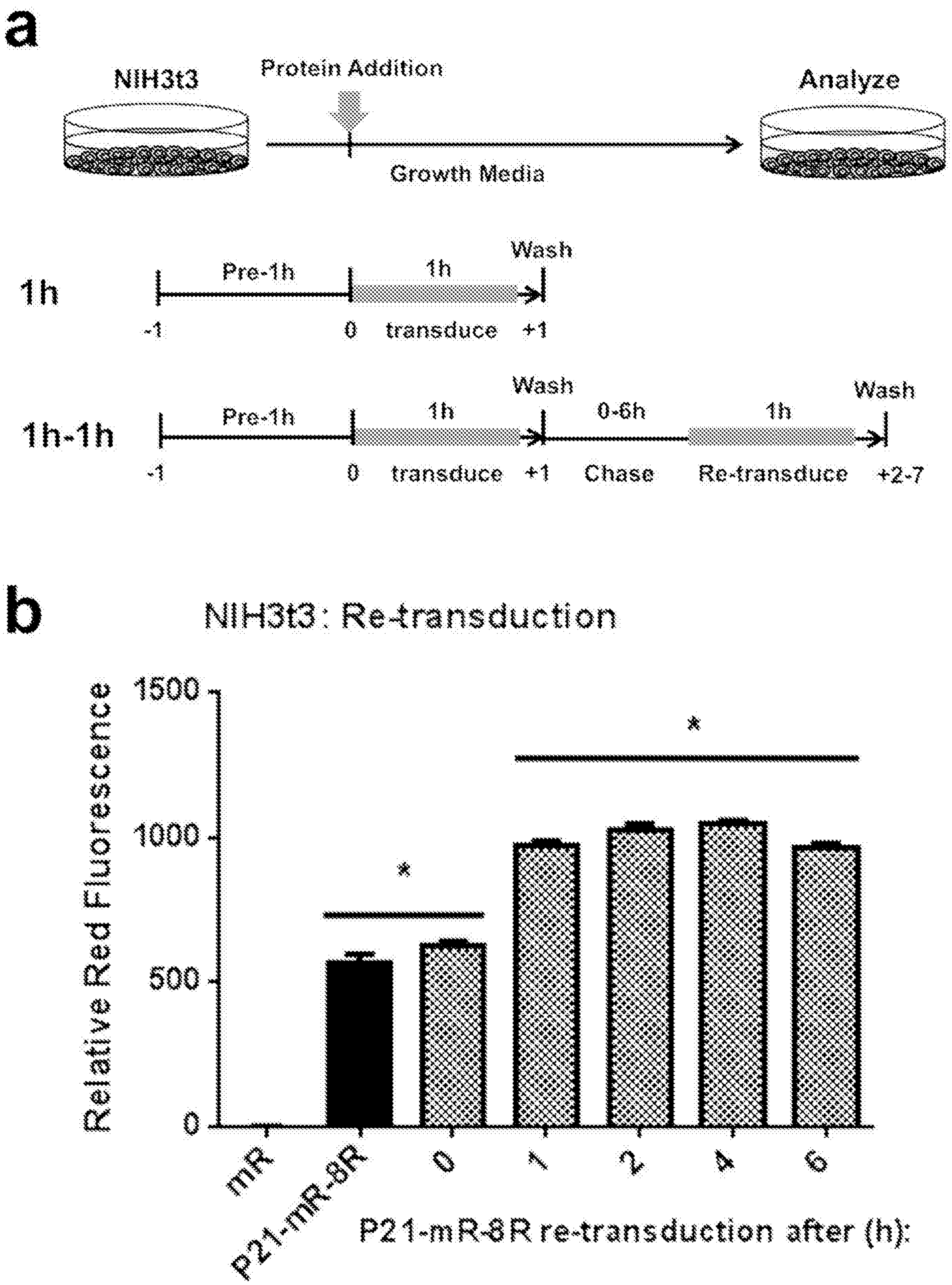
a
NIH3t3
Protein Addition
Analyze
Growth Media
1h
Pre-1h
1h
Wash
-1
0
transduce
+1
1h-1h
Pre-1h
1h
Wash
0-6h
1h
Wash
-1
0
transduce
+1
Chase
Re-transduce
+2-7
b
NIH3t3: Re-transduction
Relative Red Fluorescence
1500
1000
500
0
*
*
mR
P21-mR-8R
0
1
2
4
6
P21-mR-8R re-transduction after (h):

Figure 29

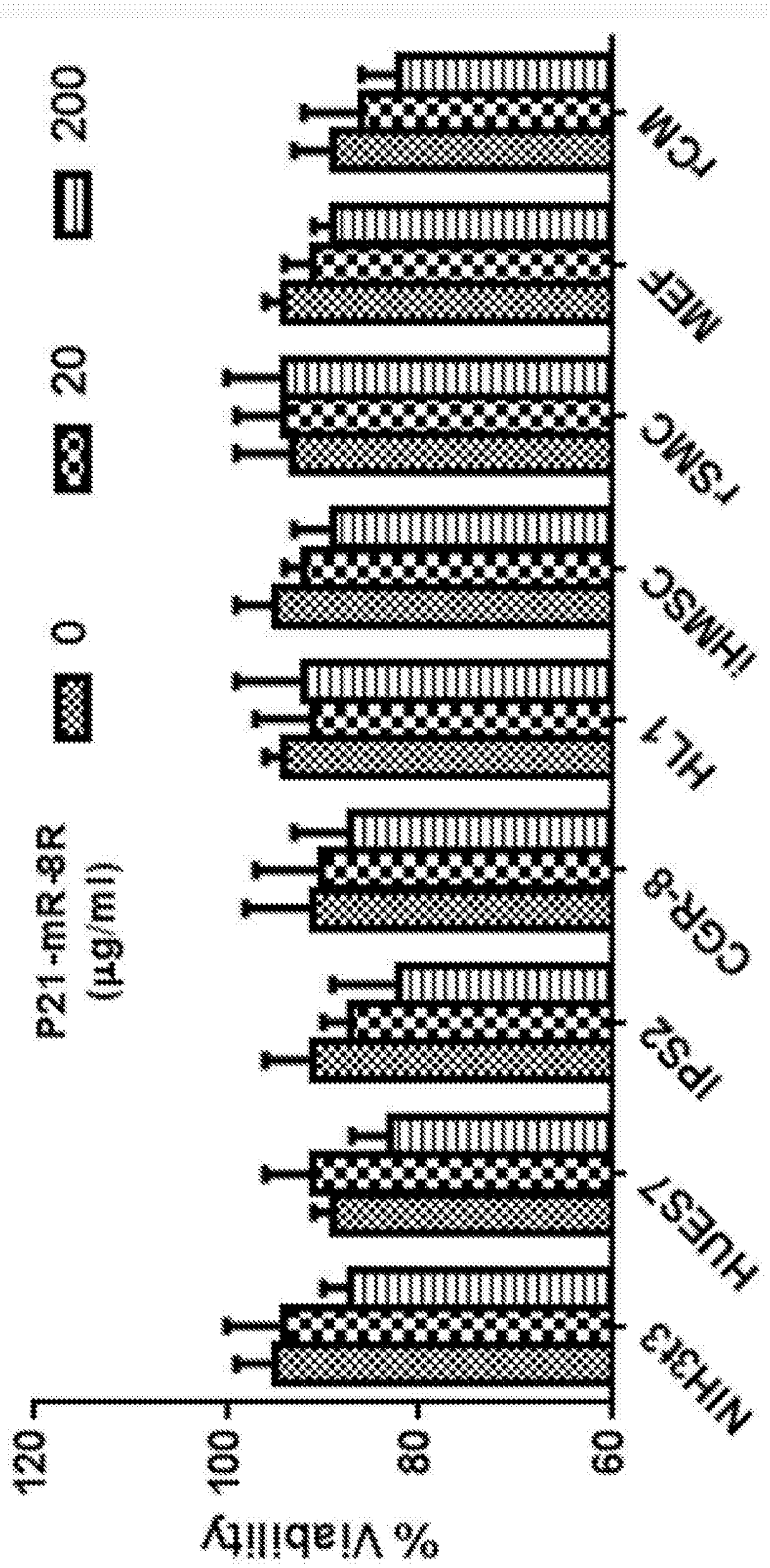
P21-mR-8R
(µg/ml)
0
20
200
% Viability
120
100
80
60
NIH3t3
HUES7
iPS2
CGR-8
HL-1
hMSC
rSMC
MEF
rCM

Figure 30

| | NIH 3t3: Incubated for 24 hours with 50 µg Nanomag-D (250 nm) | | | | | | | | |
|---|---|---|---|---|---|---|---|---|---|
| Peptide Conc (µg/ml) | 0 | 0.01 | 0.05 | 0.1 | 0.5 | 1 | 2 | 5 | 10 |
| P21 | | | | | | | | | |
| 8R | | | | | | | | | |
| P218R | | | | | | | | | |

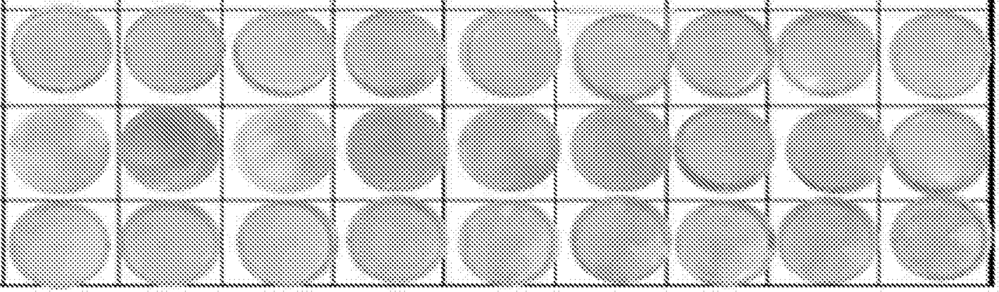

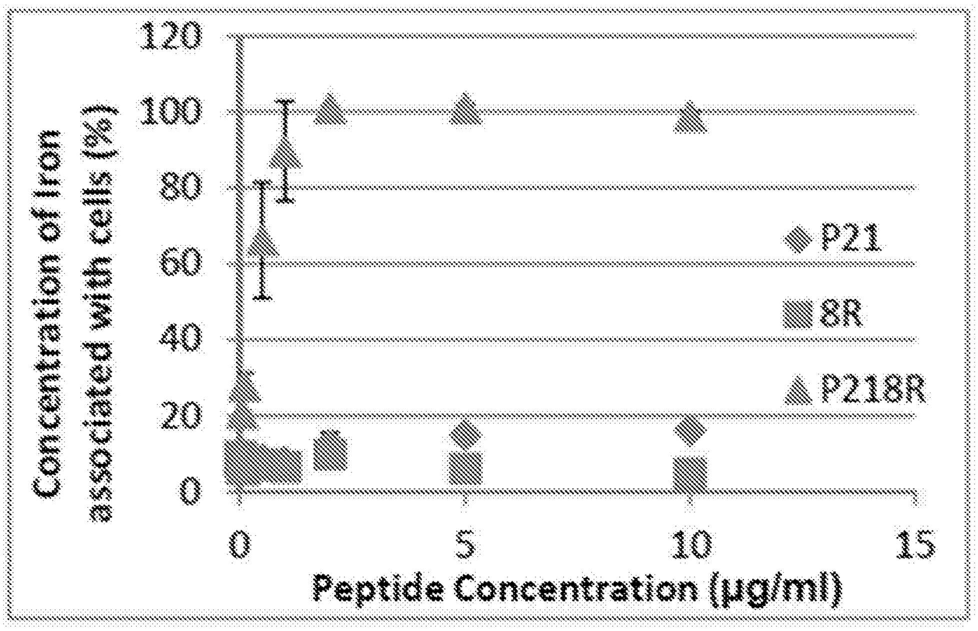

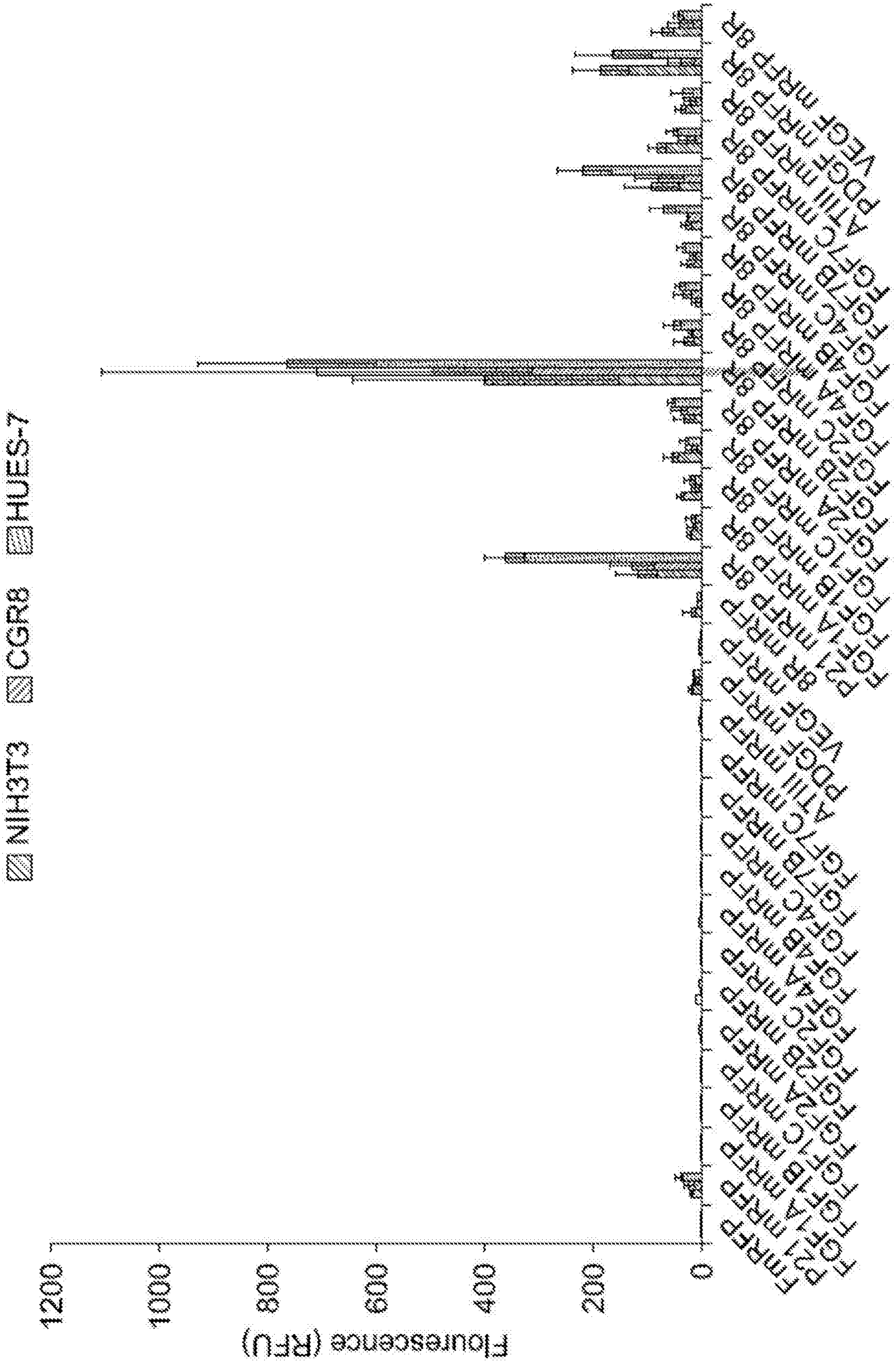
NIH3T3
CGR8
HUES-7
Flourescence (RFU)
0
200
400
600
800
1000
1200

Figure 31

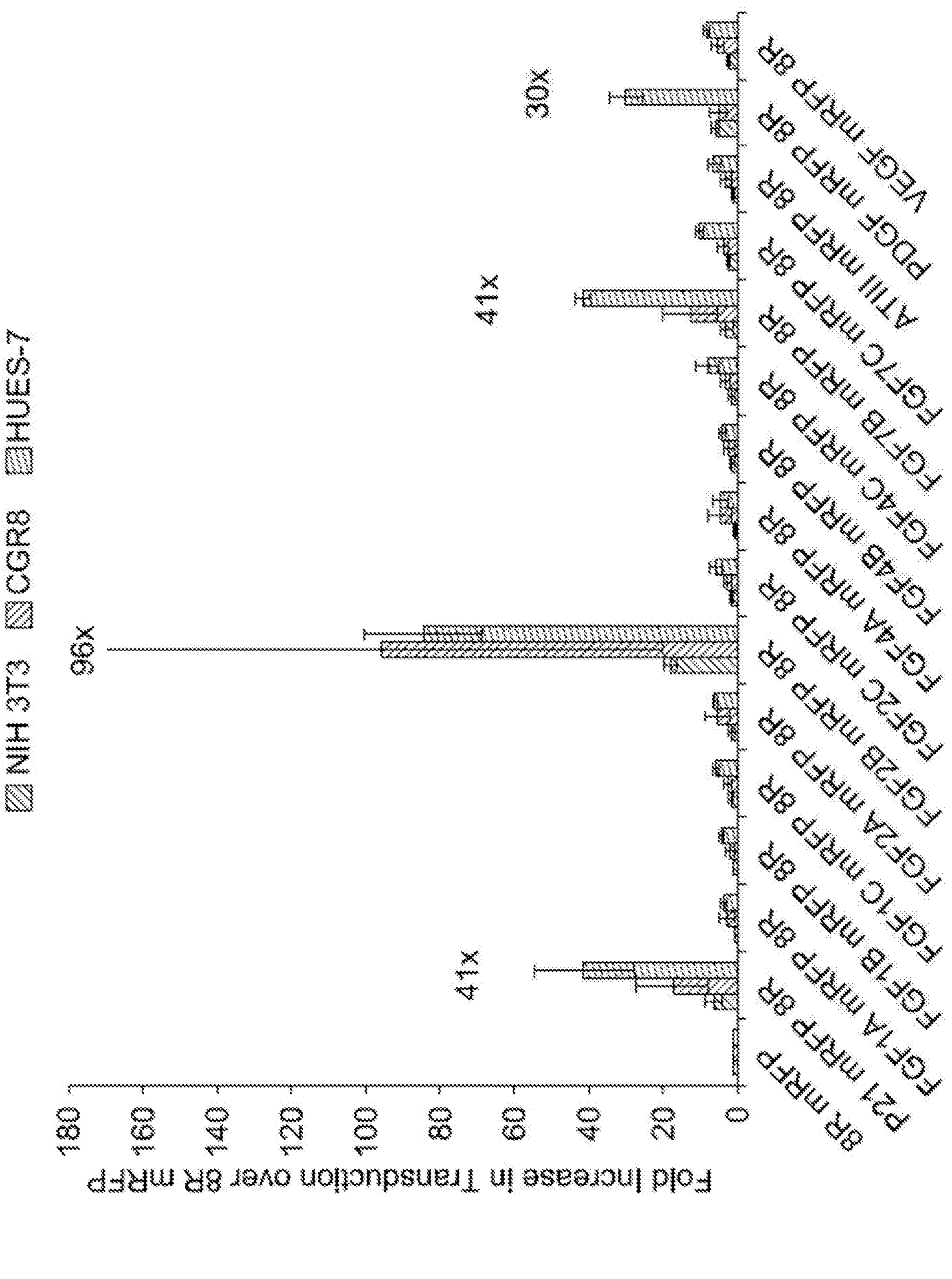
NIH 3T3
CGR8
HUES-7
Fold Increase in Transduction over 8R mRFP
180
160
140
120
100
80
60
40
20
0
41x
96x
41x
30x
8R mRFP
P21 mRFP 8R
FGF1A mRFP 8R
FGF1B mRFP 8R
FGF1C mRFP 8R
FGF2A mRFP 8R
FGF2B mRFP 8R
FGF2C mRFP 8R
FGF4A mRFP 8R
FGF4B mRFP 8R
FGF4C mRFP 8R
FGF7B mRFP 8R
FGF7C mRFP 8R
ATIII mRFP 8R
PDGF mRFP 8R
VEGF mRFP 8R

Figure 32

Figure 33

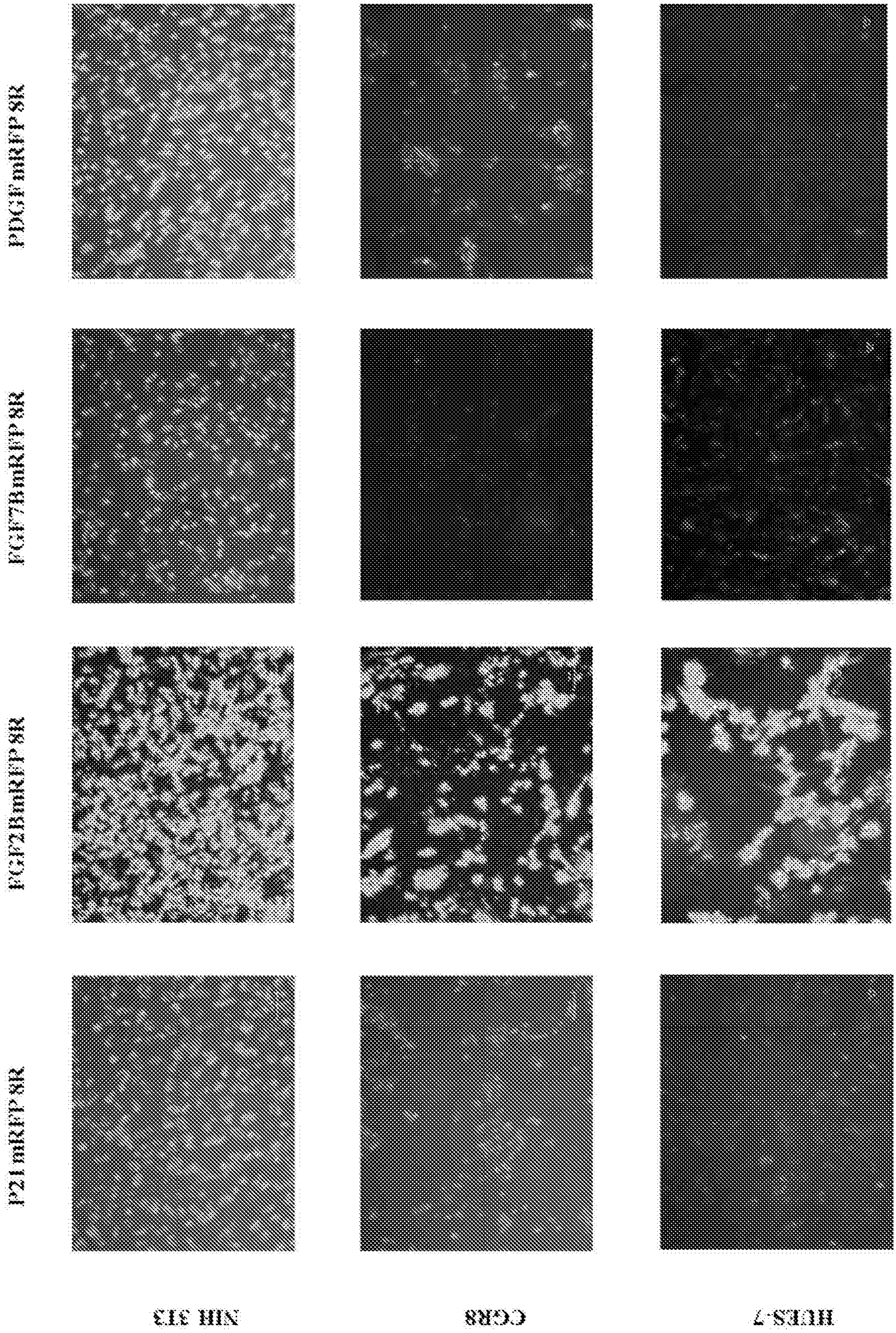
PDGF mRFP 8R
FGF7BmRFP 8R
FGF2BmRFP 8R
P21 mRFP 8R
NIH 3T3
CGR8
HUES-7

Figure 34

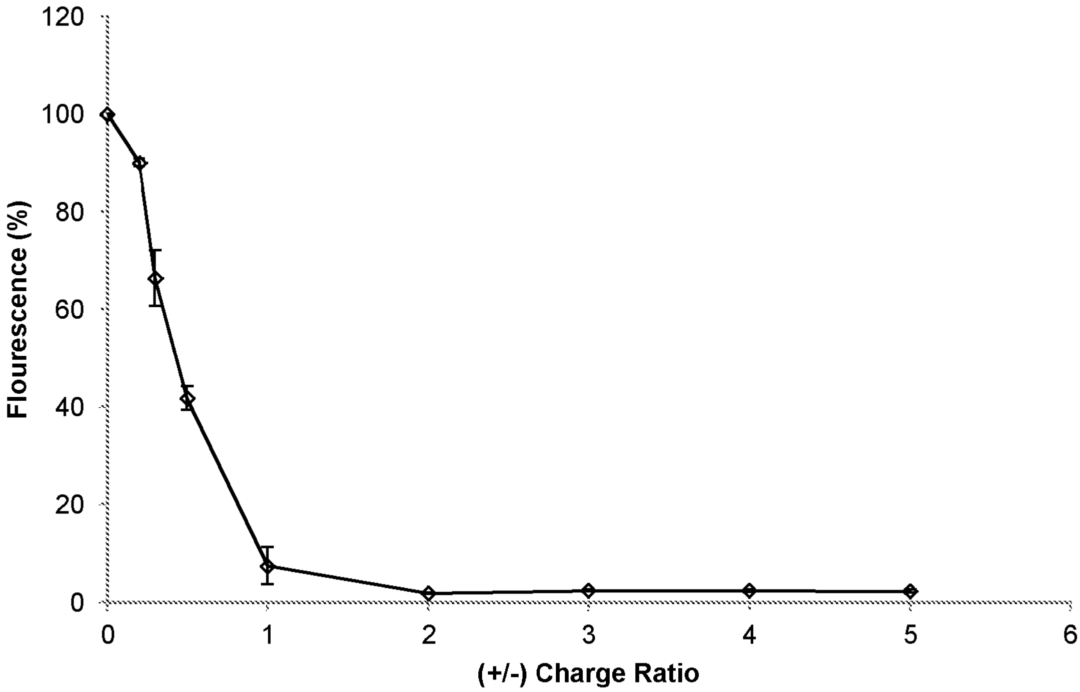

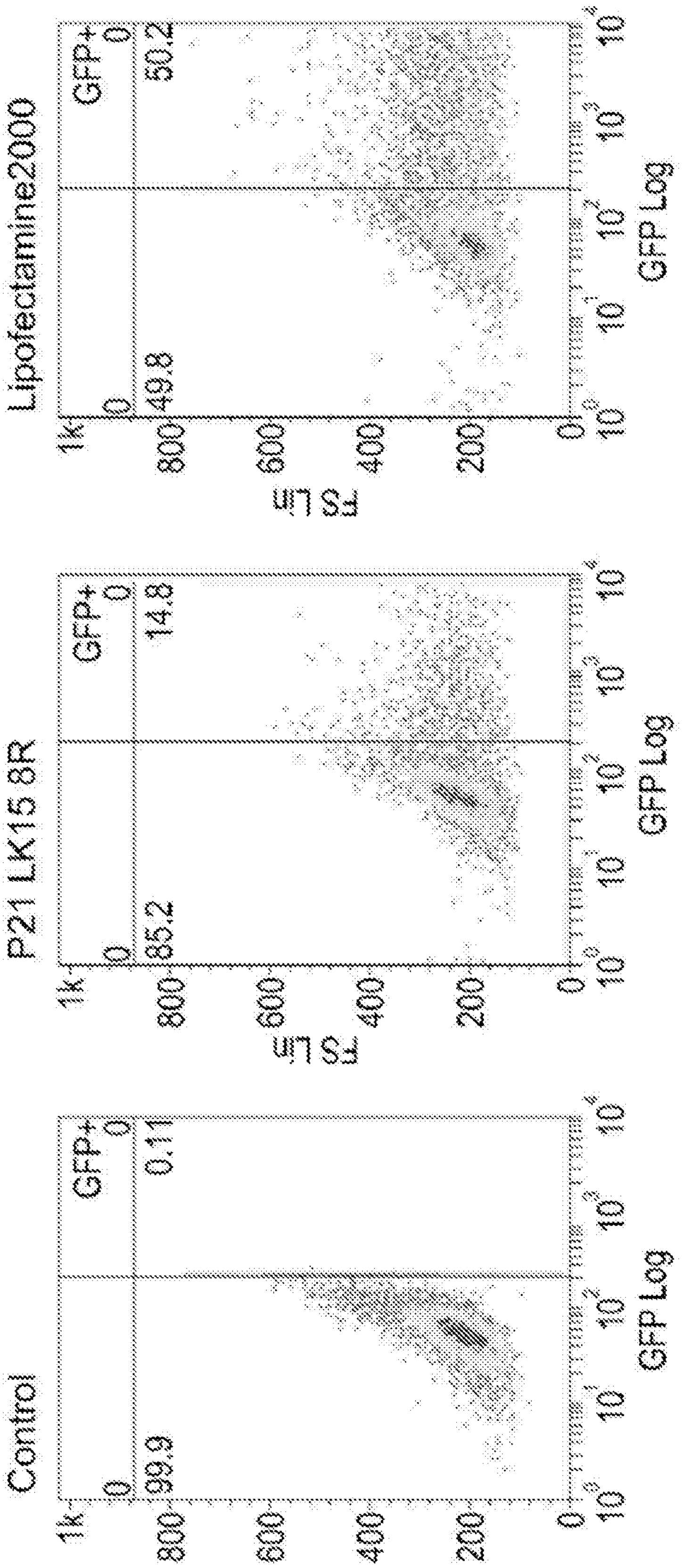
Control
99.9
0
GFP+
0
0.11
P21 LK15 8R
85.2
0
GFP+
0
14.8
Lipofectamine2000
49.8
0
GFP+
0
50.2
FS Lin
GFP Log
1k
800
600
400
200
0
$10^0$
$10^1$
$10^2$
$10^3$
$10^4$

Figure 35

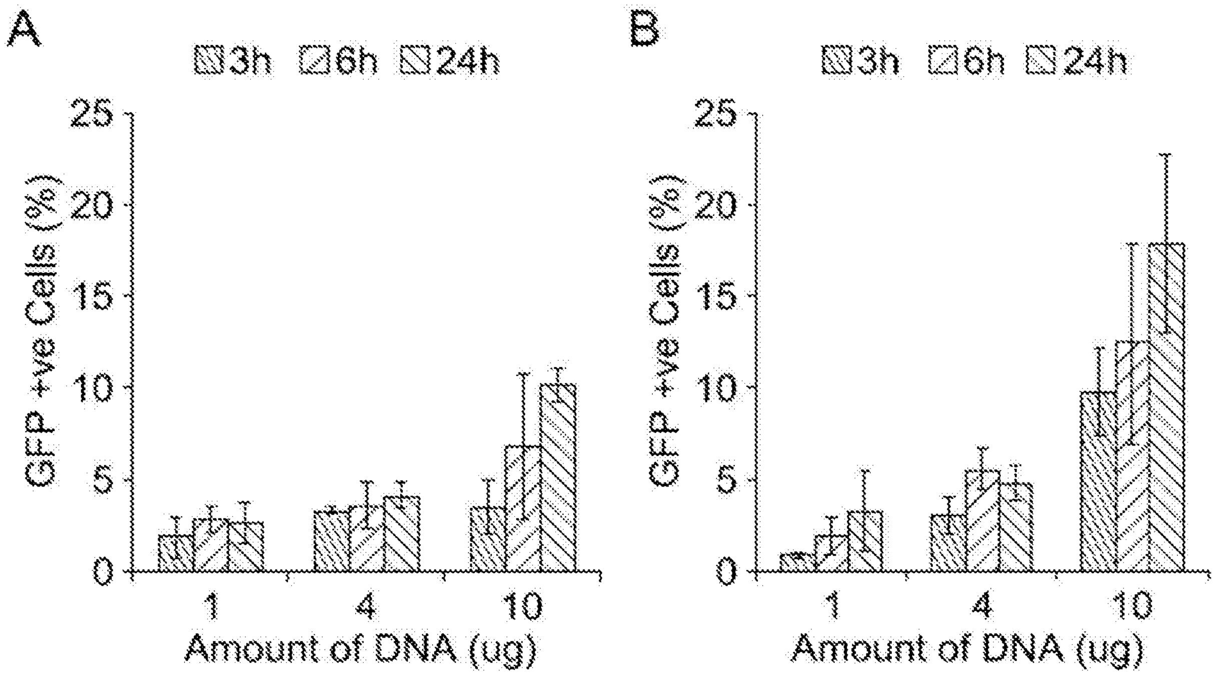


Figure 36

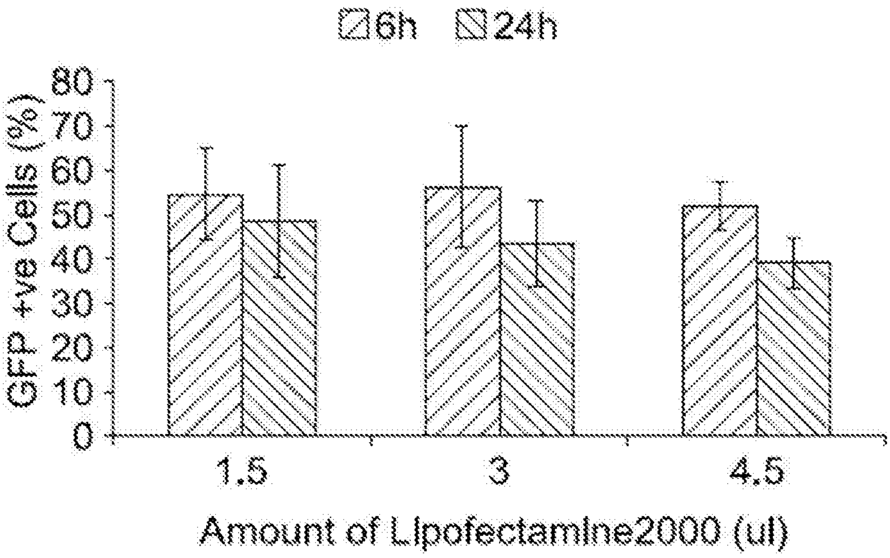


Figure 37

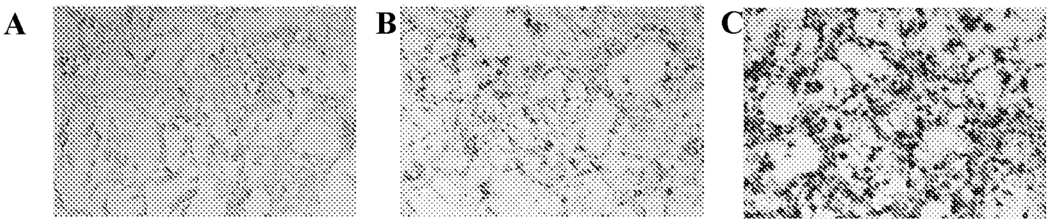


Figure 38

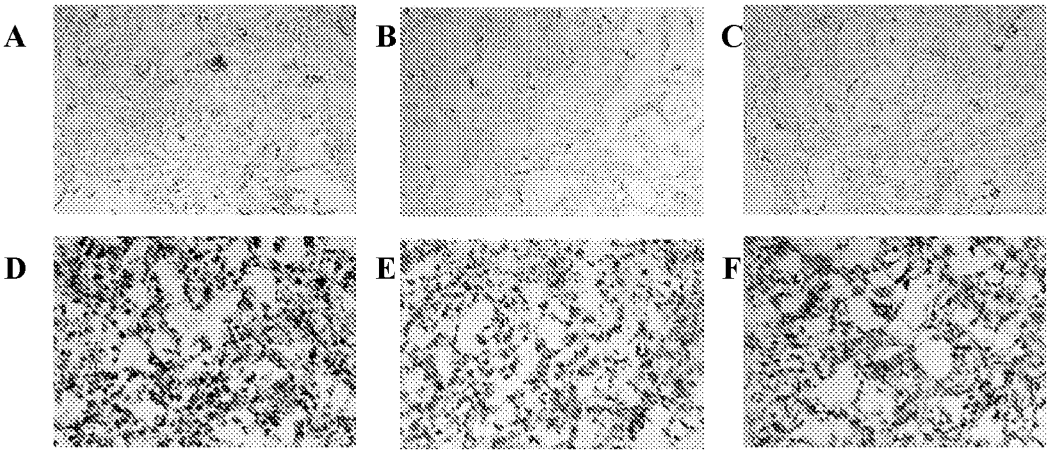


Figure 39

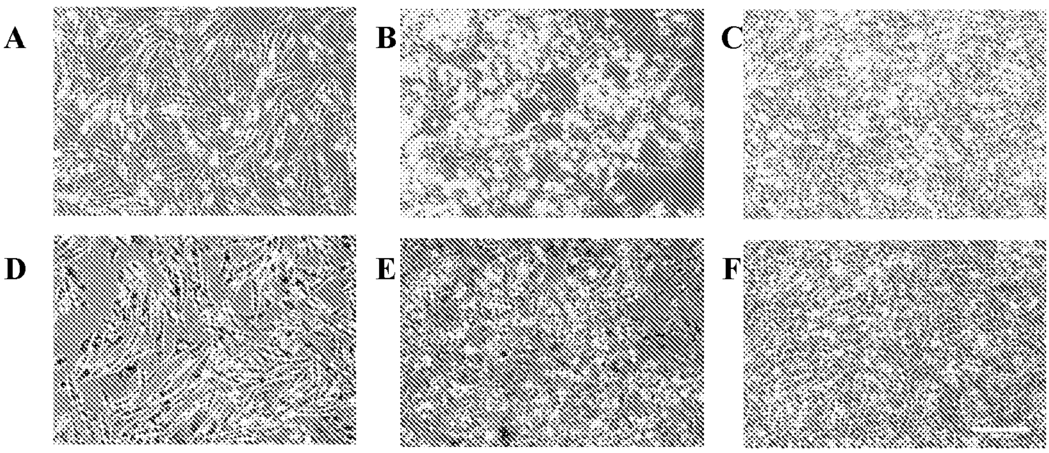


Figure 40

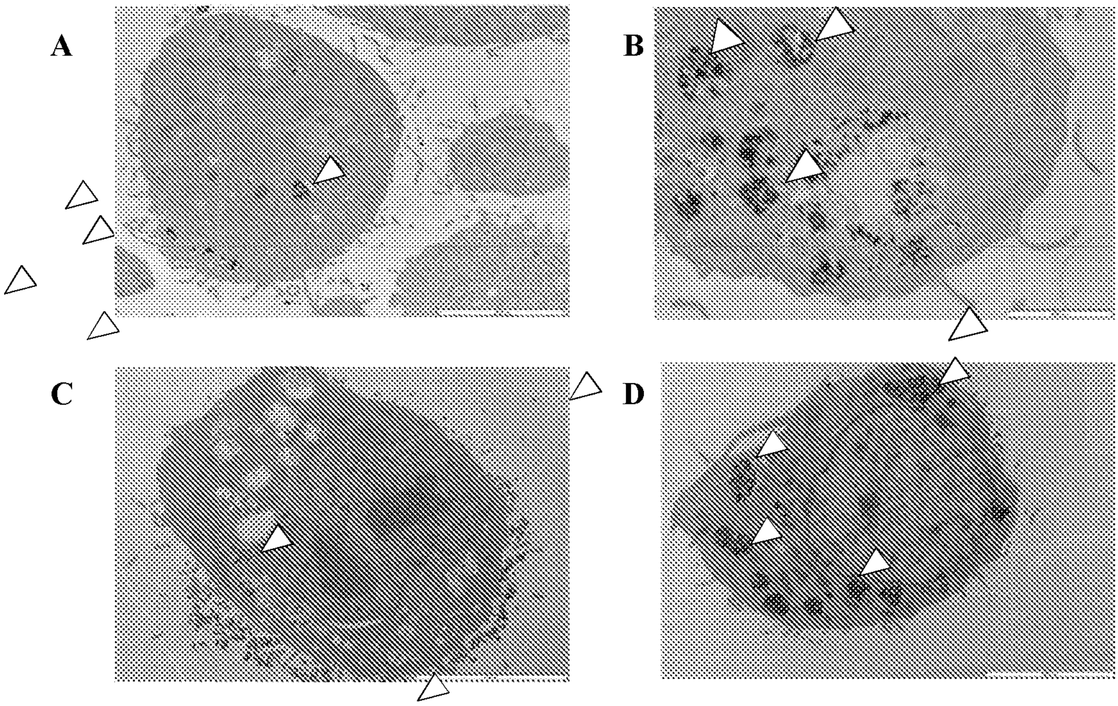


Figure 41

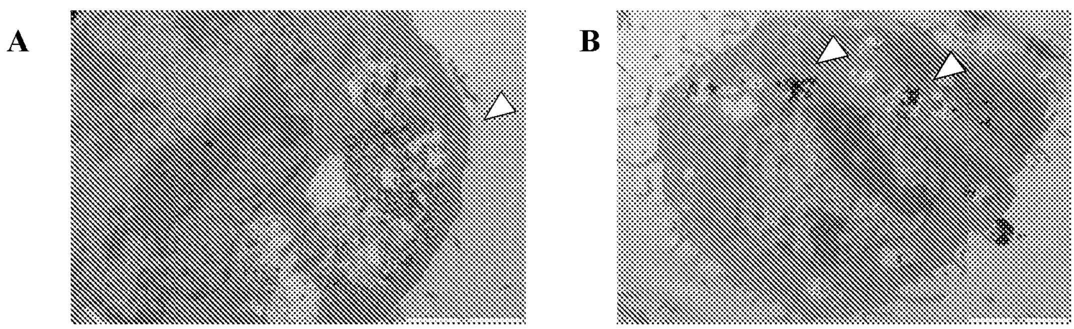


Figure 42

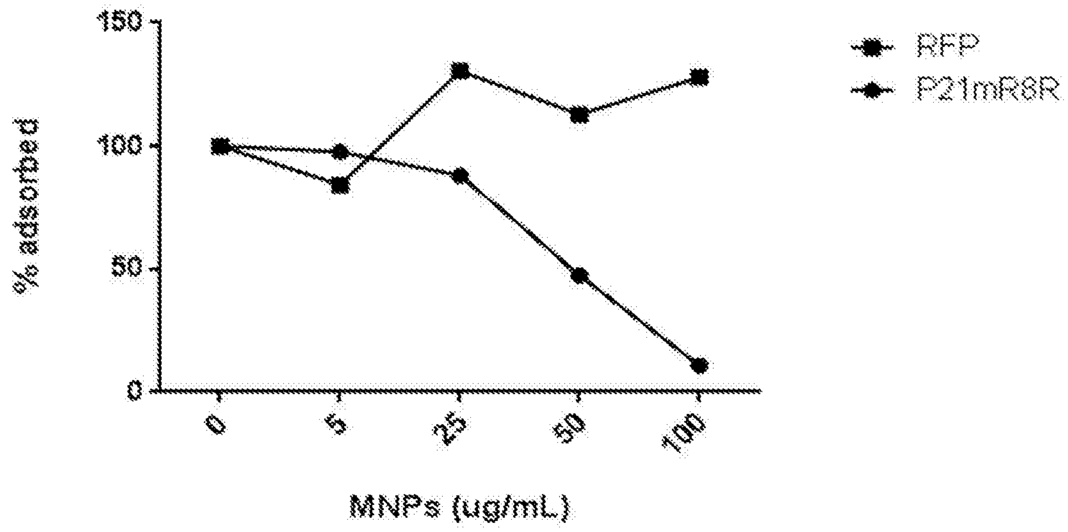


Figure 43

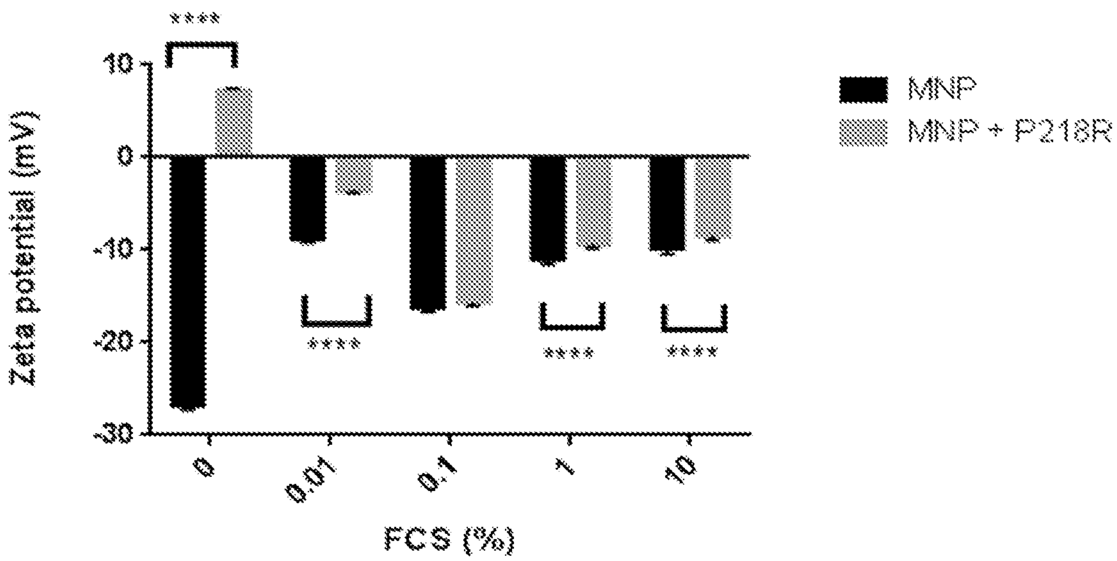


Figure 44

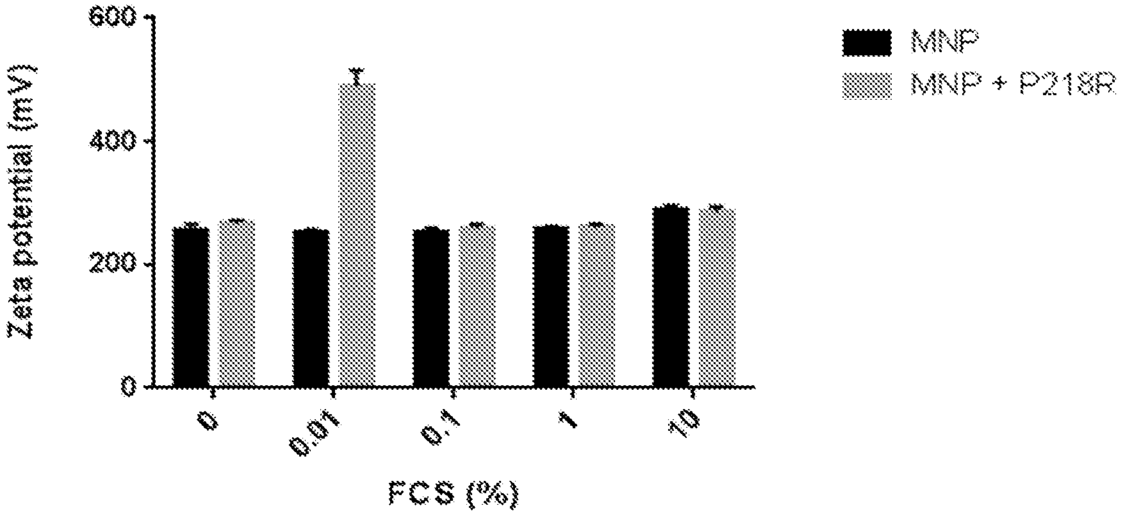


Figure 45

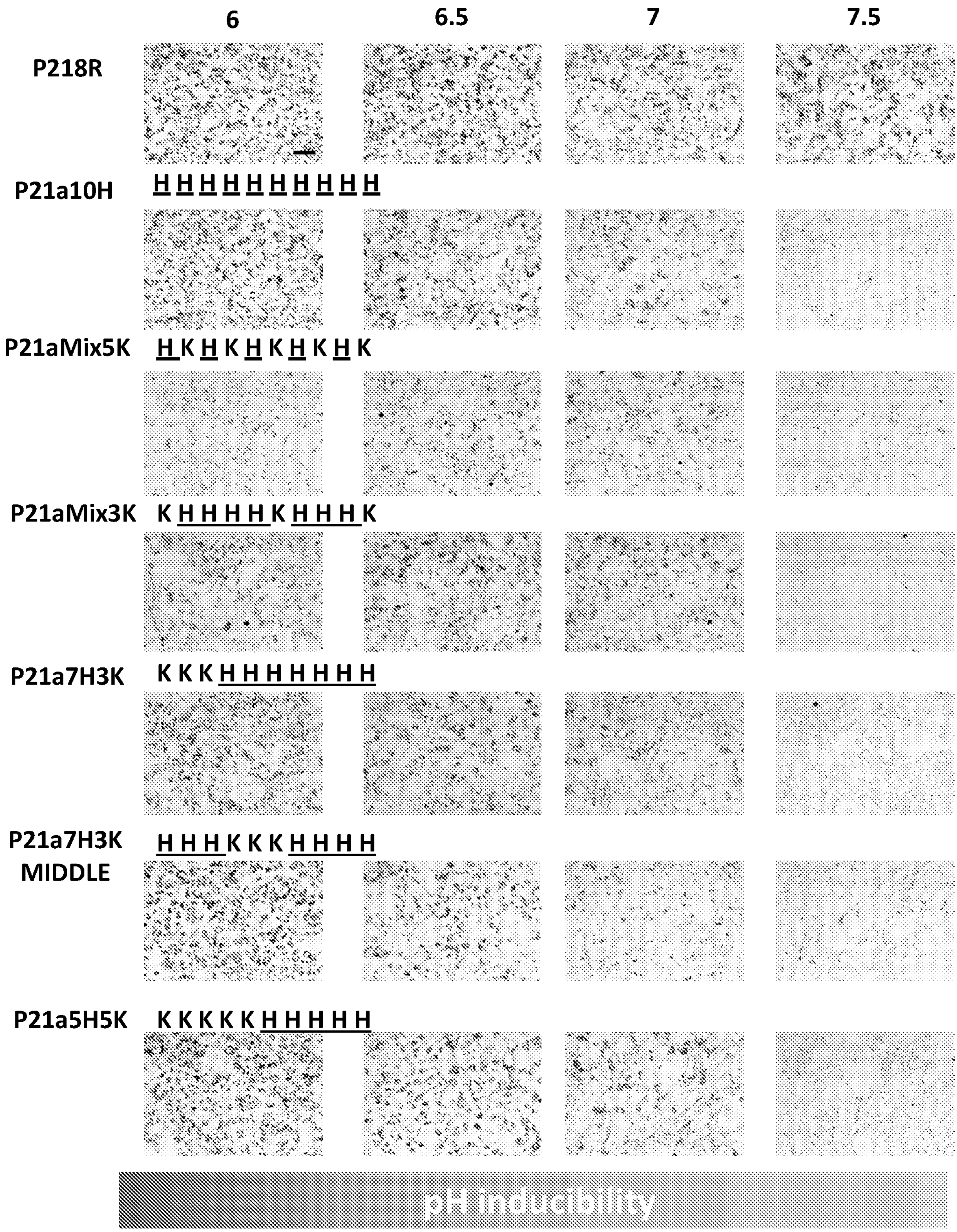
6
6.5
7
7.5
P218R
P21a10H
H H H H H H H H H H
P21aMix5K
H K H K H K H K H K
P21aMix3K
K H H H H K H H H K
P21a7H3K
K K K H H H H H H H
P21a7H3K MIDDLE
H H H K K K H H H H
P21a5H5K
K K K K K H H H H H
pH inducibility

Figure 46

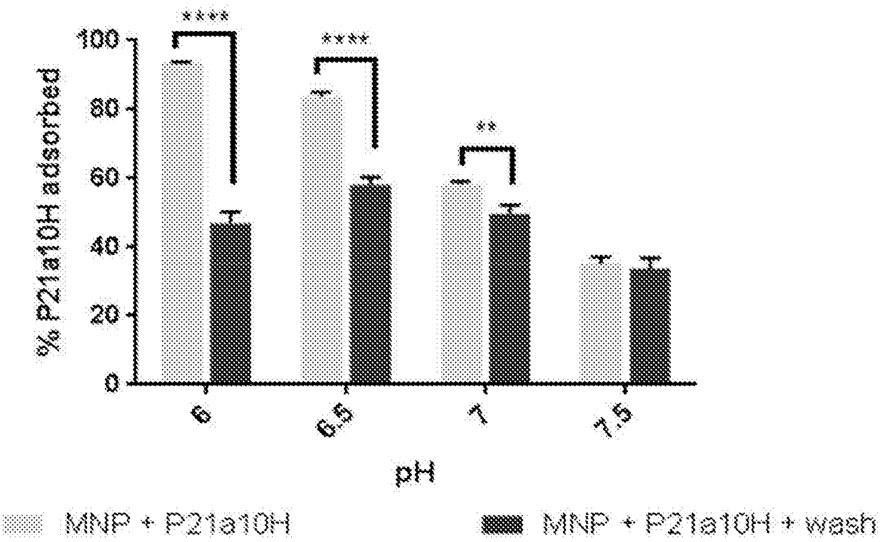


Figure 47

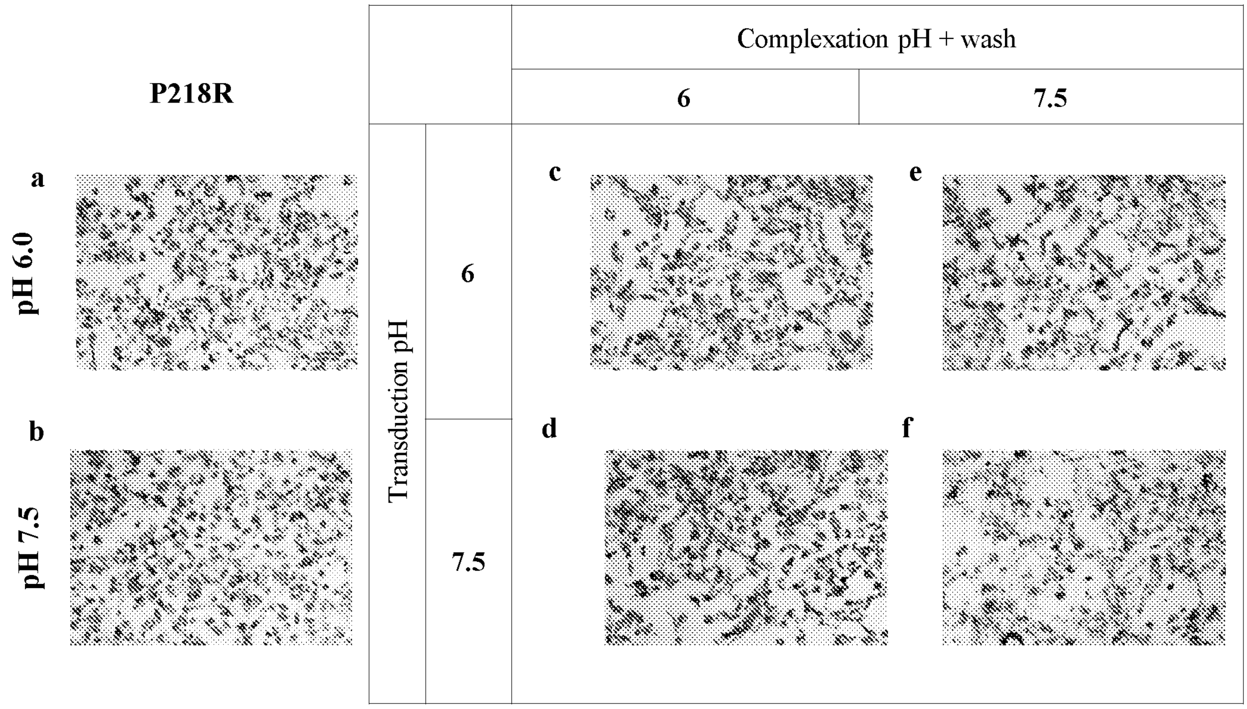


Figure 48

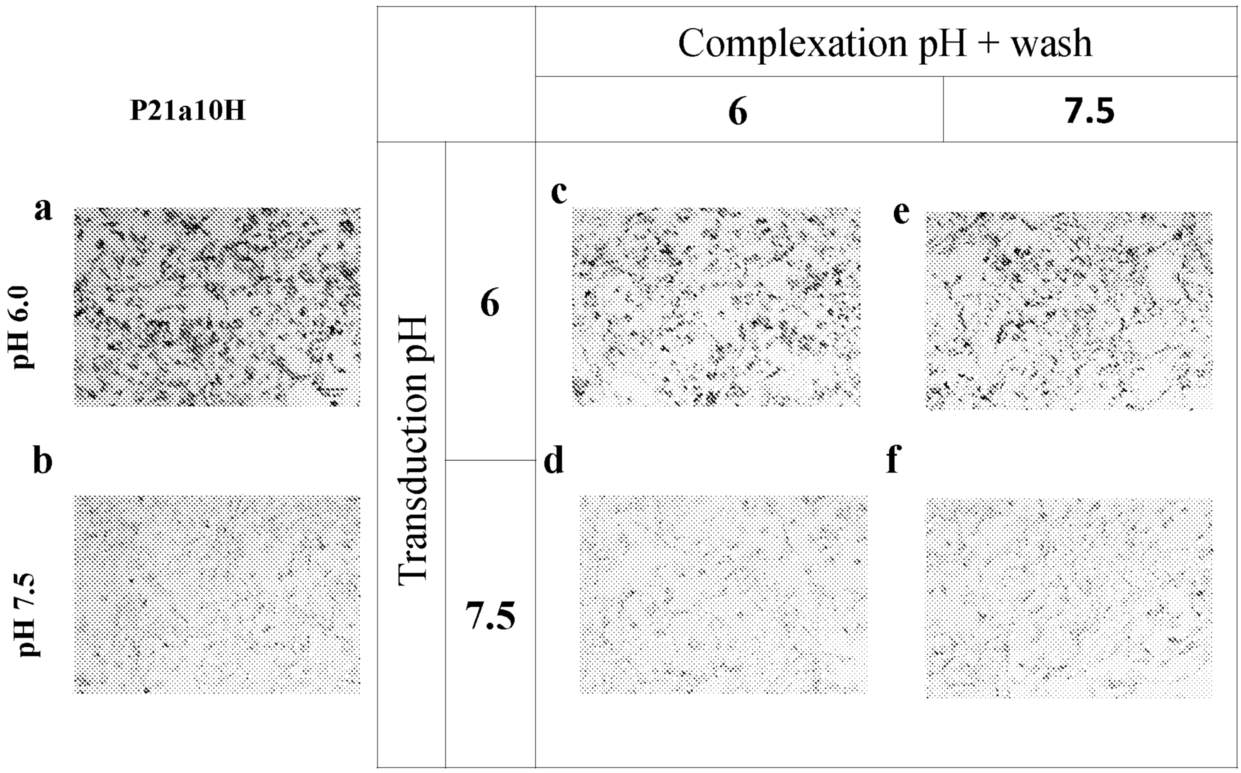


Figure 49

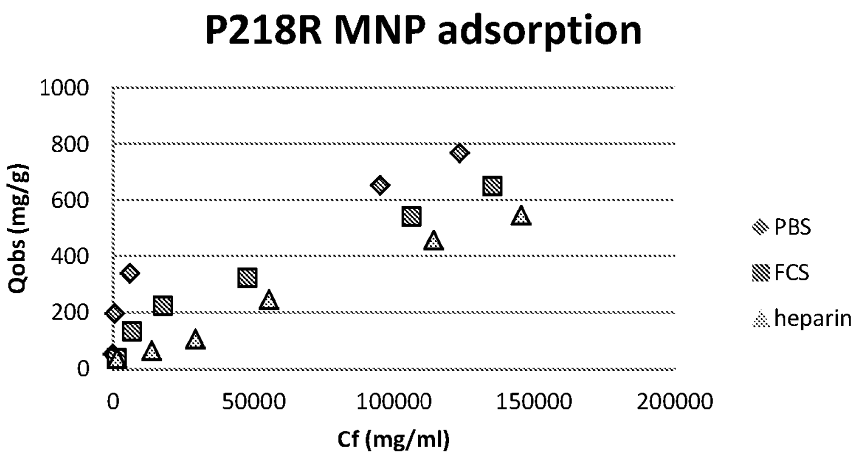


Figure 50

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015092417 A
- WO 2007042775 A
- WO 2013189663 A
- GB 1511159 A

### Non-patent literature cited in the description


- **ZHANG et al.** *Biomaterials,* 2013, vol. 34 (32), 7980-7993
- **BRUNDEN et al.** *J Neurochem,* 1993, vol. 61 (6), 2147-2154
- **DIXON et al.** *PNAS,* 2016, vol. 113 (5), E291-E299
- **LINDBERG et al.** *International Journal of Pharmaceutics,* 2013, vol. 441, 242-247
- **BERG et al.** *J. Photochemistry and Photobiology,* 1997, vol. 65, 403-409
- **WANG et al.** *Journal of Controlled Release,* 2012, vol. 157 (2), 305-313
- **BEERENS, A. ; AL HADITHY ; A., ROTS, M. ; HAISMA, H.** Protein Transduction Domains and their Utility in Gene Therapy. *Current Gene Therapy,* 2003, vol. 3 (5), 486-494
- **BYRNE, J. M. ; COKER, V. S. ; CESPEDES, E. ; WINCOTT, P. L. ; VAUGHAN, D. J. ; PATTRICK, R. A. D. ; TELLING, N. D.** Biosynthesis of Zinc Substituted Magnetite Nanoparticles with Enhanced Magnetic Properties. *Advanced Functional Materials,* 2014, vol. 24 (17), 2518-2529
- **DIXON, J. E. ; MORRIS, G. ; LANE, N. ; DENNING, C. et al.** *Highly efficient delivery of functional 545 proteins by the synergistic effect of GAG binding motifs and cell-penetrating peptides,* 2014
- **FALK, M. H. ; ISSELS, R.D.** Hyperthermia in Oncology. *International Journal of Hyperthermia,* 2001, vol. 17 (1), 1-18
- **GRIFFITHS, J. R.** Are cancer cells acidic. *British Journal of Cancer,* April 1991, vol. 64, 425-427
- **HERGT, R. ; DUTZ, S. ; MÜLLER, R. ; ZEISBERGER, M.** Magnetic particle hyperthermia: nanoparticle magnetism and materials development for cancer therapy. *Journal of Physics: Condensed Matter,* 2006, vol. 18 (38), S2919-S2934
- **KUMAR, C. S. S. R. ; MOHAMMAD, F.** Magnetic nanomaterials for hyperthermia-based therapy and controlled drug delivery. *Advanced Drug Delivery Reviews,* 2011, vol. 63 (9), 789-808
- **VAUPEL, P.** Tumor microenvironmental physiology and its implications for radiation oncology. *Seminars in Radiation Oncology,* 2004, vol. 14 (3), 198-206
- **WANG, Z. ; CUSCHIERI, A.** Tumour cell labelling by magnetic nanoparticles with determination of intracellular iron content and spatial distribution of the intracellular iron. *International Journal of Molecular Sciences,* 2013, vol. 14 (5), 9111-25
- **GUMP JM ; DOWDY SF.** TAT transduction: the molecular mechanism and therapeutic prospects. *Trends in molecular medicine,* 2007, vol. 13 (10), 443-448
- **EL-ANDALOUSSI S ; HOLM T ; LANGEL U.** Cell-penetrating peptides: Mechanisms and applications. *Curr Pharm Design,* 2005, vol. 11 (28), 3597-3611
- **GOUN EA ; PILLOW TH ; JONES LR ; ROTHBARD JB ; WENDER PA.** Molecular transporters: Synthesis of oligoguanidinium transporters and their application to drug delivery and real-time imaging. *Chembiochem,* 2006, vol. 7 (10), 1497-1515
- **MEADE BR ; DOWDY SF.** Exogenous siRNA delivery using peptide transduction domains/cell penetrating peptides. *Adv Drug Deliver Rev,* 2007, vol. 59, 134-140
- **FISCHER R ; FOTIN-MLECZEK M ; HUFNAGEL H ; BROCK R.** Break on through to the other side - Biophysics and cell biology shed light on cell-penetrating peptides. *Chembiochem,* 2005, vol. 6 (12), 2126-2142
- **NAKASE I ; TAKEUCHI T ; TANAKA G ; FUTAKI S.** Methodological and cellular aspects that govern the internalization mechanisms of arginine-rich cell-penetrating peptides. *Adv Drug Deliver Rev,* 2008, vol. 60 (4-5), 598-607
- **HEITZ F ; MORRIS MC ; DIVITA G.** Twenty years of cell-penetrating peptides: from molecular mechanisms to therapeutics. *Brit J Pharmacol,* 2009, vol. 157 (2), 195-206

- **GUMP JM ; JUNE RK ; DOWDY SF.** Revised Role of Glycosaminoglycans in TAT Protein Transduction Domain-mediated Cellular Transduction. *J Biol Chem,* 2010, vol. 285 (2), 1500-1507
- **NORBURY CC ; HEWLETT LJ ; PRESCOTT AR ; SHASTRI N ; WATTS C.** Class I MHC presentation of exogenous soluble antigen via macropinocytosis in bone marrow macrophages. *Immunity,* 1995, vol. 3 (6), 783-791
- **MEIER O et al.** Adenovirus triggers macropinocytosis and endosomal leakage together with its clathrin-mediated uptake. *J Cell Biol,* 2002, vol. 158 (6), 1119-1131
- **CONNER SD ; SCHMID SL.** Regulated portals of entry into the cell. *Nature,* 2003, vol. 422 (6927), 37-44
- **WADIA JS ; STAN RV ; DOWDY SF.** Transducible TAT-HA fusogenic peptide enhances escape of TAT-fusion proteins after lipid raft macropinocytosis. *Nat Med,* 2004, vol. 10 (3), 310-315
- **SKEHEL JJ ; CROSS K ; STEINHAUER D ; WILEY DC.** Influenza fusion peptides. *Biochem Soc T,* 2001, vol. 29, 623-626
- **HAN X ; BUSHWELLER JH ; CAFISO DS ; TAMM LK.** Membrane structure and fusion-triggering conformational change of the fusion domain from influenza hemagglutinin. *Nat Struct Biol,* 2001, vol. 8 (8), 715-720
- **SAKUMA T ; HIGASHIYAMA S ; HOSOE S ; HAYASHI S ; TANIGUCHI N.** CD9 antigen interacts with heparin-binding EGF-like growth factor through its heparin-binding domain. *Journal of biochemistry,* 1997, vol. 122 (2), 474-480
- **HIGASHIYAMA S ; ABRAHAM JA ; KLAGSBRUN M.** Heparin-Binding Egf-Like Growth-Factor Stimulation of Smooth-Muscle Cell-Migration - Dependence on Interactions with Cell-Surface Heparan-Sulfate. *J Cell Biol,* 1993, vol. 122 (4), 933-940
- **THOMPSON SA et al.** Characterization of Sequences within Heparin-Binding Egf-Like Growth-Factor That Mediate Interaction with Heparin. *J Biol Chem,* 1994, vol. 269 (4), 2541-2549
- **KAPLAN IM ; WADIA JS ; DOWDY SF.** *Cationic TAT peptide transduction domain enters cells by macropinocytosis,* 2005, vol. 102, 247
- *J Control Release,* 2005, vol. 107 (3), 571-572
- **LAWRENCE R ; LU H ; ROSENBERG RD ; ESKO JD ; ZHANG LJ.** Disaccharide structure code for the easy representation of constituent oligosaccharides from glycosaminoglycans. *Nat Methods,* 2008, vol. 5 (4), 291-292
- **LIN X et al.** Disruption of gastrulation and heparan sulfate biosynthesis in EXT1-deficient mice. *Dev Biol,* 2000, vol. 224 (2), 299-311
- **DICK E ; MATSA E ; YOUNG LE ; DARLING D ; DENNING C.** Faster generation of hiPSCs by coupling high-titer lentivirus and column-based positive selection. *Nat Protoc,* 2011, vol. 6 (6), 701-714
- **ANDERSON RGW.** The caveolae membrane system. *Annu Rev Biochem,* 1998, vol. 67, 199-225
- **NICHOLS BJ ; LIPPINCOTT-SCHWARTZ J.** Endocytosis without clathrin coats. *Trends Cell Biol,* 2001, vol. 11 (10), 406-412
- **LIU NQ et al.** Human immunodeficiency virus type 1 enters brain microvascular endothelia by macropinocytosis dependent on lipid rafts and the mitogen-activated protein kinase signaling pathway. *J Virol,* 2002, vol. 76 (13), 6689-6700
- **WEST MA ; BRETSCHER MS ; WATTS C.** Distinct Endocytotic Pathways in Epidermal Growth Factor-Stimulated Human Carcinoma A431 Cells. *J Cell Biol,* 1989, vol. 109 (6), 2731-2739
- **SAMPATH P ; POLLARD TD.** Effects of Cytochalasin, Phalloidin, and Ph on the Elongation of Actin-Filaments. *Biochemistry-Us,* 1991, vol. 30 (7), 1973-1980
- **OLIVER JM ; BERLIN RD ; DAVIS BH.** Use of Horseradish-Peroxidase and Fluorescent Dextrans to Study Fluid Pinocytosis in Leukocytes. *Method Enzymol,* 1984, vol. 108, 336-347
- **ARAKI N ; JOHNSON MT ; SWANSON JA.** A role for phosphoinositide 3-kinase in the completion of macropinocytosis and phagocytosis by macrophages. *J Cell Biol,* 1996, vol. 135 (5), 1249-1260
- **SEGLEN PO ; GRINDE B ; SOLHEIM AE.** Inhibition of the Lysosomal Pathway of Protein-Degradation in Isolated Rat Hepatocytes by Ammonia, Methylamine, Chloroquine and Leupeptin. *Eur JBiochem,* 1979, vol. 95 (2), 215-225
- **EUSTICE DC ; WILHELM JM.** Mechanisms of Action of Aminoglycoside Antibiotics in Eukaryotic Protein-Synthesis. *Antimicrob Agents Ch,* 1984, vol. 26 (1), 53-60
- **YU JY ; CHAU KF ; VODYANIK MA ; JIANG JL ; JIANG Y.** Efficient Feeder-Free Episomal Reprogramming with Small Molecules. *Plos One,* 2011, vol. 6 (3
- **WARREN L et al.** Highly Efficient Reprogramming to Pluripotency and Directed Differentiation of Human Cells with Synthetic Modified mRNA. *Cell Stem Cell,* 2010, vol. 7 (5), 618-630
- **KIM D et al.** Generation of human induced pluripotent stem cells by direct delivery of reprogramming proteins. *Cell Stem Cell,* 2009, vol. 4 (6), 472-476
- **ZHOU HY et al.** *Generation of Induced Pluripotent Stem Cells Using Recombinant Proteins,* 2009, vol. 4, 381
- *Cell Stem Cell,* 2009, vol. 4 (6), 581-581
- **TAKAHASHI K et al.** Induction of pluripotent stem cells from adult human fibroblasts by defined factors. *Cell,* 2007, vol. 131 (5), 861-872

- **DIXON JE et al.** Axolotl Nanog activity in mouse embryonic stem cells demonstrates that ground state pluripotency is conserved from urodele amphibians to mammals. *Development,* 2010, vol. 137 (18), 2973-2980
- **CHAMBERS I et al.** Functional expression cloning of Nanog, a pluripotency sustaining factor in embryonic stem cells. *Cell,* 2003, vol. 113 (5), 643-655
- **ROBINTON DA ; DALEY GQ.** The promise of induced pluripotent stem cells in research and therapy. *Nature,* 2012, vol. 481 (7381), 295-305
- **DO KWON Y et al.** Cellular manipulation of human embryonic stem cells by TAT-PDX1 protein transduction. *Mol Ther,* 2005, vol. 12 (1), 28-32
- **HIDEMA S ; TONOMURA Y ; DATE S ; NISHIMORI K.** Effects of protein transduction with intact myogenic transcription factors tagged with HIV-1 Tat-PTD (T-PTD) on myogenic differentiation of mouse primary cells. *J Biosci Bioeng,* 2012, vol. 113 (1), 5-11
- **LIANG QL ; MO ZY ; LI XF ; WANG XX ; LI RM.** Pdx1 protein induces human embryonic stem cells into the pancreatic endocrine lineage. *Cell Biol Int,* 2013, vol. 37 (1), 2-10
- **BICHSEL C et al.** Direct Reprogramming of Fibroblasts to Myocytes via Bacterial Injection of MyoD Protein. *Cell Reprogram,* 2013, vol. 15 (2), 117-125
- **CHAN EM et al.** Live cell imaging distinguishes bona fide human iPS cells from partially reprogrammed cells. *Nat Biotechnol,* 2009, vol. 27 (11), 1033-U1100
- **SMITH KP ; LUONG MX ; STEIN GS.** Pluripotency: Toward a Gold Standard for Human ES and iPS Cells. *J Cell Physiol,* 2009, vol. 220 (1), 21-29
- **BURRIDGE PW et al.** A Universal System for Highly Efficient Cardiac Differentiation of Human Induced Pluripotent Stem Cells That Eliminates Interline Variability. *Plos One,* 2011, vol. 6 (4
- **CAMPBELL RE et al.** A monomeric red fluorescent protein. *Proceedings of the National Academy of Sciences of the United States of America,* 2002, vol. 99 (12), 7877-7882
- **OKAMOTO T et al.** Clonal heterogeneity in differentiation potential of immortalized human mesenchymal stem cells. *Biochem Bioph Res Co,* 2002, vol. 295 (2), 354-361
- **ANDERSON D et al.** Transgenic enrichment of cardiomyocytes from human embryonic stem cells. *Mol Ther,* 2007, vol. 15 (11), 2027-2036
- **CLAYCOMB WC et al.** HL-1 cells: A cardiac muscle cell line that contracts and retains phenotypic characteristics of the adult cardiomyocyte. *Proceedings of the National Academy of Sciences of the United States of America,* 1998, vol. 95 (6), 2979-2984
- **DIXON JE ; DICK E ; RAJAMOHAN D ; SHAKESHEFF KM ; DENNING C.** Directed differentiation of human embryonic stem cells to interrogate the cardiac gene regulatory network. *Mol Ther,* 2011, vol. 19 (9), 1695-1703
- **MEDINA D ; MOSKOWITZ N ; KHAN S ; CHRISTOPHER S ; GERMINO J.** Rapid purification of protein complexes from mammalian cells. *Nucleic Acids Res,* 2000, vol. 28 (12
- **NOVAK A ; GUO CY ; YANG WY ; NAGY A ; LOBE CG.** Z/EG, a double reporter mouse line that expresses enhanced green fluorescent protein upon Cre-mediated excision. *Genesis,* 2000, vol. 28 (3-4), 147-155
- **BAYOUSSEF Z ; DIXON JE ; STOLNIK S ; SHAKESHEFF KM.** Aggregation promotes cell viability, proliferation, and differentiation in an in vitro model of injection cell therapy. *J Tissue Eng Regen M,* 2012, vol. 6 (10), e61-e73
- **SINGH, A ; SAHOO, S.** Magnetic Nanoparticles: a novel platform for theranostics. *Drug Disc Today,* 2013
- **ARRUEBO, M et al.** Magnetic Nanoparticles for drug delivery. *Nanotoday,* 2007, vol. 3, 22
- **WANG, Z ; CUSCHIERI A.** Tumor cell labelling by magnetic nanoparticles with determination of intracellular iron content and spatial distribution of the intracellular iron. *Int. J. Mol. Sci,* 2013, vol. 14, 9111
- **SUN, C ; LEE, J ; ZHANG, M.** Magnetic Nanoparticles in MR Imaging and drug delivery. *Adv Drug Deli Rev,* 2008, vol. 60, 1253
- **SCHLORF, T et al.** Biological properties of iron oxide nanoparticles for cellular and molecular magnetic resonance imaging. *Int. J. Mol. Sci.,* 2011, vol. 12, 12
- **MARKIDES, H et al.** Whole body tracking of superparamagnetic iron oxide nanoparticle labelled cells - a rheumatoid arthritis mouse model. *Stem cell Res & ther,* 2013, vol. 4, 126
- **PENG et al.** targeted magnetic iron oxide nanoparticles for tumour imaging and therapy. *Int. J. of Nanomed.,* 2008, vol. 3, 311
- **ZHANG, C et al.** Specific Targeting of Tumor Angiogenesis by RGD-Conjugated Ultrasmall Superparamagnetic Iron Oxide Particles Using a Clinical 1.5-T Magnetic Resonance Scanner. *Can. Res.,* 2007, vol. 67, 1555
- **JI, S et al.** RGD-conjugated albumin nanoparticles as a novel delivery vehicle in pancreatic cancer therapy. *Cancer Biology & Therapy,* 2012, vol. 13 (4), 206
- **BECHARA C ; SAGAN S.** Cell-penetrating peptides: 20 years later, where do we stand?. *Febs Letters,* 2013, vol. 587, 1693-702

- **TANAKA K ; KANAZAWA T ; OGAWA T ; SUDA Y ; TAKASHIMA Y ; FUKUDA T et al.** A Novel, Bio-Reducible Gene Vector Containing Arginine and Histidine Enhances Gene Transfection and Expression of Plasmid DNA. *Chemical & Pharmaceutical Bulletin,* 2011, vol. 59, 202-7
- **MITCHELL DJ ; KIM DT ; STEINMAN L ; FATHMAN CG ; ROTHBARD JB.** Polyarginine enters cells more efficiently than other polycationic homopolymers. *Journal of Peptide Research,* 2000, vol. 56, 318-25
- **NAKASE I ; NIWA M ; TAKEUCHI T ; SONOMURA K ; KAWABATA N ; KOIKE Y et al.** Cellular uptake of arginine-rich peptides: Roles for macropinocytosis and actin rearrangement. *Molecular Therapy,* 2004, vol. 10, 1011-22
- **MA DX ; SHI NQ ; QI XR.** Distinct transduction modes of arginine-rich cell-penetrating peptides for cargo delivery into tumor cells. *International Journal of Pharmaceutics,* 2011, vol. 419, 200-8
- **EL-SAYED A ; FUTAKI S ; HARASHIMA H.** Delivery of Macromolecules Using Arginine-Rich Cell-Penetrating Peptides: Ways to Overcome Endosomal Entrapment. *Aaps Journal,* 2009, vol. 11, 13-22
- **SHIRAISHI T ; NIELSEN PE.** Enhanced delivery of cell-penetrating peptide-peptide nucleic acid conjugates by endosomal disruption. *Nature Protocols,* 2006, vol. 1, 633-6
- **MATSUBARA Y ; CHIBA T ; KASHIMADA K ; MORIO T ; TAKADA S ; MIZUTANI S et al.** Transcription activator-like effector nuclease-mediated transduction of exogenous gene into IL2RG locus. *Scientific Reports,* 2014, 4
- **PARELKAR SS ; LETTERI R ; CHAN-SENG D ; ZOLOCHEVSKA O ; ELLIS J ; FIGUEIREDO M et al.** Polymer-Peptide Delivery Platforms: Effect of Oligopeptide Orientation on Polymer-Based DNA Delivery. *Biomacromolecules,* 2014, vol. 15, 1328-36
- **YANG HY ; VONK LA ; LICHT R ; VAN BOXTEL AMG ; BEKKERS JEJ ; KRAGTEN AHM et al.** Cell type and transfection reagent-dependent effects on viability, cell content, cell cycle and inflammation of RNAi in human primary mesenchymal cells. *European Journal of Pharmaceutical Sciences,* 2014, vol. 53, 35-44
- **MA Y ; GONG C ; MA YL ; FAN FK ; LUO MJ ; YANG F et al.** Direct cytosolic delivery of cargoes in vivo by a chimera consisting of D- and L-arginine residues. *Journal of Controlled Release,* 2012, vol. 162, 286-94
- **JAMES E. ; DIXON GM ; NINA LANE ; CHRIS DENNING ; KEVIN M.** *Shakesheff Highly Efficient Delivery of Functional Proteins by the Synergistic Effect of GAG Binding Motifs and Cell-Penetrating Peptides,* 2014
- **BALDWIN RJ ; TEN DAM GB ; VAN KUPPEVELT TH ; LACAUD G ; GALLAGHER JT ; KOUSKOFF V et al.** A Developmentally Regulated Heparan Sulfate Epitope Defines a Subpopulation with Increased Blood Potential During Mesodermal Differentiation. *Stem Cells,* 2008, vol. 26, 3108-18
- **BRADFORD MM.** RAPID AND SENSITIVE METHOD FOR QUANTITATION OF MICROGRAM QUANTITIES OF PROTEIN UTILIZING PRINCIPLE OF PROTEIN-DYE BINDING. *Analytical Biochemistry,* 1976, vol. 72, 248-54
- **SCHAMHART DHJ ; KURTH KH.** Role of proteoglycans in cell adhesion of prostate cancer cells: From review to experiment. *Urological Research,* 1997, vol. 25, S89-S96
- **DELEHEDDE M ; DEUDON E ; BOILLY B ; HONDERMARCK H.** Proteoglycans in breast cancer. *Pathologie Biologie,* 1997, vol. 45, 305-11
- **SHAO C ; SHI XF ; PHILLIPS JJ ; ZAIA J.** Mass Spectral Profiling of Glycosaminoglycans from Histological Tissue Surfaces. *Analytical Chemistry,* 2013, vol. 85, 10984-91
- **THOMPSON KE ; BASHOR CJ ; LIM WA ; KEATING AE.** SYNZIP Protein Interaction Toolbox: in Vitro and in Vivo Specifications of Heterospecific Coiled-Coil Interaction Domains. *Acs Synthetic Biology,* 2012, vol. 1, 118-29
- **SALEH AF ; AOJULA H ; ARTHANARI Y ; OFFERMAN S ; ALKOTAJI M ; PLUEN A.** Improved Tat-mediated plasmid DNA transfer by fusion to LK15 peptide. *Journal of Controlled Release,* 2010, vol. 143, 233-42
- **DUFOURCQ J ; NERI W ; HENRY-TOULME N.** Molecular assembling of DNA with amphipathic peptides. *Febs Letters,* 1998, vol. 421, 7-11
- **ZHANG X ; HU HM ; LIU TB ; YANG YY ; PENG YF ; CAI QQ et al.** Multi-armed poly(L-glutamic acid)-graft-polypropyleneinime as effective and serum resistant gene delivery vectors. *International Journal of Pharmaceutics,* 2014, vol. 465, 444-54
- **WU HM ; PAN SR ; CHEN MW ; WU Y ; WANG C ; WEN YT et al.** A serum-resistant polyamidoamine-based polypeptide dendrimer for gene transfection. *Biomaterials,* 2011, vol. 32, 1619-34